# EUROPÄISCHE PATENTSCHRIFT

(11) **EP 0 754 175 B1**
(45) Veröffentlichungstag und Bekanntmachung des Hinweises auf die Patenterteilung: **29.01.2003**
(21) Anmeldenummer: 95913154.1
(22) Anmeldetag: 23.03.1995
(51) Int. Cl.: C07D 207/38, C07D 491/10, C07D 209/54, A01N 43/36, C07F 9/572, C07C 235/34, C07C 235/36, C07C 255/46, C07C 255/29

(54) **ALKOXY-ALKYL-SUBSTITUIERTE 1-H-3-ARYL-PYRROLIDIN-2,4-DIONE ALS HERBIZIDE UND PESTIZIDE**
ALKOXY-ALKYL-SUBSTITUTED 1-H-3-ARYL-PYRROLIDINE-2,4-DIONES USED AS HERBICIDES AND PESTICIDES
1-H-3-ARYLE-PYRROLIDINE-2,4-DIONES A SUBSTITUTION ALCOXY-ALKYLE, UTILISES COMME HERBICIDES ET PESTICIDES

(30) Priorität: 05.04.1994 DE 4411669; 14.11.1994 DE 4440594
(43) Veröffentlichungstag der Anmeldung: 22.01.1997
(73) Patentinhaber: Bayer CropScience AG, 40789 Monheim (DE)
(72) Erfinder: FISCHER, Reiner, D-40789 Monheim (DE); BRETSCHNEIDER, Thomas, D-53797 Lohmar (DE); KRÜGER, Bernd-Wieland, D-51467 Bergisch Gladbach (DE); RUTHER, Michael, D-40789 Monheim (DE); ERDELEN, Christoph, D-42799 Leichlingen (DE); WACHENDORFF-NEUMANN, Ulrike, D-40789 Monheim (DE); SANTEL, Hans-Joachim, D-51371 Leverkusen (DE); DOLLINGER, Markus, D-51381 Leverkusen (DE)
(86) Internationale Anmeldenummer: EP9501100
(87) Internationale Veröffentlichungsnummer: WO95026954

(56) Entgegenhaltungen:
- EP-A- 0 456 063
- EP-A- 0 521 334

## Beschreibung

Die Erfindung betrifft neue 1-H-3-Aryl-pyrrolidin-2,4-dion-Derivate, mehrere Verfahren zu ihrer Herstellung und ihre Verwendung als Schädlingsbekämpfungsmittel, insbesondere als Insektizide und Akarizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmieren und H. Mildenberger (Liebigs Ann. Chem. 1985 1095) synthetisiert. Eine biologische Wirksamkeit dieser Verbindungen wurde nicht beschrieben.

In EP-A 0 262 399 und GB-A 2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A 355 599 und EP 415 211) sowie substituierte monocyclische 3-Aryl-pyrrolidin-2,4-dion-Derivate (EP-A 377 893 und EP 442 077).

Weiterhin bekannt sind polycyclische 3-Arylpyrrolidin-2,4-dion-Derivate (EP 442 073) sowie 1-H-3-Arylpyrrolidin-dion-Derivate (EP 456 063 und EP 521 334).

Es wurden nun neue substituierte 1-H-3-Aryl-pyrrolidin-2,4-dion-Derivate der Formel (I) 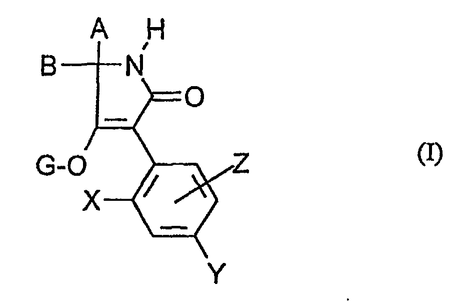 gefunden,
in welcher
- A: für Wasserstoff oder für jeweils gegebenenfalls einfach oder mehrfach durch Halogen substituiertes C₁-C₁₂-Alkyl, C₂-C₈-Alkenyl, C₁-C₁₀-Alkoxy-C₁-C₈-alkyl, C₁-C₈-Polyalkoxy-C₁-C₈-alkyl, C₁-C₁₀-Alkylthio-C₁-C₆-alkyl, gegebenenfalls durch Halogen, C₁-C₄-Alkyl oder C₁-C₄-Alkoxy substituiertes Cycloalkyl mit 3 bis 8 Ringatomen, das durch Sauerstoff und/oder Schwefel unterbrochen sein kann oder für jeweils gegebenenfalls einfach bis mehrfach durch Halogen, C₁-C₆-Alkyl, C₁-C₆-Halogenalkyl, C₁-C₆-Alkoxy und/oder Nitro substituiertes Phenyl, Naphthyl, Pyridyl, Imidazolyl, Indolyl, Thiazolyl, Furanyl, Thienyl, Phenyl-C₁-C₆-alkyl oder Naphthyl-C₁-C₆-alkyl steht,
- B: für C₁-C₁₂-Alkyl oder C₁-C₈-Alkoxy-C₁-C₆-alkyl steht oder
- A, B: und das Kohlenstoffatom an das sie gebunden sind, für einen gesättigten oder ungesättigten C₃-C₁₀-Spirocyclus stehen, der gegebenenfalls einfach oder mehrfach durch C₁-C₆-Alkyl, C₃-C₈-Cycloalkyl, C₁-C₄-Halogenalkyl, C₁-C₆-Alkoxy, C₁-C₆-Alkylthio, Halogen oder Phenyl substituiert und gegebenenfalls durch Sauerstoff oder Schwefel unterbrochen ist oder
- A, B: und das Kohlenstoffatom, an das sie gebunden sind, für einen C₃-C₆-Spirocyclus stehen, der durch eine gegebenenfalls durch ein oder zwei Sauerstoff- und/oder Schwefelatome unterbrochene Alkylendiyl-, oder durch eine Alkylendioxyl- oder durch eine Alkylendithioyl-gruppe substituiert ist, die mit dem Kohlenstoffatom, an das sie gebunden ist, einen weiteren fünf- bis achtgliedrigen Spirocyclus bildet oder
- A, B: und das Kohlenstoffatom, an das sie gebunden sind, für einen C₃-C₈-Spirocyclus stehen, bei dem zwei Substituenten gemeinsam für einen gegebenenfalls durch C₁-C₄-Alkyl, C₁-C₄-Alkoxy oder Halogen substituierten gesättigten oder ungesättigten füng- bis achtgliedrigen Cyclus stehen, der durch Sauerstoff oder Schwefel unterbrochen sein kann,
- X: für C₁-C₆-Alkyl oder C₁-C₆-Alkoxy steht,
- Y: für Wasserstoff, C₁-C₆-Alkyl oder C₁-C₆-Alkoxy steht,
- Z: für Wasserstoff, C₁-C₆-Alkyl oder C₁-C₆-Alkoxy steht,
- G: für Wasserstoff (a) oder für die Gruppen 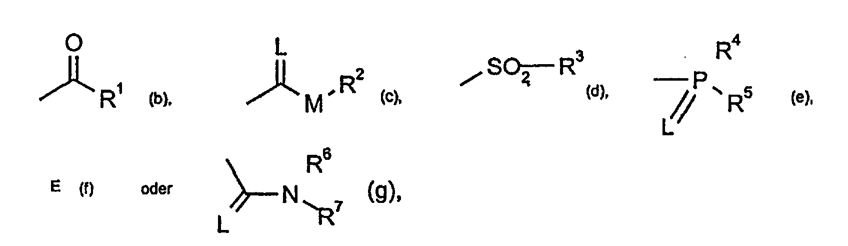
steht,
in welchen
- E: für ein Metallionäquivalent oder ein Ammoniumion steht und
- L: für Sauerstoff oder Schwefel steht,
- M: für Sauerstoff oder Schwefel steht,
- R¹: für jeweils gegebenenfalls einfach oder mehrfach durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, C₁-C₈-Polyalkoxy-C₂-C₈-alkyl oder gegebenenfalls durch Halogen oder C₁-C₆-Alkyl substituiertes Cycloalkyl mit 3 bis 8 Ringatomen, das durch mindestens ein Sauerstoff- und/oder Schwefelatom unterbrochen sein kann,
für gegebenenfalls einfach bis fünffach durch Halogen, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls einfach bis fünffach durch Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls einfach bis dreifach durch Halogen oder C₁-C₆-Alkyl substituiertes Pyridyl, Thienyl, Furanyl, Pyrazolyl, Pyrimidyl oder Thiazolyl,
für gegebenenfalls einfach bis dreifach durch Halogen oder C₁-C₆-Alkyl substituiertes Phenoxy-C₁-C₆-alkyl oder
für gegebenenfalls einfach bis zweifach durch Halogen, Amino oder C₁-C₆-Alkyl substituiertes Pyridinyloxy-C₁-C₆-alkyl, Pyrimidinyloxy-C₁-C₆-alkyl oder Thiazolyloxy-C₁-C₆-alkyl steht,
- R²: für jeweils gegebenenfalls einfach oder mehrfach durch Halogen substituiertes C₁-C₂₀-Alkyl, C₃-C₂₀-Alkenyl, C₁-C₈-Alkoxy-C₂-C₈-alkyl, C₁-C₈-Polyalkoxy-C₂-C₈-alkyl,
für gegebenenfalls einfach oder mehrfach durch Halogen, C₁-C₄-Alkyl oder C₁-C₄-Alkoxy substituiertes C₃-C₈-Cycloalkyl, oder
für gegebenenfalls einfach bis dreifach durch Halogen, Nitro, C₁-C₆-Alkyl, C₁-C₆-Alkoxy oder C₁-C₆-Halogenalkyl substituiertes Phenyl oder Benzyl steht,
- R³, R⁴ und R⁵: unabhängig voneinander für jeweils gegebenenfalls einfach oder mehrfach durch Halogen substituiertes C₁-C₈-Alkyl, C₁-C₈-Alkoxy, C₁-C₈-Alkylamino, Di-(C₁-C₈)-alkylamino, C₁-C₈-Alkylthio, C₃-C₆-Alkenylthio, C₃-C₇-Cycloalkylthio, für jeweils gegebenenfalls einfach oder mehrfach durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen und
- R⁶ und R⁷: unabhängig voneinander für Wasserstoff, für jeweils gegebenenfalls einfach oder mehrfach durch Halogen substituiertes C₁-C₈-Alkyl, C₃-C₈-Cycloalkyl, C₁-C₈-Alkoxy, C₃-C₈-Alkenyl, C₁-C₈-Alkoxy-C₂-C₈-alkyl, für gegebenenfalls einfach bis dreifach durch Halogen, C₁-C₈-Halogenalkyl, C₁-C₈-Alkyl oder C₁-C₈-Alkoxy substituiertes Phenyl, gegebenenfalls einfach bis dreifach durch Halogen, C₁-C₈-Alkyl, C₁-C₈-Halogenalkyl und/oder C₁-C₈-Alkoxy substituiertes Benzyl oder gemeinsam mit dem N-Atom, an das sie gebunden sind, für einen gegebenenfalls durch Sauerstoff oder Schwefel unterbrochenen C₃-C₆-Alkylenring stehen,
mit der Maßgabe, dass mindestens einer der Substituenten Y und Z für Alkoxy steht, wenn X für Alkyl steht.

Unter Einbeziehung der verschiedenen Bedeutungen (a), (b), (c), (d), (e), (f) und (g) der Gruppe G ergeben sich folgende hauptsächlichen Strukturen (Ia) bis (Ig): 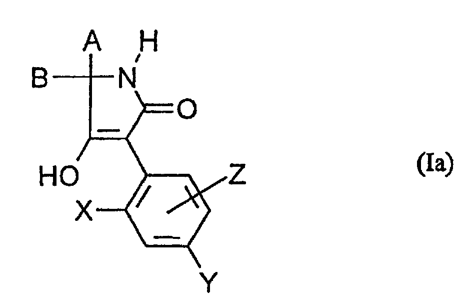 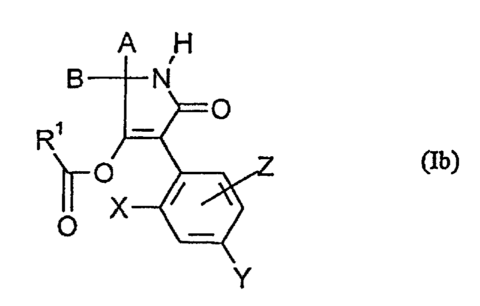 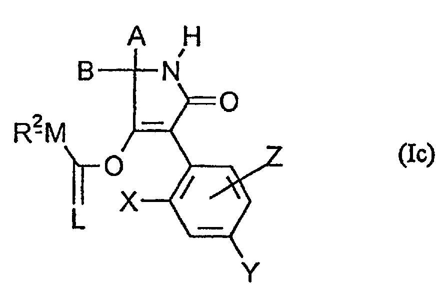 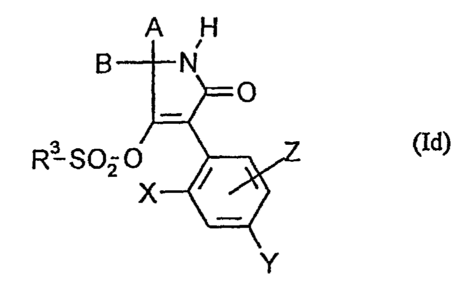 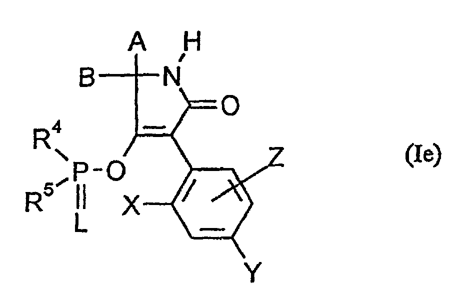 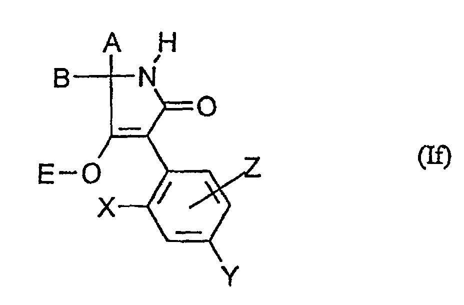 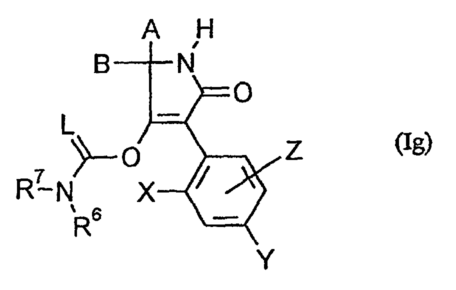 worin
A, B, E, L, M, X, Y, Z, R¹, R², R³, R⁴, R⁵, R⁶ und R⁷ die oben angegebenen Bedeutungen besitzen.

Aufgrund eines oder mehrerer Chiralitätszentren fallen die Verbindungen der Formel (Ia) - (Ig) im allgemeinen als Stereoisomerengemisch an, die gegebenenfalls in üblicher Art und Weise getrennt werden können. Sie können sowohl in Form ihrer Diastereomerengemische als auch als reine Diastereomere oder Enantiomere verwendet werden. Im folgenden wird der Einfachheit halber stets von Verbindungen der Formel (Ia) bis (Ig) gesprochen, obwohl sowohl die reinen Verbindungen, als auch die Gemische mit unterschiedlichen Anteilen an isomeren, enantiomeren und stereomeren Verbindungen gemeint sind.

Weiterhin wurde gefunden, daß man die neuen substituierten 1-H-3-Arylpyrrolidin-2,4-dion-Derivate der Formel (I) nach einem der im folgenden beschriebenen Verfahren erhält.
(A) Man erhält 1-H-3-Aryl-pyrrolidin-2,4-dione bzw. deren Enole der Formel (Ia) 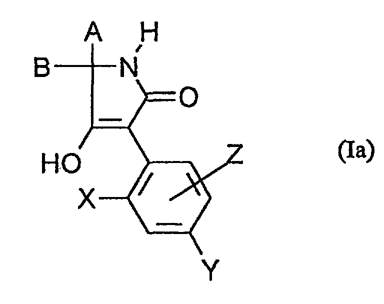 in welcher
   - A, B, X, Y und Z: die oben angegebene Bedeutung haben,
   wenn man
   N-Acylaminosäureester der Formel (II) 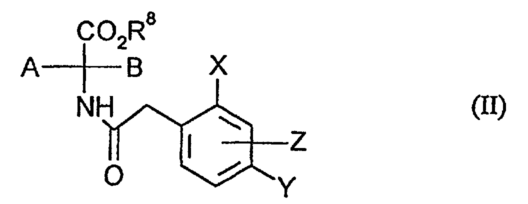 in welcher
   - A, B, X, Y und Z: die oben angegebene Bedeutung haben,
   und
   - R⁸: für Alkyl, insbesondere C₁-C₆-Alkyl steht,
   in Gegenwart eines Verdünnungsmittels und in Gegenwart einer Base intramolekular kondensiert;
   oder
(B) man erhält Verbindungen der Formel (Ib) 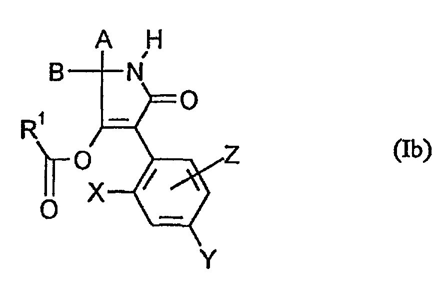 in welcher
   - A, B, X, Y, Z und R¹: die oben angegebene Bedeutung haben,
   wenn man Verbindungen der Formel (Ia), 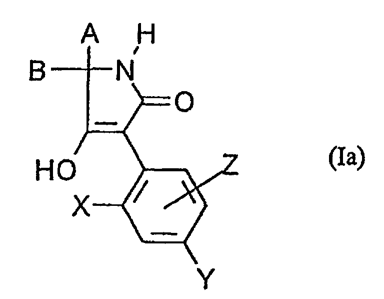 in welcher
   - A, B, X, Y und Z: die oben angegebene Bedeutung haben,

   α) mit Säurehalogeniden der Formel (III) 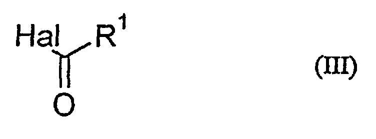 in welcher
      - R¹: die oben angegebene Bedeutung hat und
      - Hal: für Halogen, insbesondere Chlor oder Brom steht,
      gegebenenfalls in Gegenwart eines Verdünnungsmittels und gegebenenfalls in Gegenwart eines Säurebindemittels umsetzt
      oder
   β) mit Carbonsäureanhydriden der Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels,
      umsetzt;
      oder
(C) man erhält Verbindungen der Formel (Ic-a) 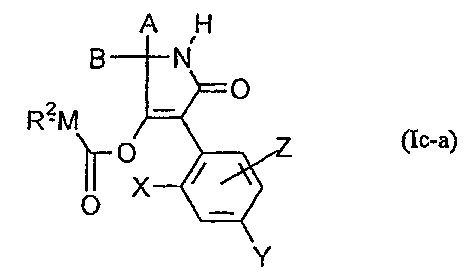 in welcher
   - A, B, X, Y, Z und R²: die oben angegebene Bedeutung haben,
   und
   - M: für Sauerstoff oder Schwefel steht,
   wenn man Verbindungen der Formel (Ia) 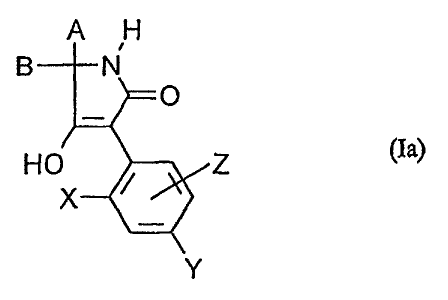 in welcher
   - A, B, X, Yund Z: die oben angegebene Bedeutung haben,
   mit Chlorameisensäureester oder Chlorameisensäurethiolester der Formel (V)

   R²-M-CO-Cl (V)

   in welcher
   - R² und M: die oben angegebene Bedeutung haben,
   gegebenenfalls in Gegenwart eines Verdünnungsmittels und gegebenenfalls in Gegenwart eines Säurebindemittels umsetzt;
   oder
(D) man erhält Verbindungen der Formel (Ic-b) 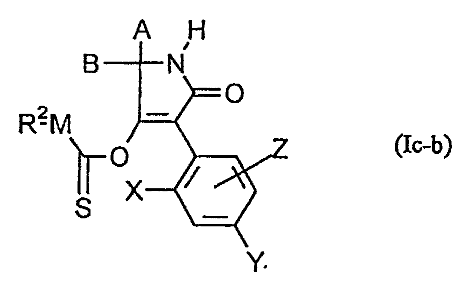 in welcher
   - A, B, R², X, Y und Z: die oben angegebene Bedeutung haben
   und
   - M: für Sauerstoff oder Schwefel steht,
   wenn man Verbindungen der Formel (Ia) 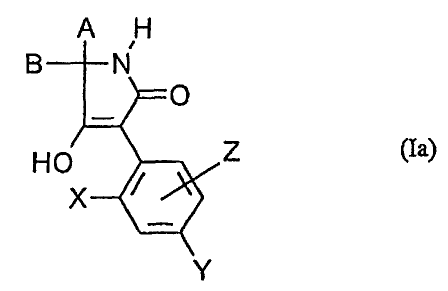 in welcher
   - A, B, X, Y und Z: die oben angegebene Bedeutung haben,

   α) mit Chlormonothioameisensäureestern oder Chlordithioameisensäureestern der Formel (VI) 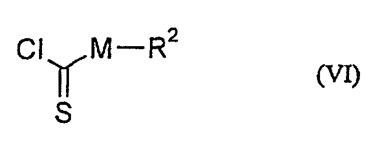 in welcher
      - M und R²: die oben angegebene Bedeutung haben,
      gegebenenfalls in Gegenwart eines Verdünnungsmittels und gegebenenfalls in Gegenwart eines Säurebindemittels umsetzt,
      oder
   β) mit Schwefelkohlenstoff und anschließend mit Alkylhalogeniden der Formel (VII)

      R²-Hal (VII)

      in welcher
      - R²: die oben angegebene Bedeutung hat
      und
      - Hal: für Chlor, Brom oder Iod steht,
      gegebenenfalls in Gegenwart eines Verdünnungsmittels und gegebenenfalls in Gegenwart einer Base umsetzt;
      oder
(E) man erhält Verbindungen der Formel (Id) 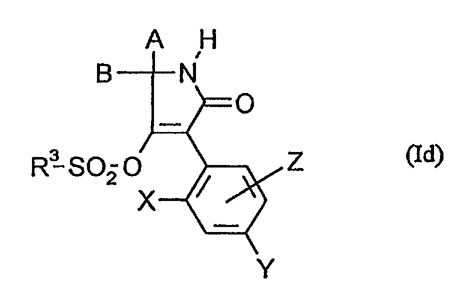 in welcher
   - A, B, X, Y, Z und R³: die oben angegebene Bedeutung haben,
   wenn man Verbindungen der Formel (Ia) 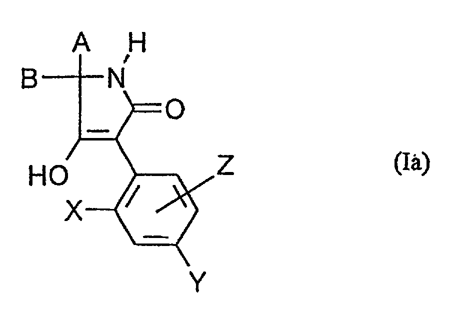 in welcher
   - A, B, X, Y und Z: die oben angegebene Bedeutung haben,
   mit Sulfonsäurechloriden der Formel (VIII)

   R³-SO₂-Cl (VIII)

   in welcher
   - R³: die oben angegebene Bedeutung hat,
   gegebenenfalls in Gegenwart eines Verdünnungsmittels und gegebenenfalls in Gegenwart eines Säurebindemittels, umsetzt;
   oder
(F) man erhält 3-Aryl-pyrrolidin-2,4-dione der Formel (Ie) 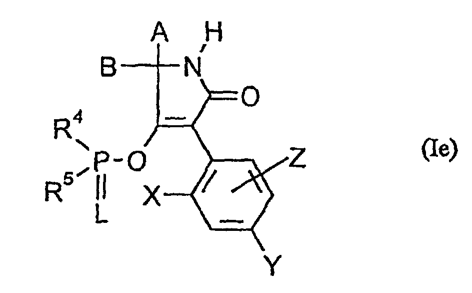 in welcher
   - A, B, L, X, Y, Z, R⁴ und R⁵: die oben angegebene Bedeutung haben,
   wenn man
   1-H-3-Aryl-pyrrolidin-2,4-dione der Formel (Ia) bzw. deren Enole 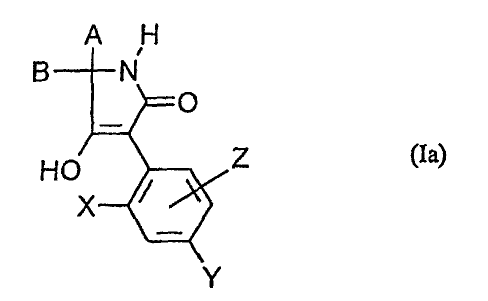 in welcher
   - A, B, X, Y und Z: die oben angegebene Bedeutung haben,
   mit Phosphorverbindungen der Formel (IX) 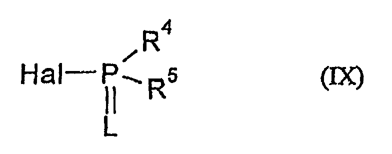 in welcher
   - L, R⁴ und R⁵: die oben angegebene Bedeutung haben
   und
   - Hal: für Halogen, insbesondere Chlor oder Brom steht,
   gegebenenfalls in Gegenwart eines Verdünnungsmittels und gegebenenfalls in Gegenwart eines Säurebindemittels umsetzt;
   oder
(G) man erhält Verbindungen der Formel (If) 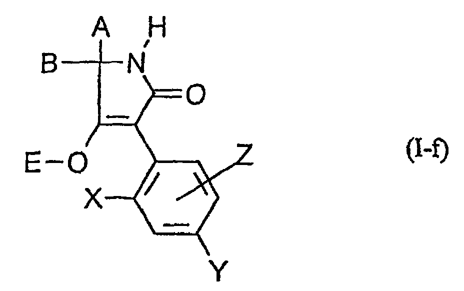 in welcher
   - A, B, X, Y und Z: die oben angegebene Bedeutung haben,
   und
   - E: für ein Metallionäquivalent oder für ein Ammoniumion steht,
   wenn man Verbindungen der Formel (Ia) 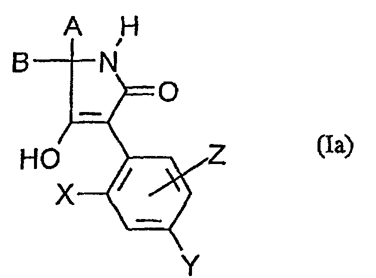 in welcher
   - A, B, X, Y und Z: die oben angegebene Bedeutung haben,
   mit Metallhydroxiden, Metallalkoxiden oder Aminen der Formeln (X) und (XI) 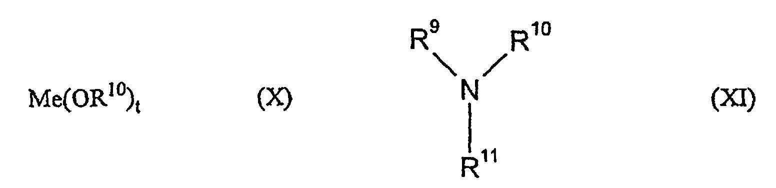 in welchen
   - Me: für ein- oder zweiwertige Metallionen, wie z.B. Lithium, Natrium, Kalium, Magnesium, Calcium,
   - t: für die Zahl 1 oder 2 und
   - R⁹, R¹⁰ und R¹¹: unabhängig voneinander für Wasserstoff und/oder Alkyl, insbesondere C₁-C₆-Alkyl
   stehen,
   gegebenenfalls in Gegenwart eines Verdünnungsmittels, umsetzt.
(H) Ferner wurde gefunden, daß man Verbindungen der Formel (Ig) 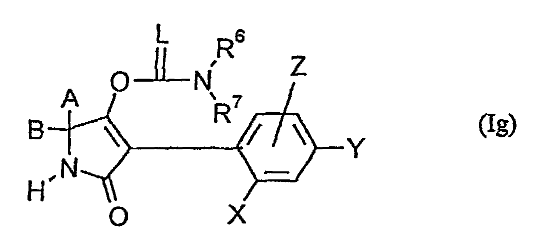 in welcher
   - A, B, L, X, Y, Z, R⁶ und R⁷: die oben angegebene Bedeutung haben,
   erhält, wenn man Verbindungen der Formel (Ia) 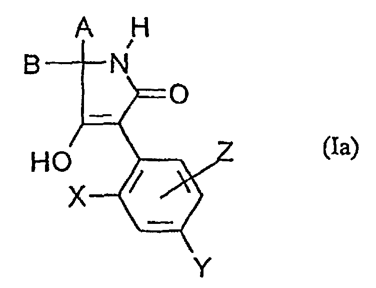 in welcher
   - A, B, X, Y und Z: die oben angegebene Bedeutung haben,

   α) mit Isocyanaten oder Isothiocyanaten der Formel (XII)

      R⁶-N=C=L (XII)

      in welcher
      - R⁶: die oben angegebene Bedeutung hat
      gegebenenfalls in Gegenwart eines Verdünnungsmittels und gegebenenfalls in Gegenwart eines Katalysators
      oder
   β) mit Carbamidsäurechloriden oder Thiocarbamidsäurechloriden der Formel (XIII) 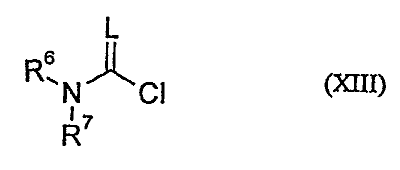 in welcher
      - L, R⁶ und R⁷: die oben angegebene Bedeutung haben
      gegebenenfalls in Gegenwart eines Verdünnungsmittels und gegebenenfalls in Gegenwart eines Säurebindemittels,
      umsetzt.

Weiterhin wurde gefunden, daß sich die neuen 1-H-3-Arylpyrrolidin-2,4-dion-Derivate der Formel (I) durch hervorragende insektizide, akarizide und herbizide Wirkungen auszeichnen.

Bevorzugte Substituenten bzw. Bereiche der in den oben und nachstehend erwähnten Formeln aufgeführten Reste werden im folgenden erläutert.
- A: steht besonders bevorzugt für Wasserstoff, für jeweils gegebenenfalls einfach bis sechsfach durch Fluor oder Chlor substituiertes C₁-C₁₀-Alkyl, C₂-C₆-Alkenyl, C₁-C₈-Alkoxy-C₁-C₆-alkyl, C₁-C₆-Polyalkoxy-C₁-C₆-alkyl, C₁-C₈-Alkylthio-C₁-C₆-alkyl gegebenenfalls durch Fluor, Chlor, C₁-C₃-Alkyl oder C₁-C₃-Alkoxy substituiertes Cycloalkyl mit 3 bis 7 Ringatomen, das durch 1 bis 2 Sauerstoff- und/oder Schwefelatome unterbrochen sein kann oder für jeweils gegebenenfalls einfach bis zweifach durch Fluor, Chlor, Brom, C₁-C₄-Alkyl, C₁-C₄-Halogenalkyl, C₁-C₄-Alkoxy oder Nitro substituiertes Phenyl, Pyridyl, Thienyl, Furanyl, Imidazolyl, Indolyl oder Phenyl-C₁-C₄-alkyl.
- B: steht besonders bevorzugt für C₁-C₁₀-Alkyl oder C₁-C₆-Alkoxy-C₁-C₄-alkyl oder
- A, B: und das Kohlenstoffatom an das sie gebunden sind, stehen besonders bevorzugt für einen gesättigten oder ungesättigten C₃-C₉-Spirocyclus, der gegebenenfalls einfach oder mehrfach durch C₁-C₆-Alkyl, C₃-C₈-Cycloalkyl, C₁-C₃-Halogenalkyl, C₁-C₆-Alkoxy, C₁-C₆-Alkylthio, Fluor, Chlor oder Phenyl substituiert und gegebenenfalls durch Sauerstoff oder Schwefel unterbrochen ist oder
- A, B: und das Kohlenstoffatom, an das sie gebunden sind, stehen besonders bevorzugt für einen C₃-C₆-Spirocyclus, der durch eine gegebenenfalls durch ein oder zwei Sauerstoff- oder Schwefelatome unterbrochenen Alkylendiyl- oder durch eine Alkylendioxyl- oder durch eine Alkylendithiol-Gruppe substituiert ist, die mit dem Kohlenstoffatom, an das sie gebunden ist, einen weiteren fünf- bis siebengliedrigen Spirocyclus bildet oder
- A,B: und das Kohlenstoffatom, an das sie gebunden sind, stehen besonders bevorzugt für einen C₃-C₆-Spirocyclus, bei dem zwei Substituenten gemeinsam für einen gegebenenfalls durch C₁-C₃-Alkyl, C₁-C₃-Alkoxy, Fluor, Chlor oder Brom substituierten gesättigten oder ungesättigten, fünf- bis siebengliedrigen Cyclus stehen, der durch Sauerstoff oder Schwefel unterbrochen sein kann.
- A: steht ganz besonders bevorzugt für Wasserstoff, für jeweils gegebenenfalls einfach bis dreifach durch Fluor und/oder Chlor substituiertes C₁-C₈-Alkyl, C₂-C₄-Alkenyl, C₁-C₆-Alkoxy-C₁-C₄-alkyl, C₁-C₄-Polyalkoxy-C₁-C₄-alkyl, C₁-C₆-Alkylthio-C₁-C₄-alkyl, gegebenenfalls durch Fluor, Chlor, Methyl, Ethyl, Methoxy oder Ethoxy substituiertes Cycloalkyl mit 3 bis 6 Ringatomen, das durch 1 bis 2 Sauerstoff- und/oder Schwefelatome unterbrochen sein kann oder für jeweils gegebenenfalls einfach oder zweifach durch Fluor, Chlor, Brom, Methyl, Ethyl, Propyl, iso-Propyl, Methoxy, Ethoxy, Trifluormethyl und/oder Nitro substituiertes Phenyl, Furanyl, Thienyl, Imidazolyl, Indolyl, Pyridyl oder Benzyl.
- B: steht ganz besonders bevorzugt für C₁-C₈-Alkyl oder C₁-C₄-Alkoxy-C₁-C₂-alkyl oder
- A, B: und das Kohlenstoffatom an das sie gebunden sind, stehen ganz besonders bevorzugt für einen gesättigten oder ungesättigten C₃-C₈-Spirocyclus, der gegebenenfalls einfach oder mehrfach durch Methyl, Ethyl, Propyl, Isopropyl, Butyl, iso-Butyl, sec.-Butyl, tert,-Butyl, Cyclohexyl, Trifluormethyl, Methoxy, Ethoxy, Propoxy, iso-Propoxy, Butoxy, iso-Butoxy, sek.-Butoxy, tert.-Butoxy, Methylthio, Ethylthio, Fluor, Chlor oder Phenyl substituiert und gegebenenfalls durch Sauerstoff oder Schwefel unterbrochen ist oder
- A, B: und das Kohlenstoffatom, an das sie gebunden sind, stehen ganz besonders bevorzugt für einen C₅-C₆-Spirocyclus, der durch eine gegebenenfalls durch ein Sauerstoff- oder Schwefelatom unterbrochene Alkylendiyl- oder durch eine Alkylendioxyl-Gruppe substituiert ist, die mit dem Kohlenstoffatom, an das sie gebunden ist, einen weiteren fünf- bis siebengliedrigen Spirocyclus bildet oder
- A, B: und das Kahlenstoffatom, an das sie gebunden sind, stehen ganz besonders bevorzugt für einen C₃-C₆-Spirocyclus, bei dem zwei Substituenten gemeinsam für einen gesättigten oder ungesättigten fünf- oder sechsgliedrigen Cyclus stehen, der durch Sauerstoff oder Schwefel unterbrochen sein kann.
- X: steht besonders bevorzugt für C₁-C₅-Alkyl oder C₁-C₄-Alkoxy.
- X: steht ganz besonders bevorzugt für Methyl, Ethyl, Propyl, iso-Propyl, Methoxy, Ethoxy, Propoxy oder iso-Propoxy.
- Y: steht besonders bevorzugt für Wasserstoff, C₁-C₅-Alkyl oder C₁-C₄-Alkoxy.
- Y: steht ganz besonders bevorzugt für Wasserstoff, Methyl, Ethyl, Propyl, isoPropyl, Butyl, iso-Butyl, sek.-Butyl, tert.-Butyl, Methoxy, Ethoxy, Propoxy oder iso-Propoxy.
- Z: steht besonders bevorzugt für Wasserstoff, C₁-C₄-Alkyl oder C₁-C₄-Alkoxy.
- Z: steht ganz besonders bevorzugt für Wasserstoff, Methyl, Ethyl, Propyl, iso-Propyl, Methoxy, Ethoxy, Propoxy oder iso-Propoxy.

Dabei gilt, daß mindestens einer der Substituenten Y und Z für Alkoxy steht, wenn X für Alkyl steht.
- G: steht besonders bevorzugt für Wasserstoff (a) oder für die Gruppen 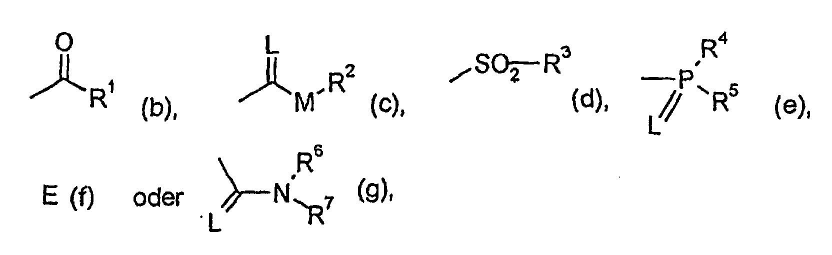 in welchen
E für ein Metallionäquivalent oder ein Ammoniumion steht,
L für Sauerstoff oder Schwefel steht und
M für Sauerstoff oder Schwefel steht.
- R¹: steht besonders bevorzugt für jeweils gegebenenfalls einfach bis sechsfach durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl, C₁-C₆-Polyalkoxy-C₂-C₆-alkyl oder gegebenenfalls durch Fluor, Chlor oder C₁-C₅-Alkyl substituiertes Cycloalkyl mit 3 bis 7 Ringatomen, das durch 1 oder 2 Sauerstoff- und/oder Schwefelatome unterbrochen sein kann,
für gegebenenfalls einfach bis dreifach durch Fluor, Chlor, Brom, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis dreifach durch Fluor, Chlor, Brom, C₁-C₄-Alkyl, C₁-C₄-Alkoxy, C₁-C₃-Halogenalkyl, C₁-C₃-Halogenalkoxy substituiertes Phenyl-C₁-C₄-alkyl,
für gegebenenfalls einfach bis zweifach durch Fluor, Chlor, Brom oder C₁-C₄-Alkyl substituiertes Pyridyl, Thienyl, Furanyl, Pyrazolyl, Pyrimidyl oder Thiazolyl,
für gegebenenfalls einfach bis zweifach durch Fluor, Chlor, Brom oder C₁-C₄-Alkyl substituiertes Phenoxy-C₁-C₅-alkyl oder
für gegebenenfalls einfach bis zweifach durch Fluor, Chlor, Brom, Amino oder C₁-C₄-Alkyl substituiertes Pyrimidinyloxy-C₁-C₅-alkyl, Pyridinyloxy-C₁-C₅-alkyl, Thiazolyloxy-C₁-C₅-alkyl.
- R²: steht besonders bevorzugt für jeweils gegebenenfalls einfach bis sechsfach durch Fluor oder Chlor substituiertes C₁-C₁₆-Alkyl, C₃-C₁₆-Alkenyl, C₁-C₆-Alkoxy-C₂-C₆-alkyl, C₁-C₆-Polyalkoxy-C₂-C₆-alkyl,
für gegebenenfalls einfach bis sechsfach durch Fluor, Chlor, C₁-C₃-Alkyl oder C₁-C₃-Alkoxy substituiertes C₃-C₇-Cycloalkyl,
für gegebenenfalls einfach bis zweifach durch Fluor, Chlor, Bröm, Nitro, C₁-C₄-Alkyl, C₁-C₃-Alkoxy oder C₁-C₃-Halogenalkyl substituiertes Phenyl oder Benzyl.
- R³, R⁴ und R⁵: stehen unabhängig voneinander besonders bevorzugt für jeweils gegebenenfalls einfach bis sechsfach durch Fluor oder Chlor substituiertes C₁-C₆-Alkyl, C₁-C₆-Alkoxy, C₁-C₆-Alkylamino, Di-(C₁-C₆)-alkylamino, C₁-C₆-Alkylthio, C₃-C₄-Alkenylthio, C₃-C₆-Cycloalkylthio, für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
- R⁶ und R⁷: stehen unabhängig voneinander besonders bevorzugt für Wasserstoff, für jeweils gegebenenfalls einfach bis sechsfach durch Fluor oder Chlor substituiertes C₁-C₆-Alkyl, C₃-C₆-Cycloalkyl, C₁-C₆-Alkoxy, C₃-C₆-Alkenyl, C₁-C₆-Alkoxy-C₂-C₆-alkyl, für gegebenenfalls einfach bis zweifach durch Fluor, Chlor, Brom, C₁-C₅-Halogenalkyl, C₁-C₅-Alkyl und/oder C₁-C₅-Alkoxy substituiertes Phenyl, für gegebenenfalls einfach bis zweifach durch Fluor, Chlor, Brom, C₁-C₅-Alkyl, C₁-C₅-Halogenalkyl oder C₁-C₅-Alkoxy substituiertes Benzyl, oder gemeinsam für einen gegebenenfalls durch Sauerstoff oder Schwefel unterbrochenen C₃-C₆-Alkylenrest.
- G: steht ganz besonders bevorzugt für Wasserstoff (a) oder für die Gruppen 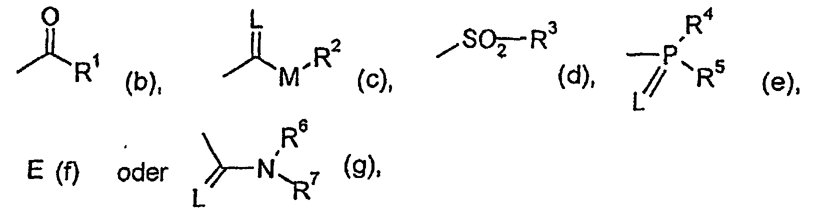 in welchen
E für ein Metallionäquivalent oder ein Ammoniumion steht,
L für Sauerstoff oder Schwefel steht und
M für Sauerstoff oder Schwefel steht.
- R¹: steht ganz besonders bevorzugt für jeweils gegebenenfalls einfach bis dreifach durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, C₁-C₄-Polyalkoxy-C₂-C₄-alkyl oder gegebenenfalls durch Fluor, Chlor, Methyl, Ethyl, Propyl, 2-Propyl, Butyl, i-Butyl oder t-Butyl subststuiertes Cycloalkyl mit 3 bis 6 Ringatomen, das durch 1 bis 2 Sauerstoff- und/oder Schwefelatome unterbrochen sein kann,
für gegebenenfalls einfach bis zweifach durch Fluor, Chlor, Brom, Methyl, Ethyl, Propyl, i-Propyl, Methoxy, Ethoxy, Trifluormethyl, Trifluormethoxy, Methylthio, Ethylthio, Methylsulfonyl, Ethylsulfonyl oder Nitro substituiertes Phenyl,
für gegebenenfalls einfach bis zweifach durch Fluor, Chlor, Brom, Methyl, Ethyl, Propyl, i-Propyl, Methoxy, Ethoxy, Trifluormethyl oder Trifluormethoxy substituiertes Phenyl-C₁-C₃-alkyl steht,
für gegebenenfalls einfach bis zweifach durch Fluor, Chlor, Brom, Methyl oder Ethyl substituiertes Thienyl, Furanyl oder Pyridyl,
für gegebenenfalls einfach bis zweifach durch Fluor, Chlor, Methyl oder Ethyl substituiertes Phenoxy-C₁-C₄-alkyl, oder
für jeweils gegebenenfalls einfach bis zweifach durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl.
- R²: steht ganz besonders bevorzugt für jeweils gegebenenfalls einfach bis dreifach durch Fluor oder Chlor substituiertes C₁-C₁₄-Alkyl, C₃-C₁₄-Alkenyl, C₁-C₄-Alkoxy-C₂-C₆-alkyl, C₁-C₄-Polyalkoxy-C₂-C₆-alkyl,
für gegebenenfalls einfach bis dreifach durch Fluor, Chlor, Methyl, Ethyl, Propyl, iso-Propyl oder Methoxy substituiertes C₃-C₆-Cycloalkyl,
oder für jeweils gegebenenfalls einfach bis zweifach durch Fluor, Chlor, Nitro, Methyl, Ethyl, Propyl, i-Propyl, Methoxy, Ethoxy oder Trifluormethyl substituiertes Phenyl oder Benzyl.
- R³, R⁴ und R⁵: stehen ganz besonders bevorzugt unabhängig voneinander für jeweils gegebenenfalls einfach bis dreifach durch Fluor oder Chlor substituiertes C₁-C₄-Alkyl, C₁-C₄-Alkoxy, C₁-C₄-Alkylamino, Di-(C₁-C₄)-alkylamino, C₁-C₄-Alkylthio, für jeweils gegebenenfalls durch einfach bis zweifach Fluor, Chlor, Brom, Nitro, Cyano, C₁-C₂-Alkoxy, C₁-C₂-Fluoralkoxy, C₁-C₂-Alkylthio, C₁-C₂-Fluoralkylthio, C₁-C₂-Alkyl oder C₁-C₂-Fluoralkyl substituiertes Phenyl, Phenoxy oder Phenylthio.
- R⁶ und R⁷: stehen ganz besonders bevorzugt unabhängig voneinander für Wasserstoff, für jeweils gegebenenfalls einfach bis dreifach durch Fluor oder Chlor substituiertes C₁-C₄-Alkyl, C₃-C₆-Cycloalkyl, C₁-C₄-Alkoxy, C₃-C₄-Alkenyl, C₁-C₄-Alkoxy-C₂-C₄-alkyl, für gegebenenfalls einfach bis zweifach durch Fluor, Chlor, Brom, C₁-C₄-Halogenalkyl, C₁-C₄-Alkyl oder C₁-C₄-Alkoxy substituiertes Phenyl, für gegebenenfalls einfach bis zweifach durch Fluor, Chlor, Brom, C₁-C₄-Alkyl, C₁-C₄-Halogenalkyl oder C₁-C₄-Alkoxy substituiertes Benzyl, oder gemeinsam für einen gegebenenfalls durch Sauerstoff oder Schwefel unterbrochenen C₃-C₆-Alkylenrest.

In den angegebenen Definitionen können gesättigte oder ungesättigte Alkylreste auch in Verbindung mit Heteroatomen, wie z.B. Alkoxy oder Alkylthio soweit möglich geradkettig oder verzweigt sein.

Die gegebenenfalls mehrfach substituierten Reste können gleich oder verschieden mit den für diese Reste genannten Substituenten substituiert sein.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß besonders bevorzugt werden die Verbindungen der allgemeinen Formel (I), in welchen eine Kombination der vorstehend als besonders bevorzugt aufgeführten Bedeutungen vorliegt.

Erfindungsgemäß ganz besonders bevorzugt werden die Verbindungen der allgemeinen Formel (I), in welchen eine Kombination dieser vorstehend als ganz besonders bevorzugt aufgeführten Bedeutungen vorliegt.

Eine weiterhin bevorzugte Gruppe von Verbindungen sind solche Verbindungen der Formel (Ih) 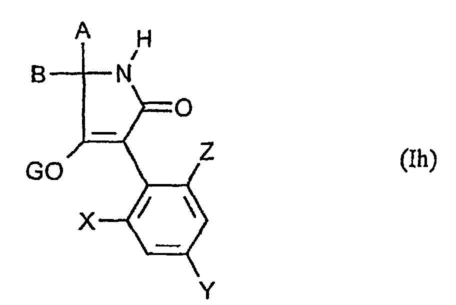 in welcher
- X und Z: für Alkyl stehen,
- Y: für Alkoxy steht und
- A, B und G: die obengenannte Bedeutung haben.

Bevorzugt sind auch Verbindungen der oben gezeigten Formel (Ih), in welcher
- X und Y: für Alkyl stehen,
- Z: für Alkoxy steht und
- A, B und G: die obengenannte Bedeutung haben.

Bevorzugt sind weiter Verbindungen der oben gezeigten Formel (Ih), in welcher
- X: für Alkyl steht,
- Y: für Wasserstoff steht,
- Z: für Alkoxy steht und
- A, B und G: die obengenannte Bedeutung haben.

Eine weitere Gruppe bevorzugter Verbindungen sind diejenigen der oben gezeigten Formel (Ih), in welcher
- X: für Alkyl steht,
- Y: für Alkoxy steht,
- Z: für Wasserstoff steht und
- A, B und G: die obengenannte Bedeutung haben.

Bevorzugt ist außerdem die Gruppe von Verbindungen der Formel (Ih), in welcher
- X: für Alkoxy steht,
- Y: für Alkyl steht,
- Z: für Wasserstoff steht und
- A, B und G: die obengenannte Bedeutung haben.

Im einzelnen seien außer bei den bei Herstellungsbeispielen genannten Verbindungen die folgenden Verbindungen der Formel (Ia) genannt:

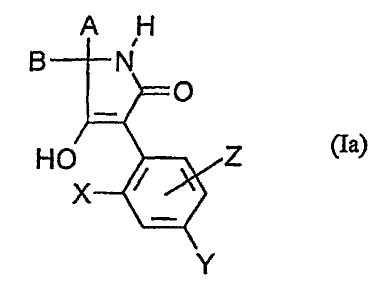

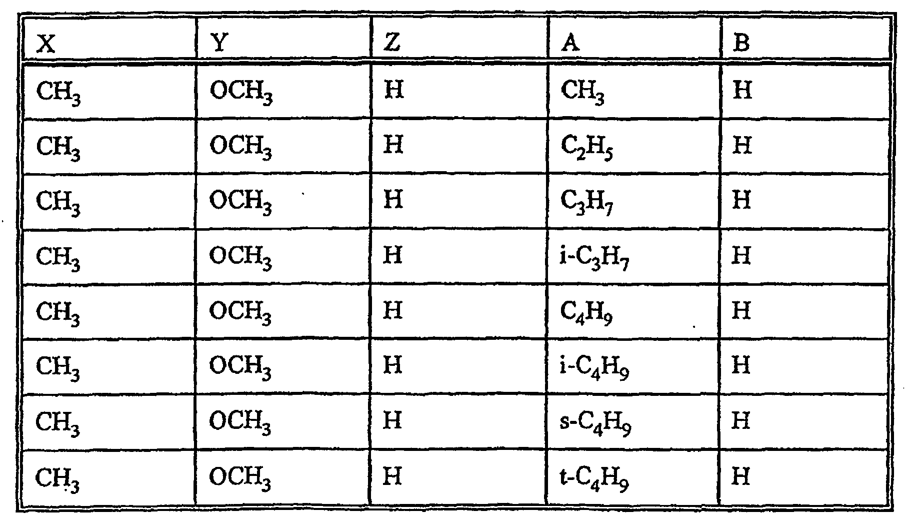

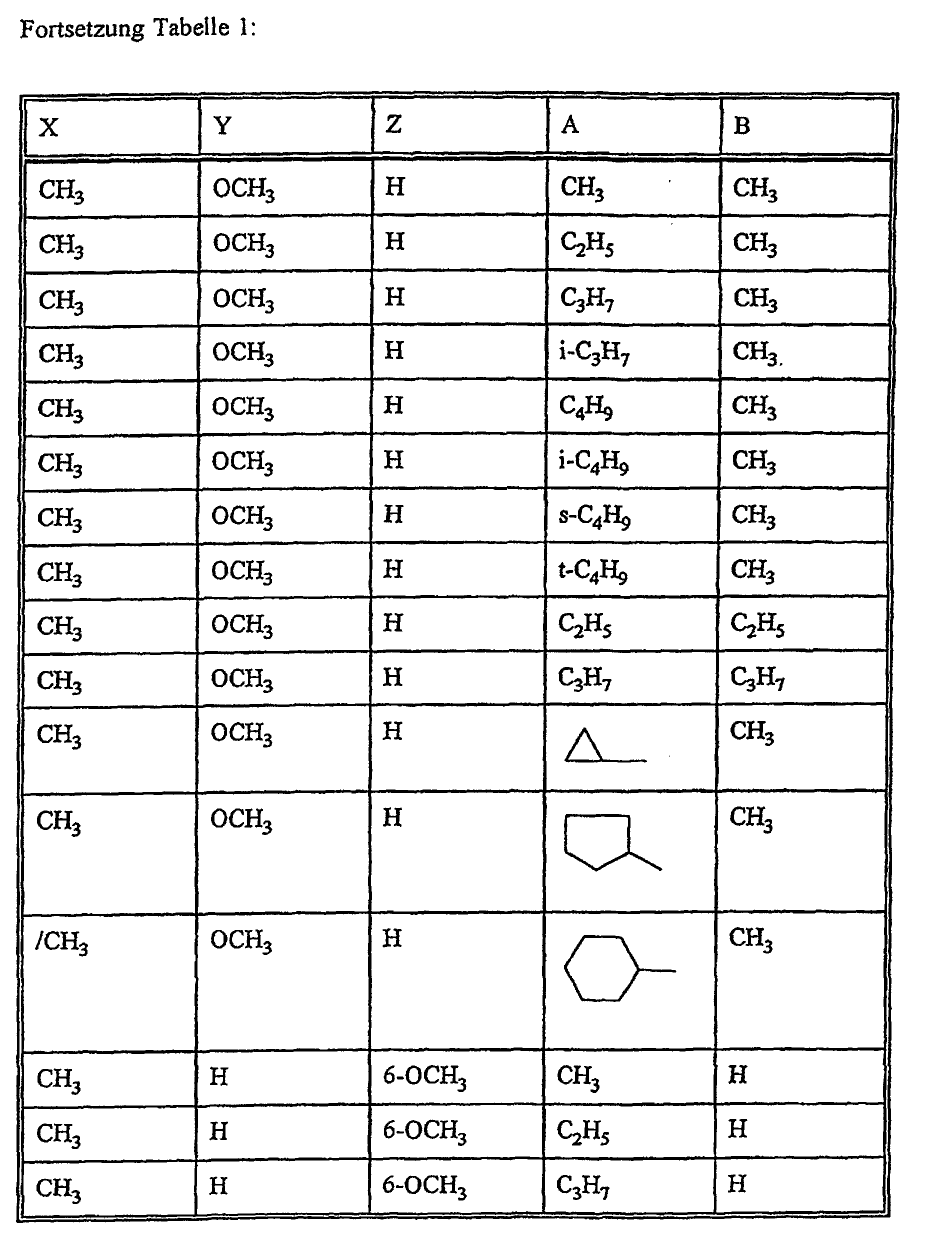

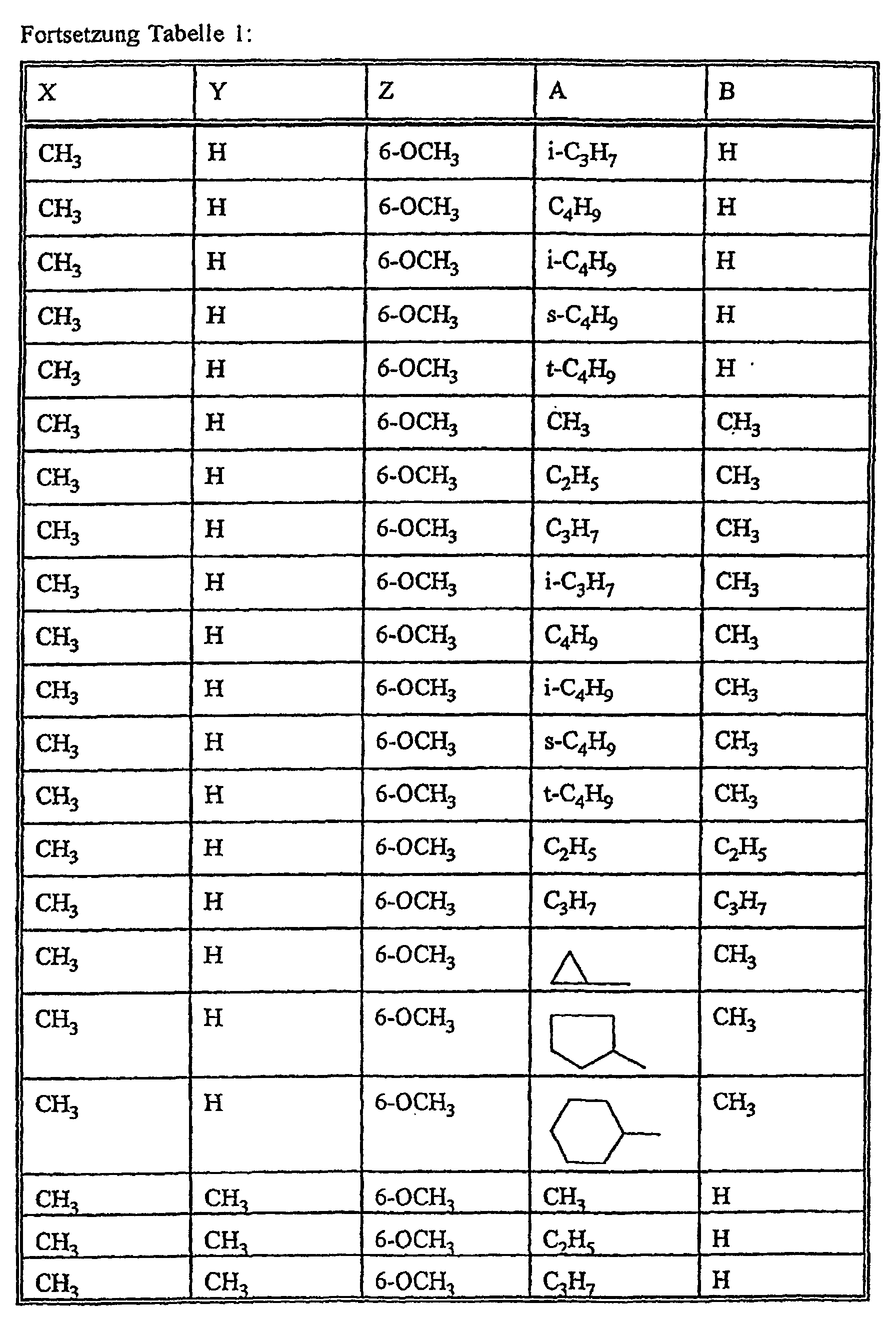

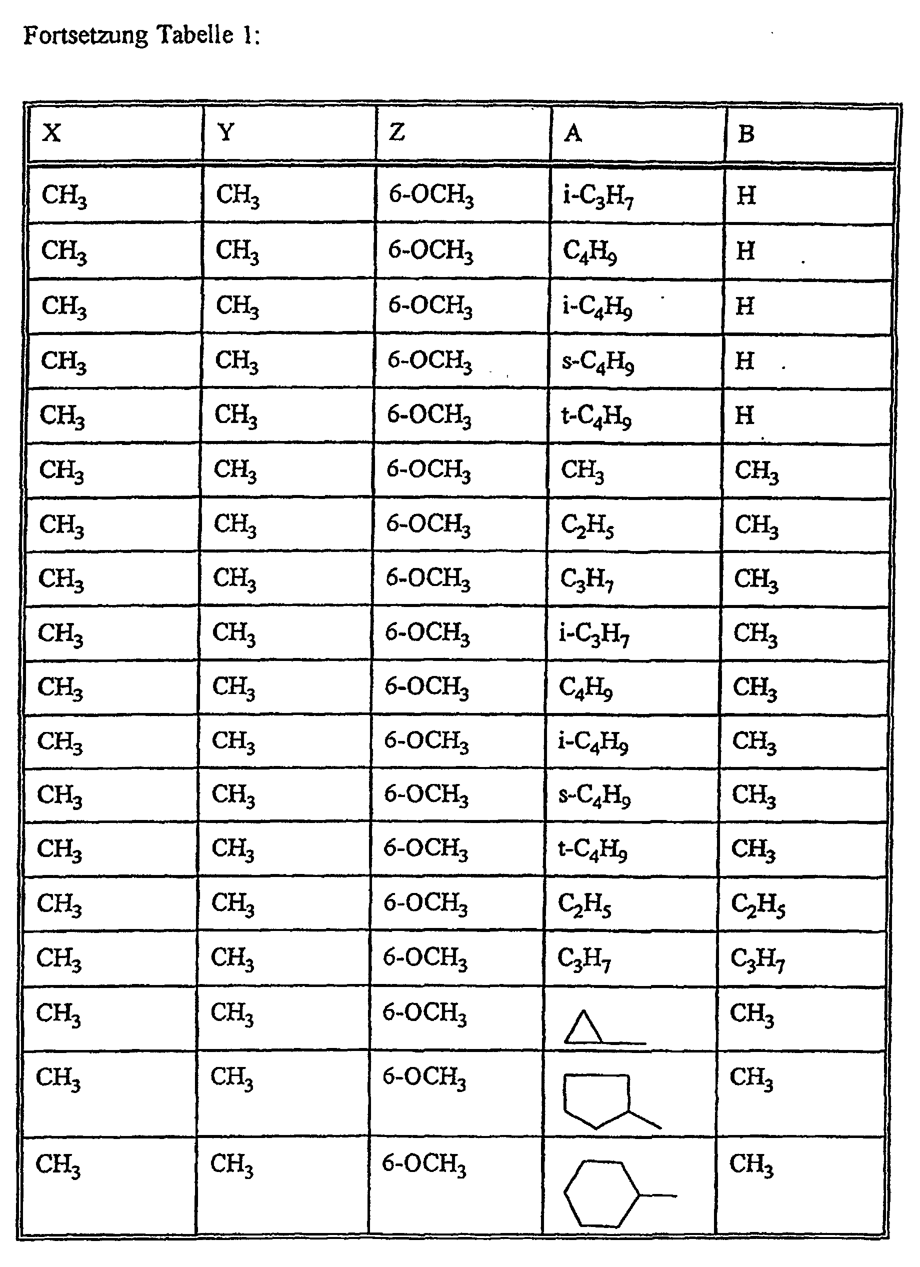

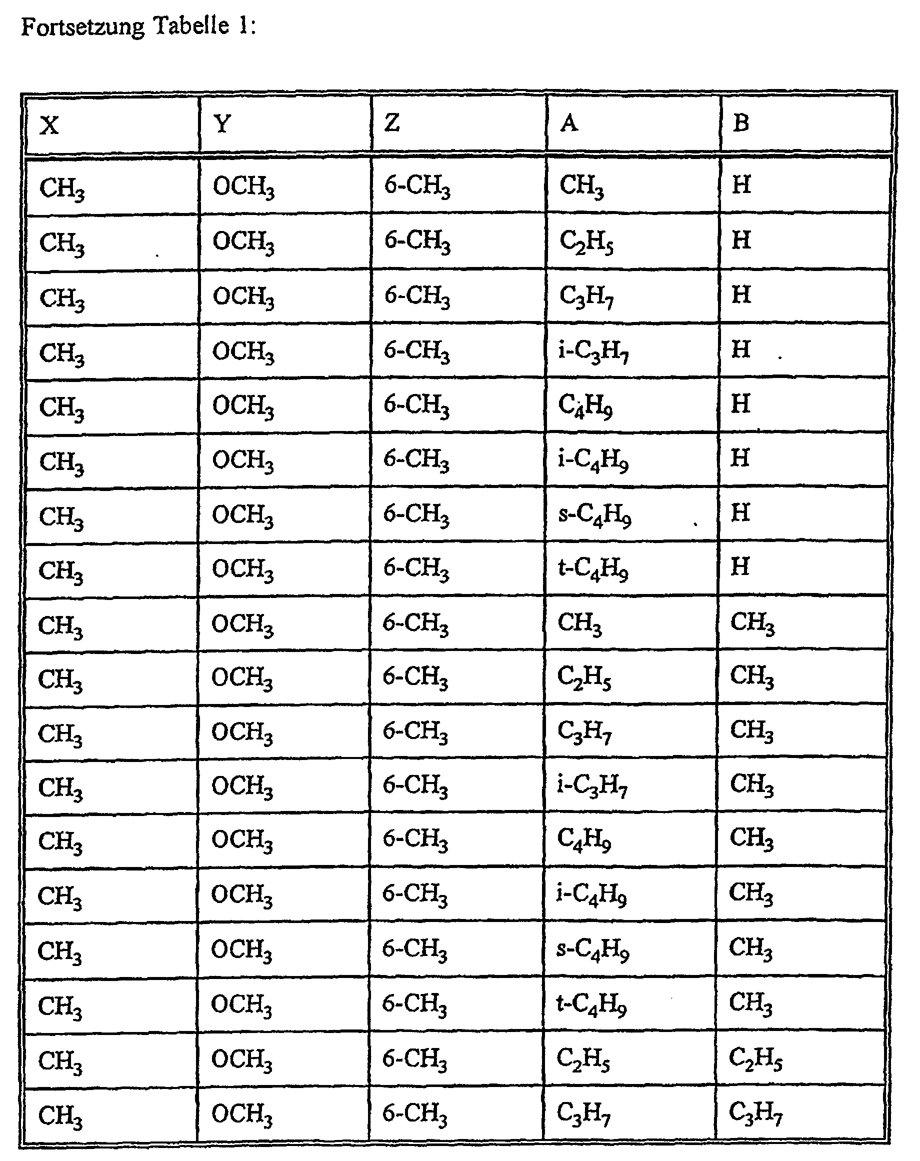

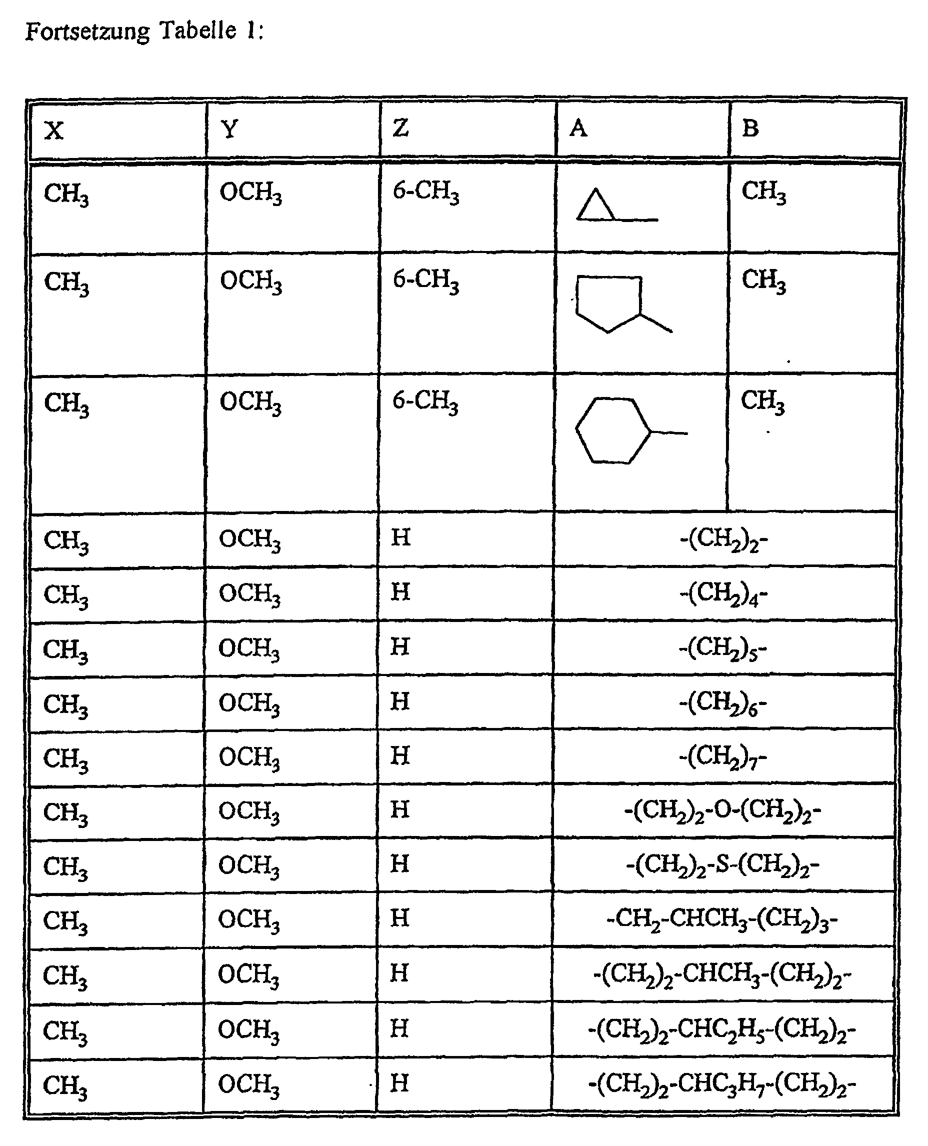

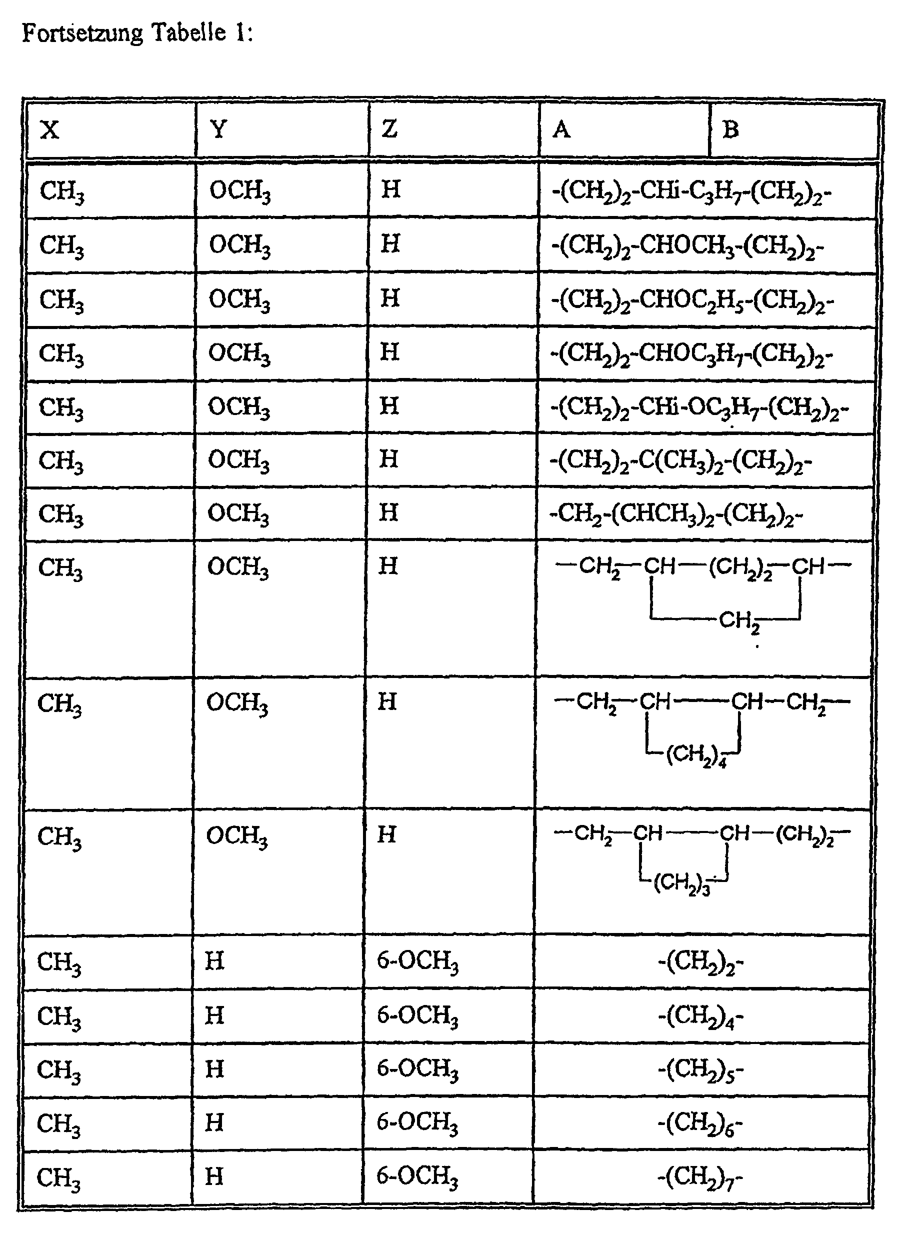

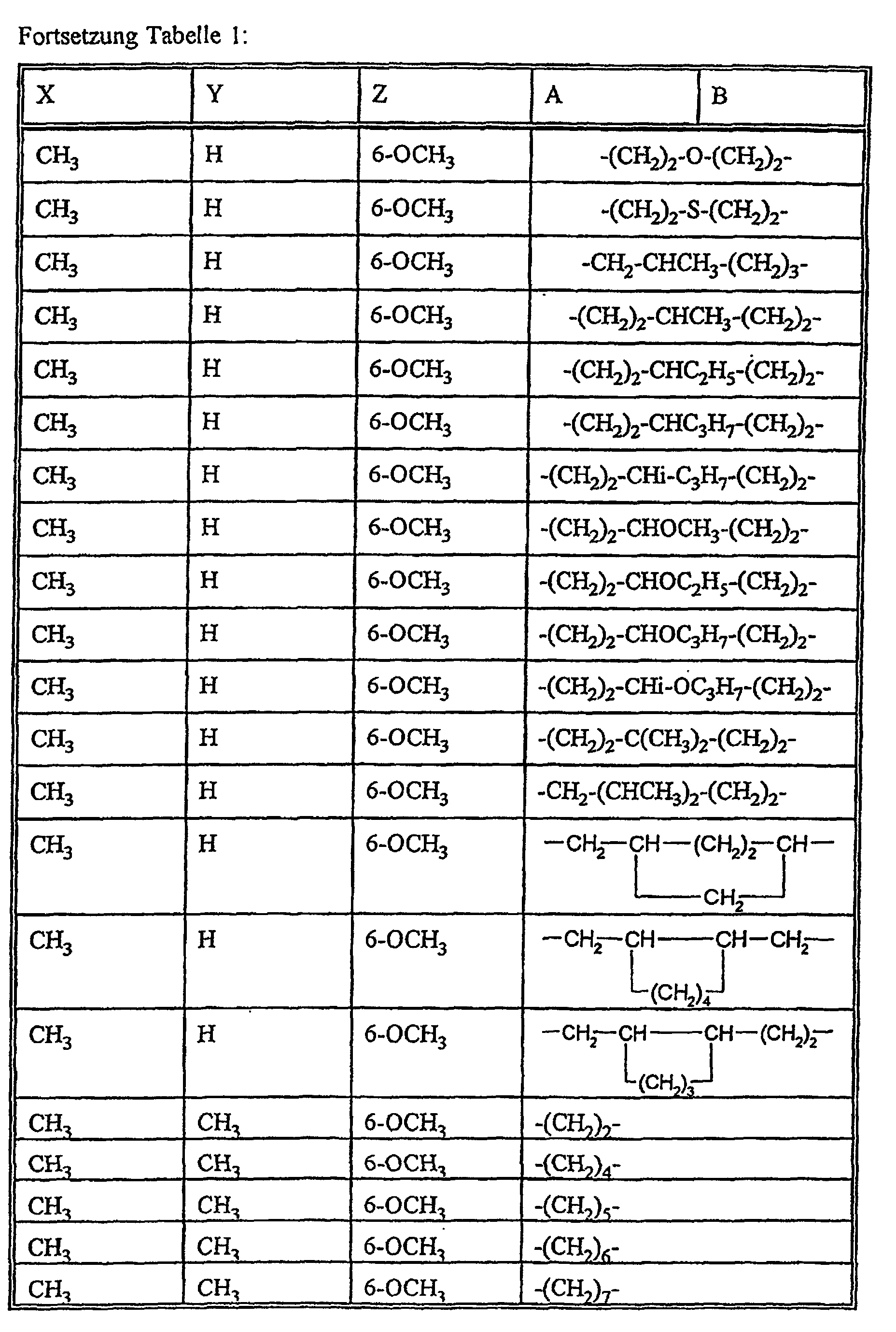

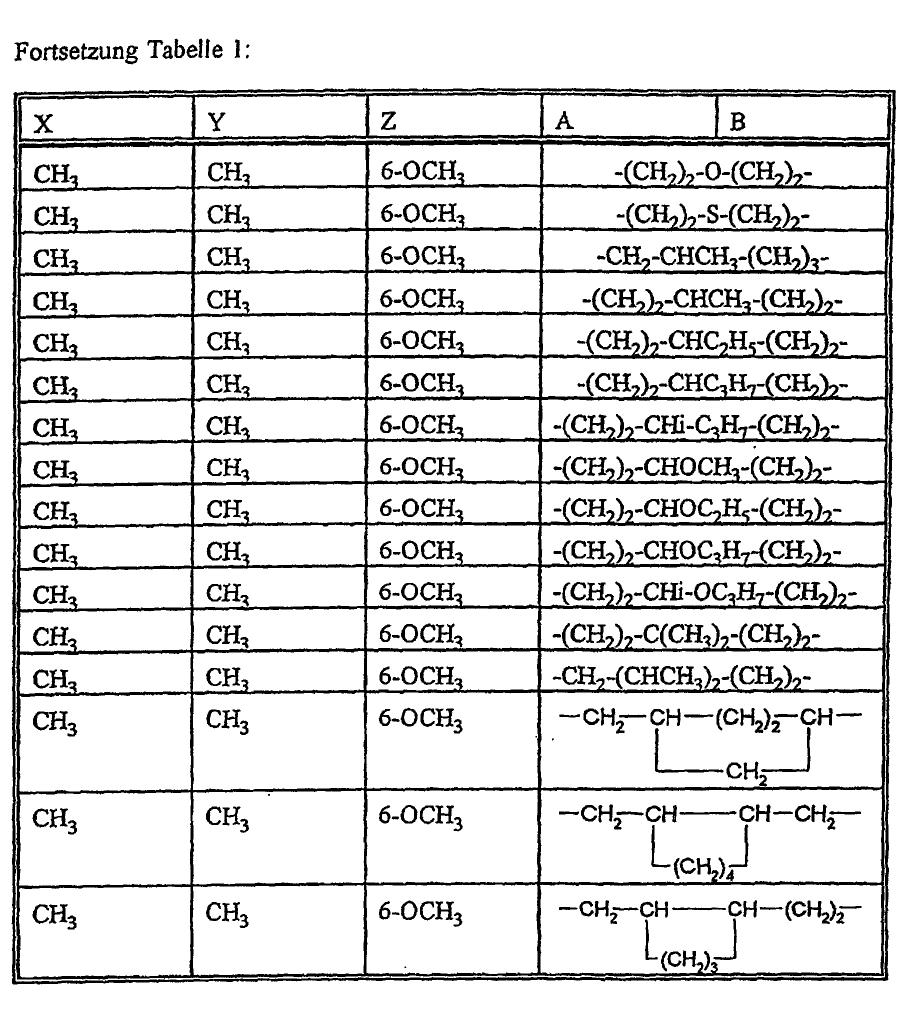

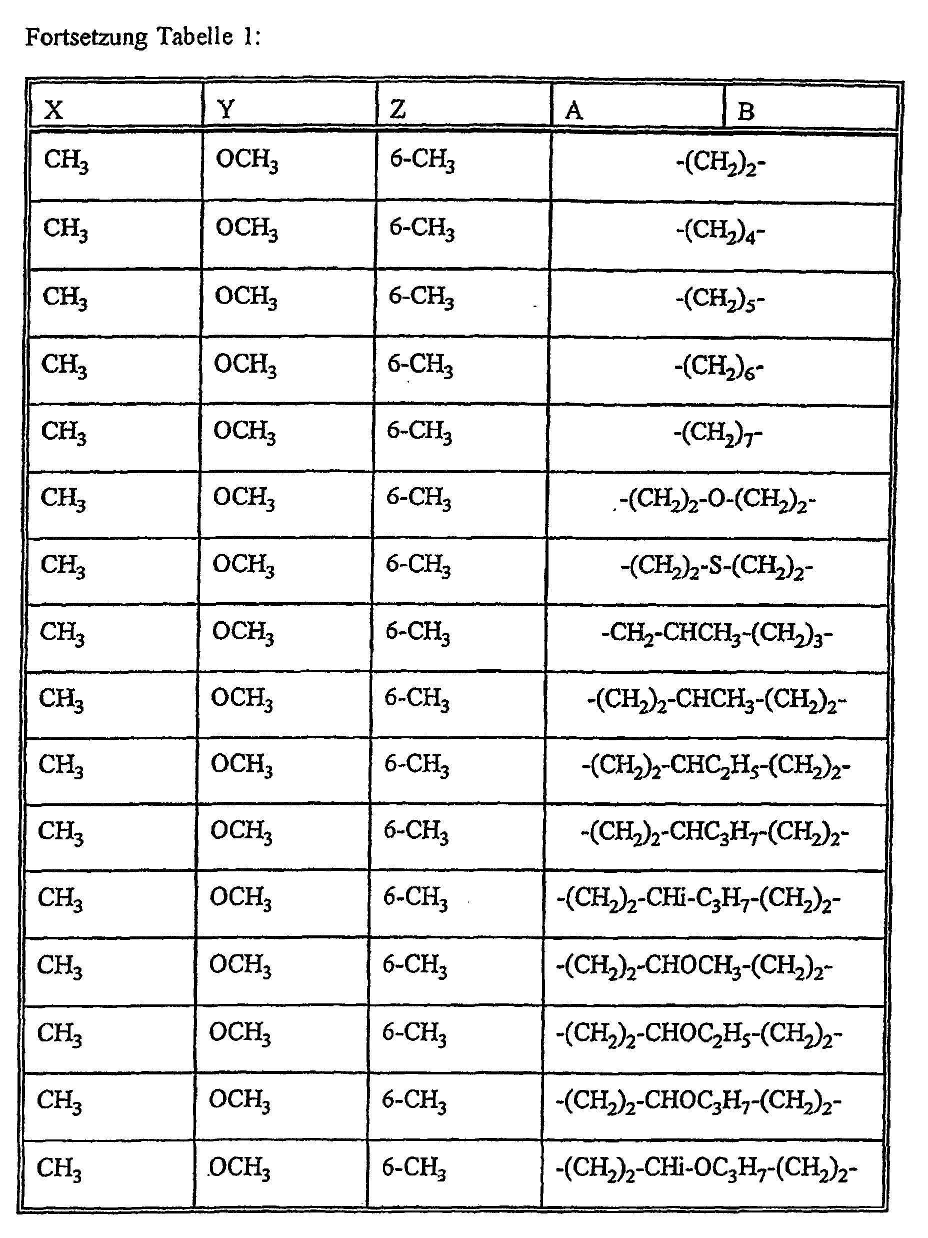

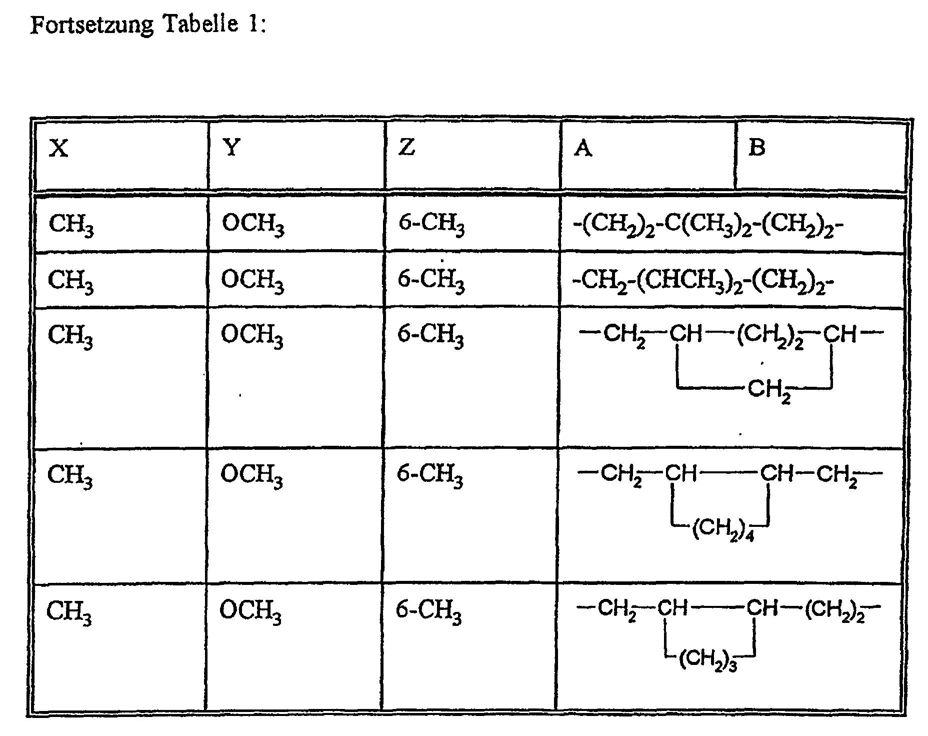

Im einzelnen seien außer den bei den Herstellungsbeispielen genannten Verbindungen die folgenden Verbindungen der Formel (Ib) genannt:

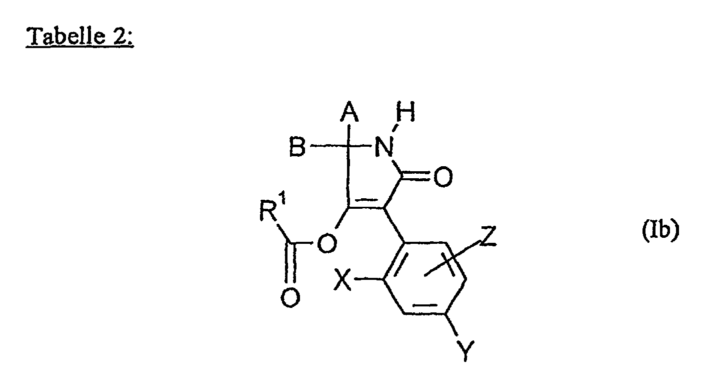

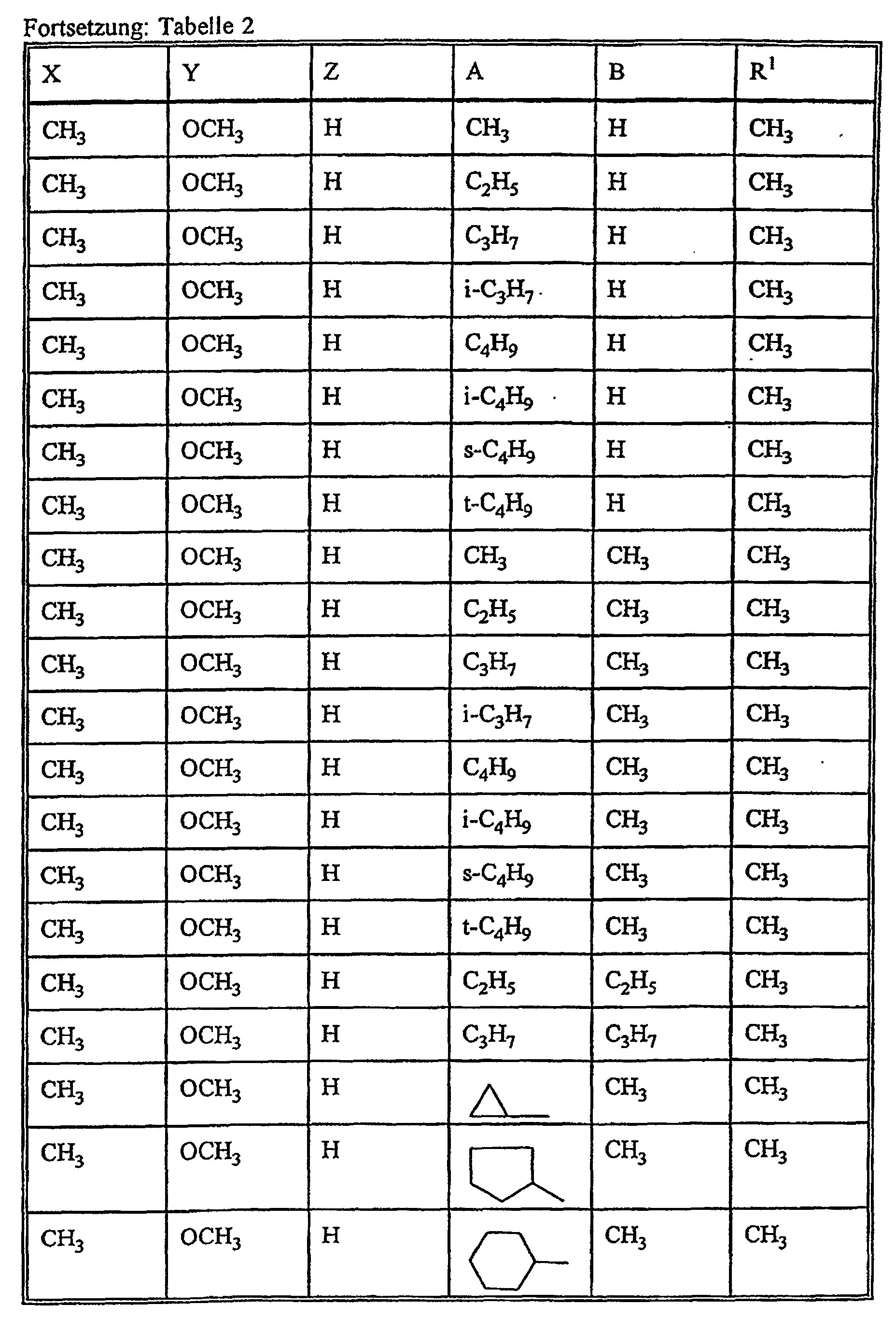

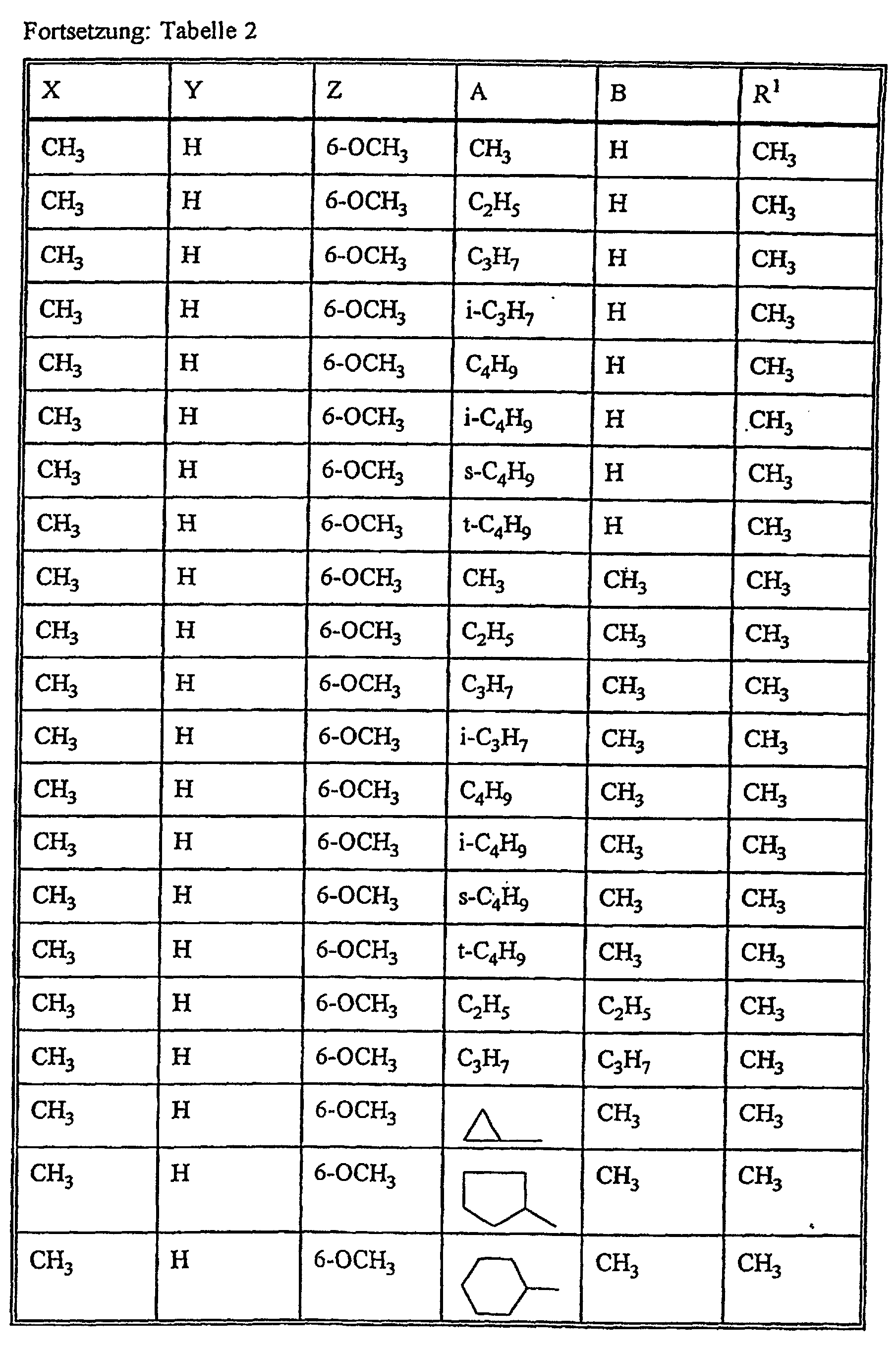

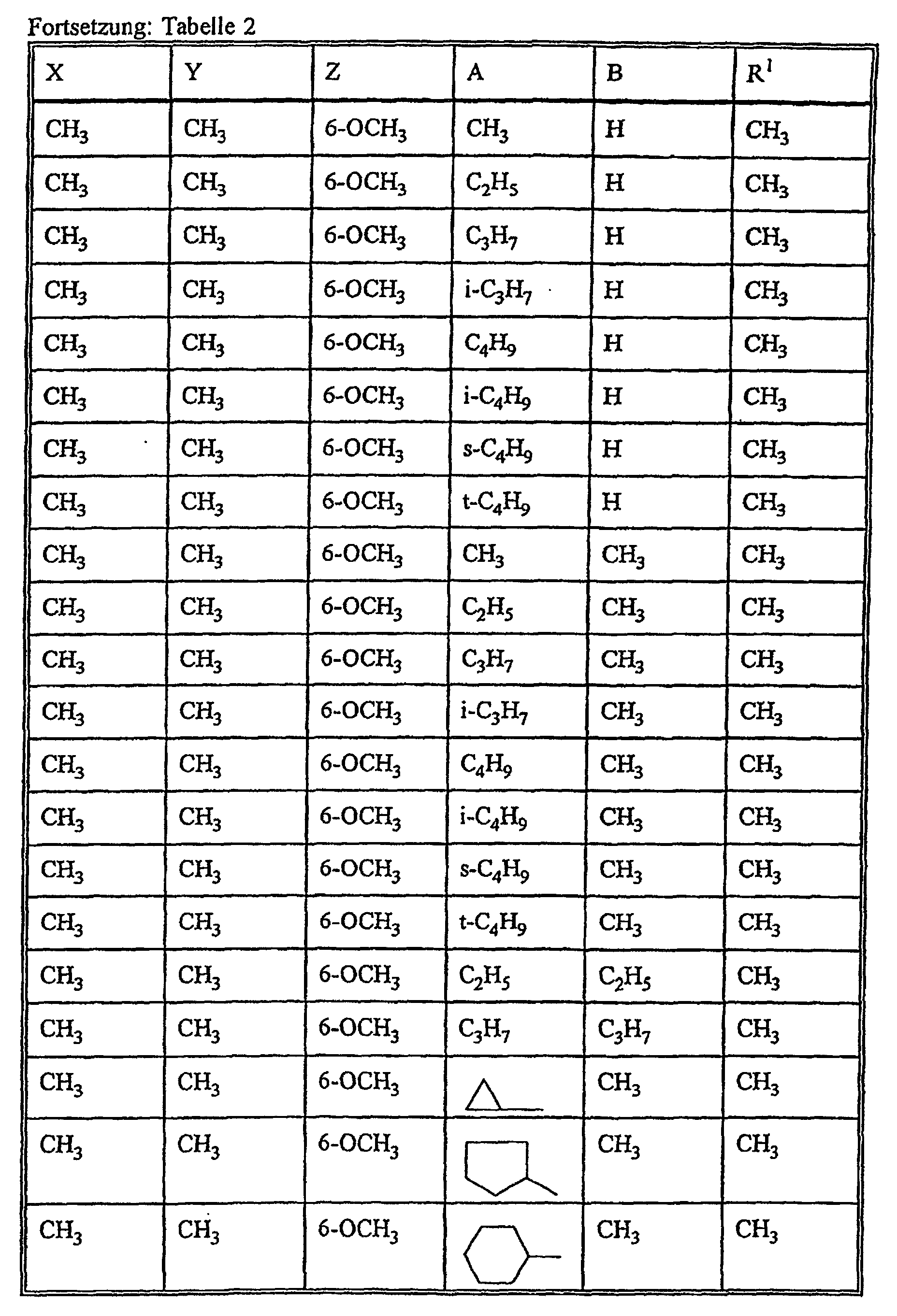

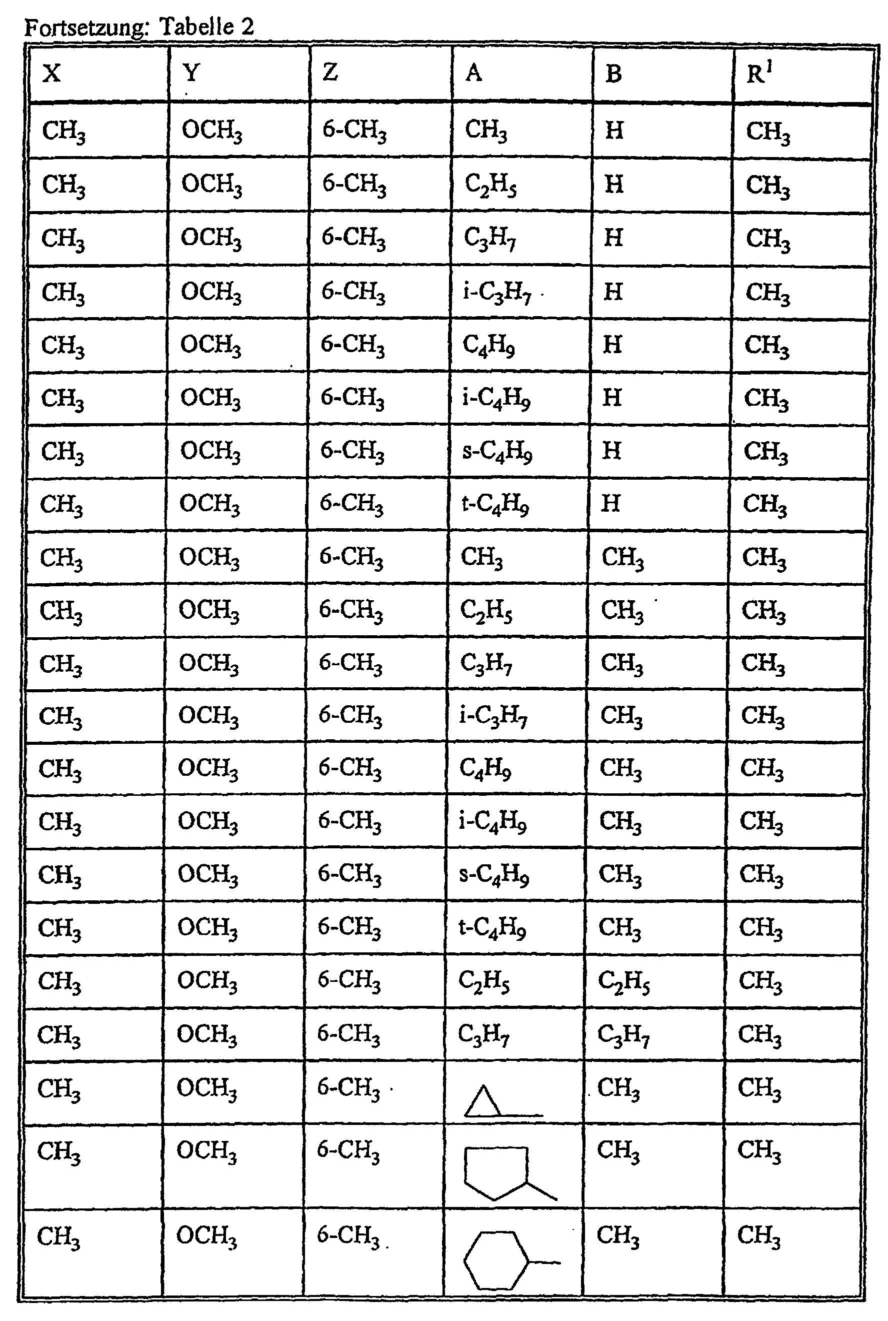

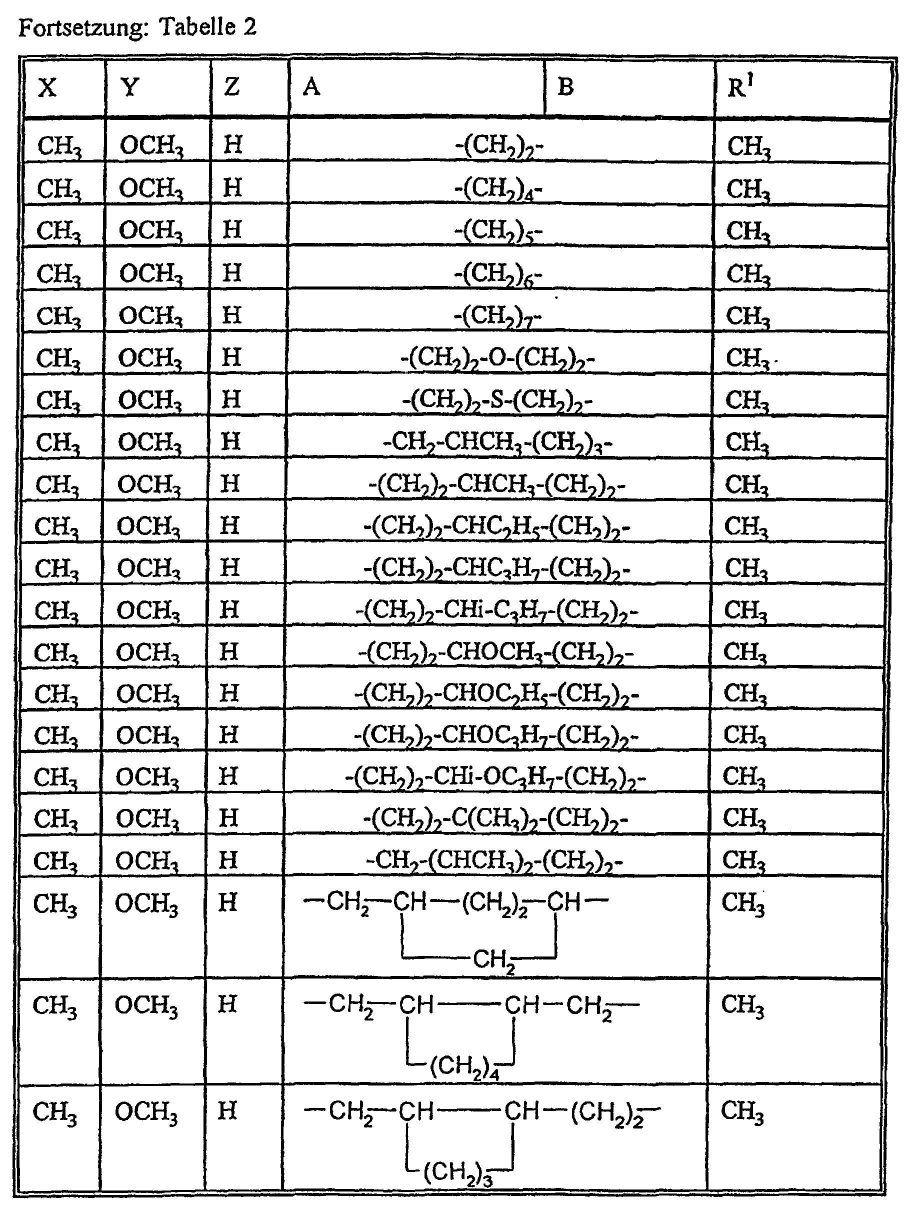

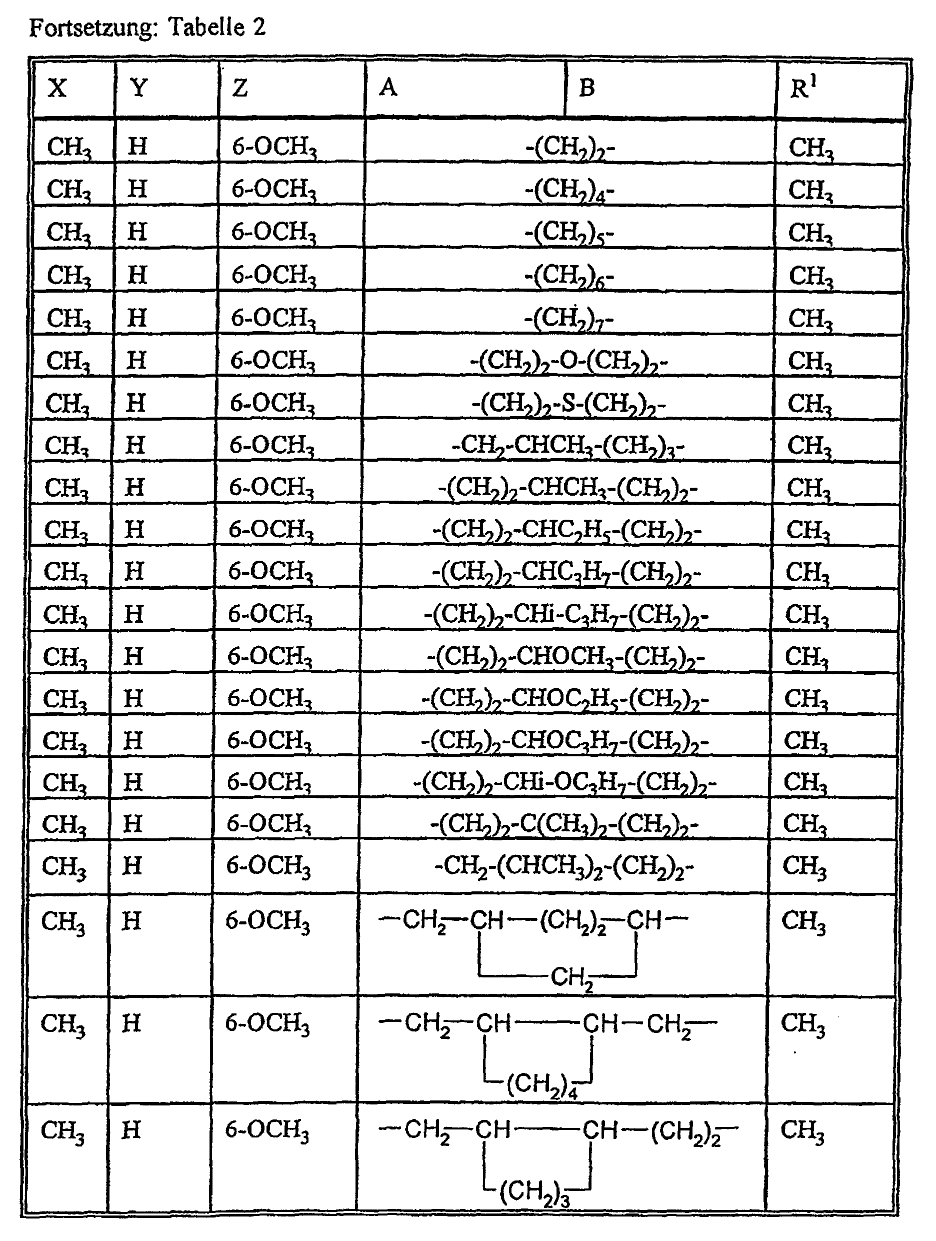

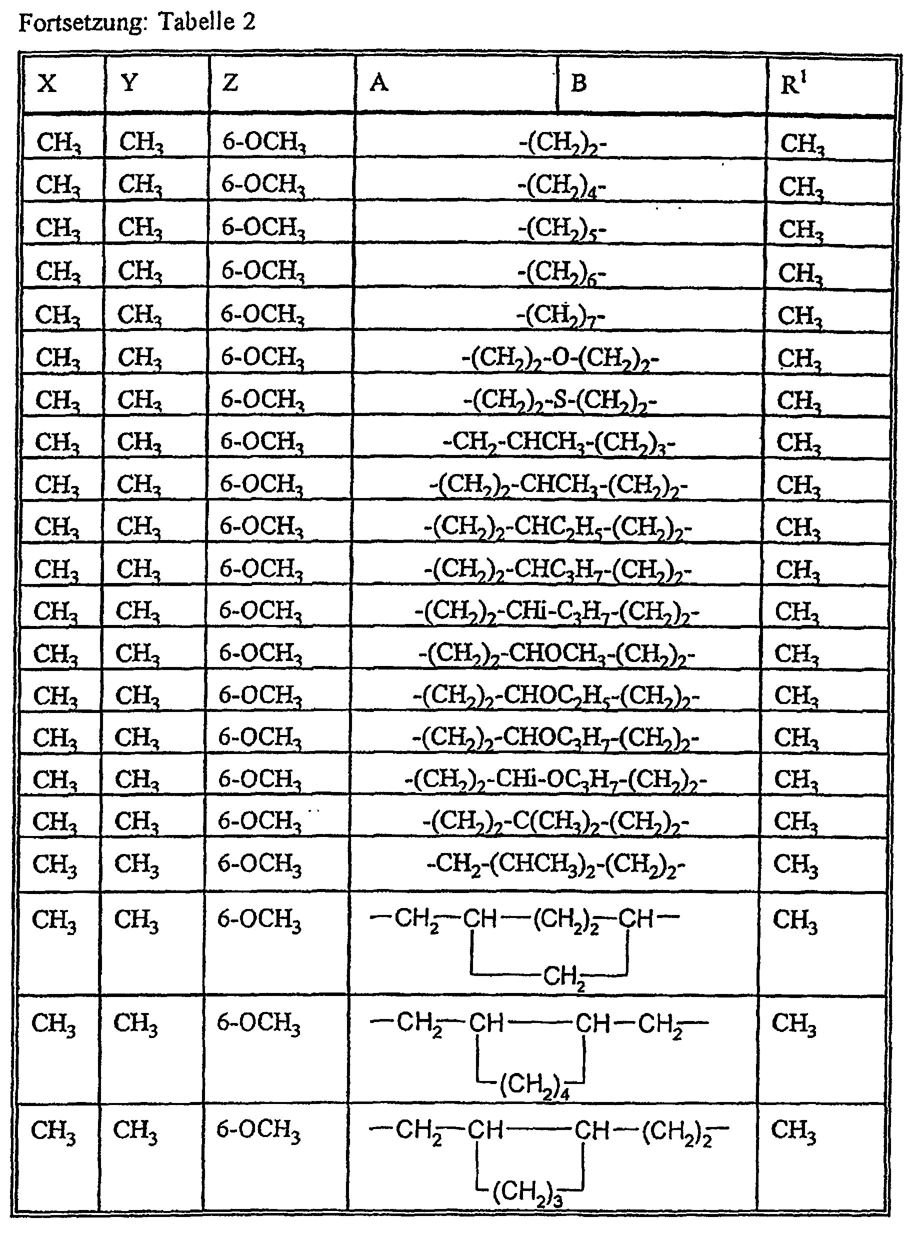

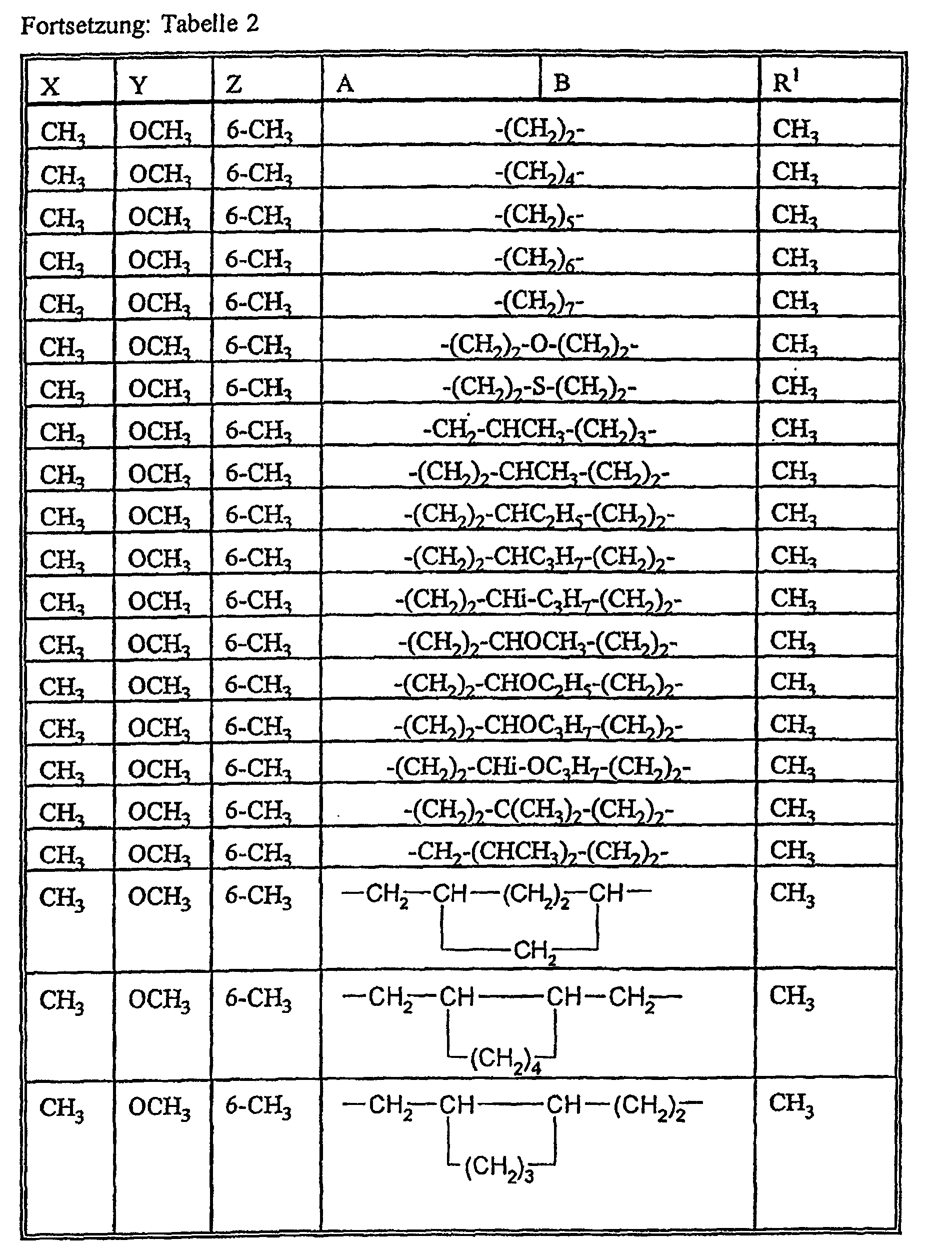

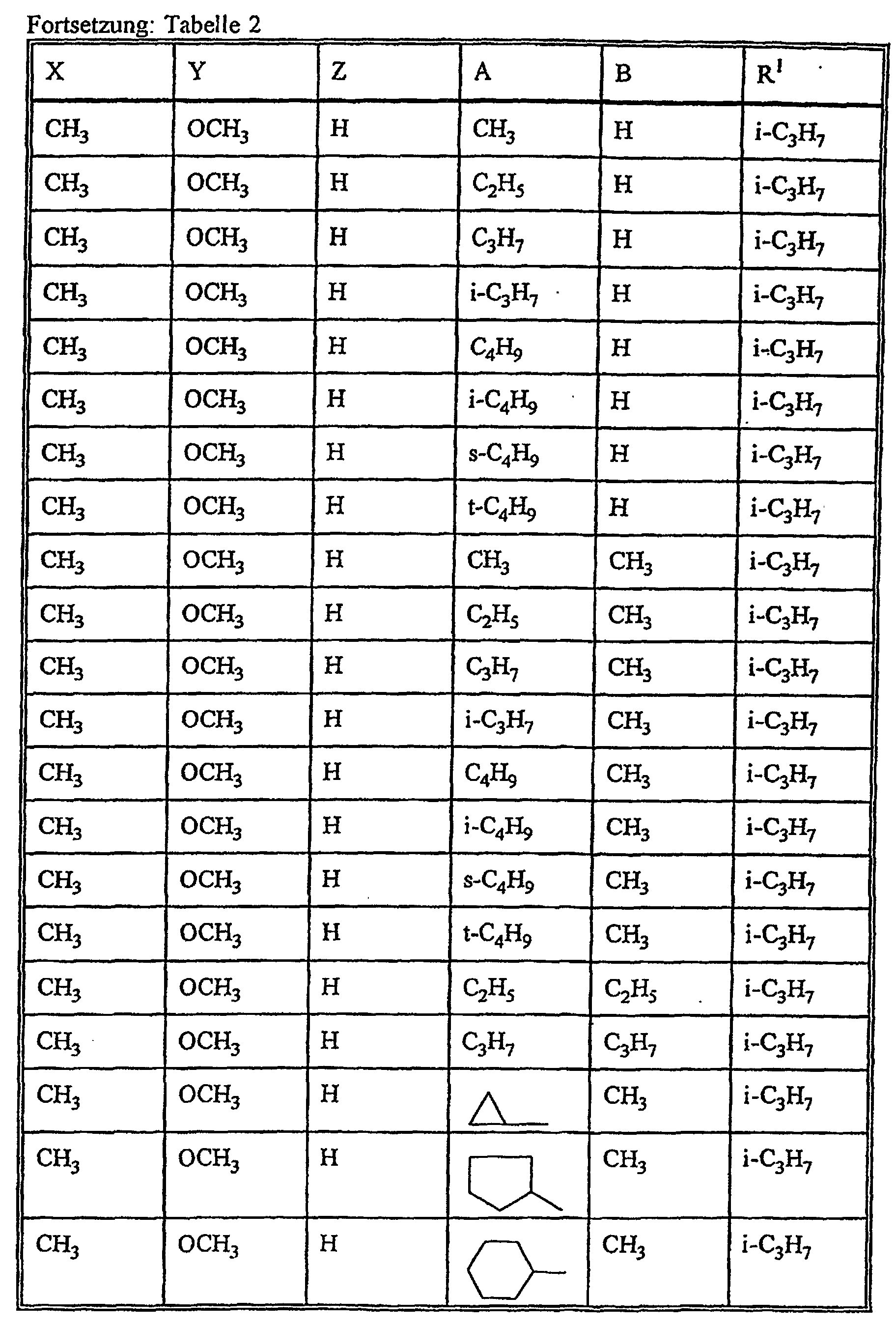

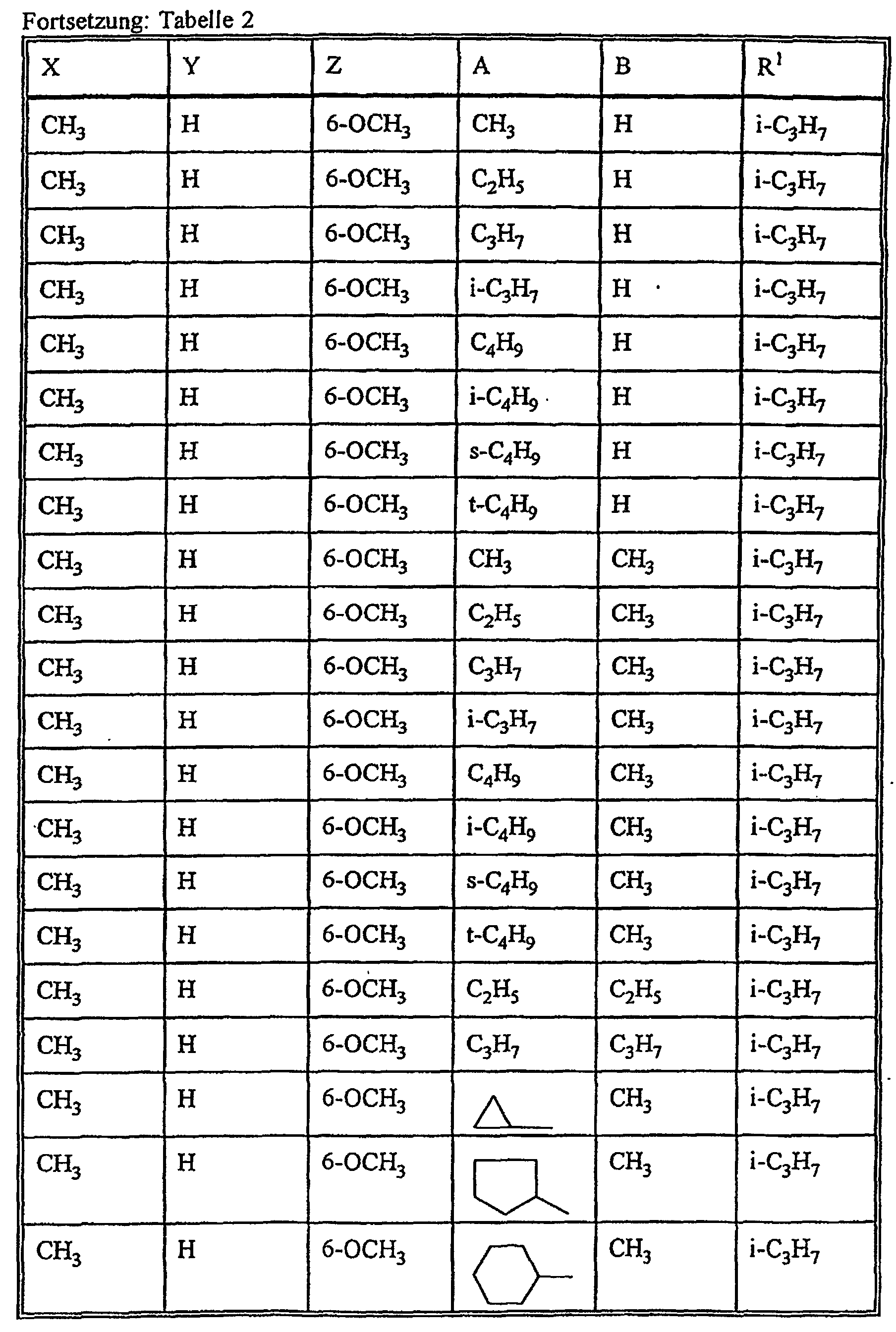

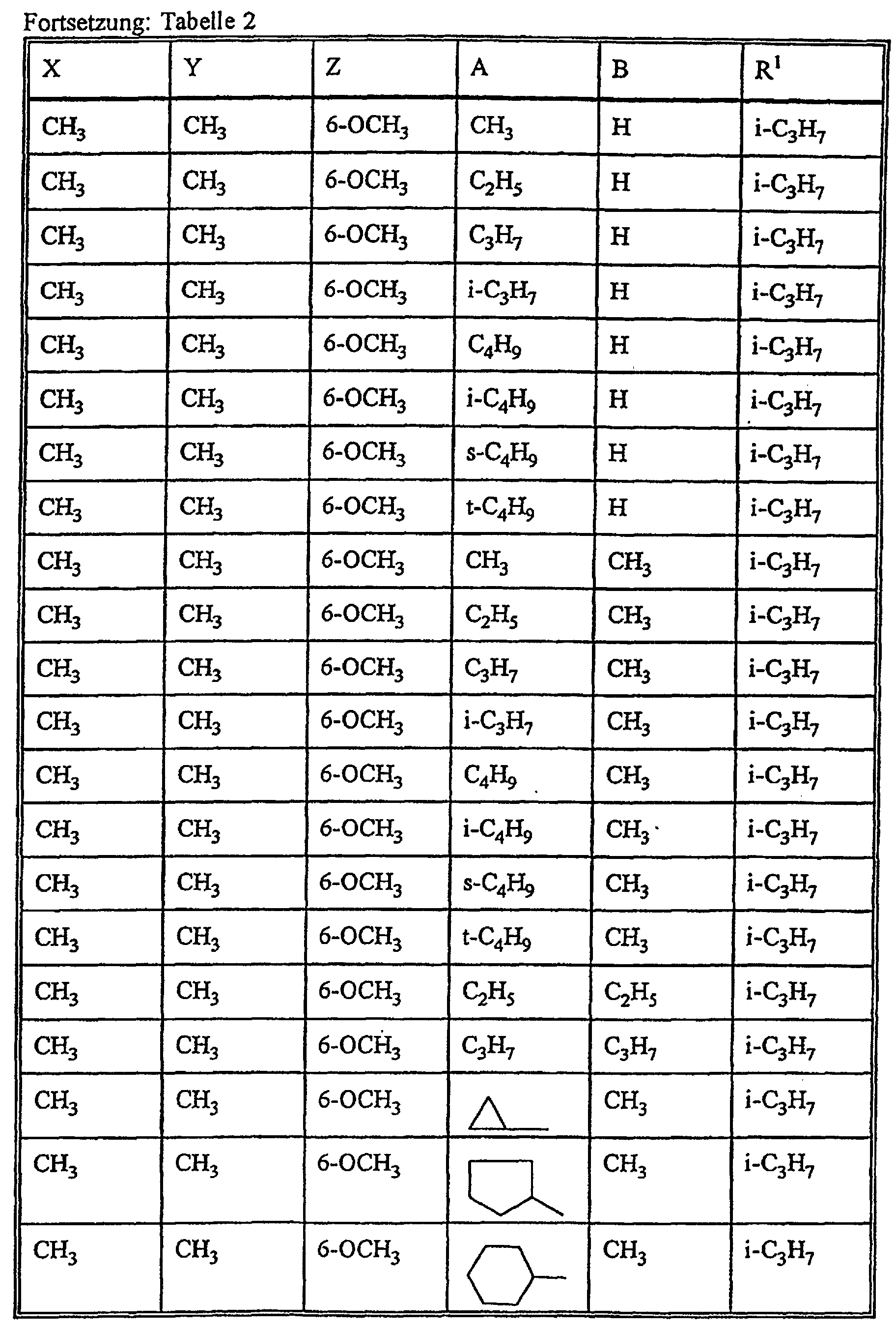

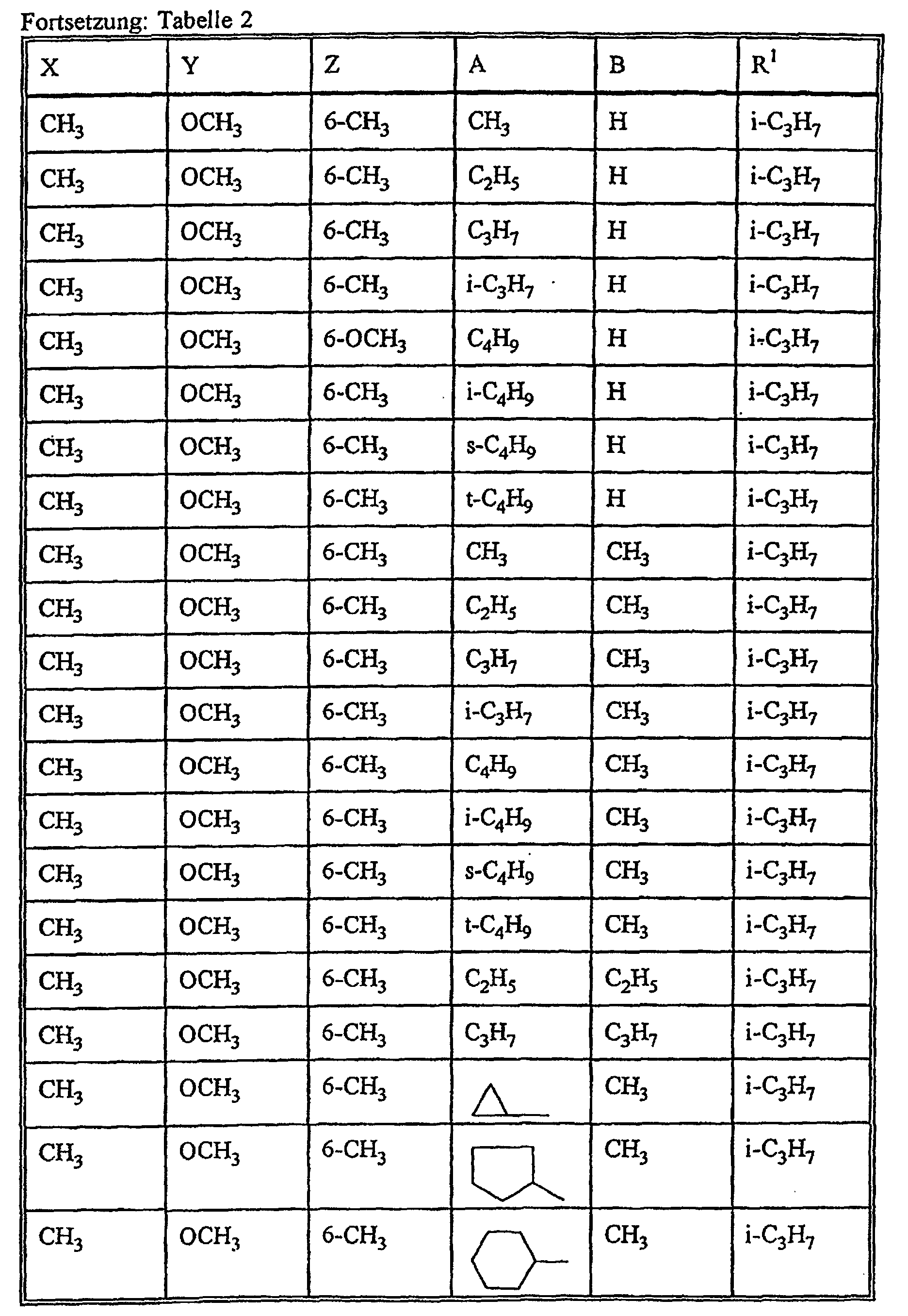

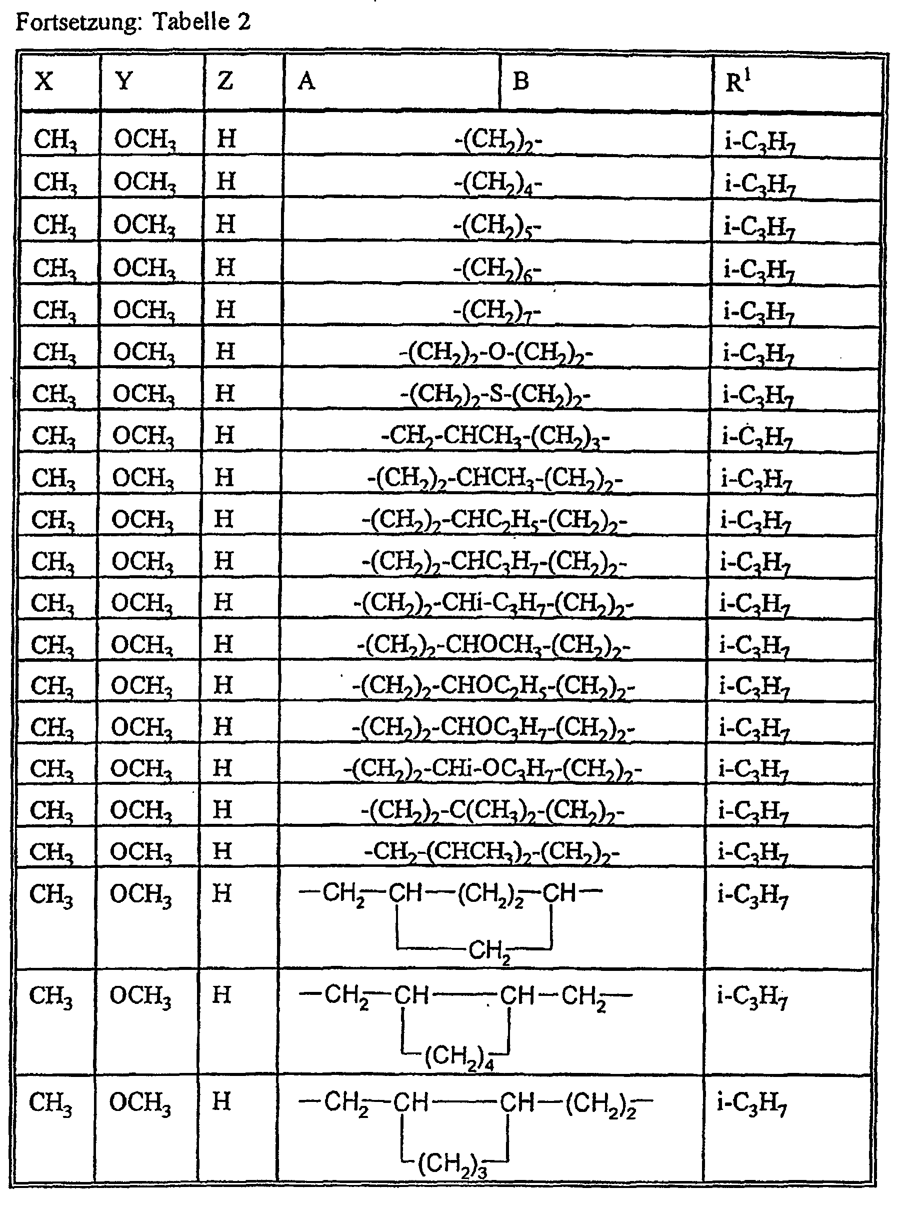

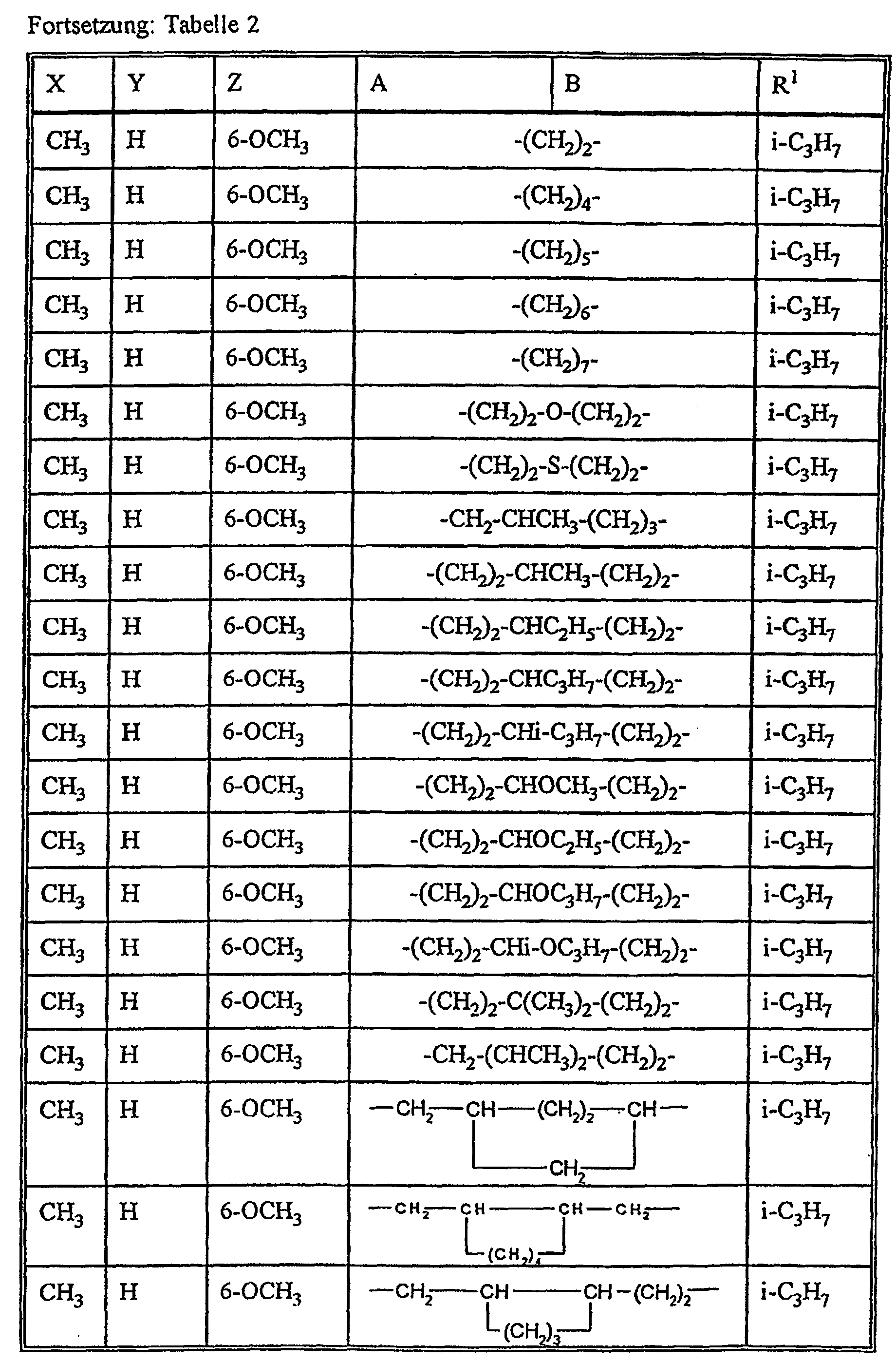

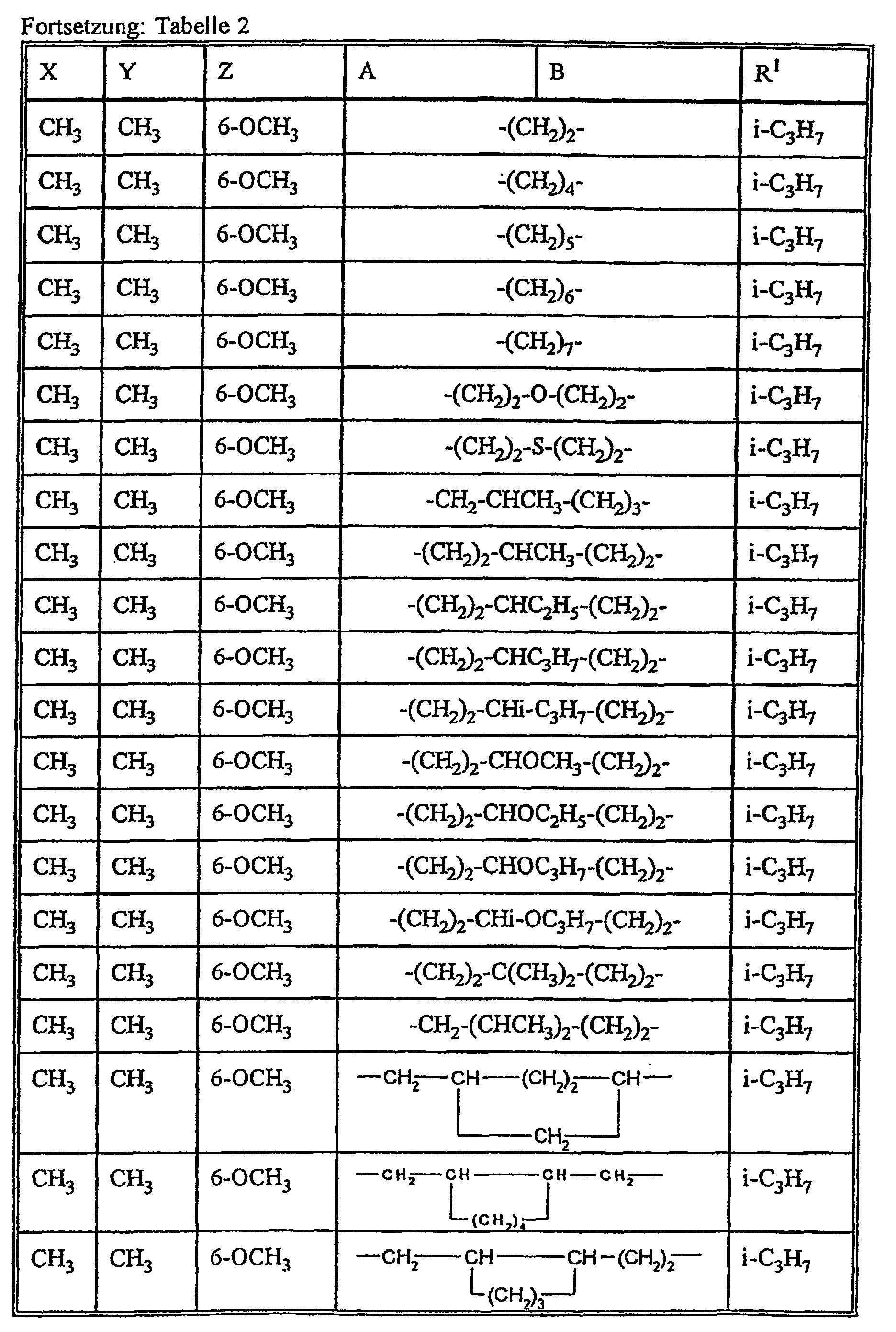

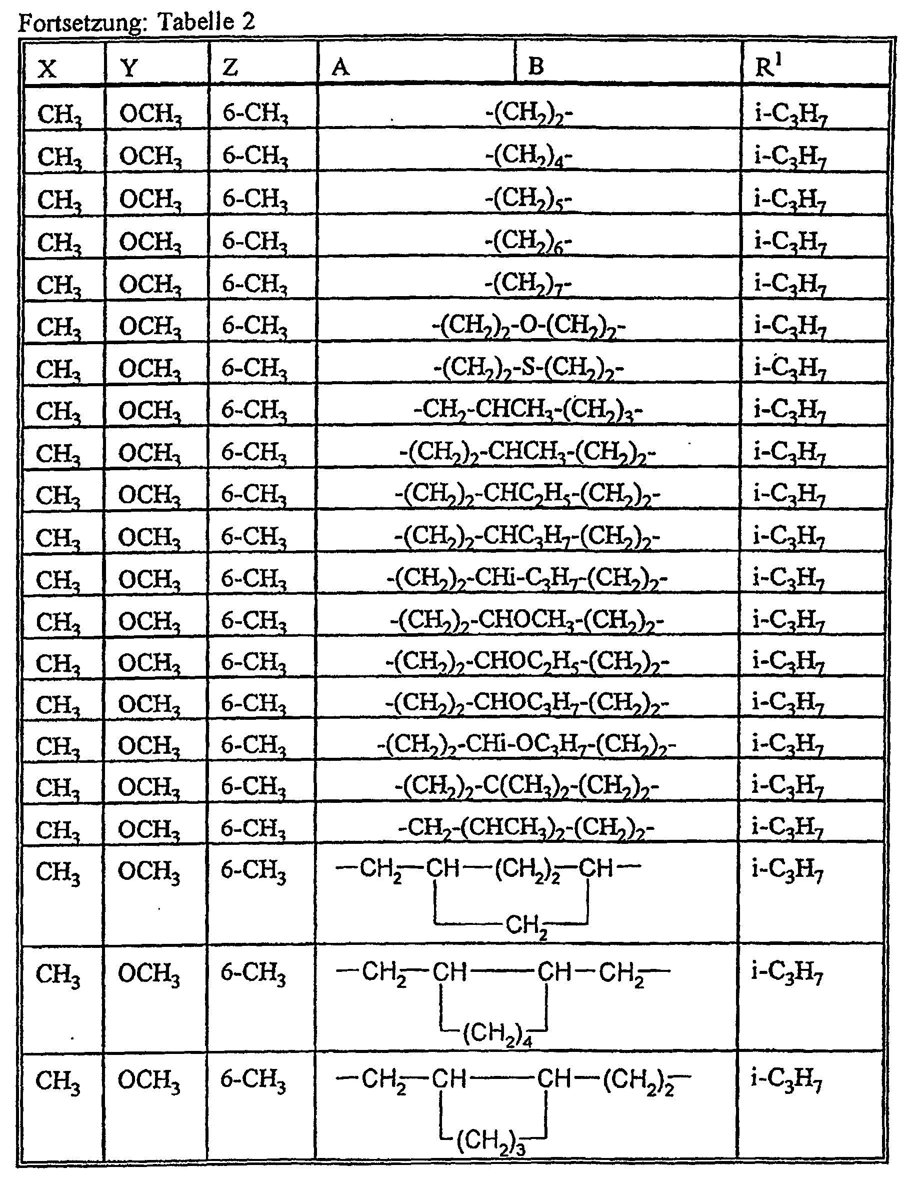

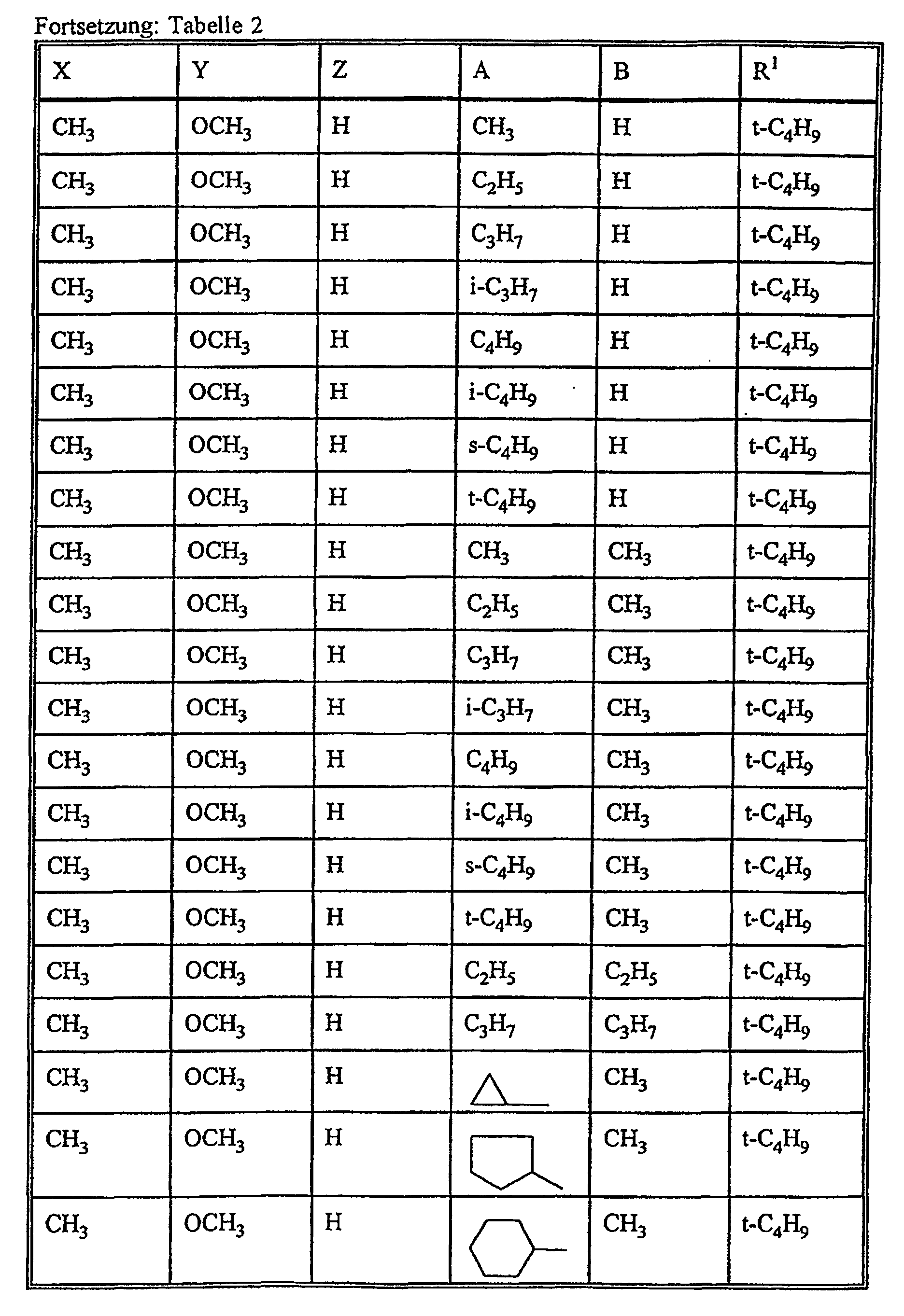

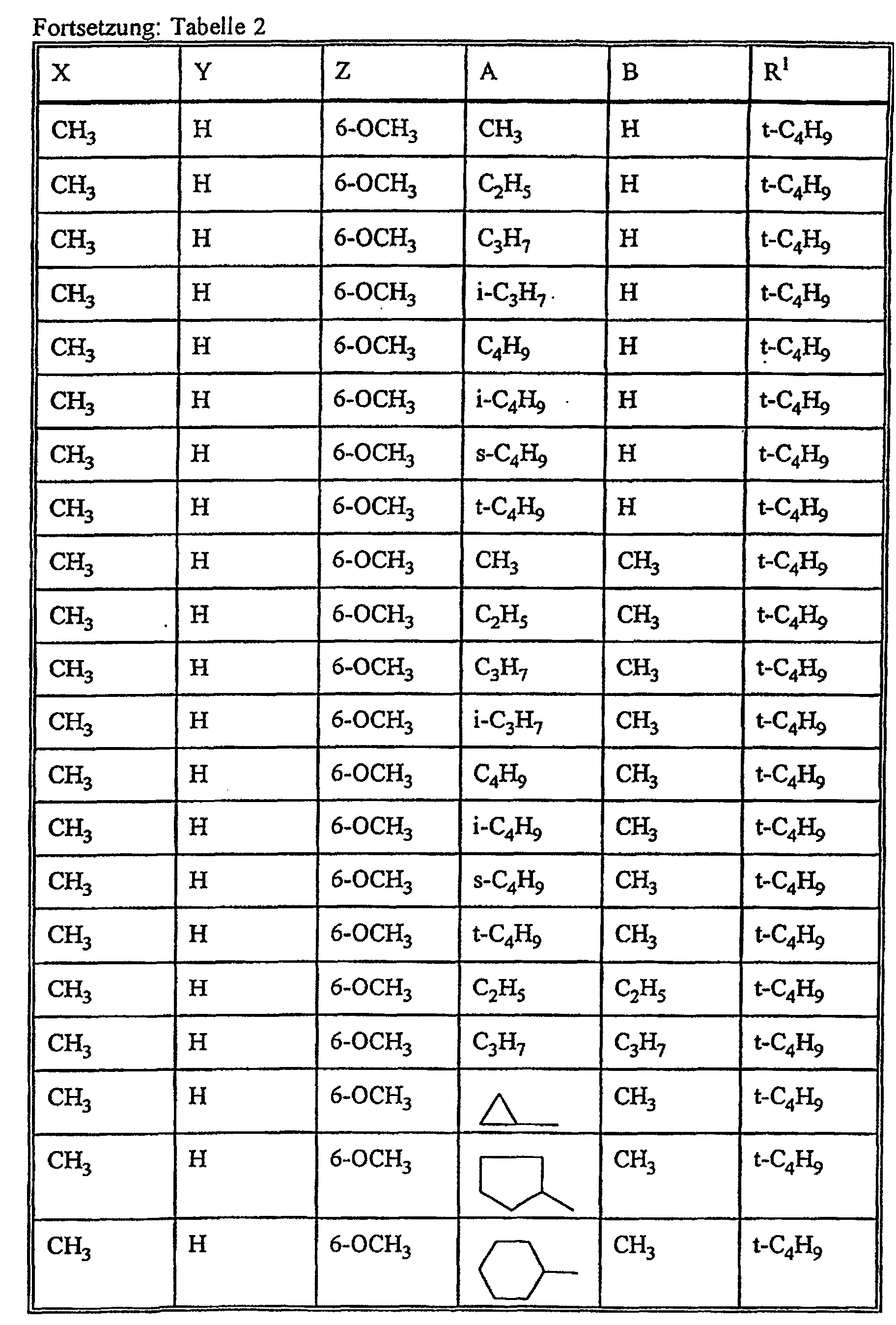

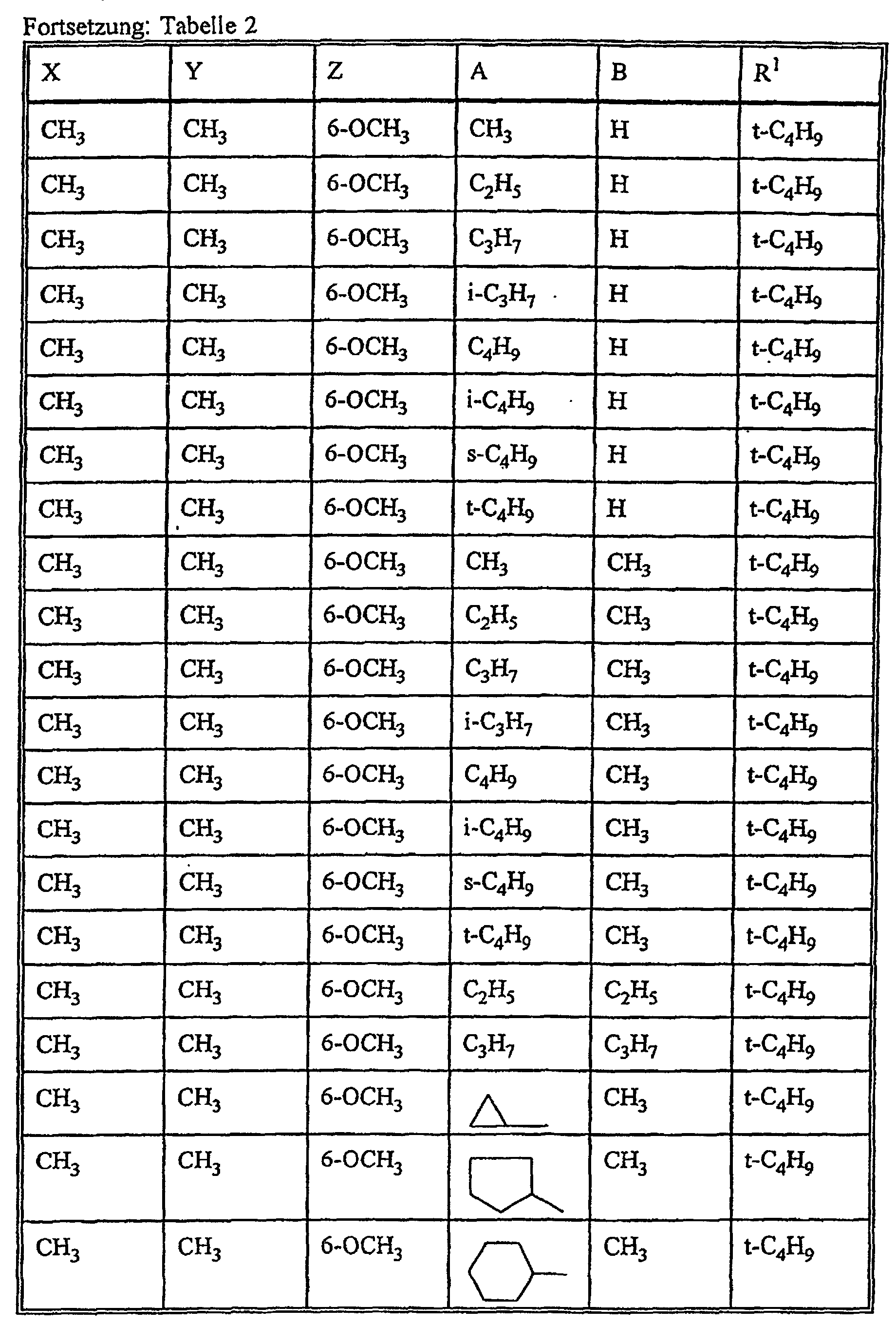

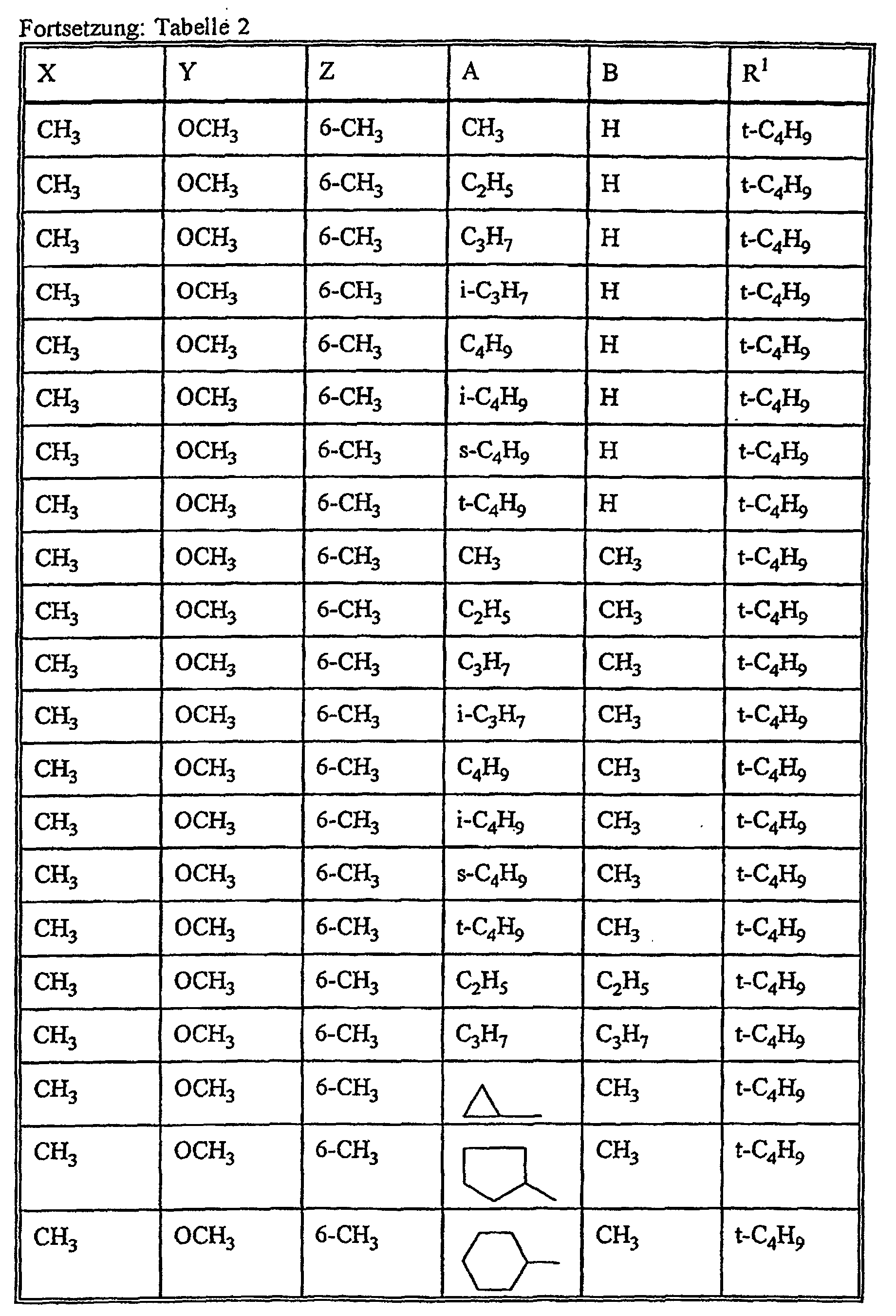

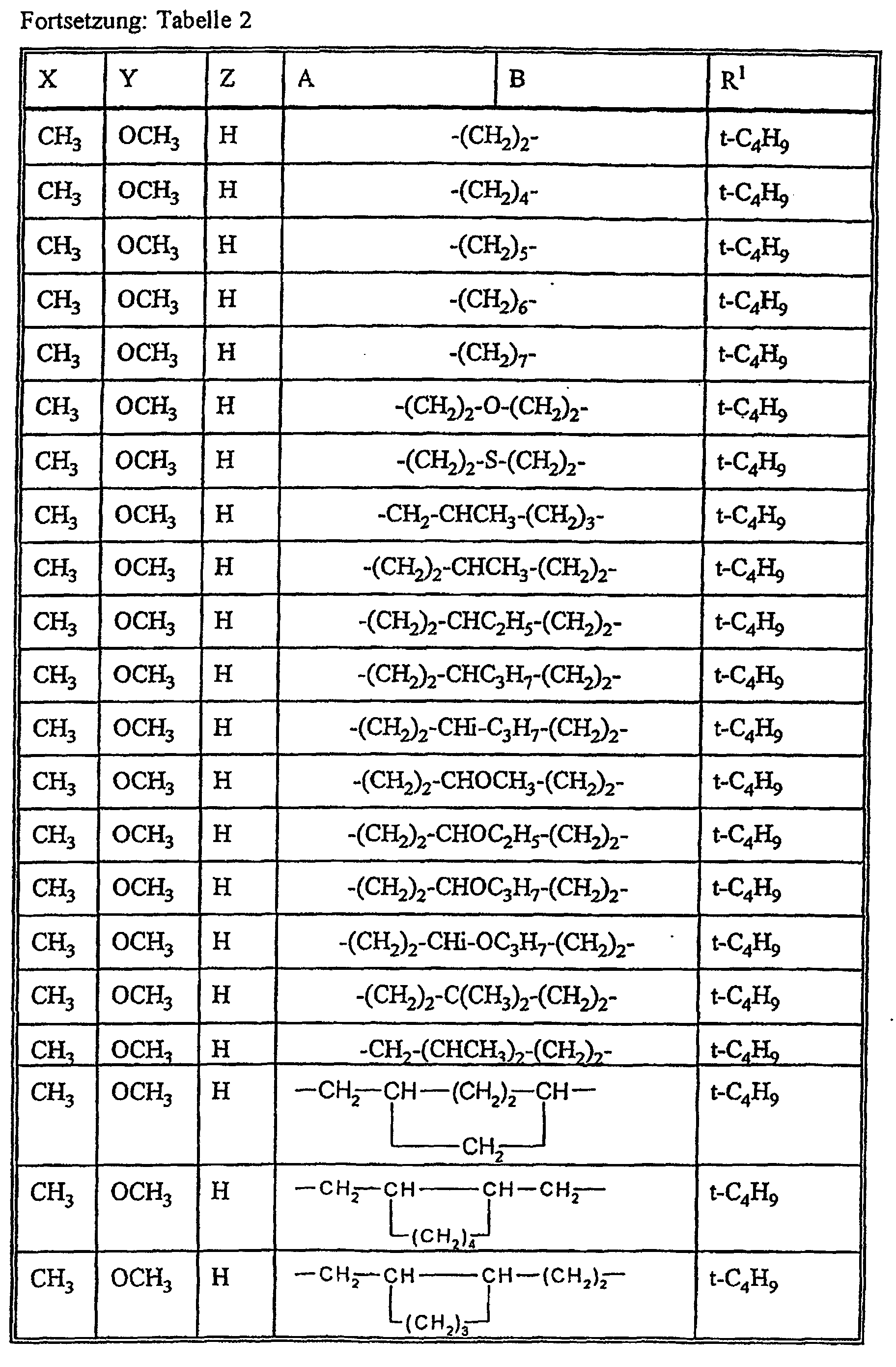

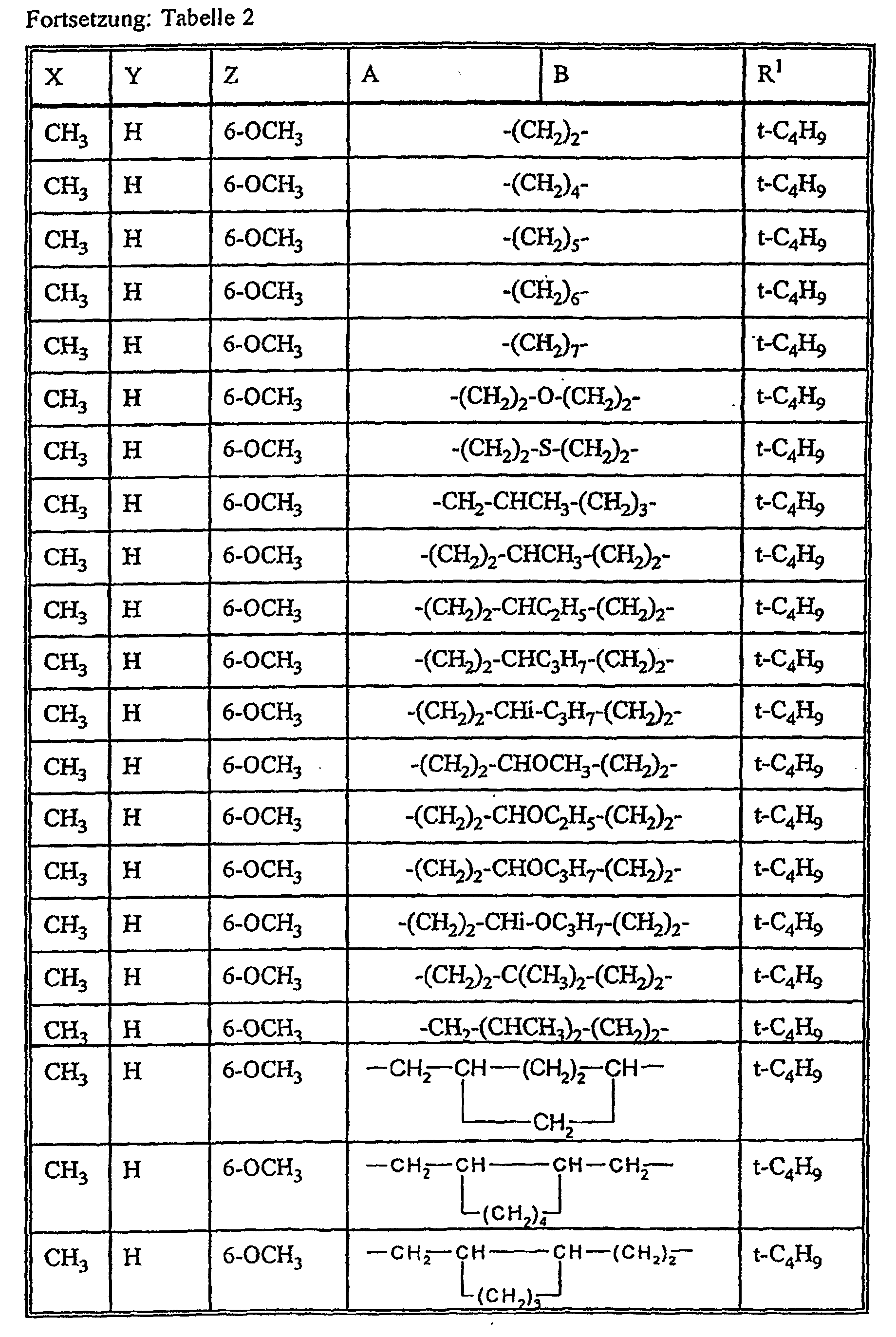

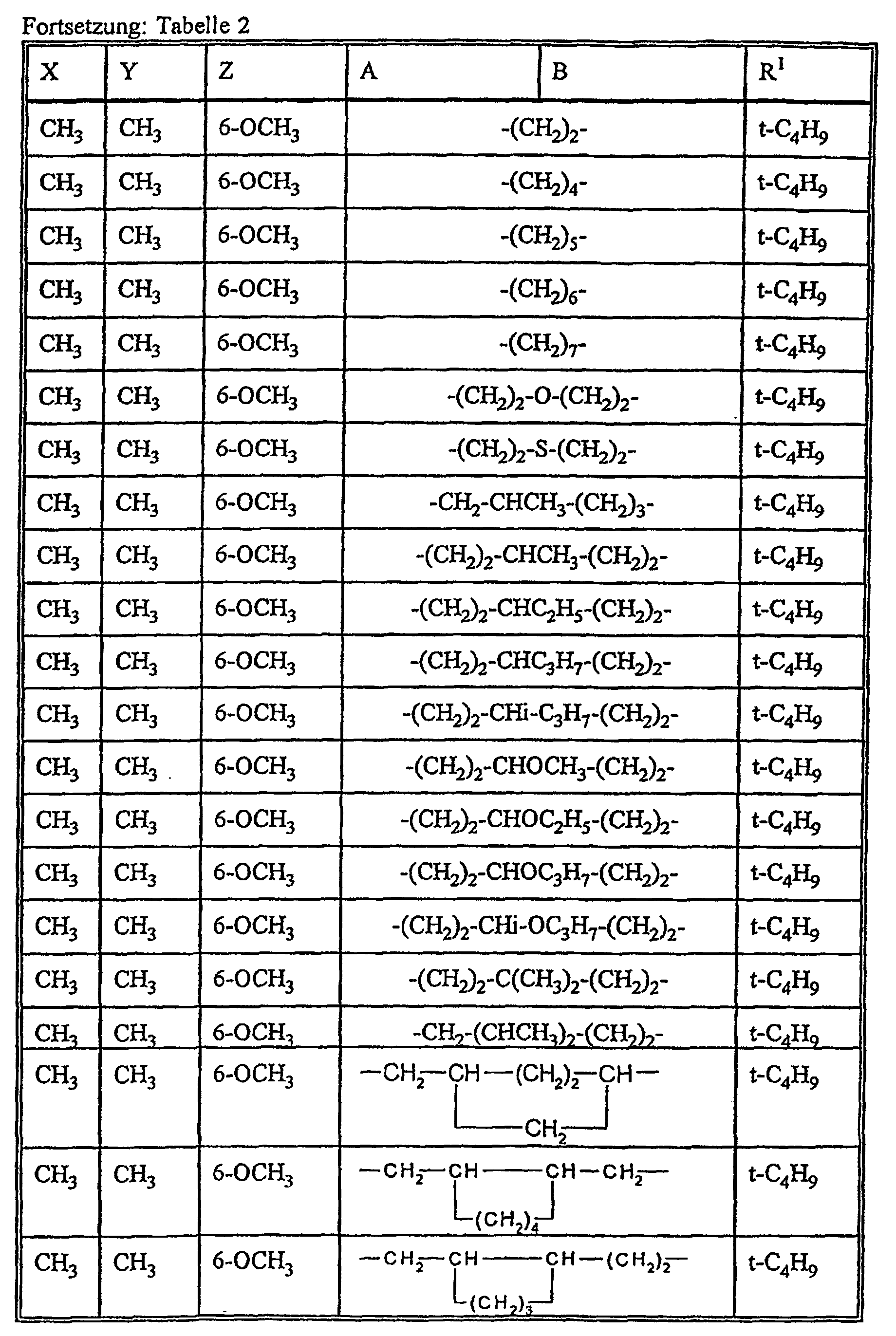

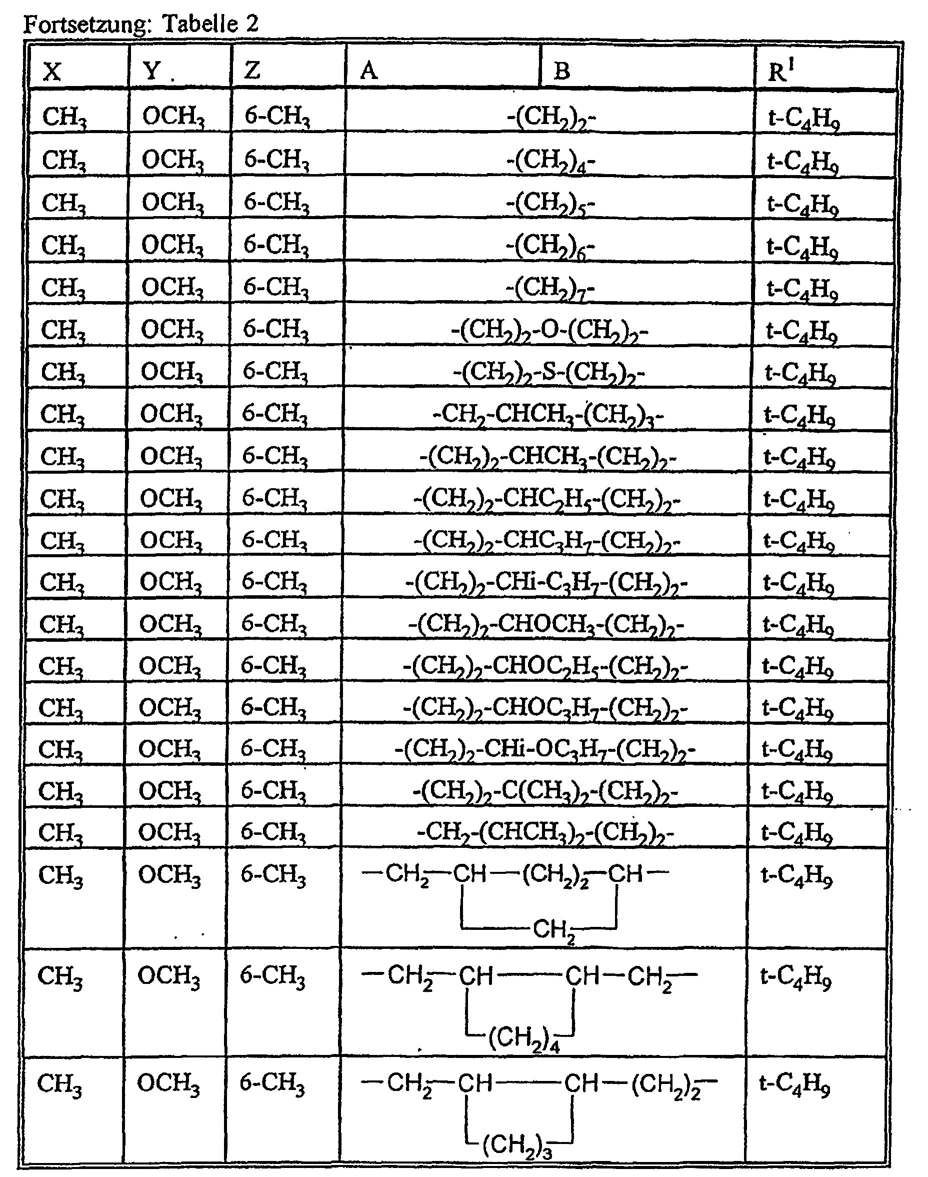

Im einzelnen seien außer den bei den Herstellungsbeispielen genannten Verbindungen die folgenden Verbindungen der Formel (Ic) genannt:

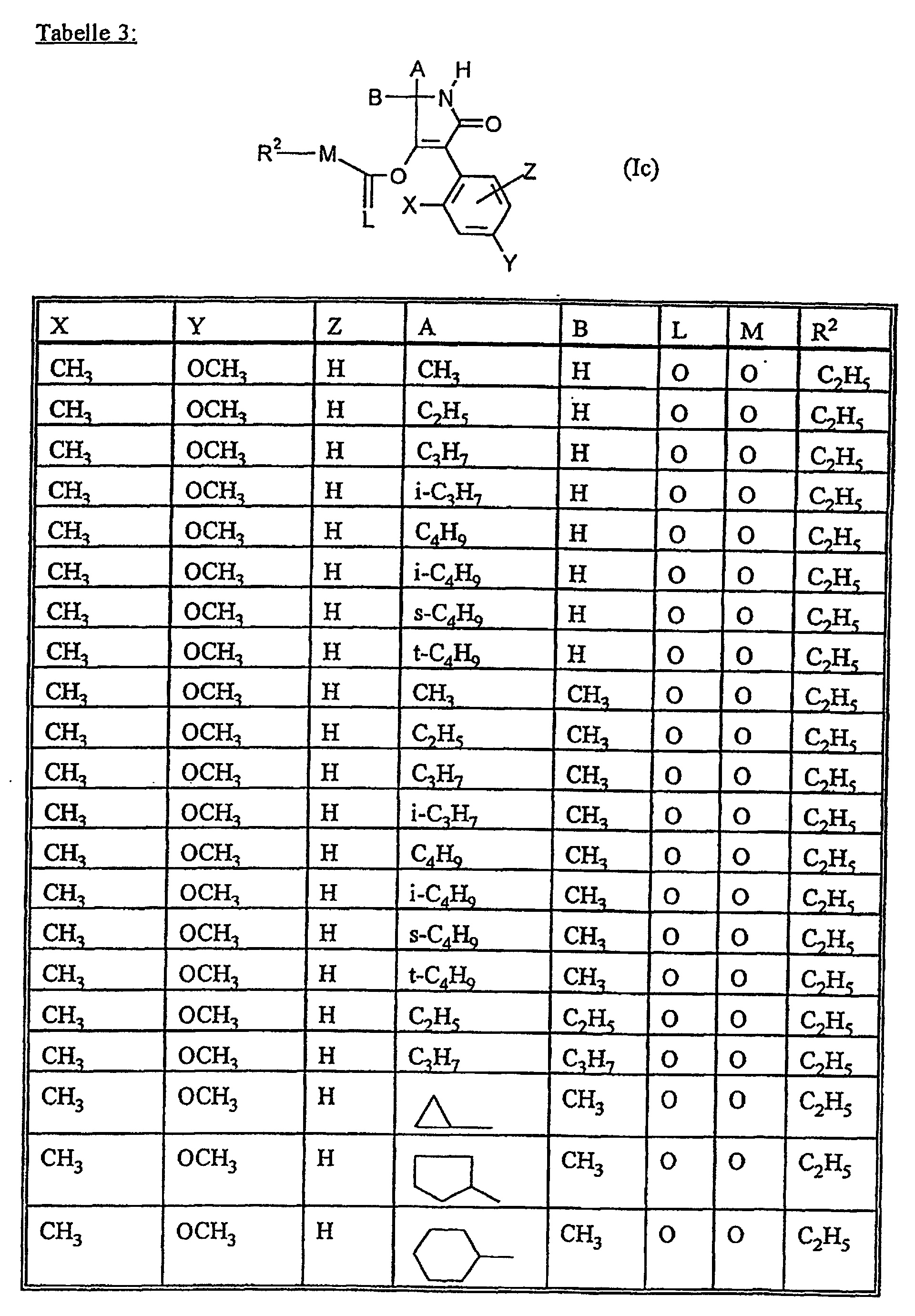

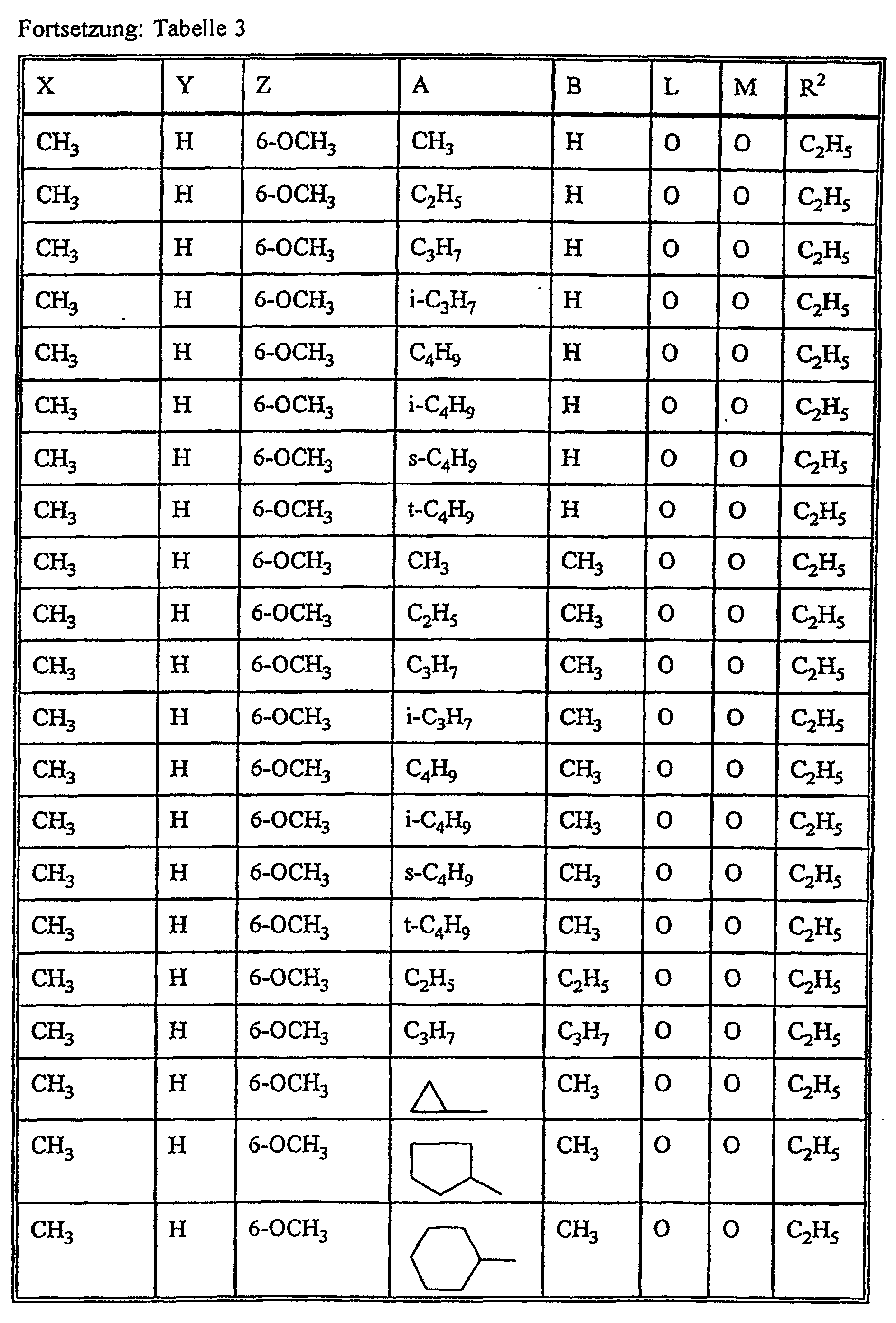

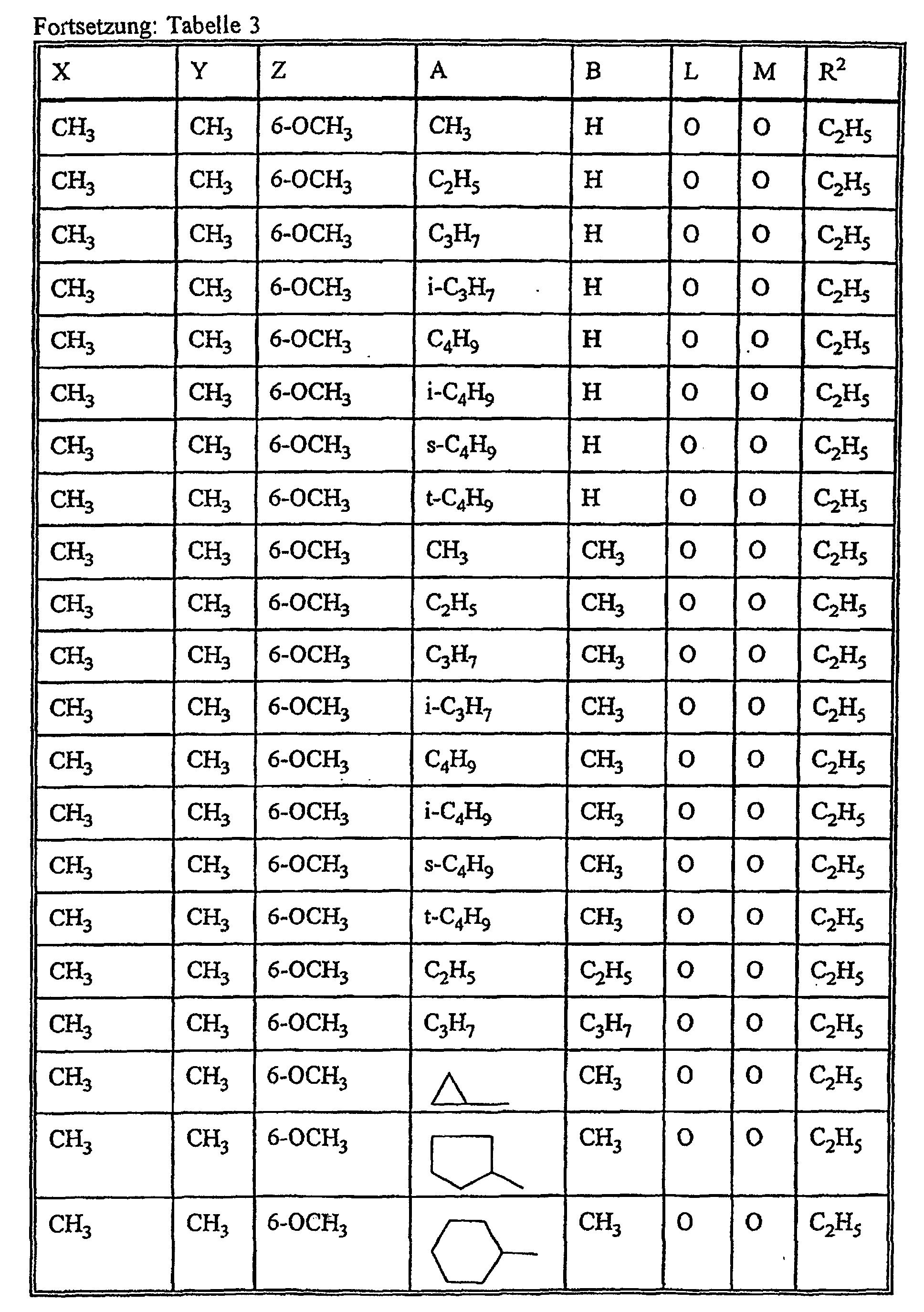

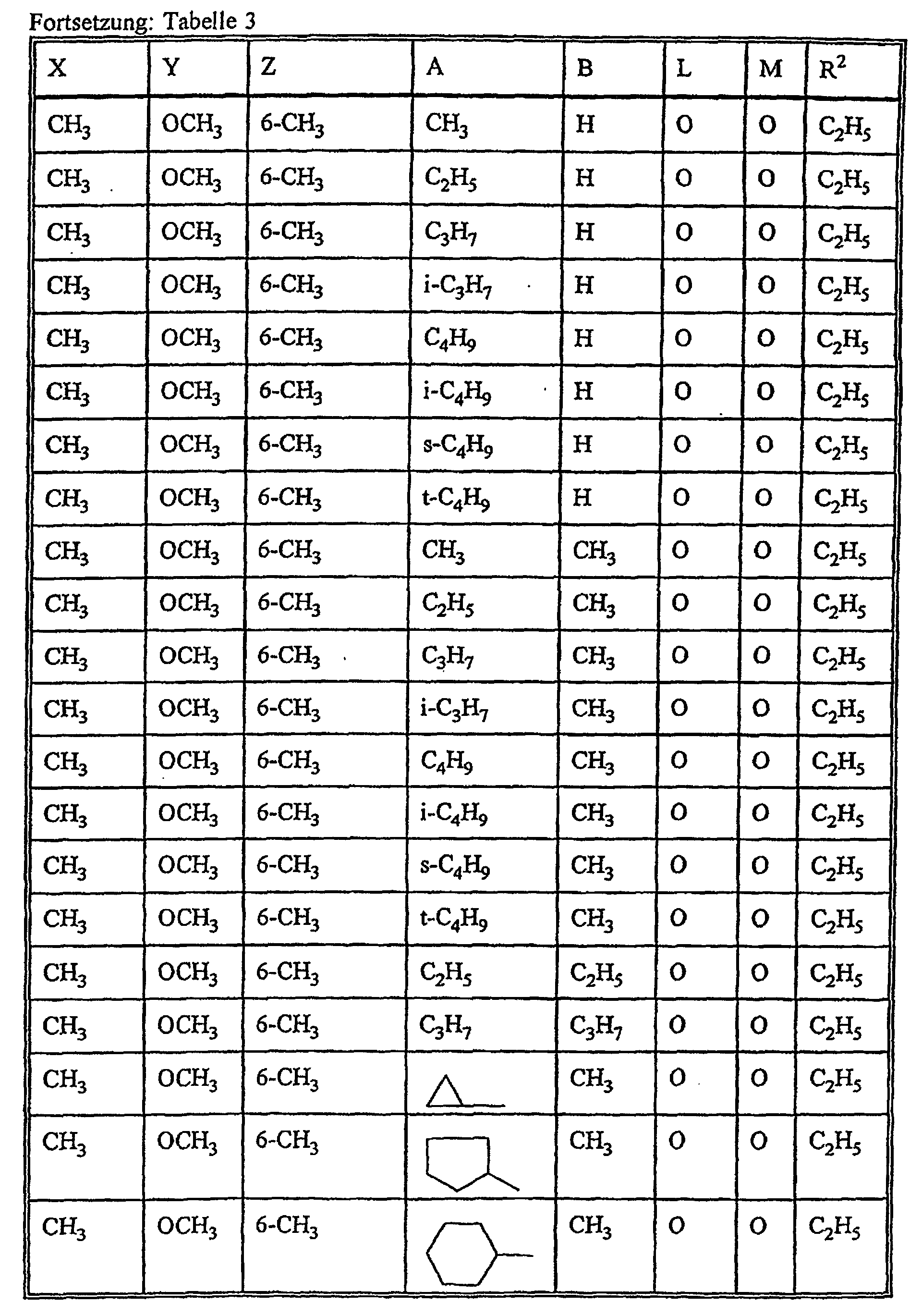

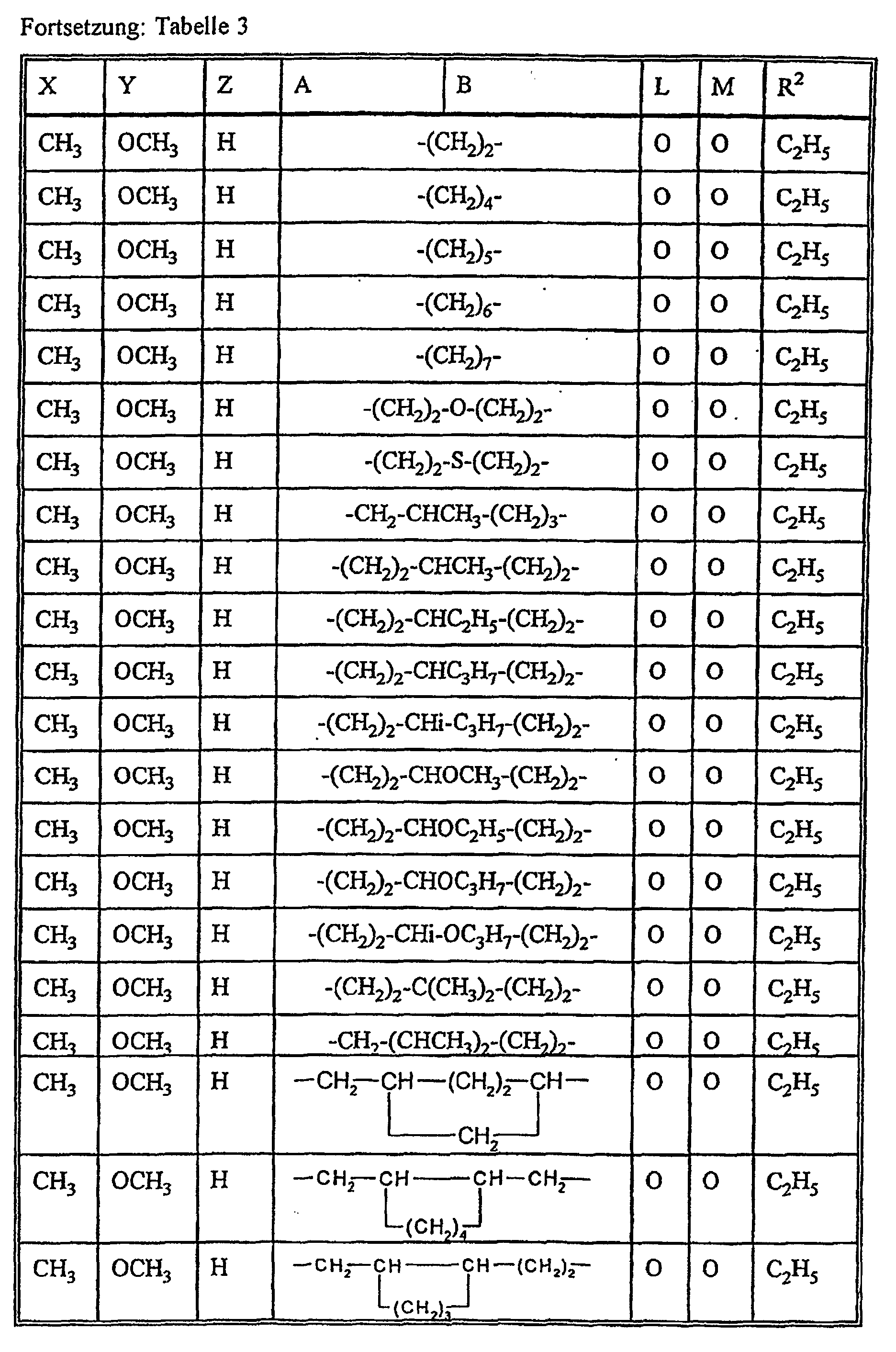

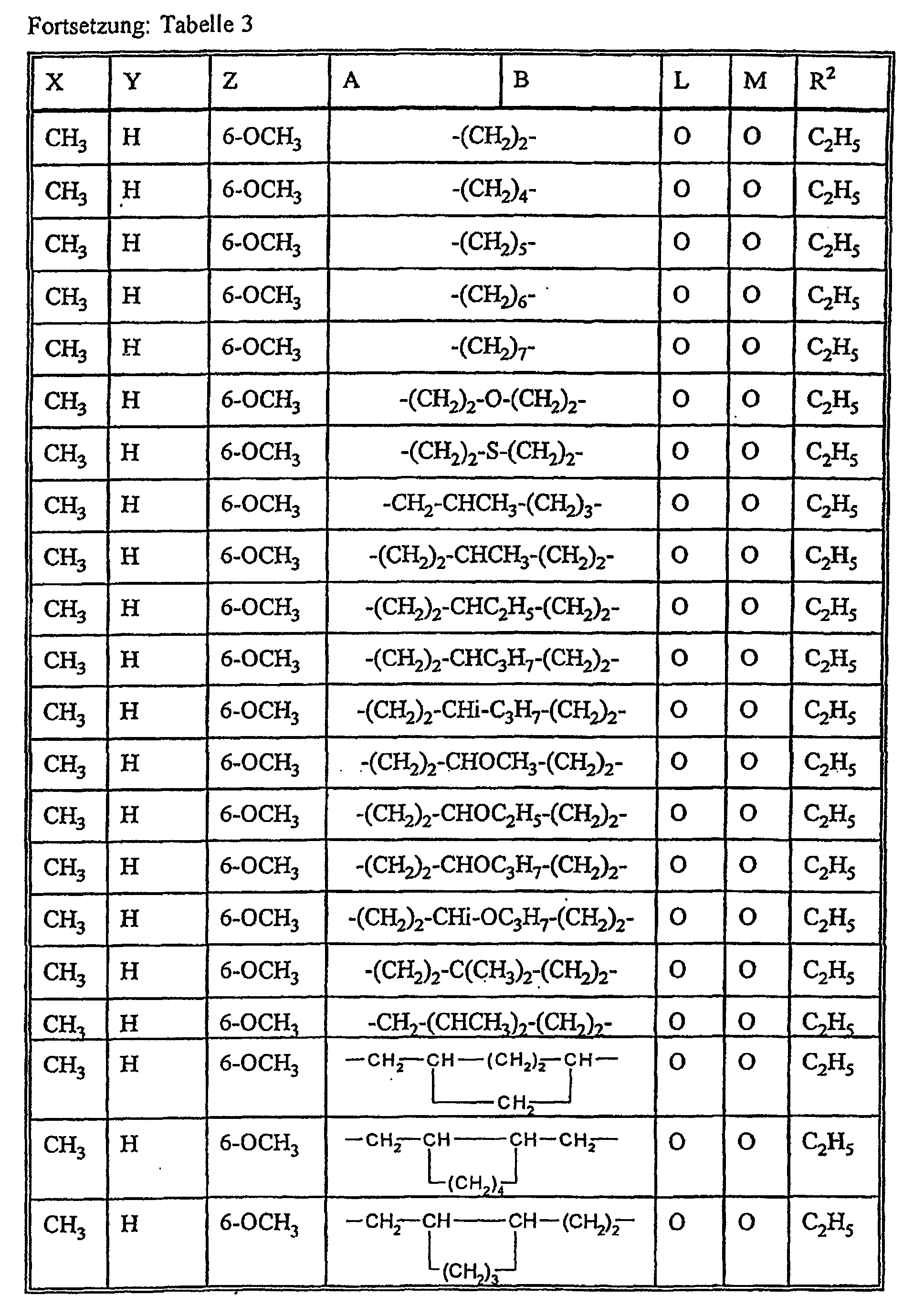

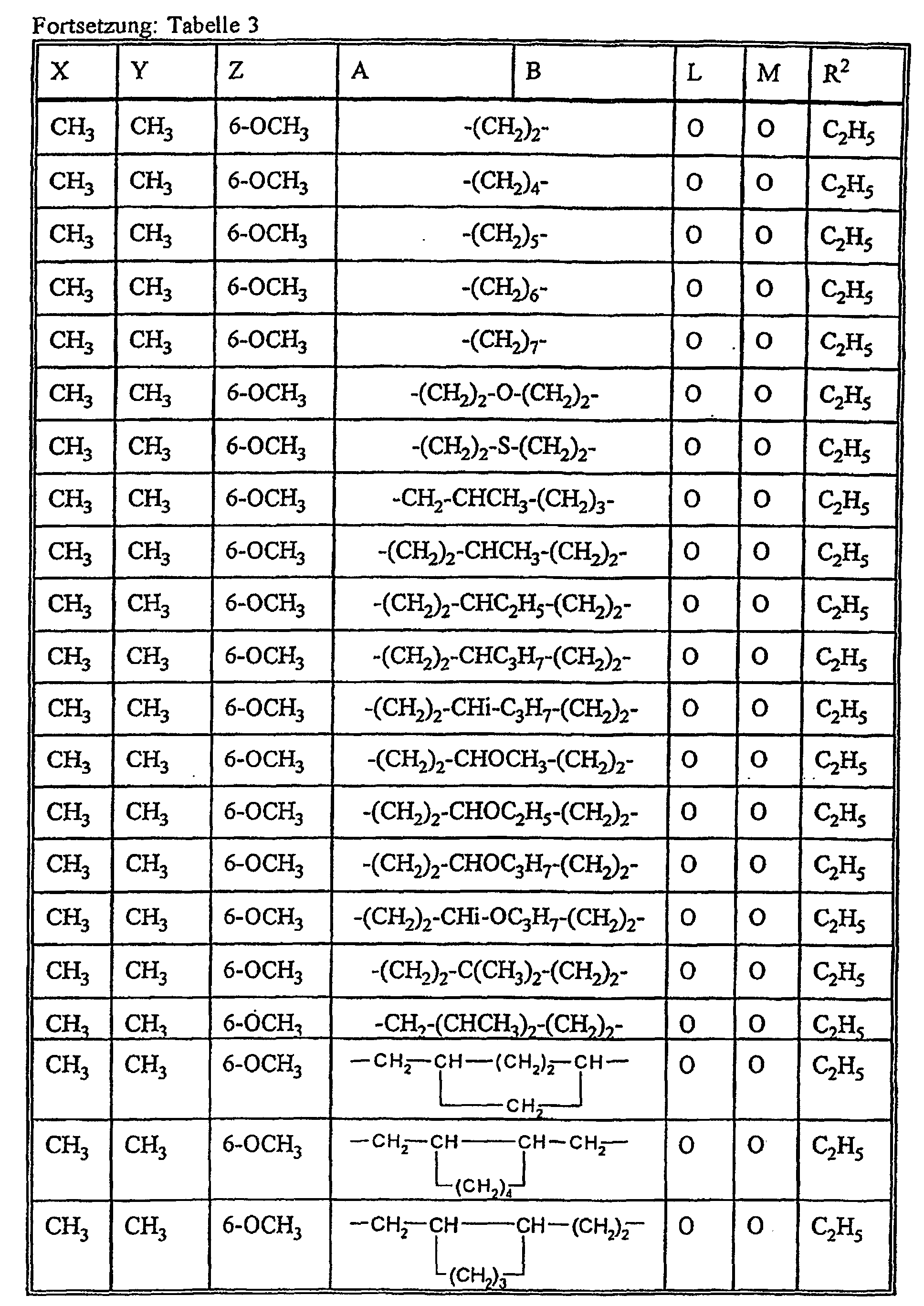

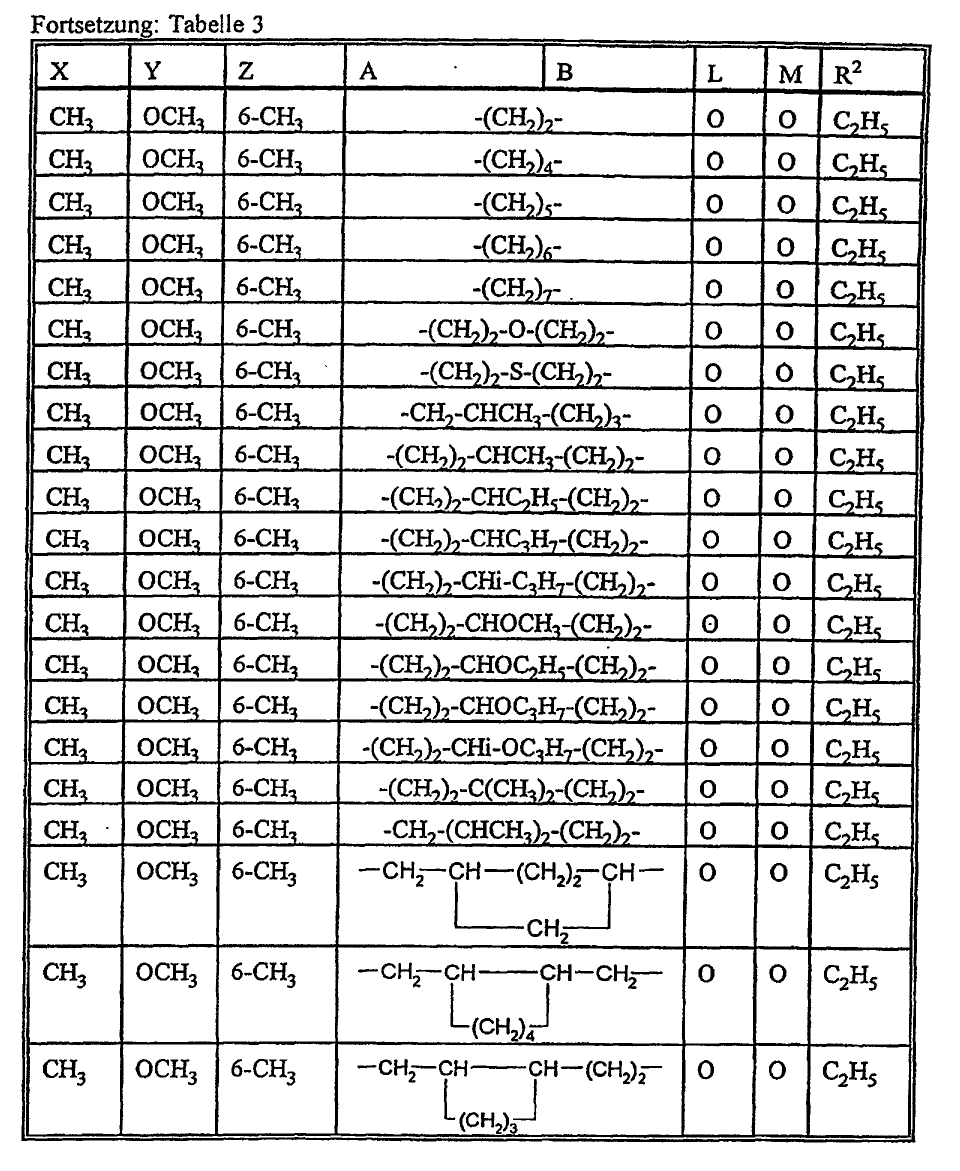

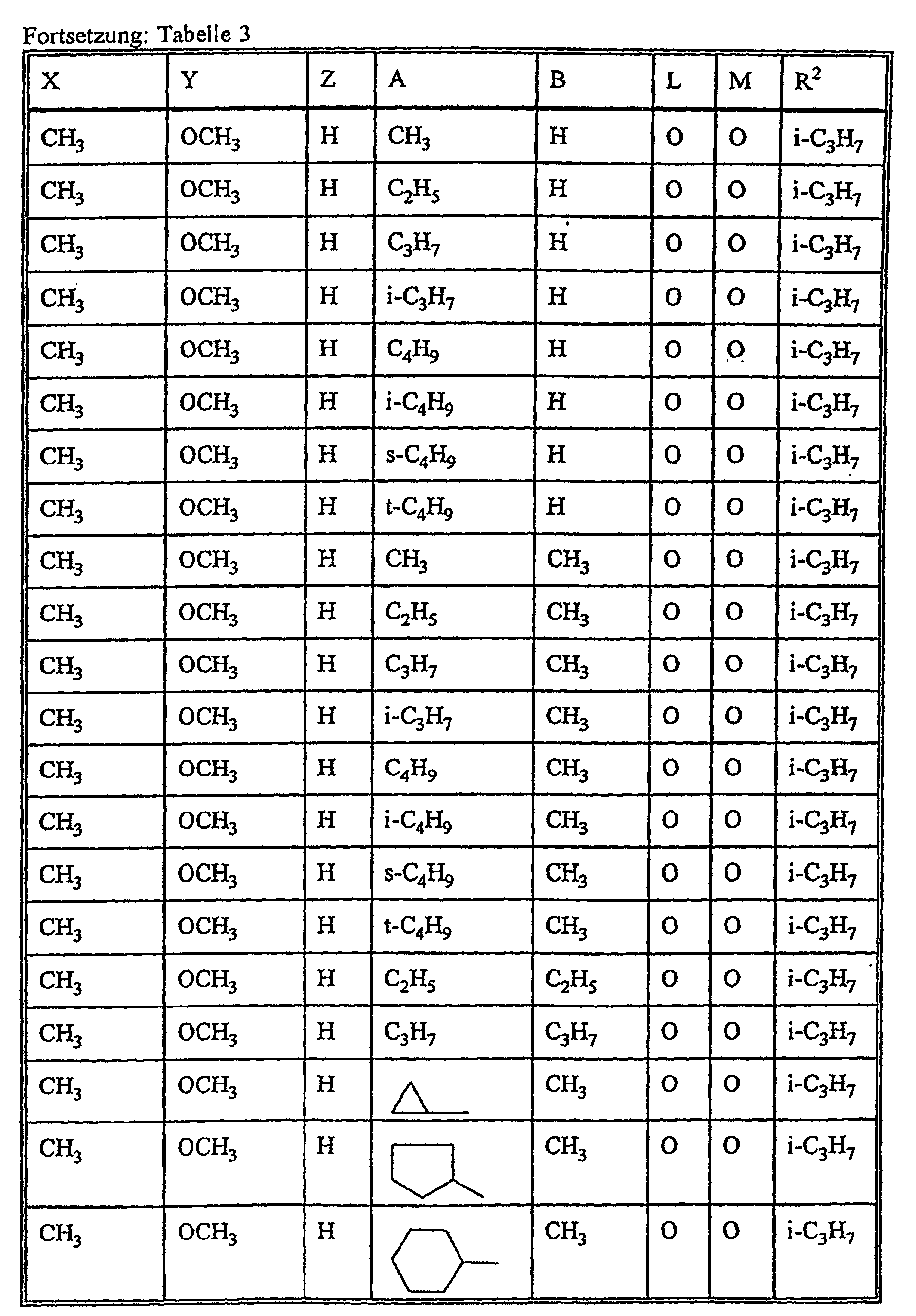

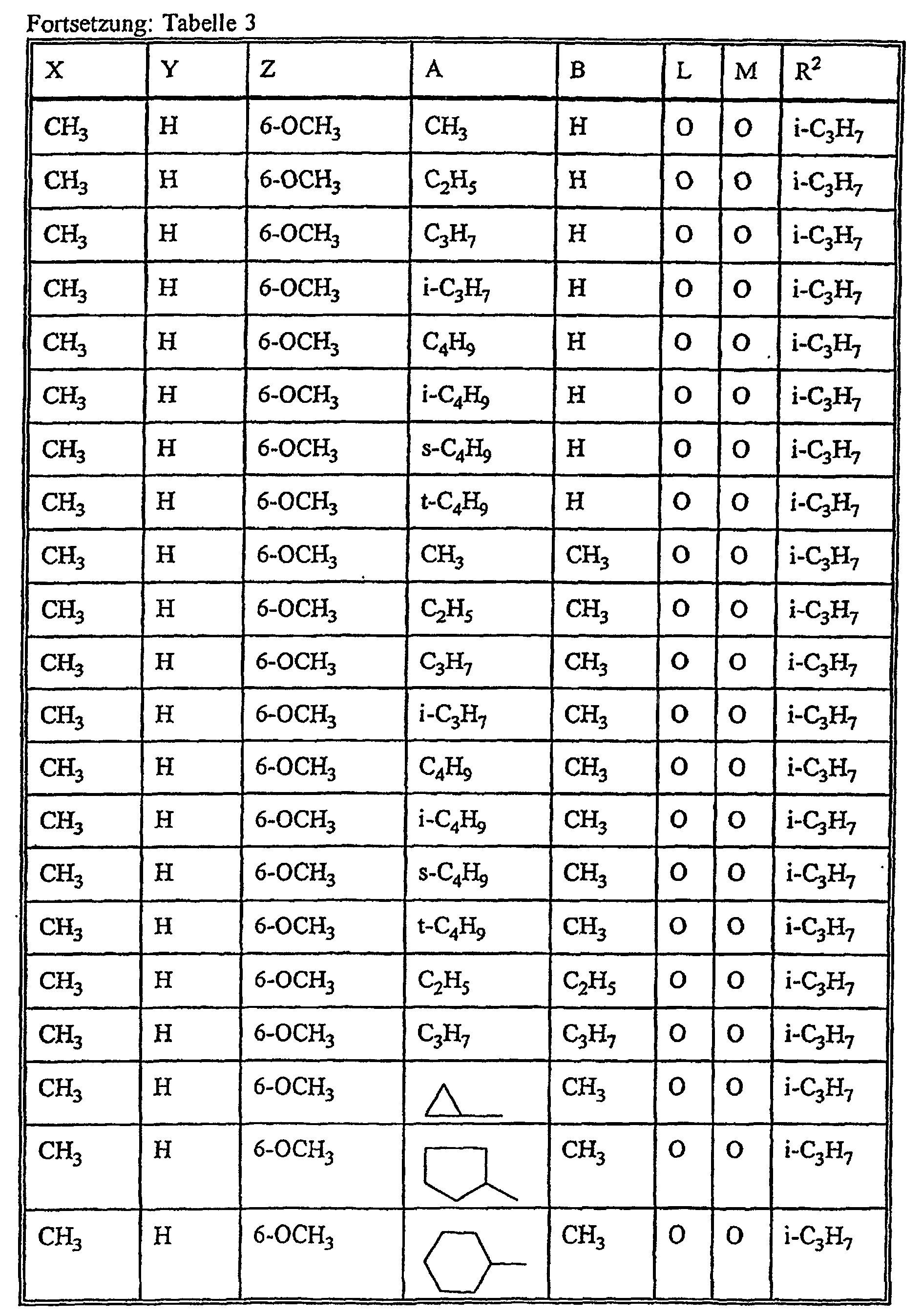

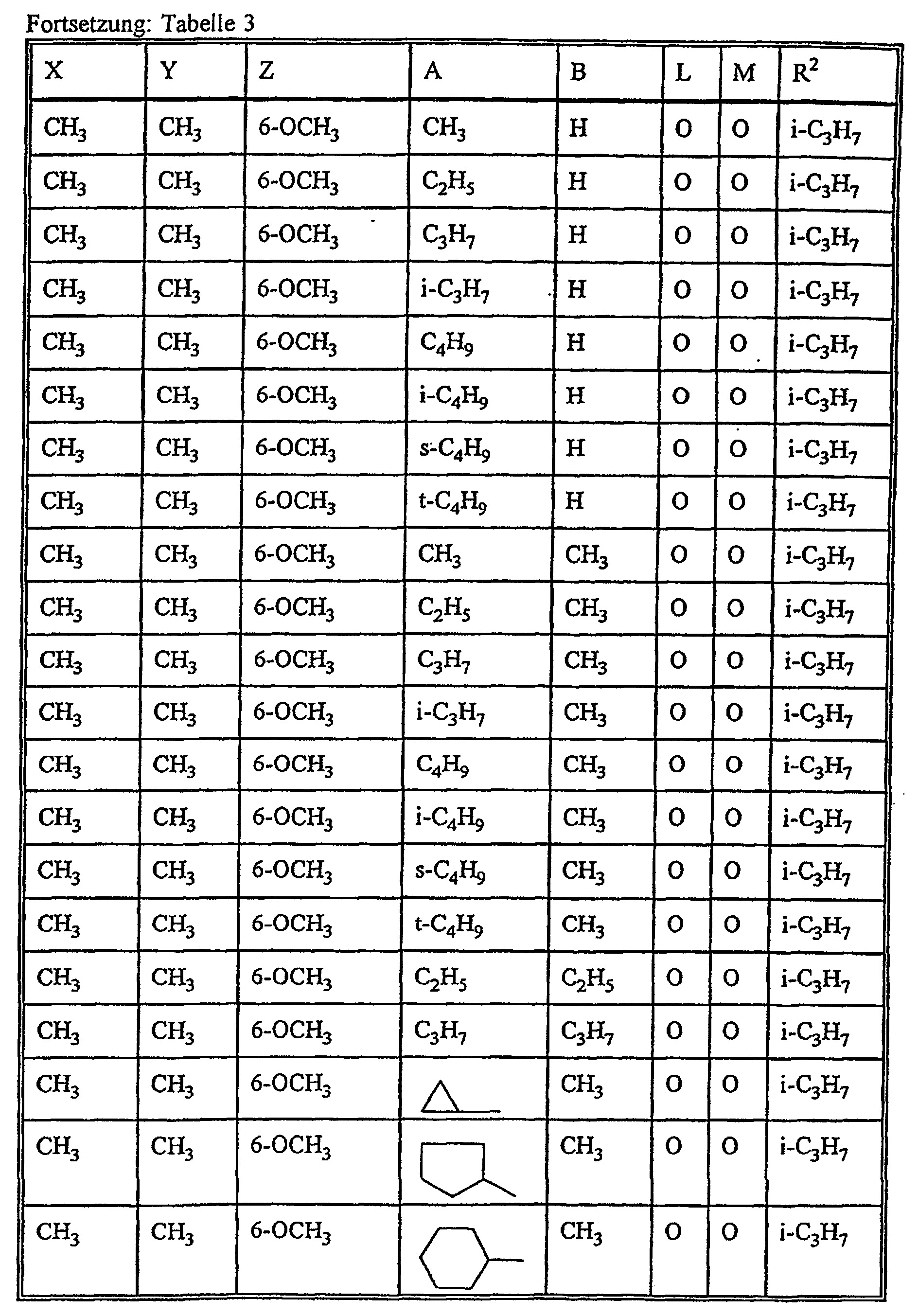

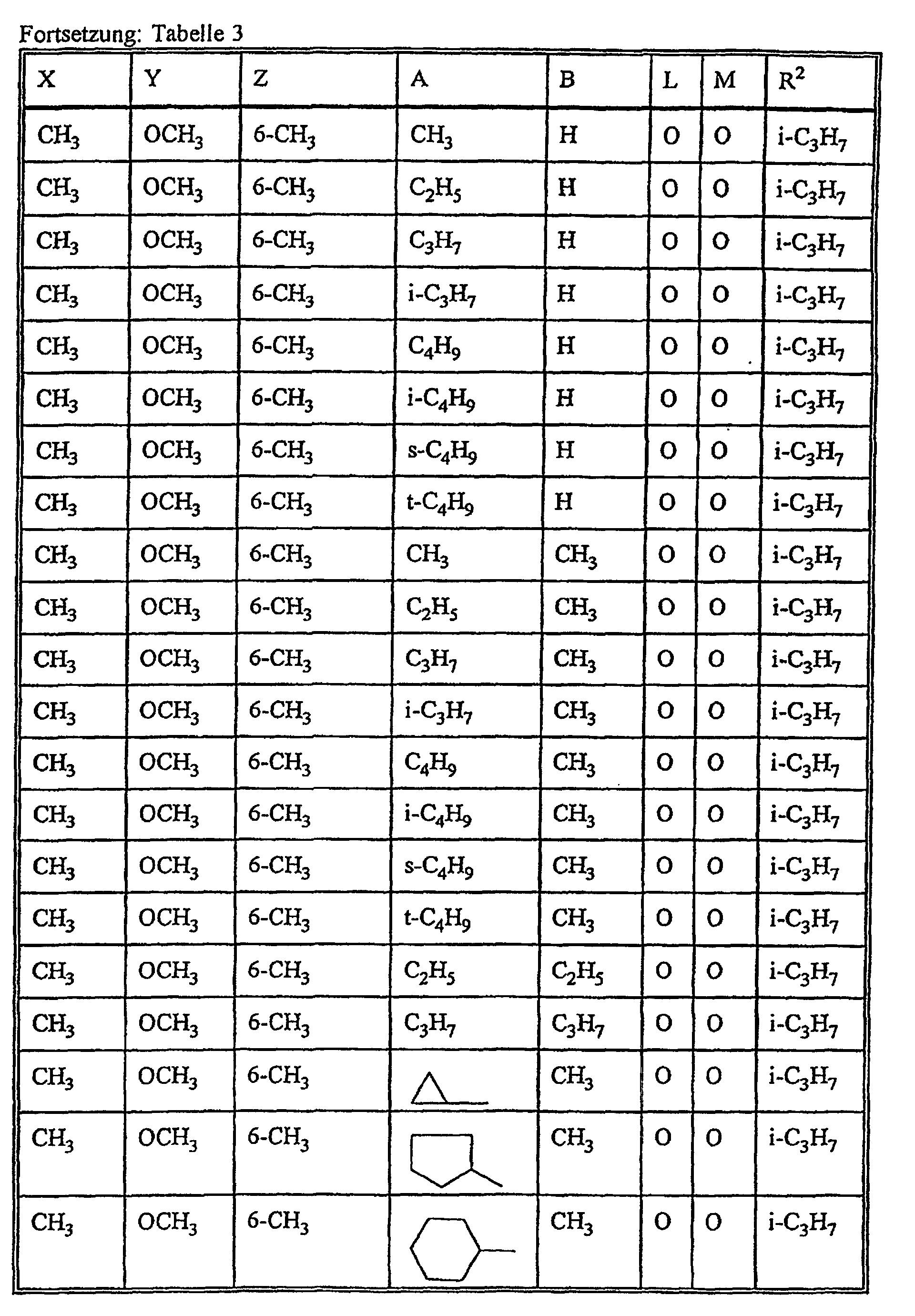

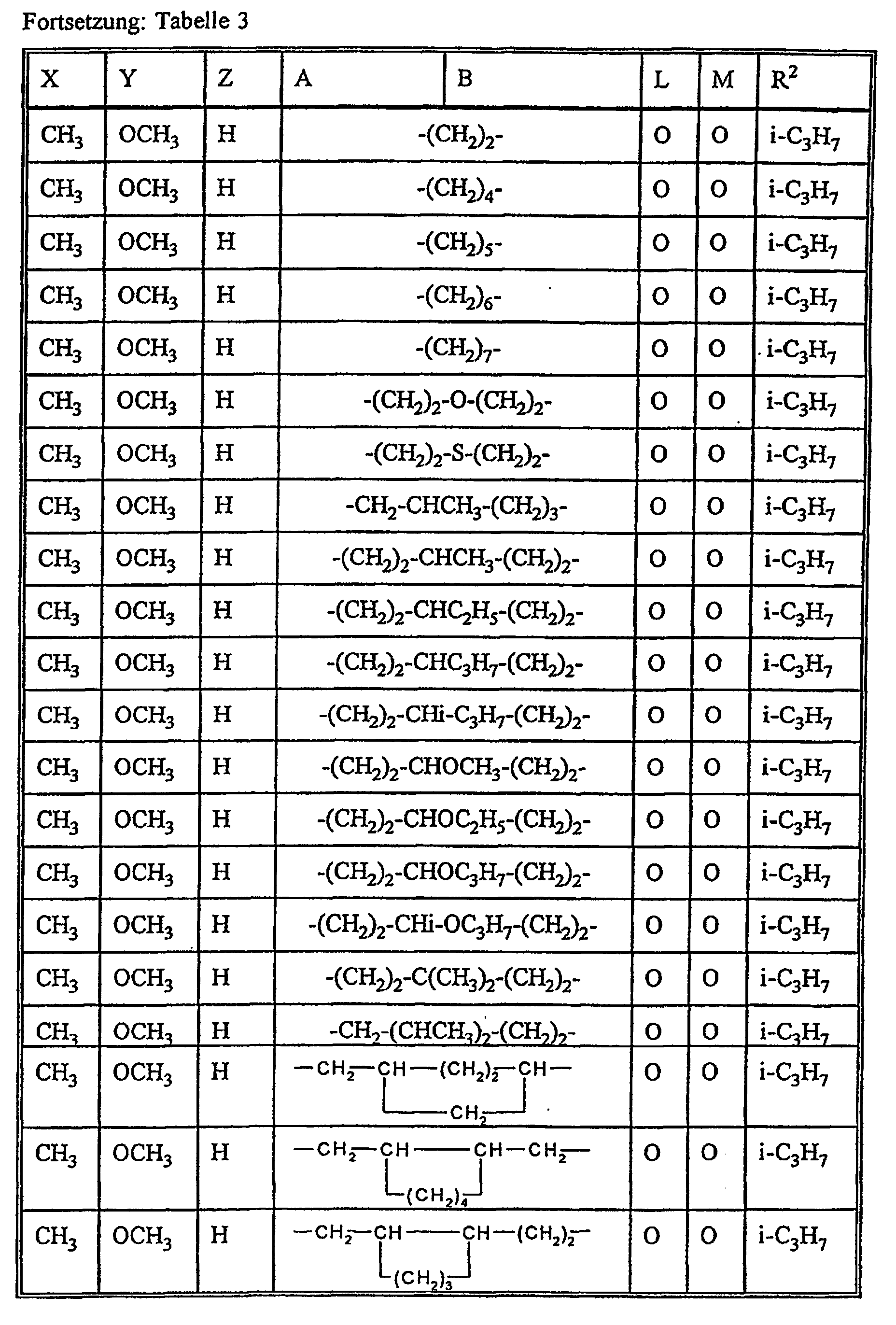

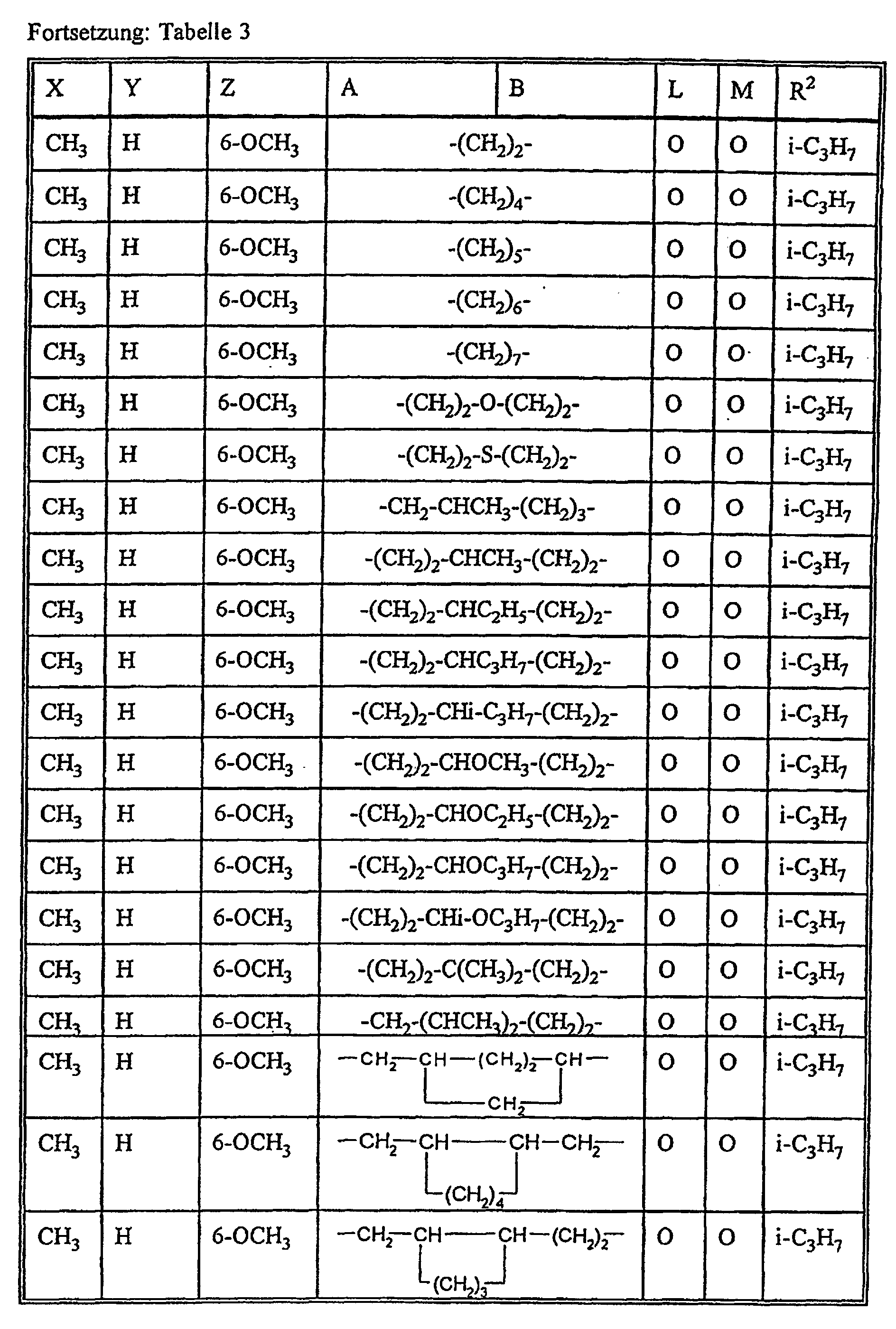

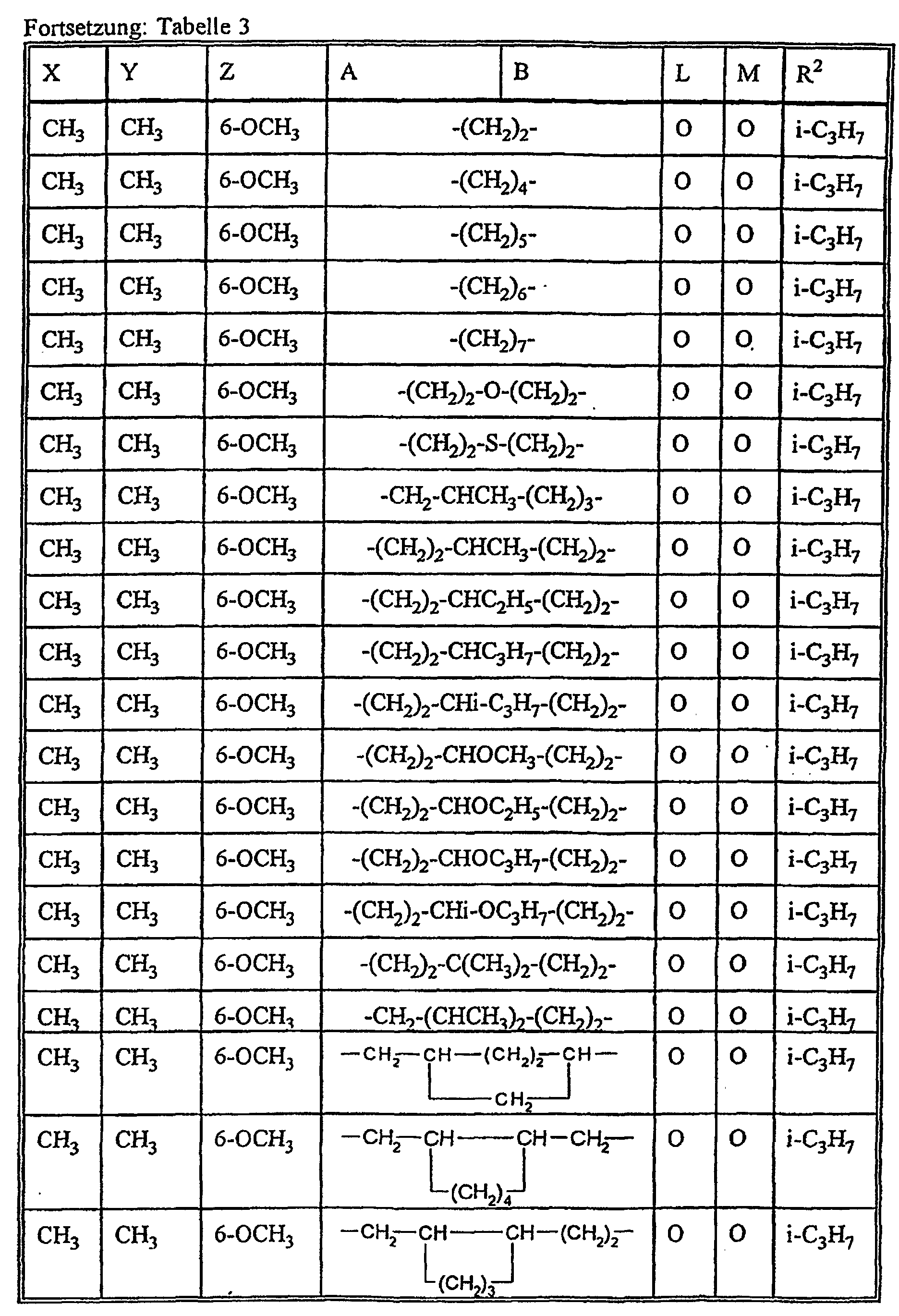

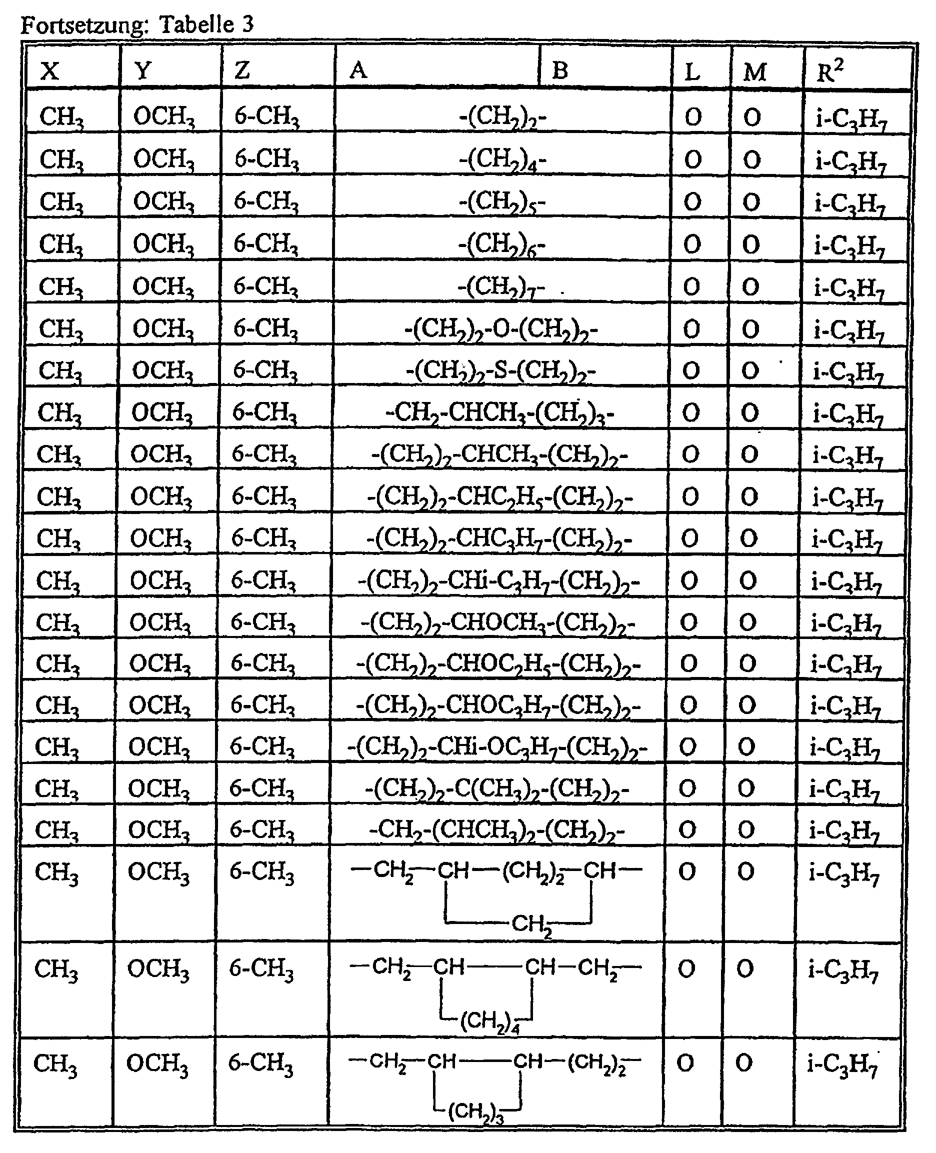

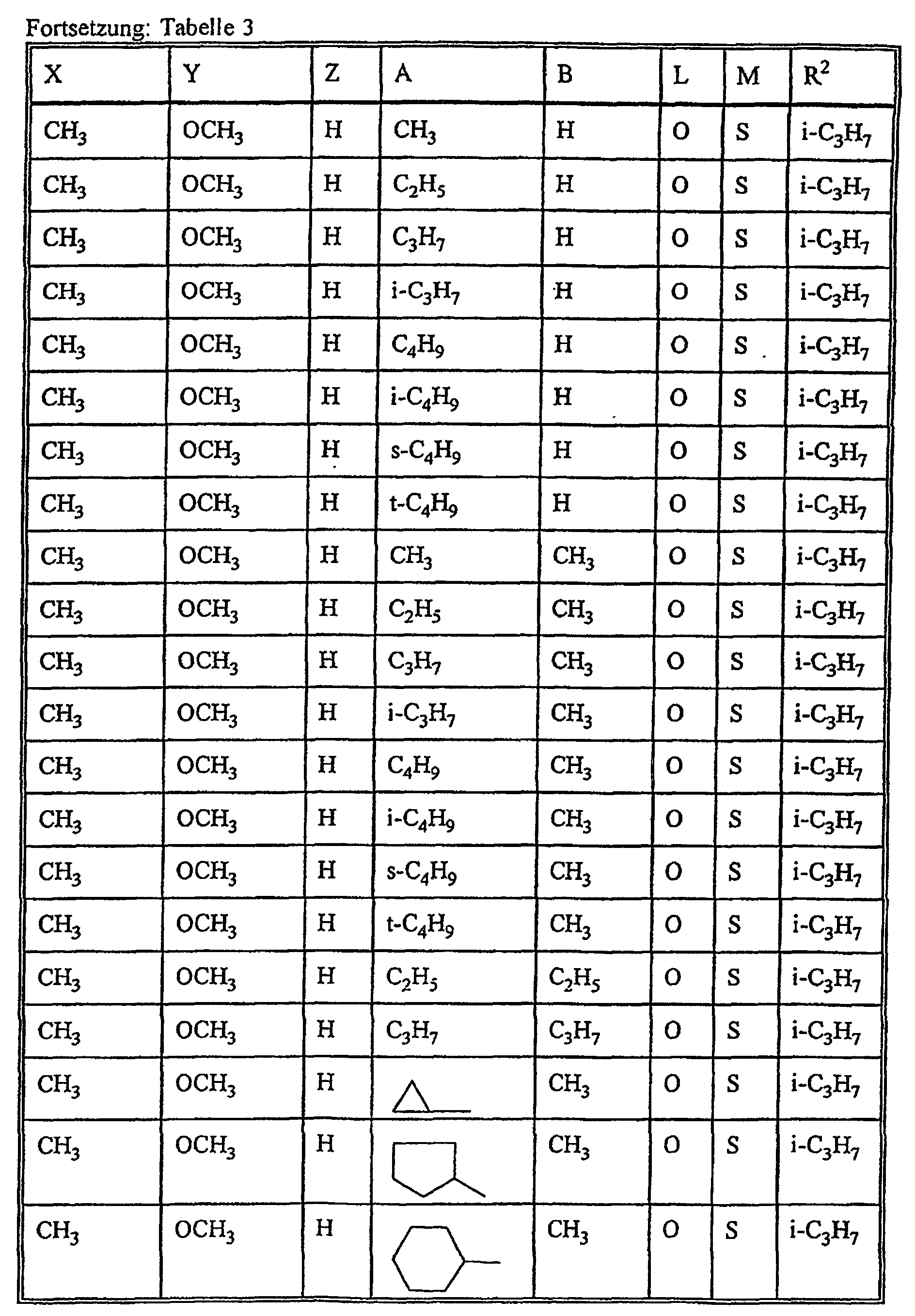

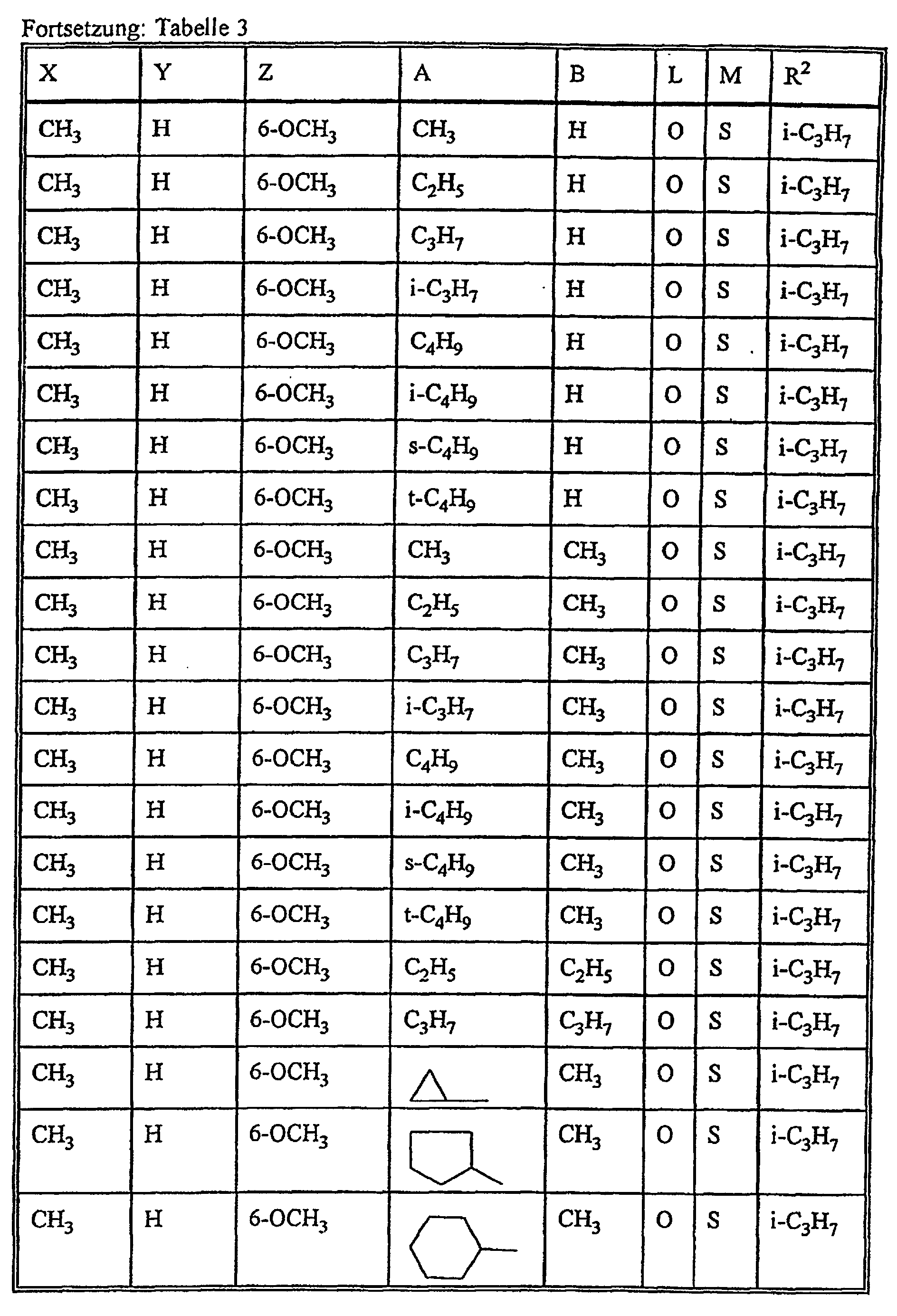

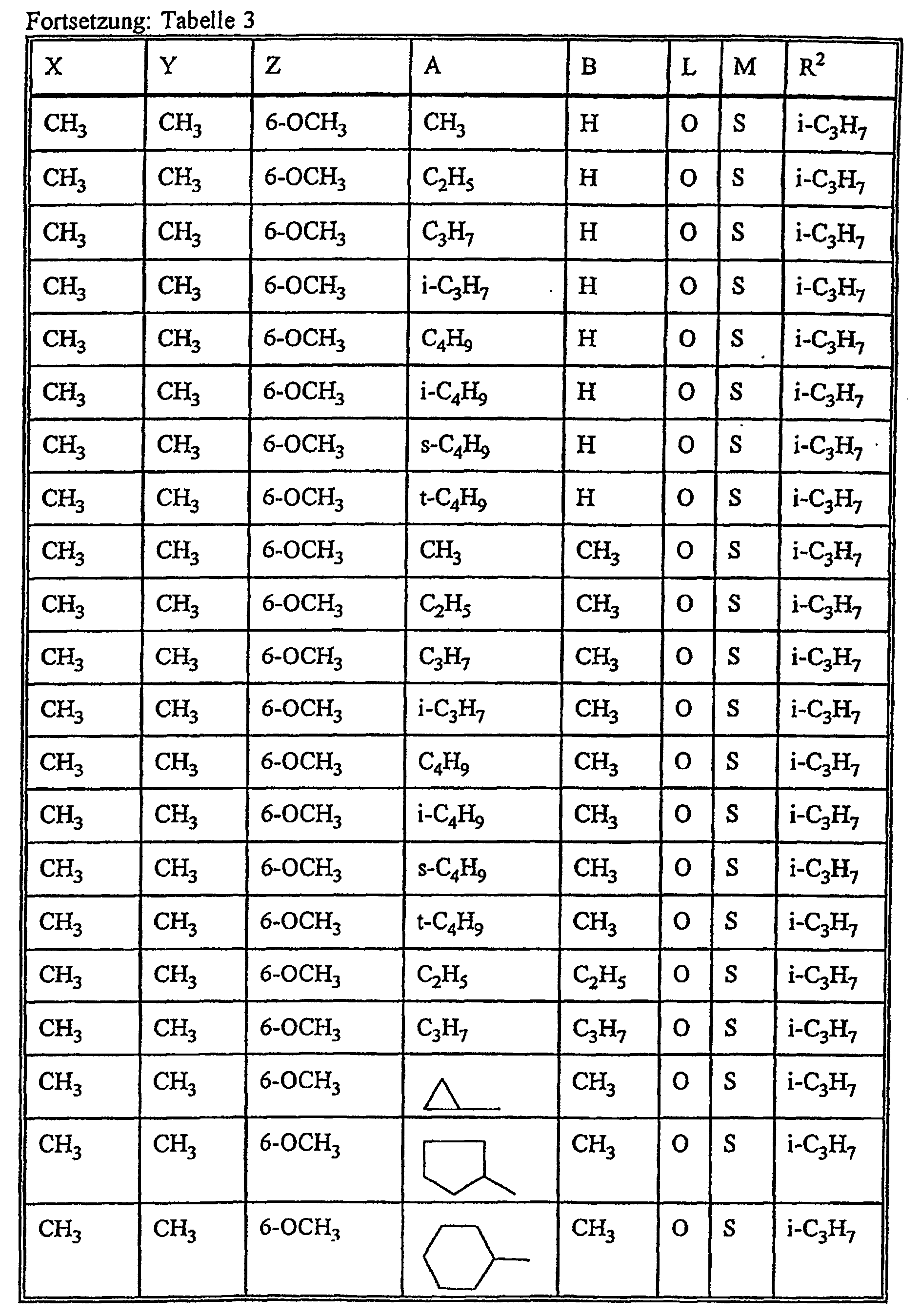

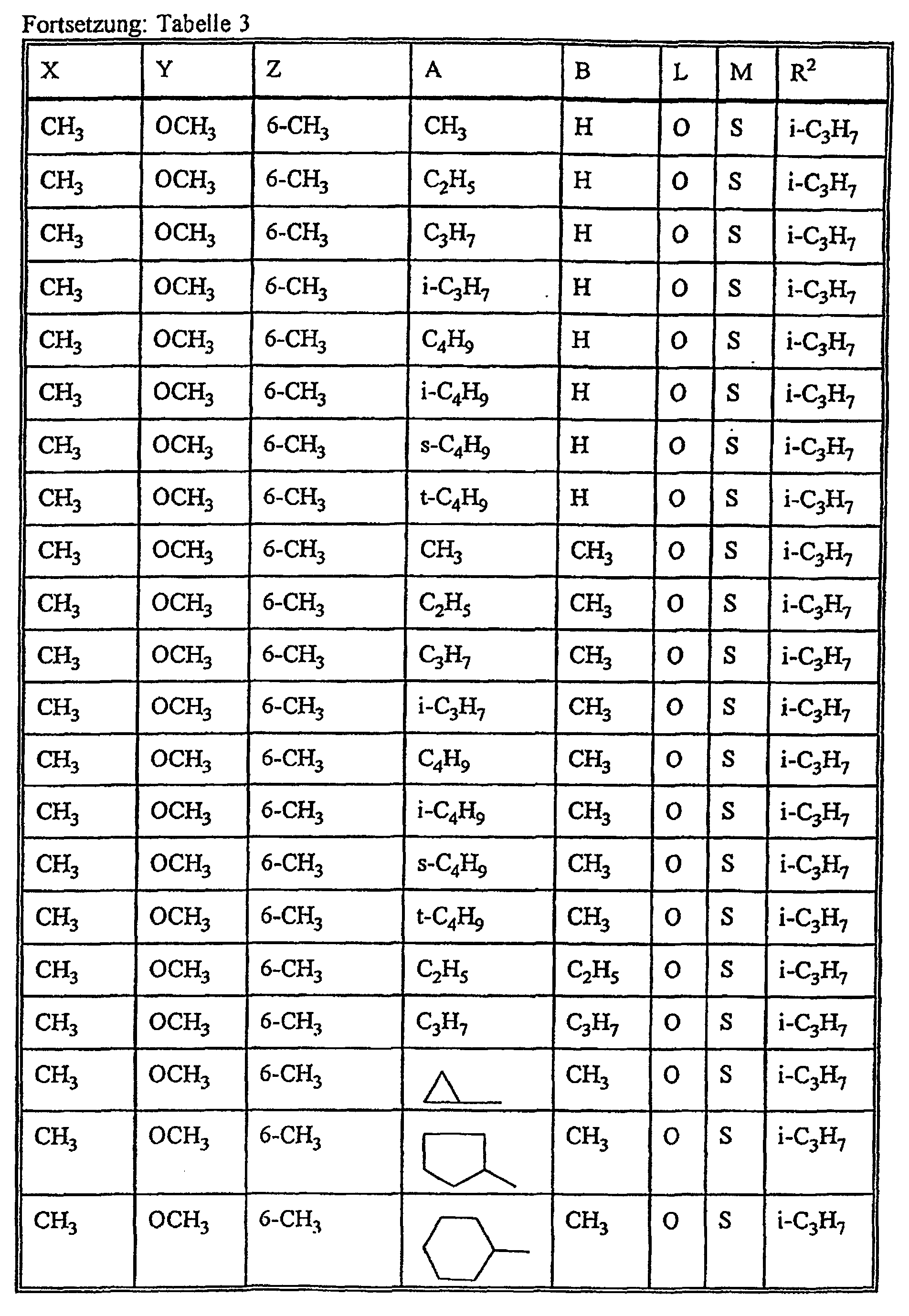

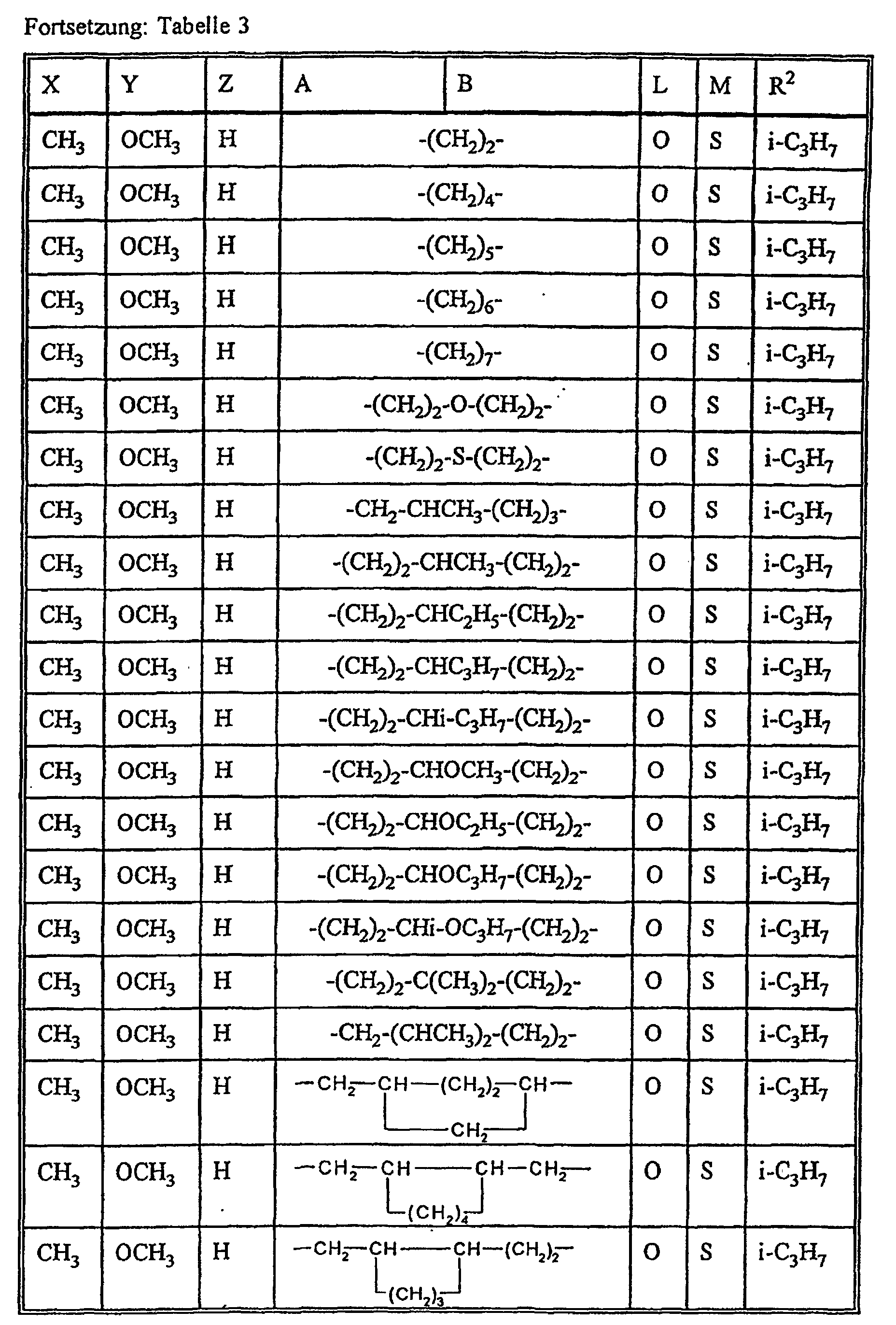

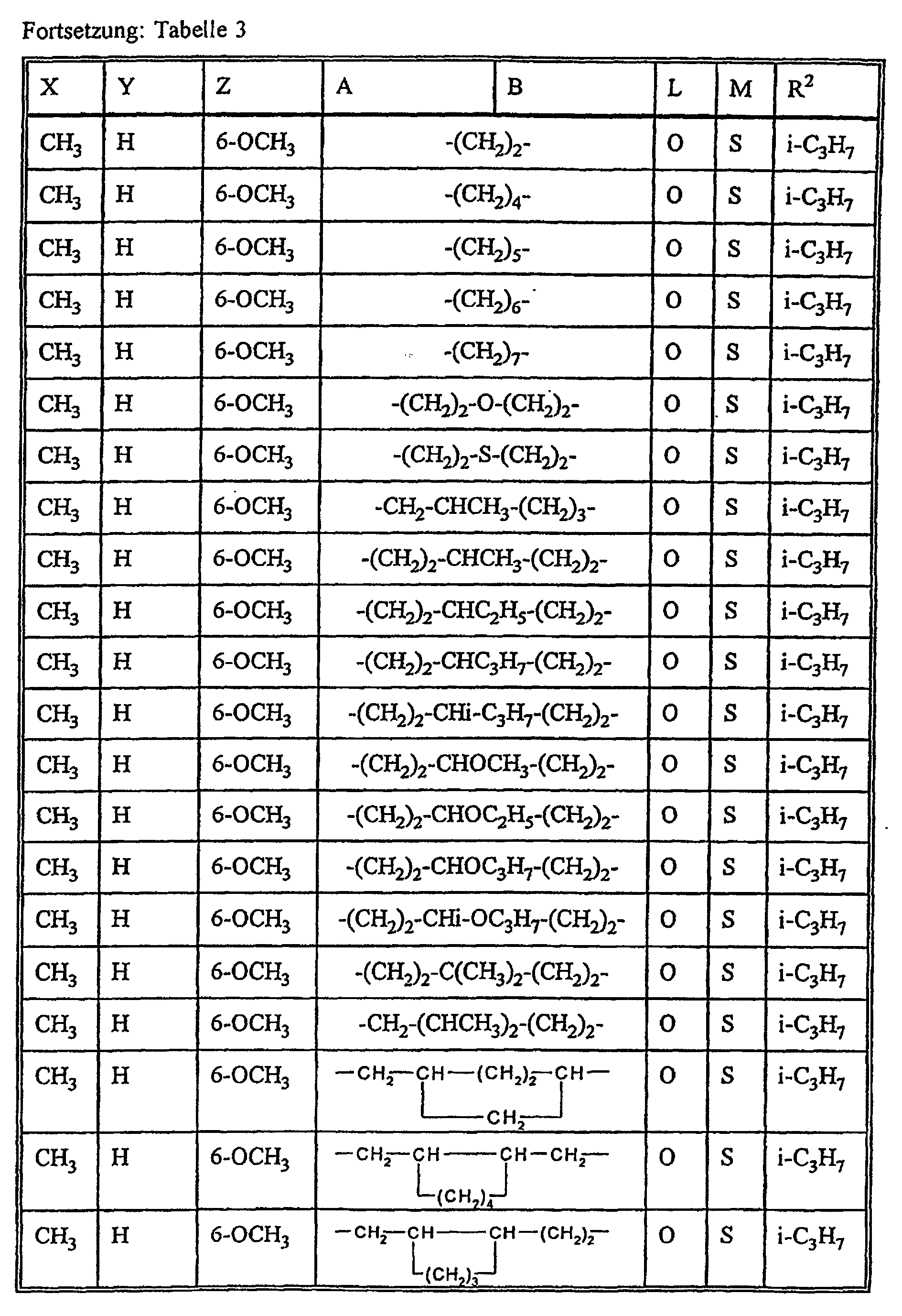

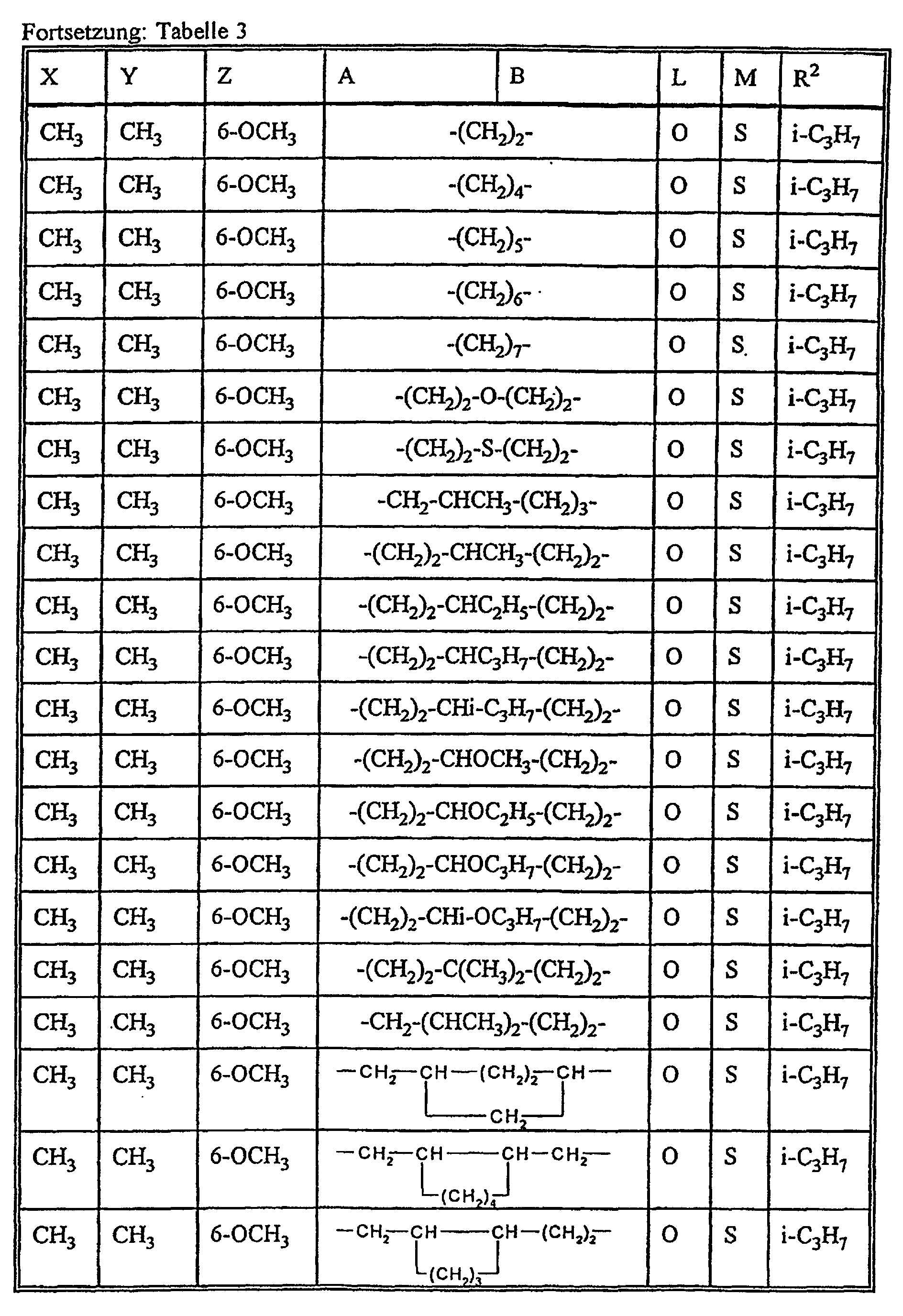

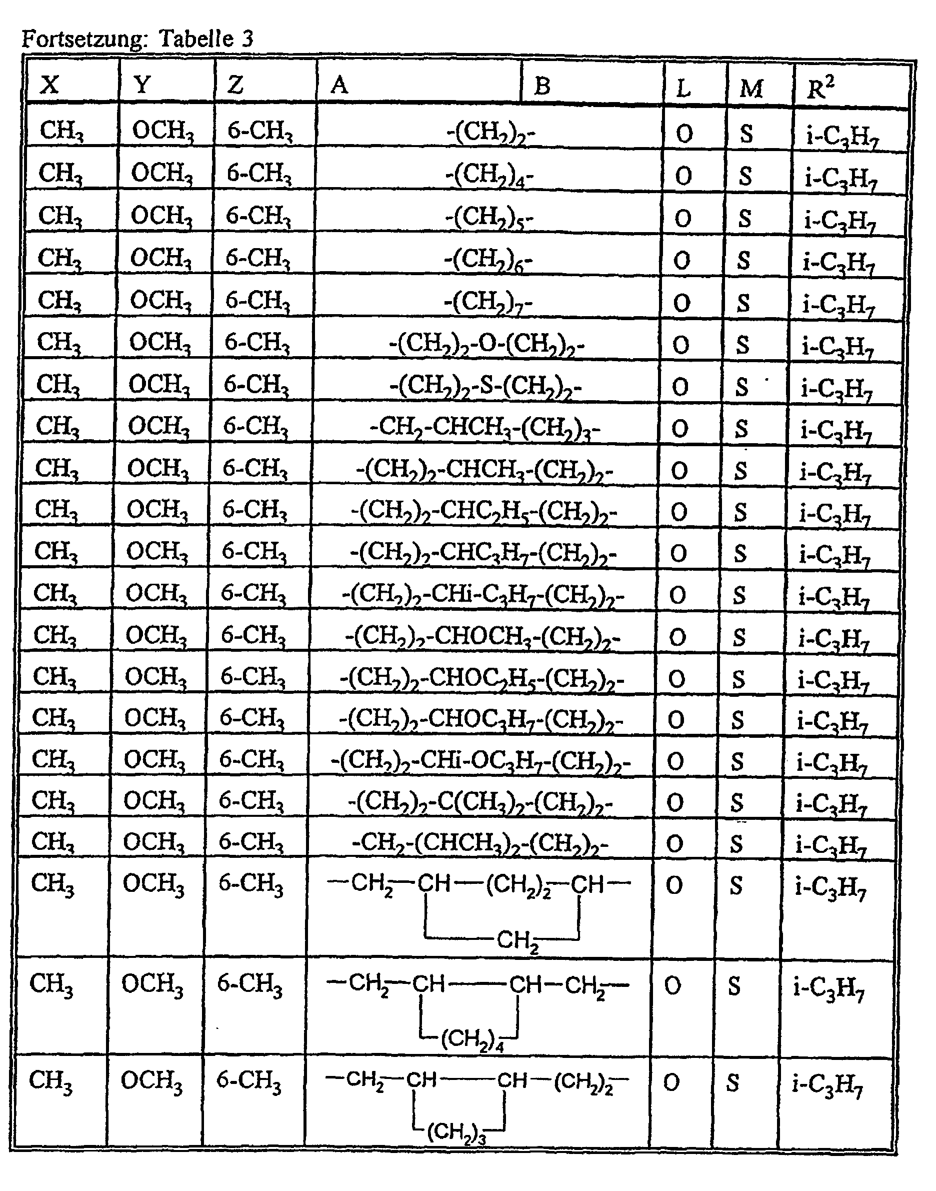

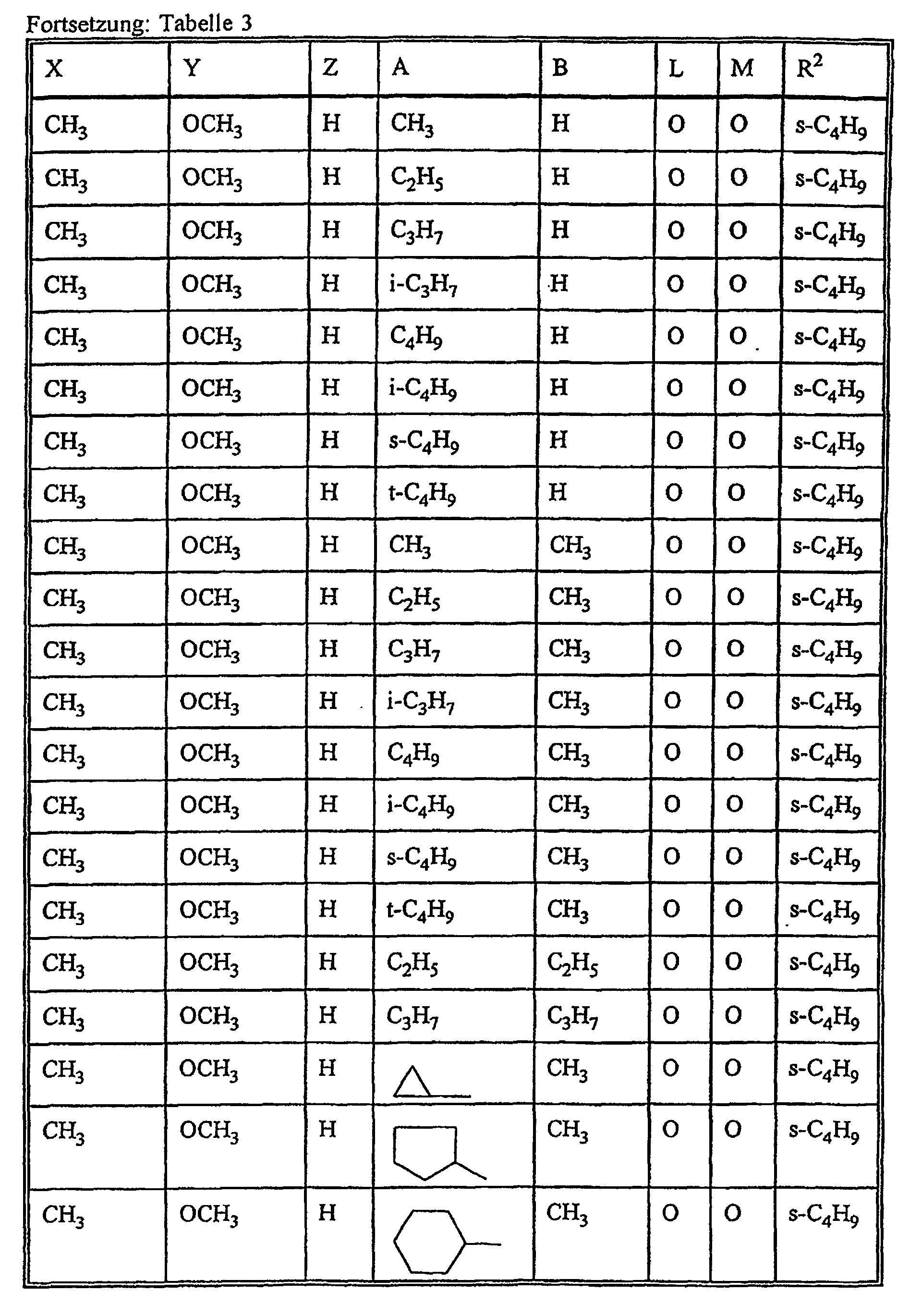

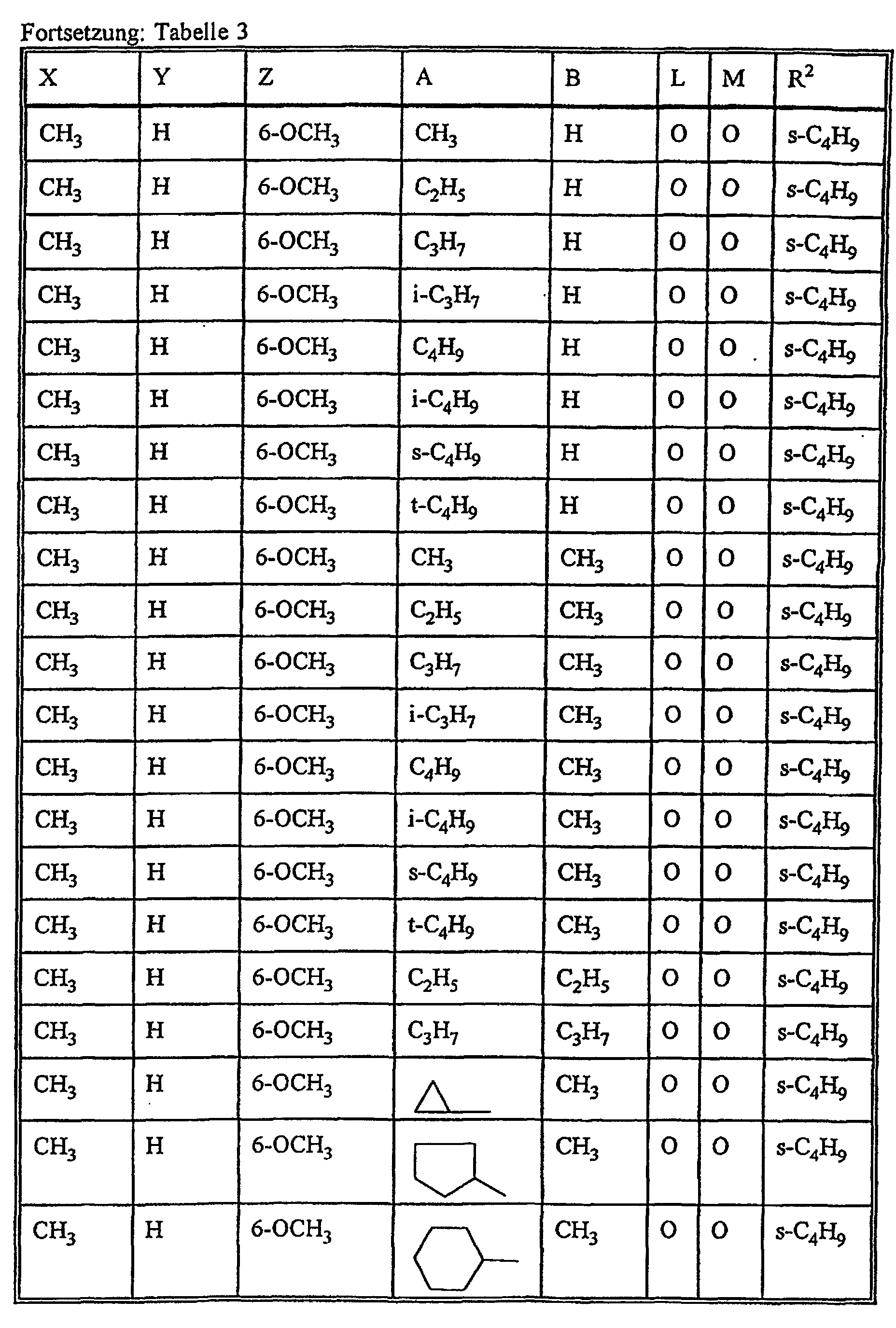

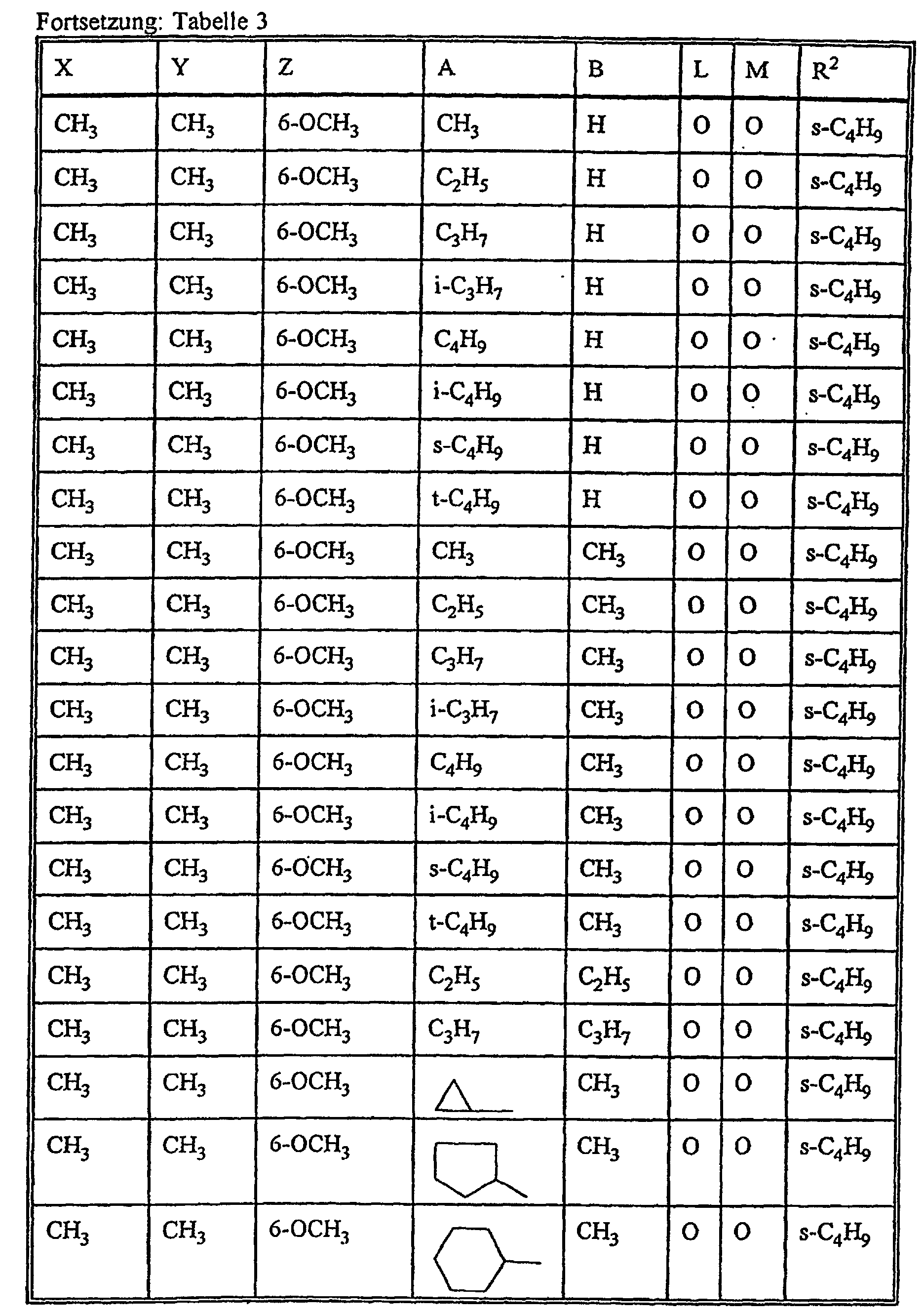

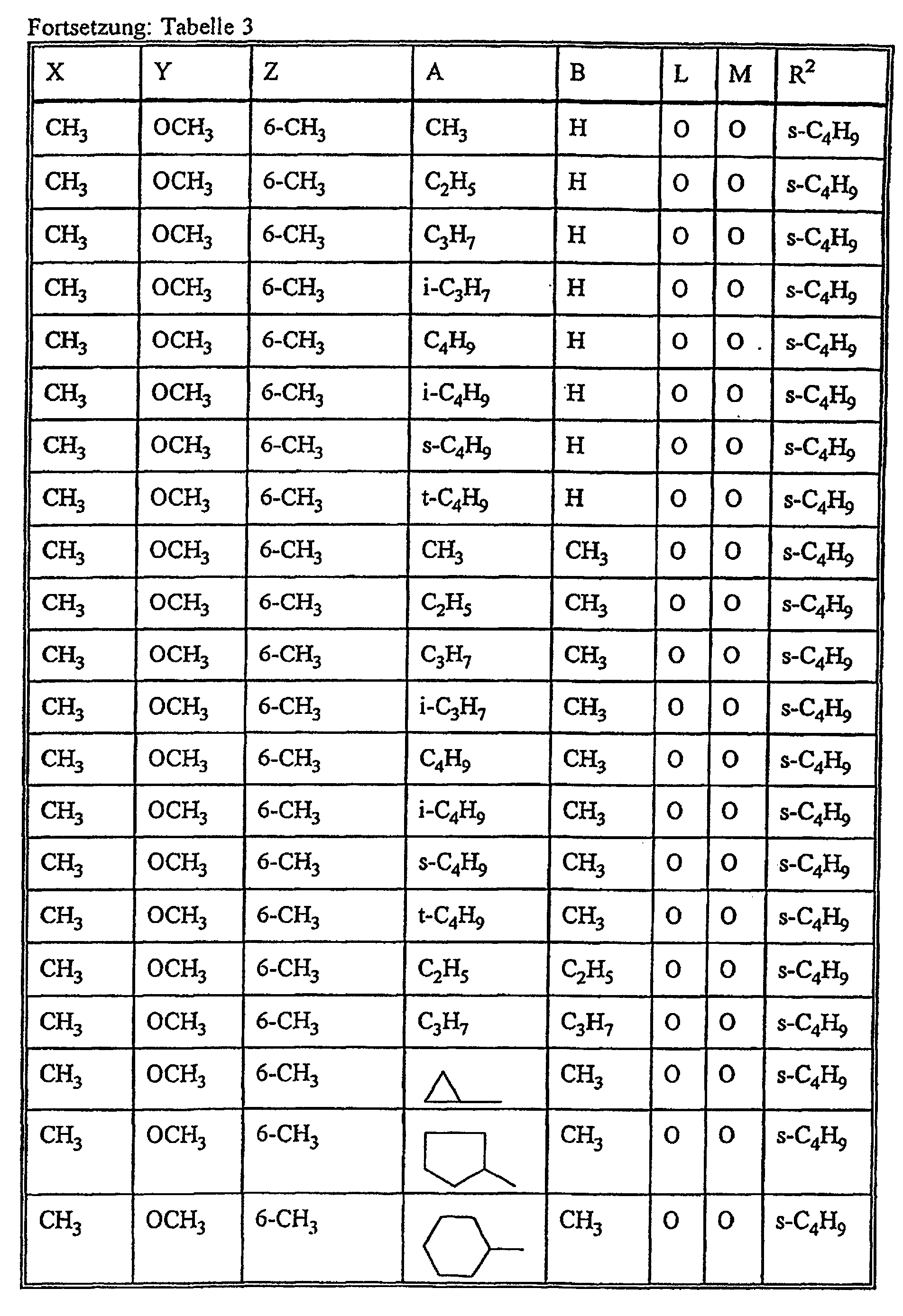

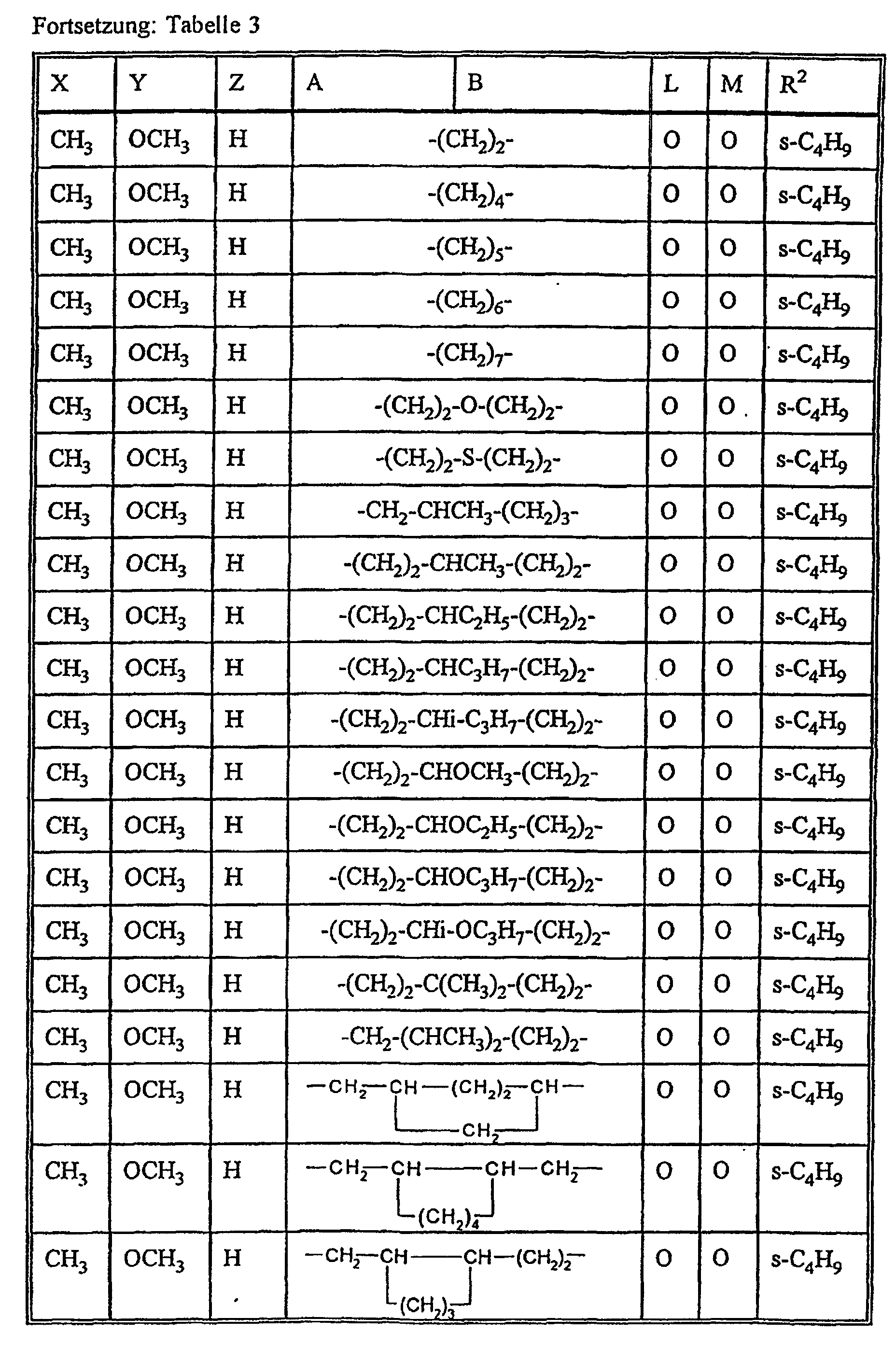

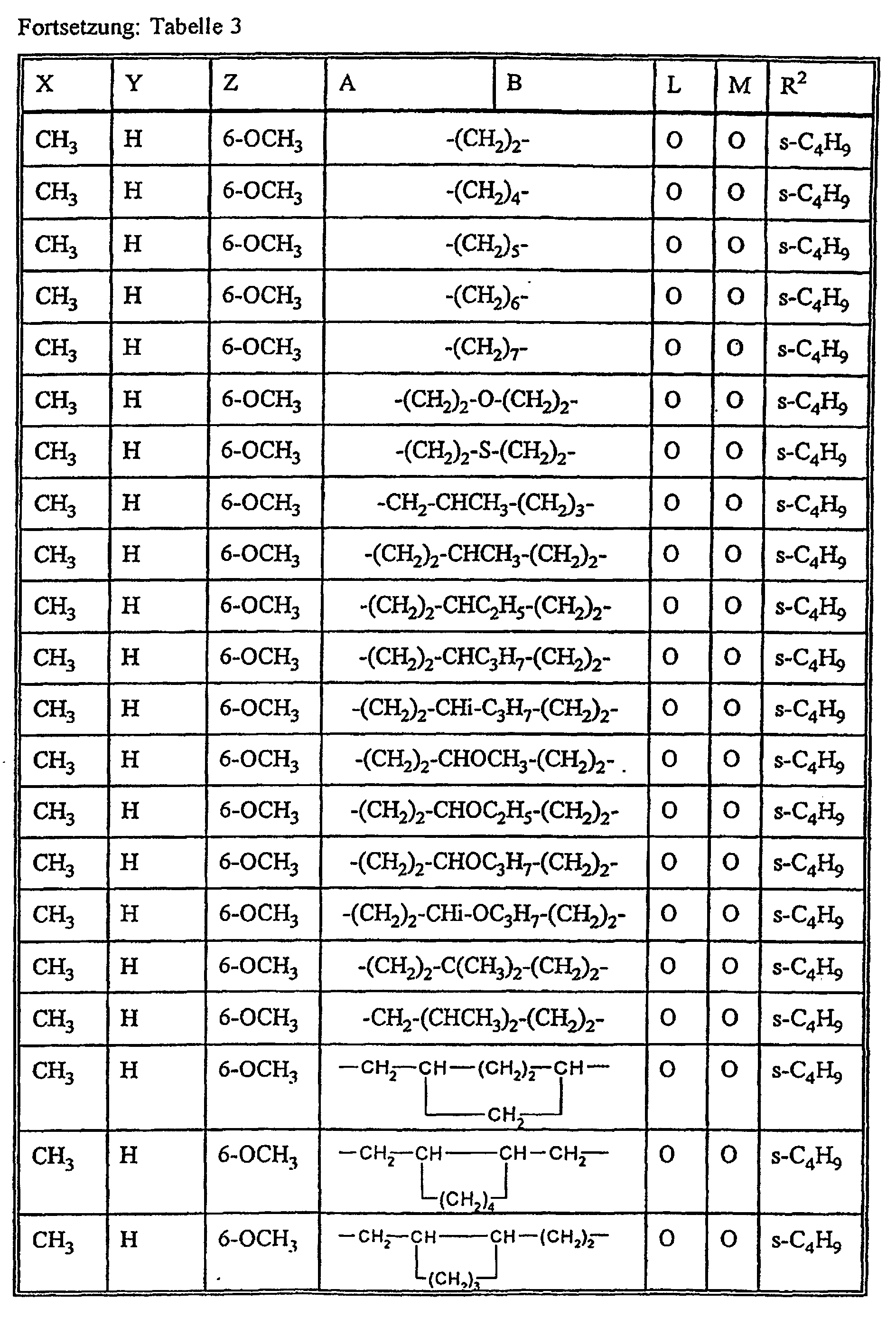

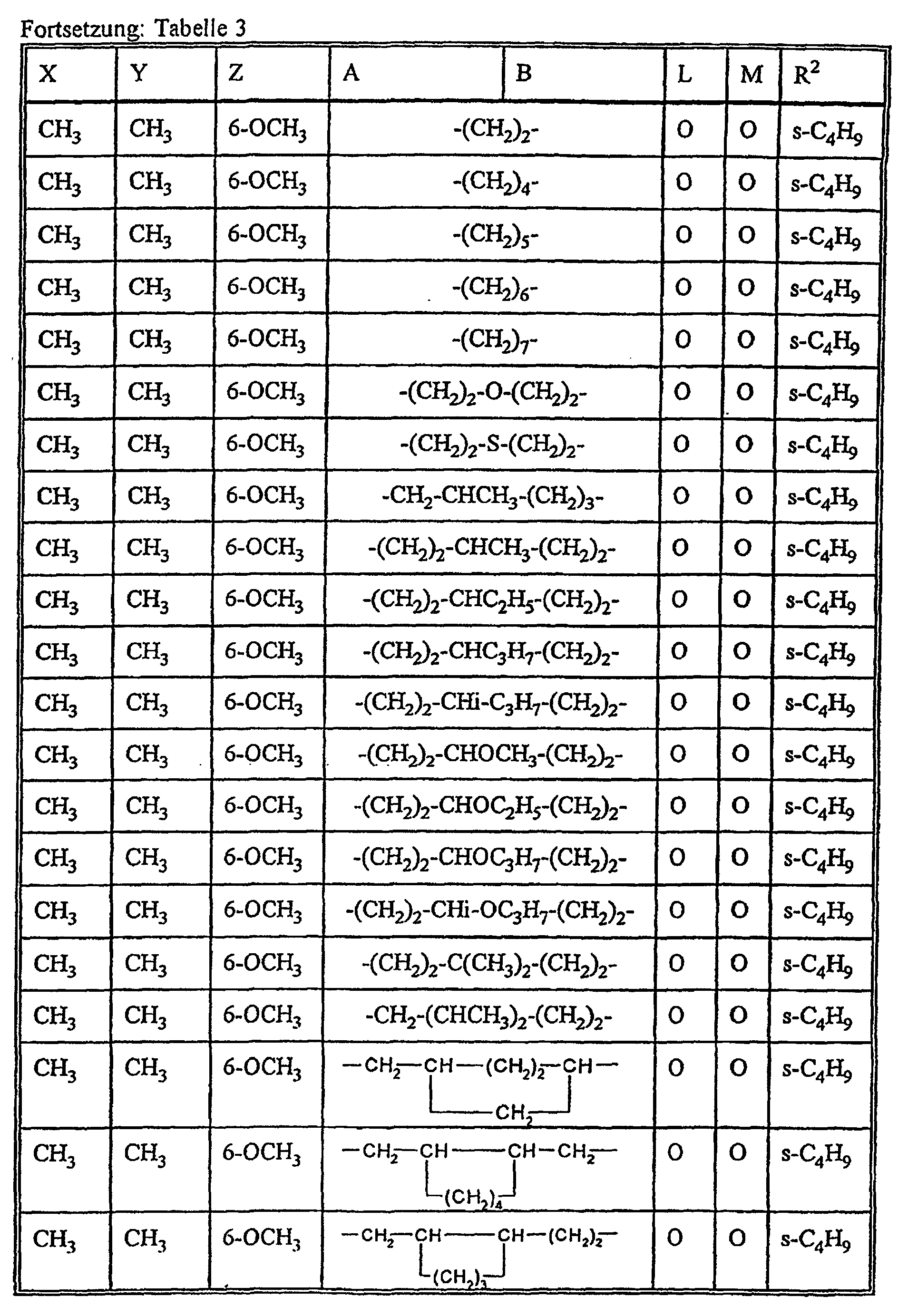

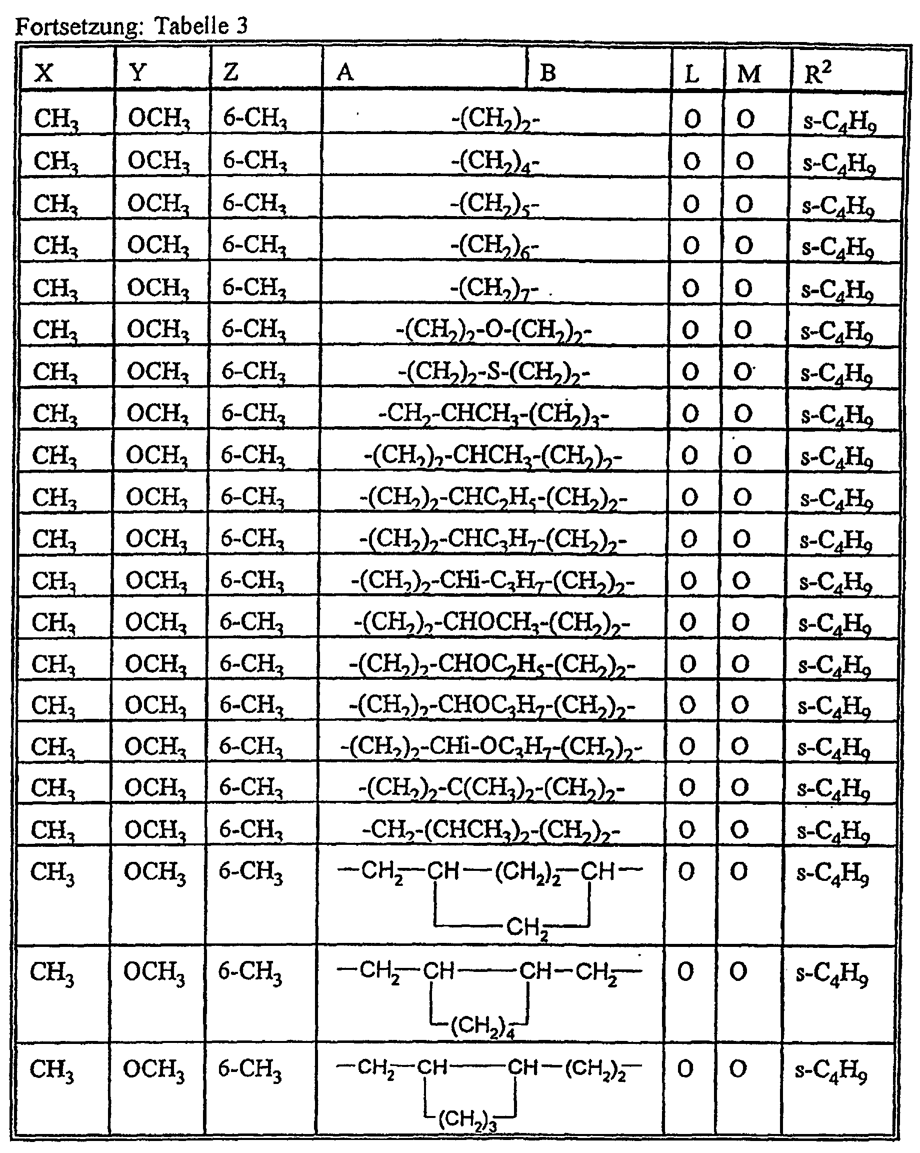

Im einzelnen seien außer den bei den Herstellungsbeispielen genannten Verbindungen die folgenden Verbindungen der Formel (Id) genannt:

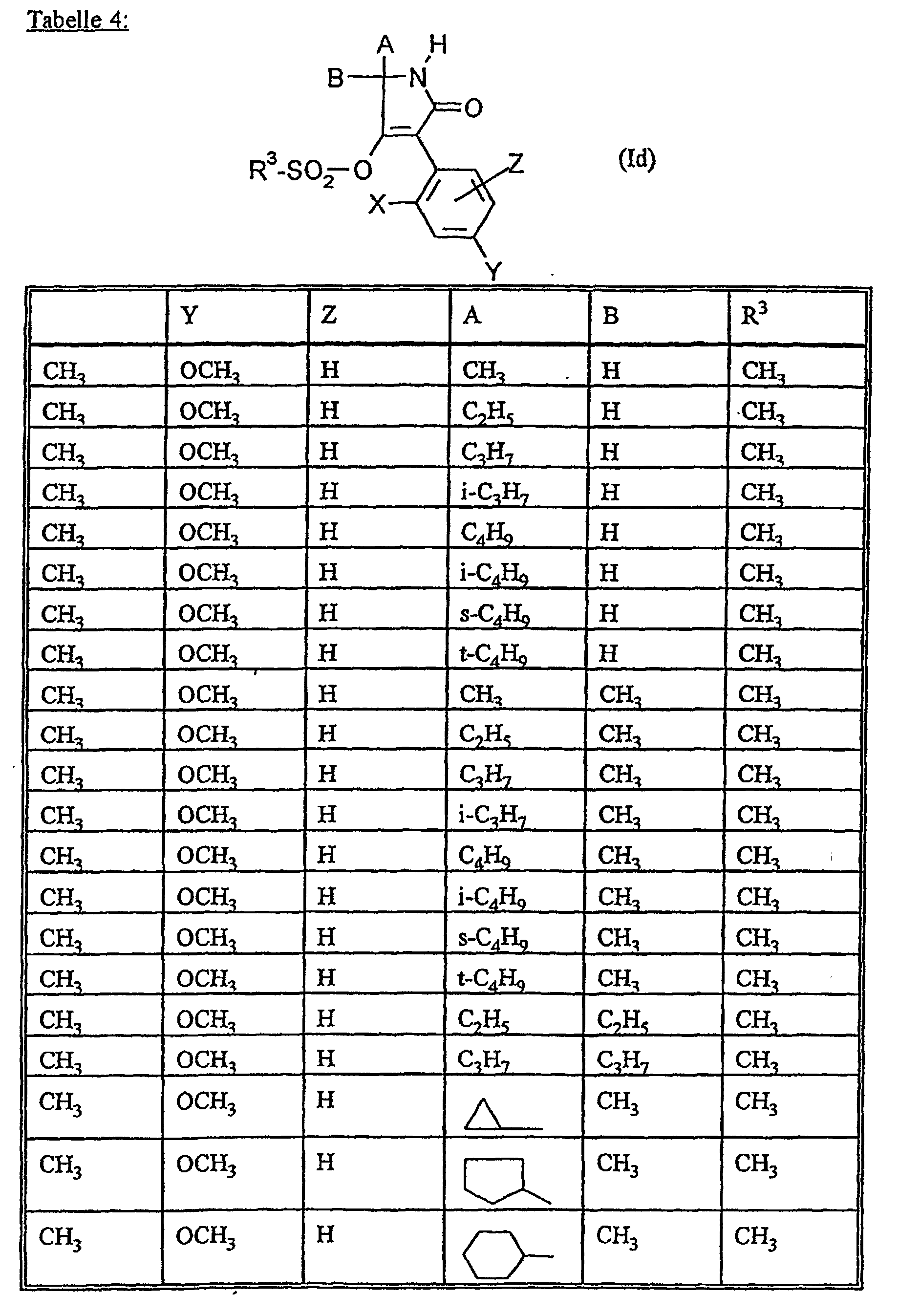

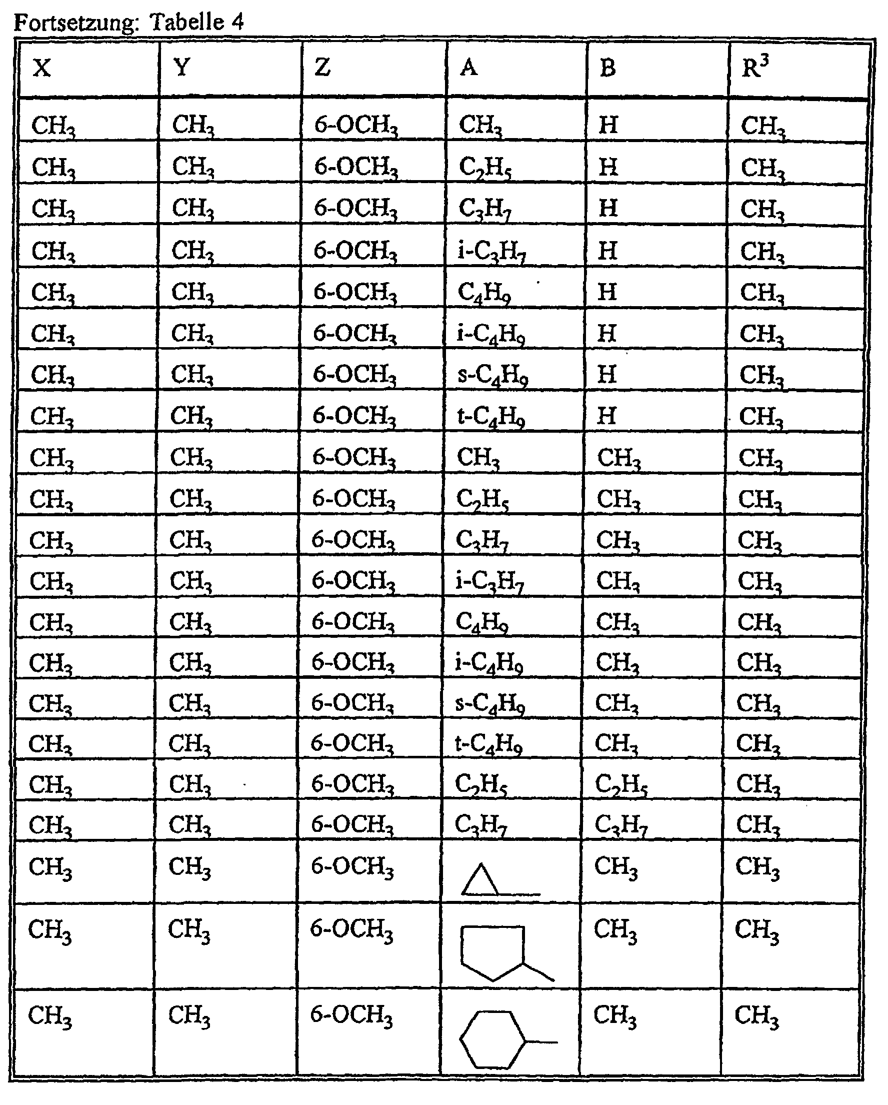

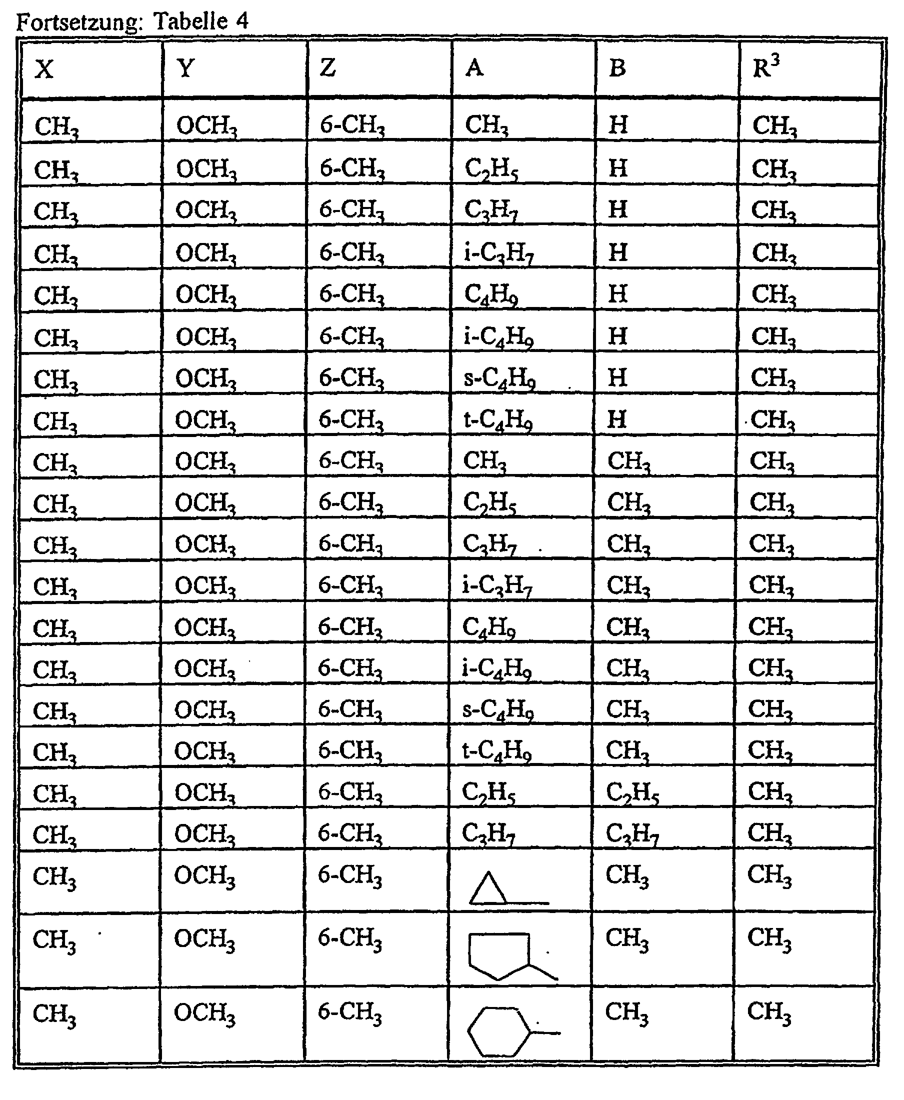

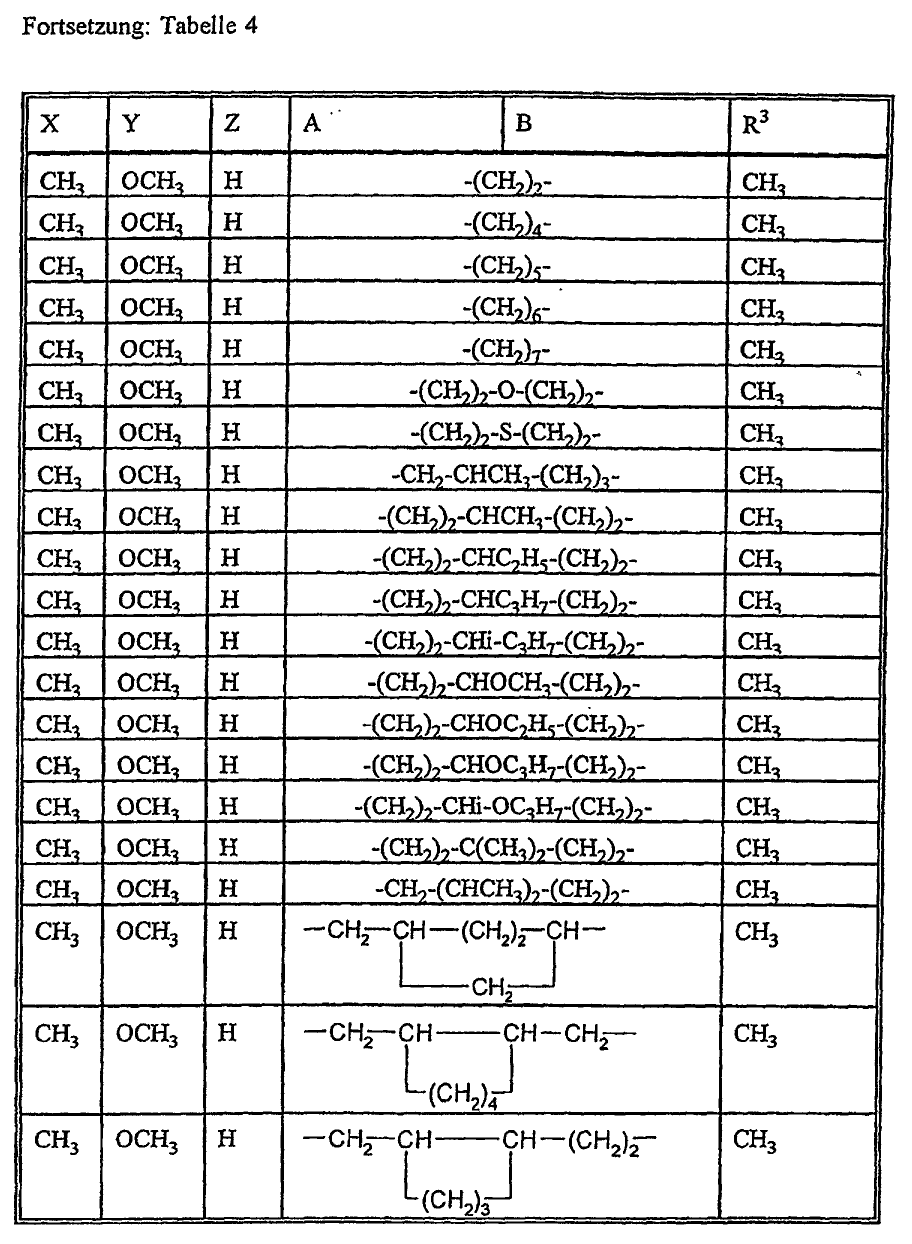

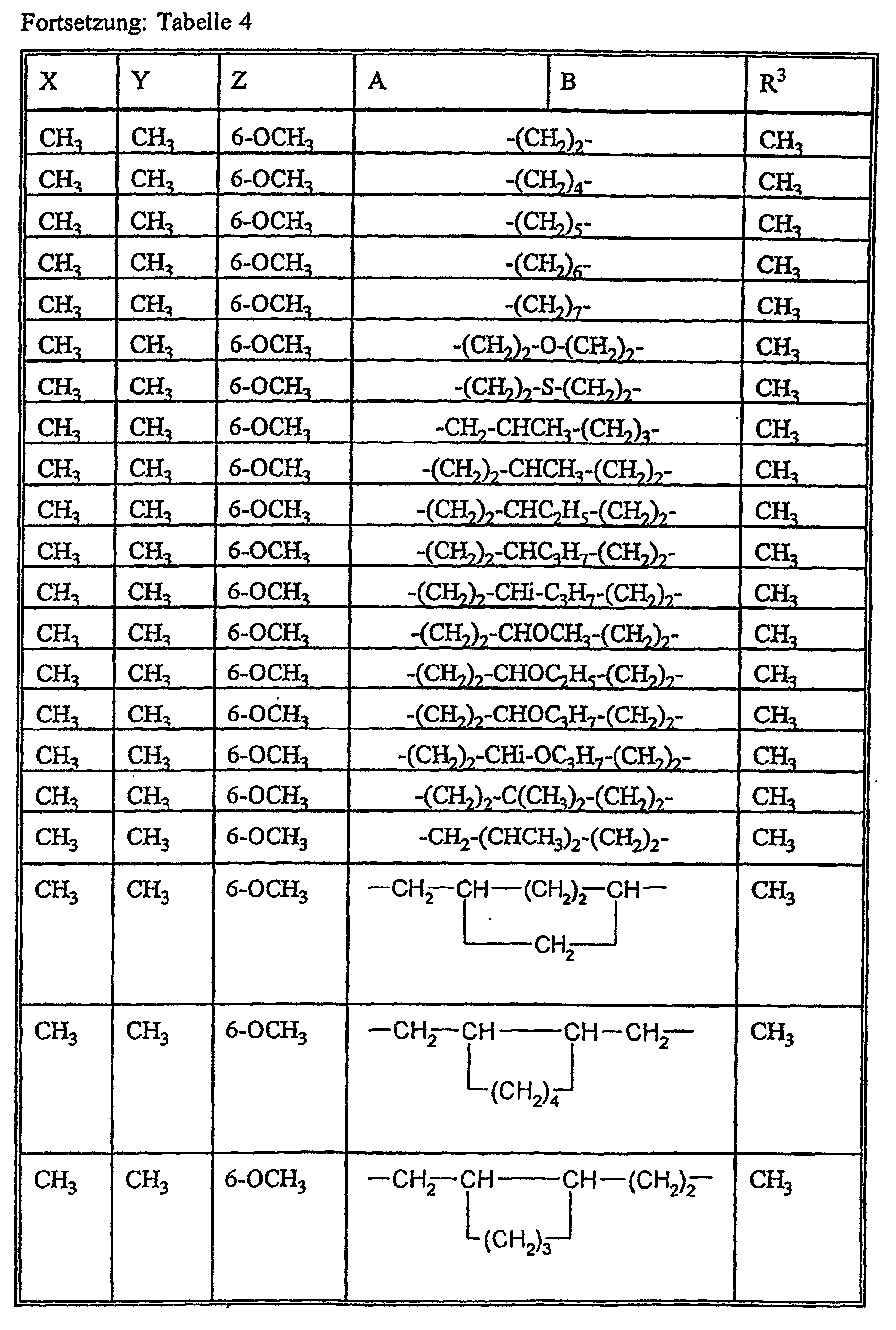

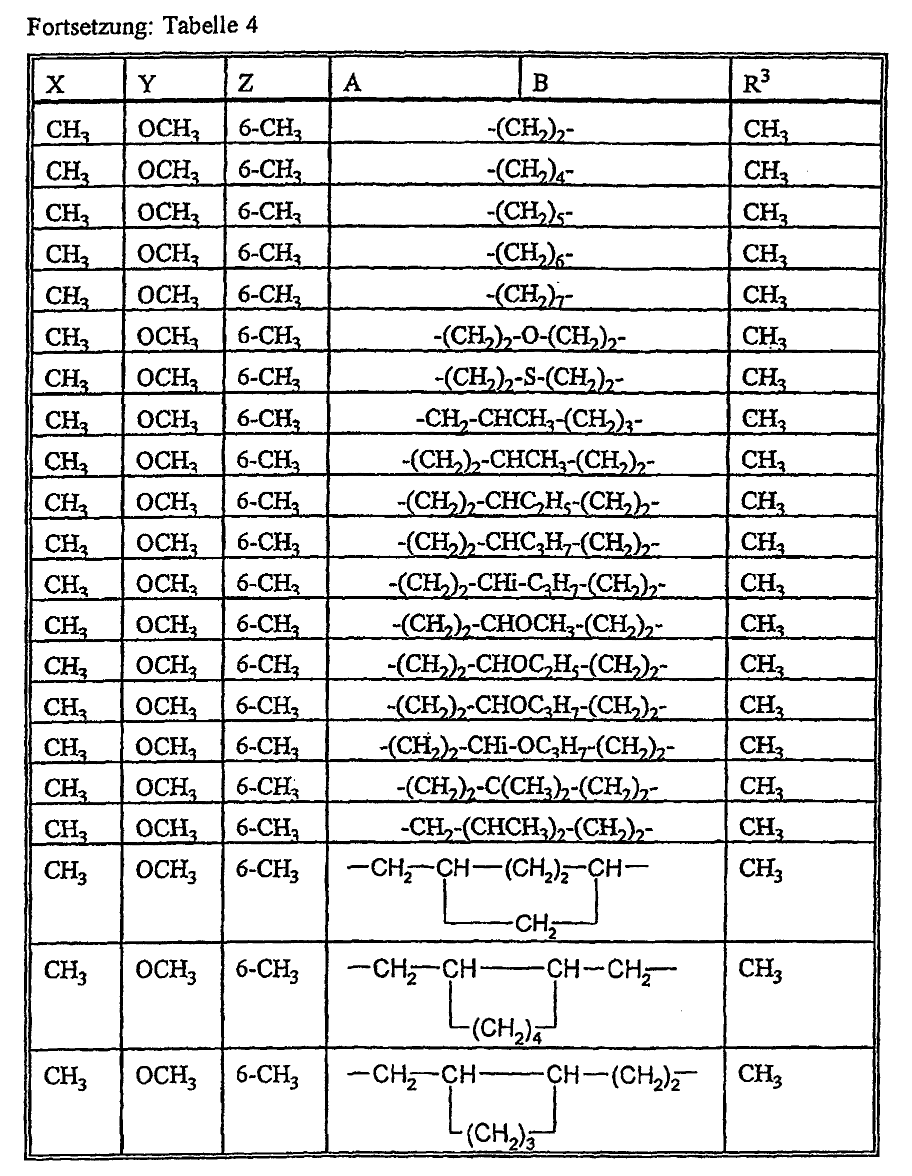

Im einzelnen seien außer den bei den Herstellungsbeispielen genannten Verbindungen die folgenden Verbindungen der Formel (Ie) genannt:

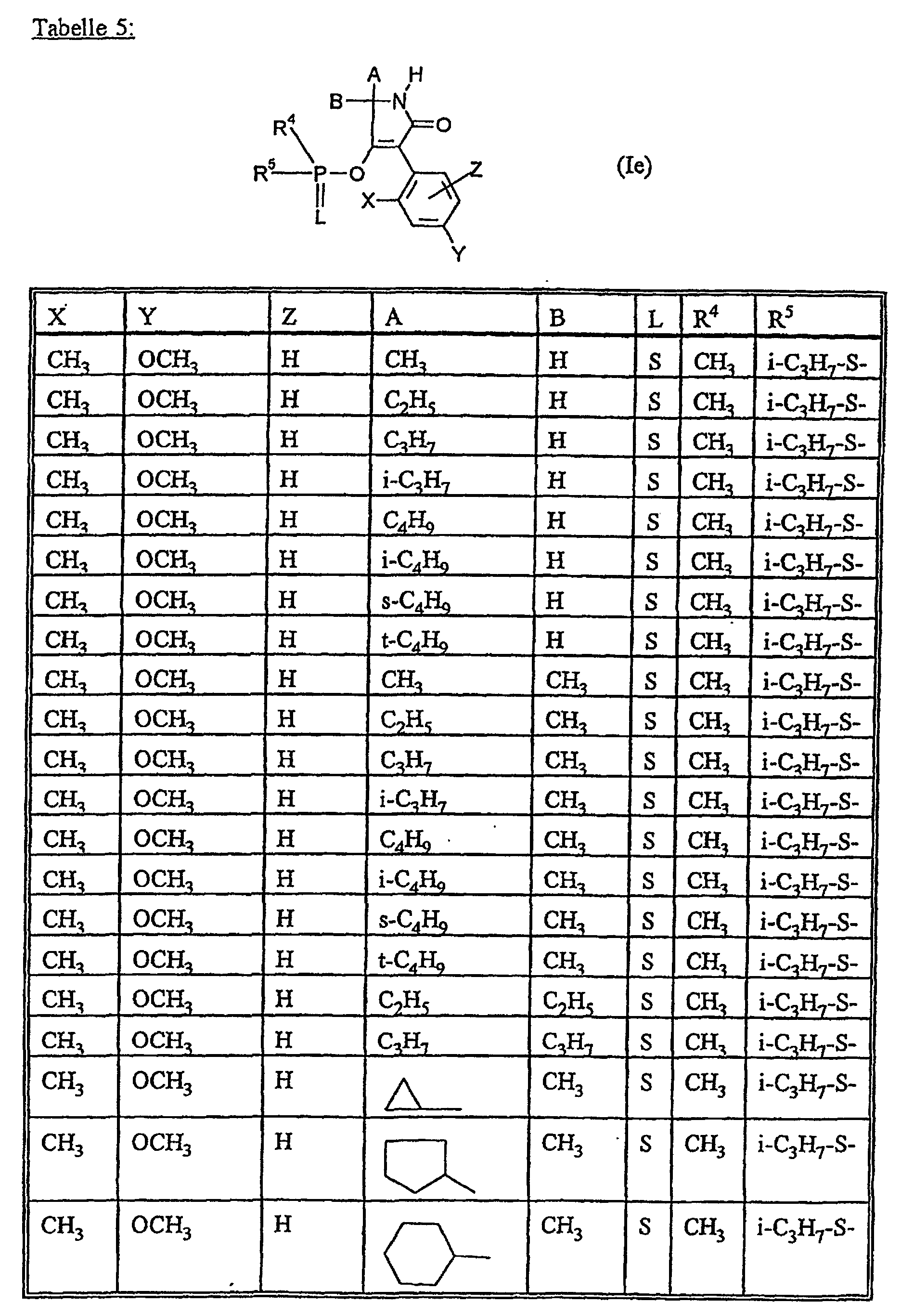

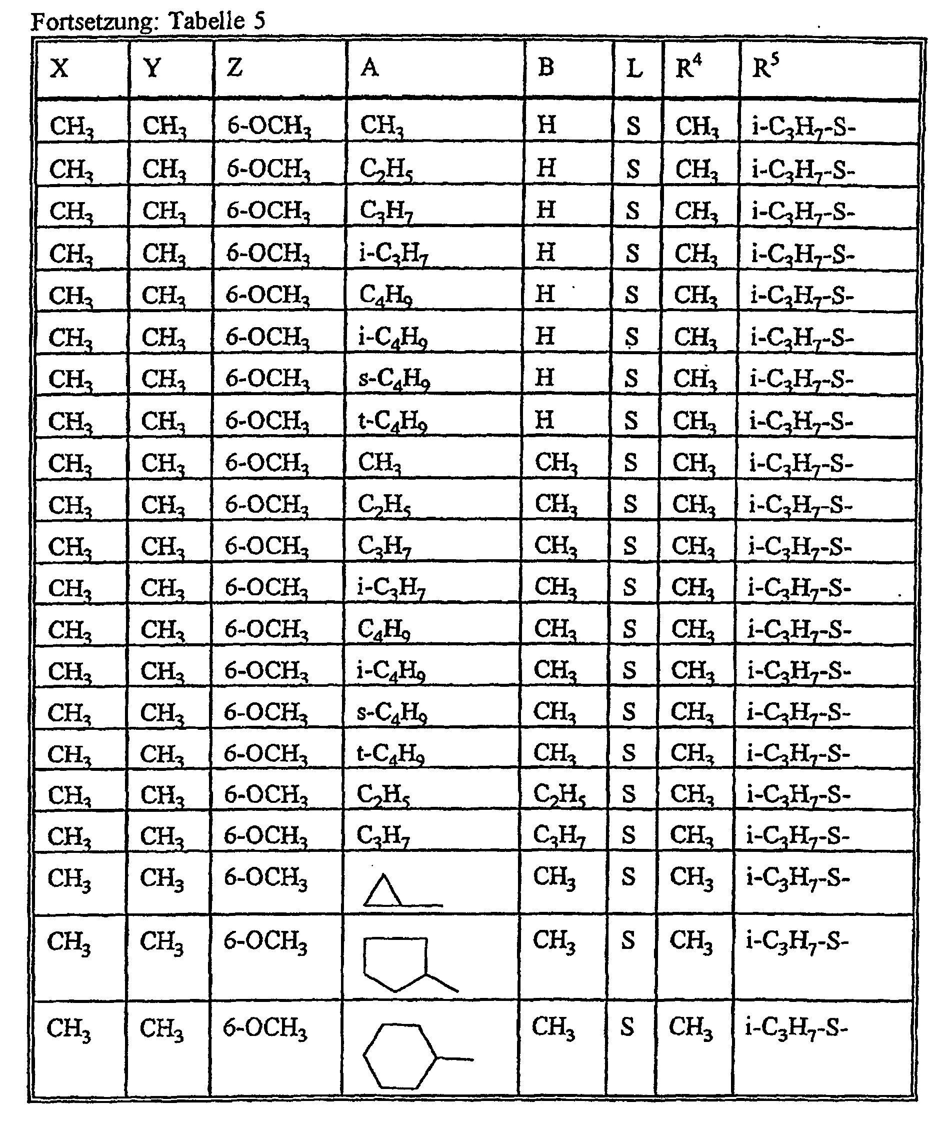

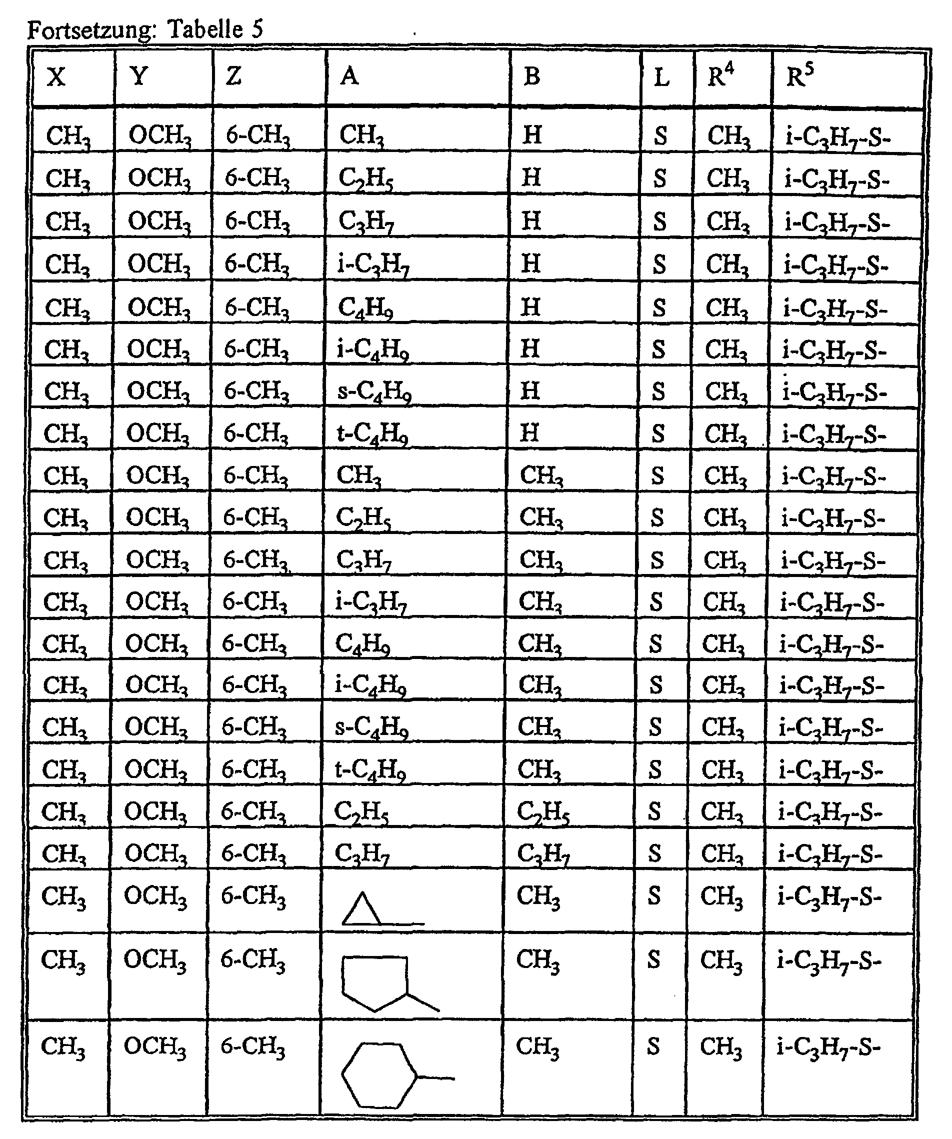

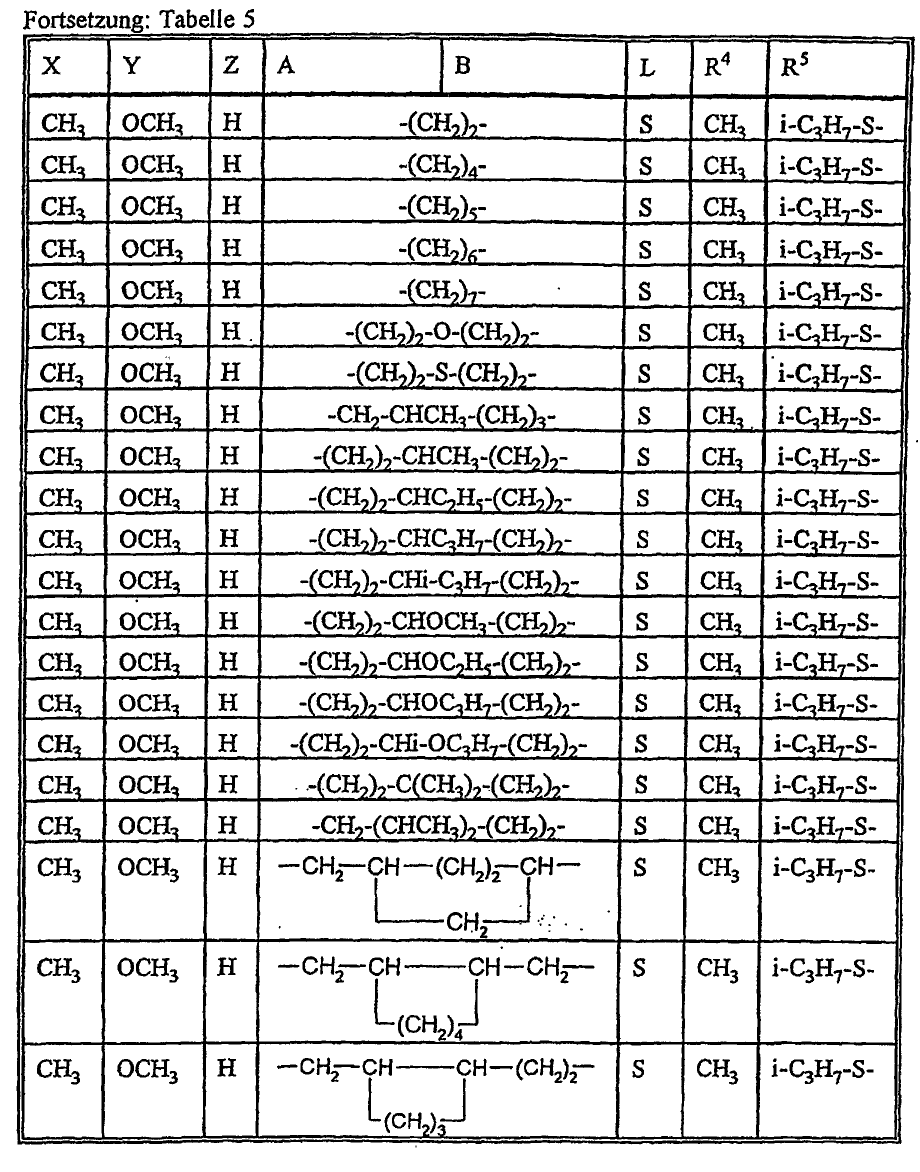

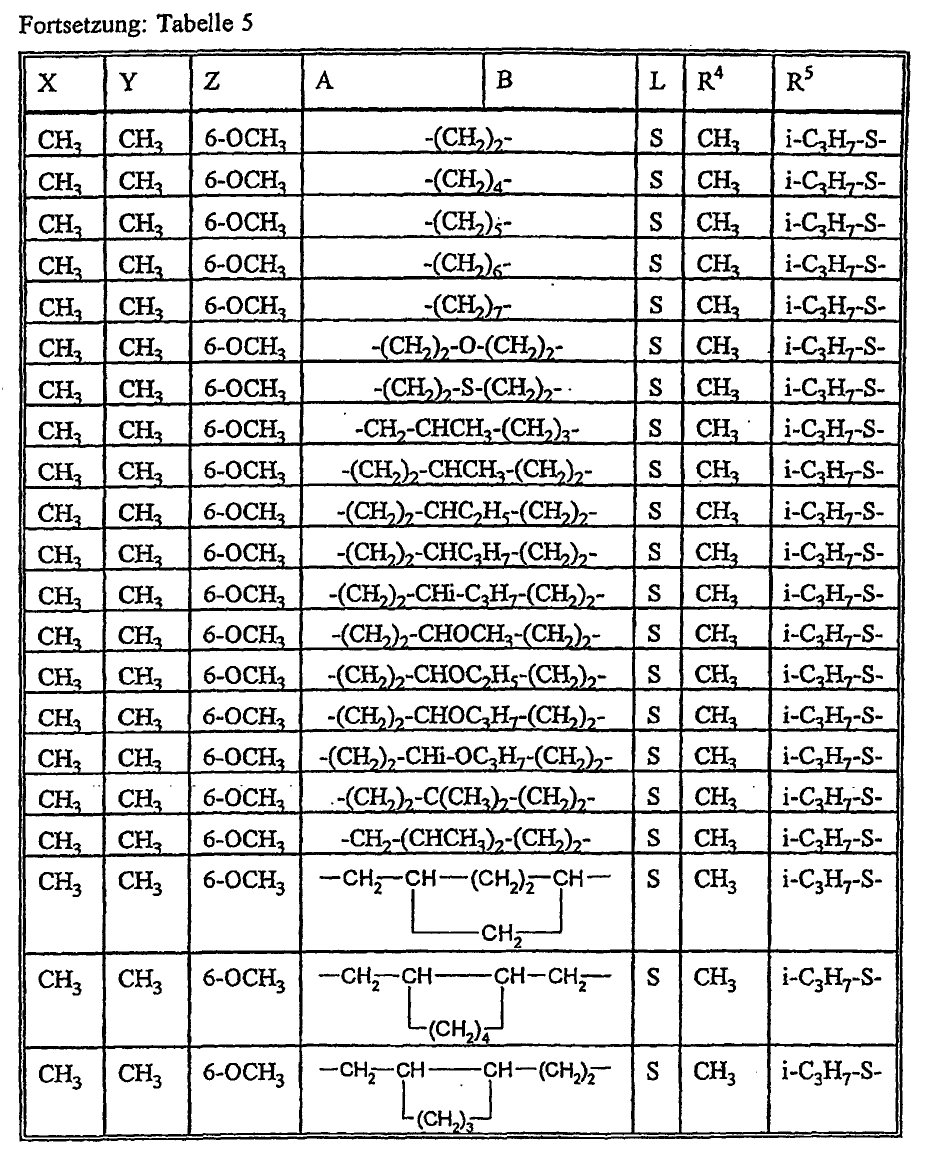

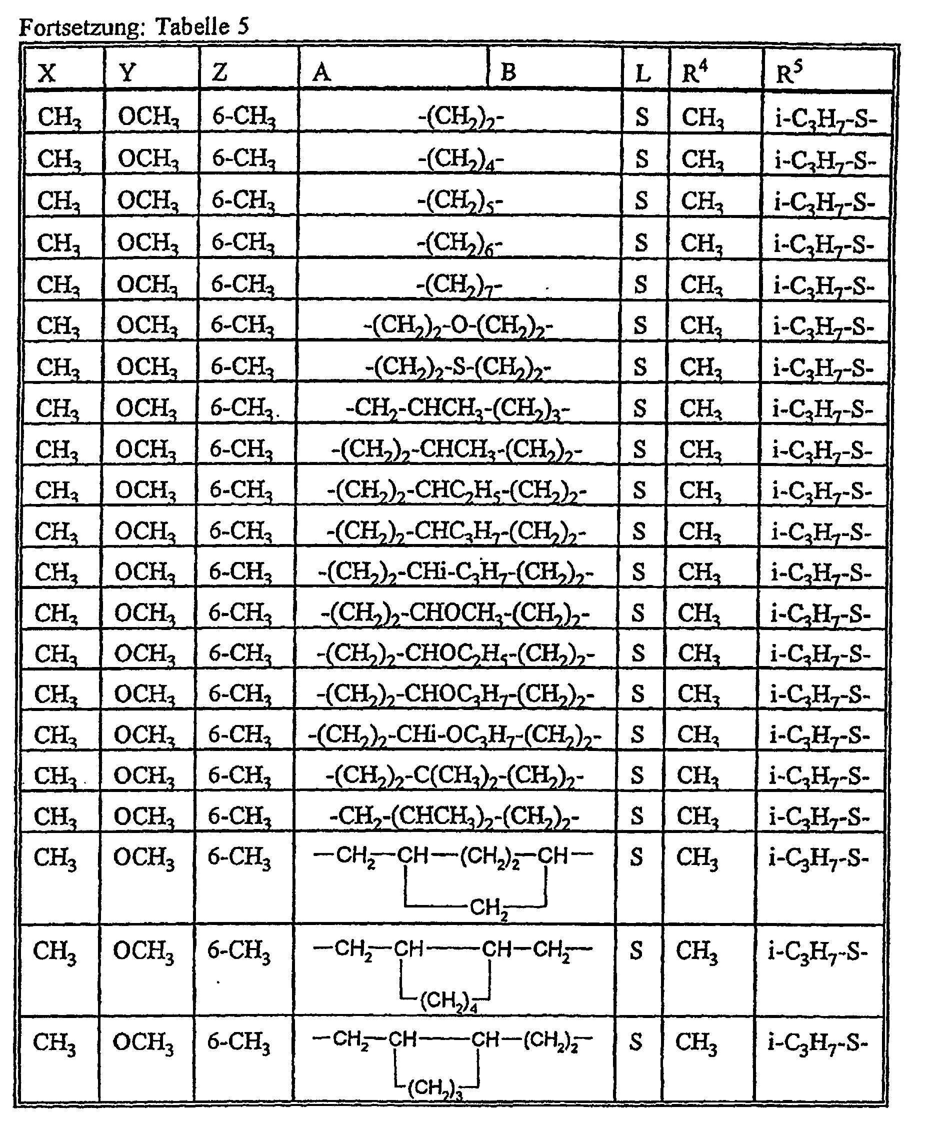

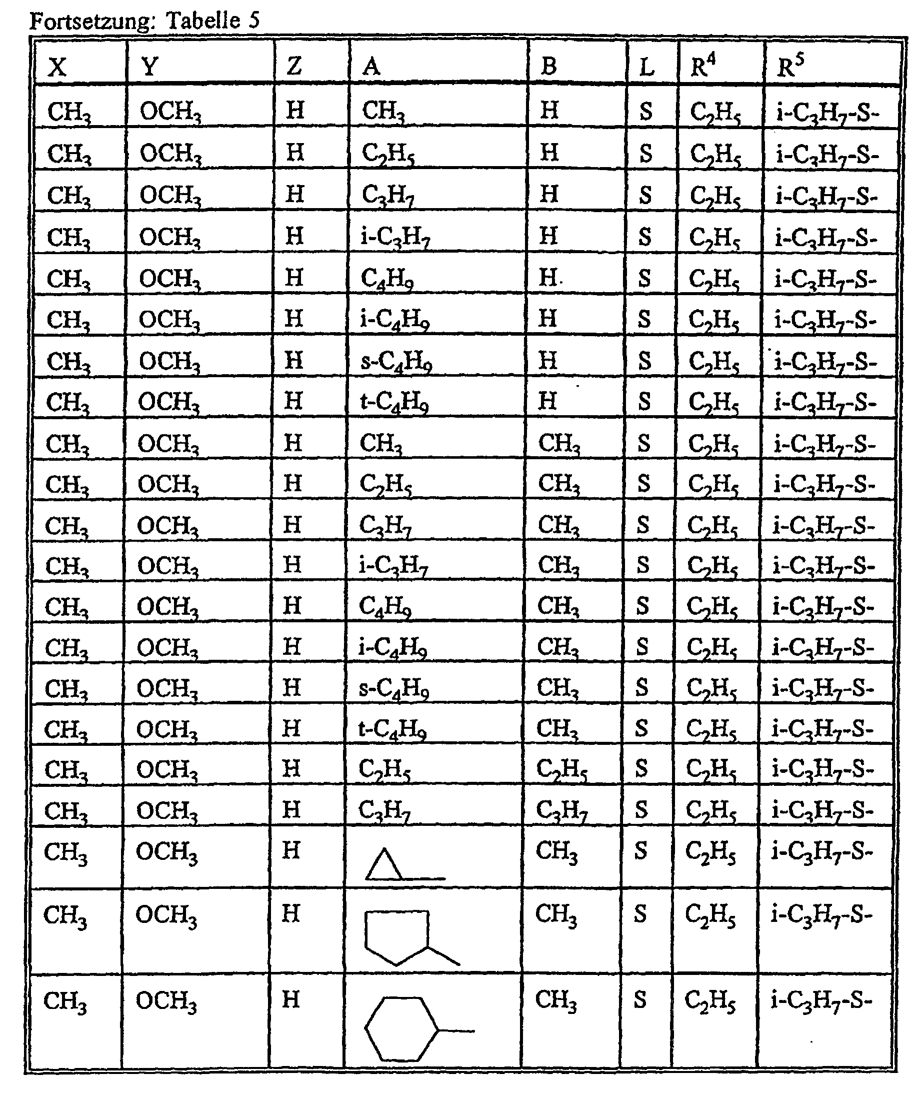

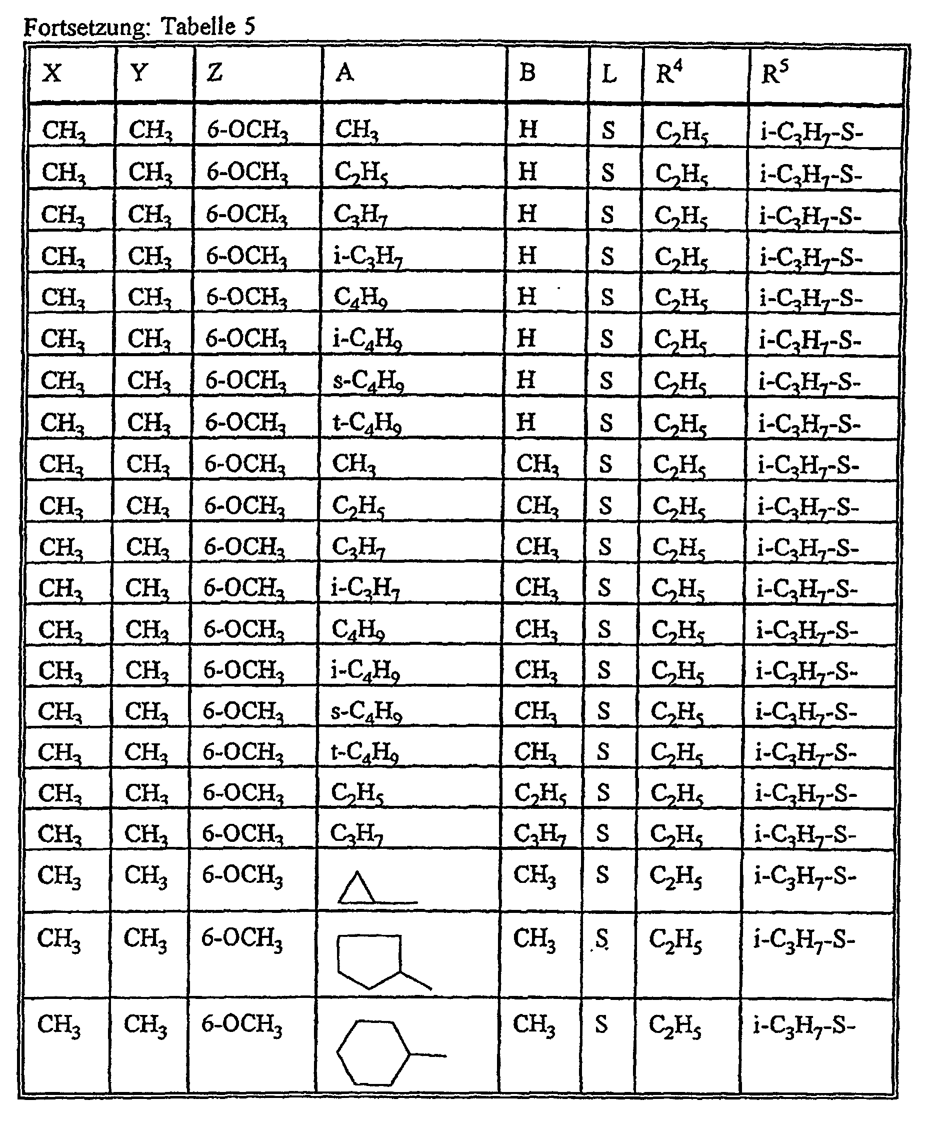

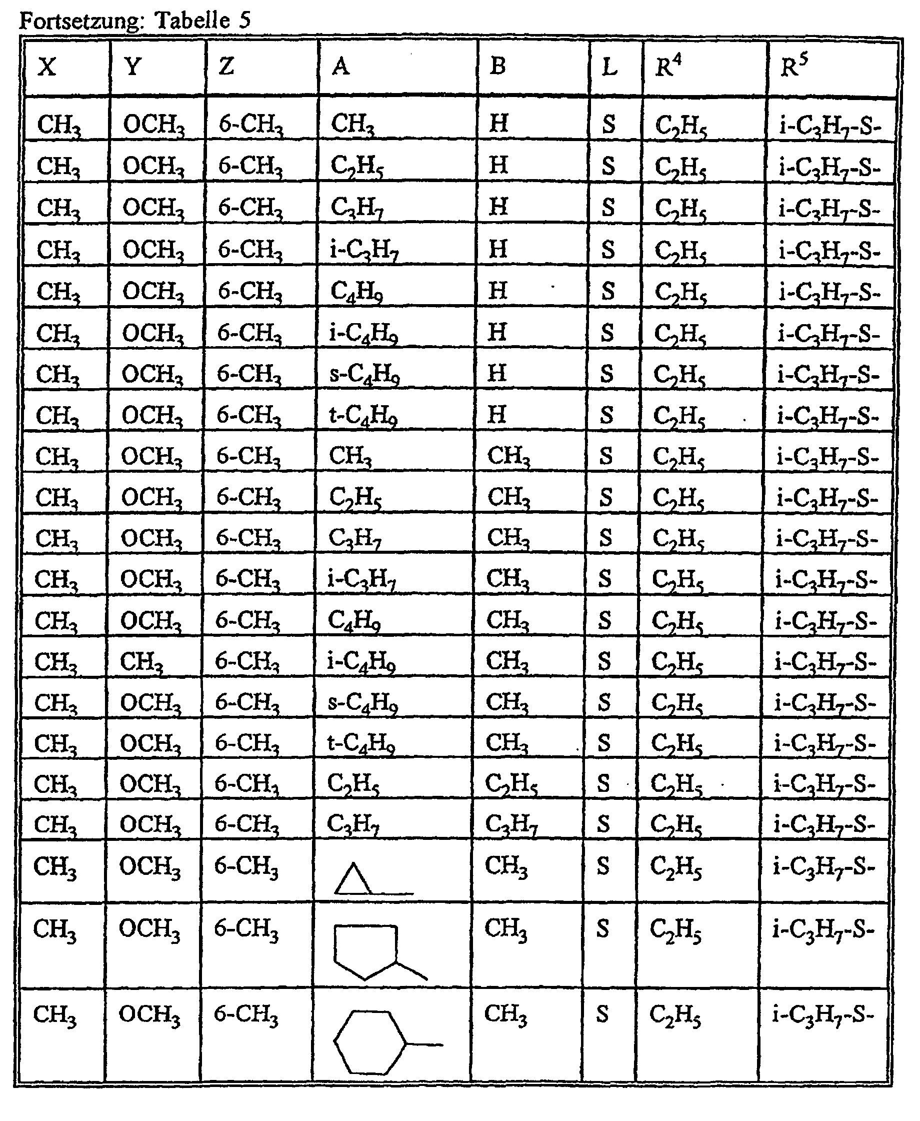

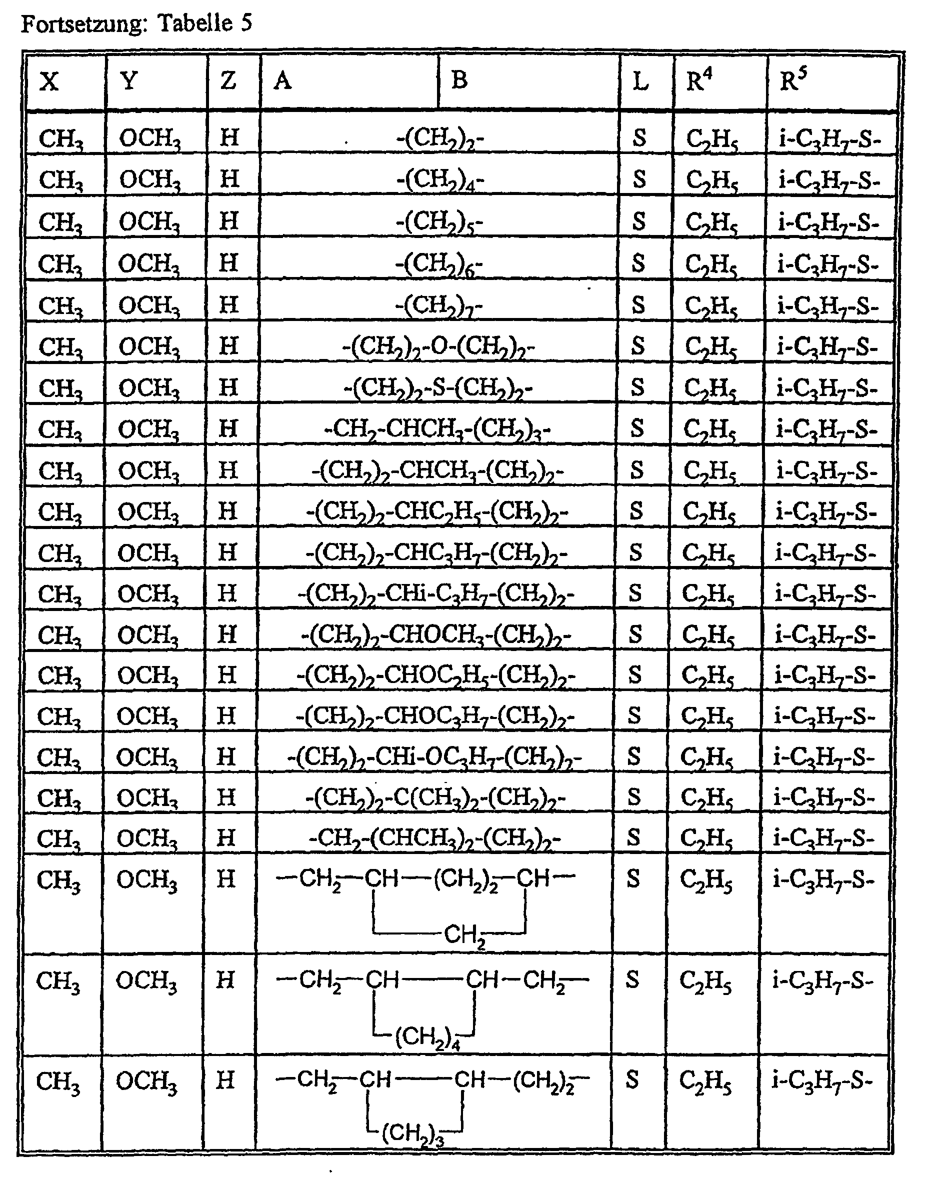

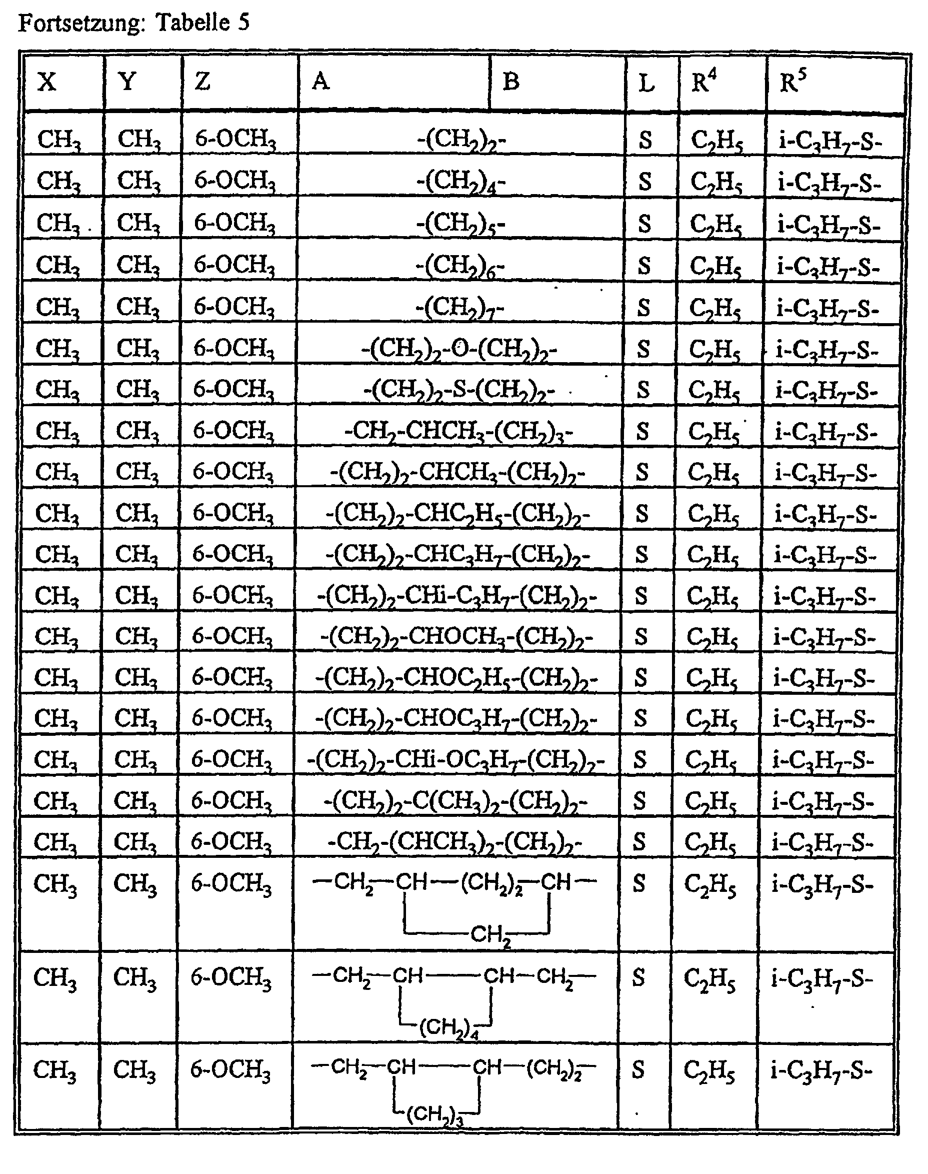

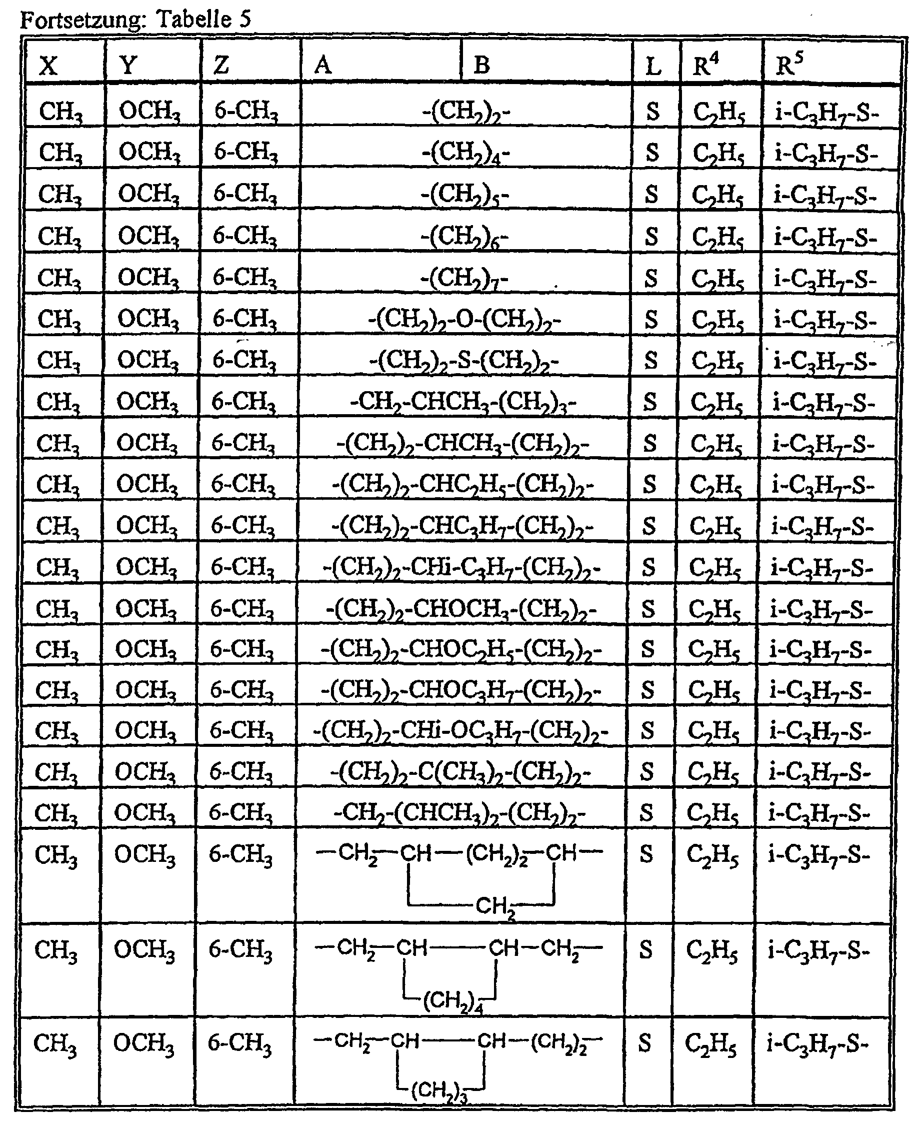

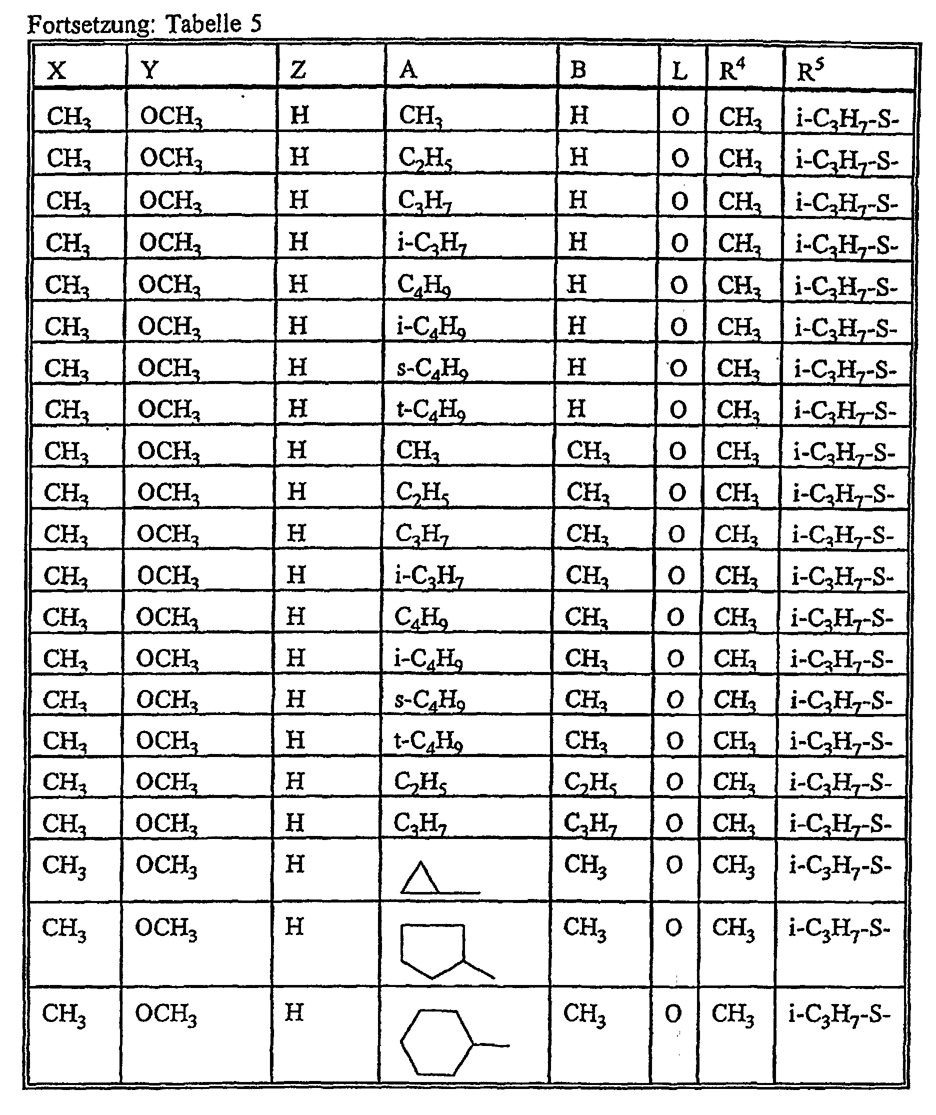

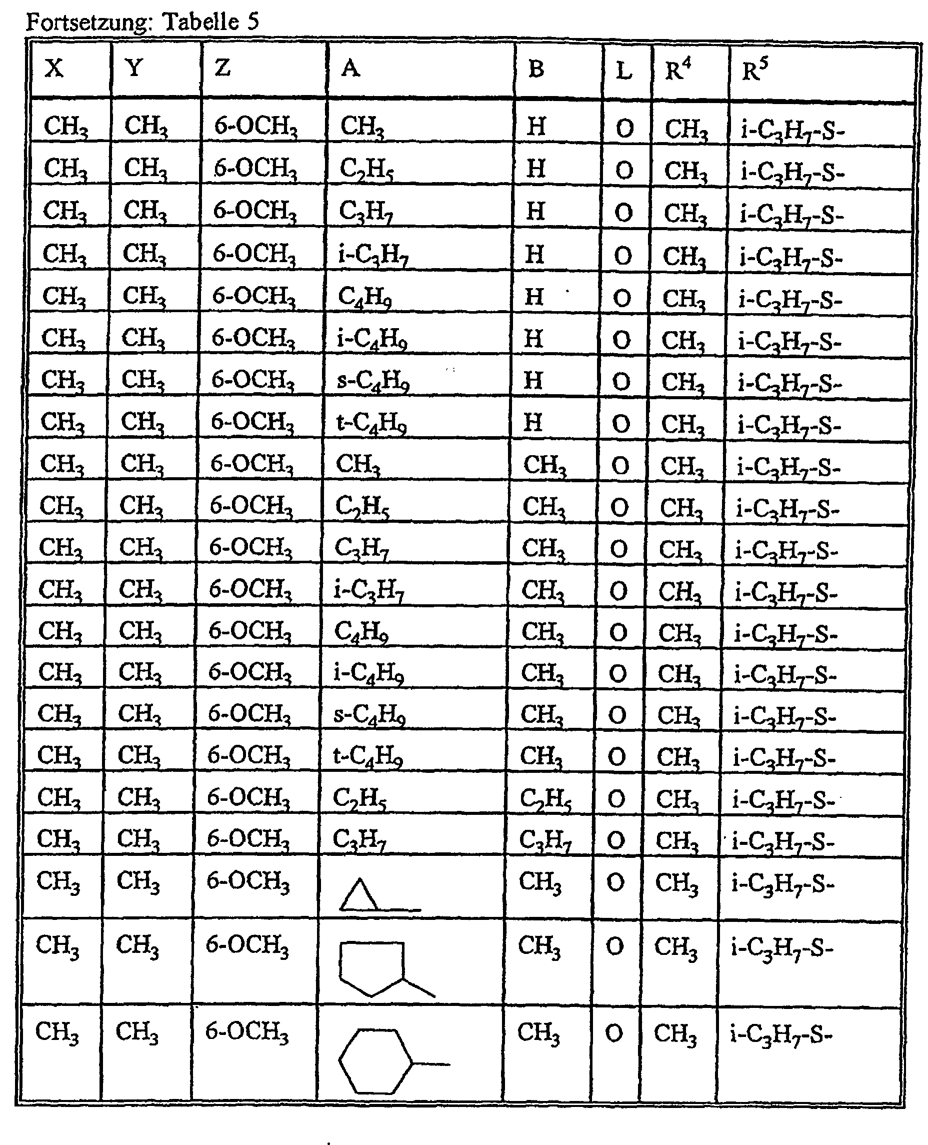

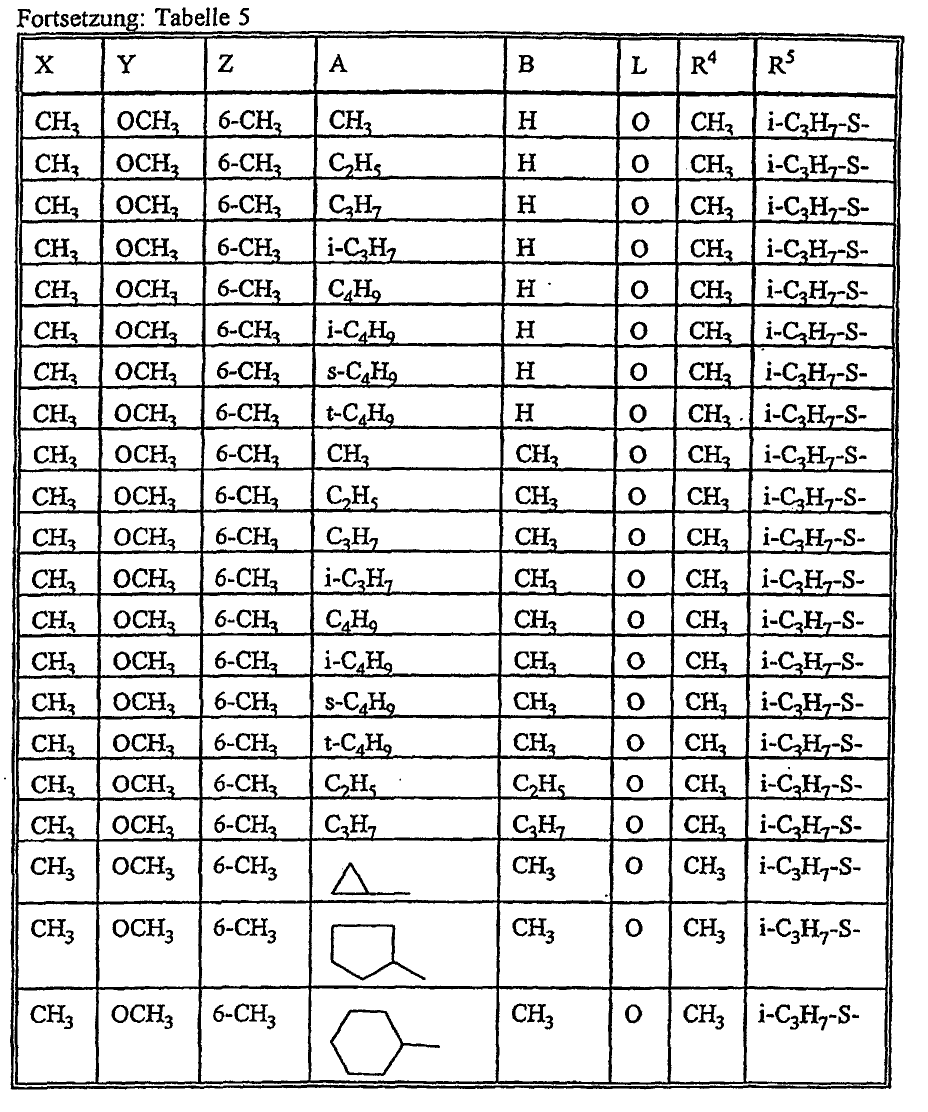

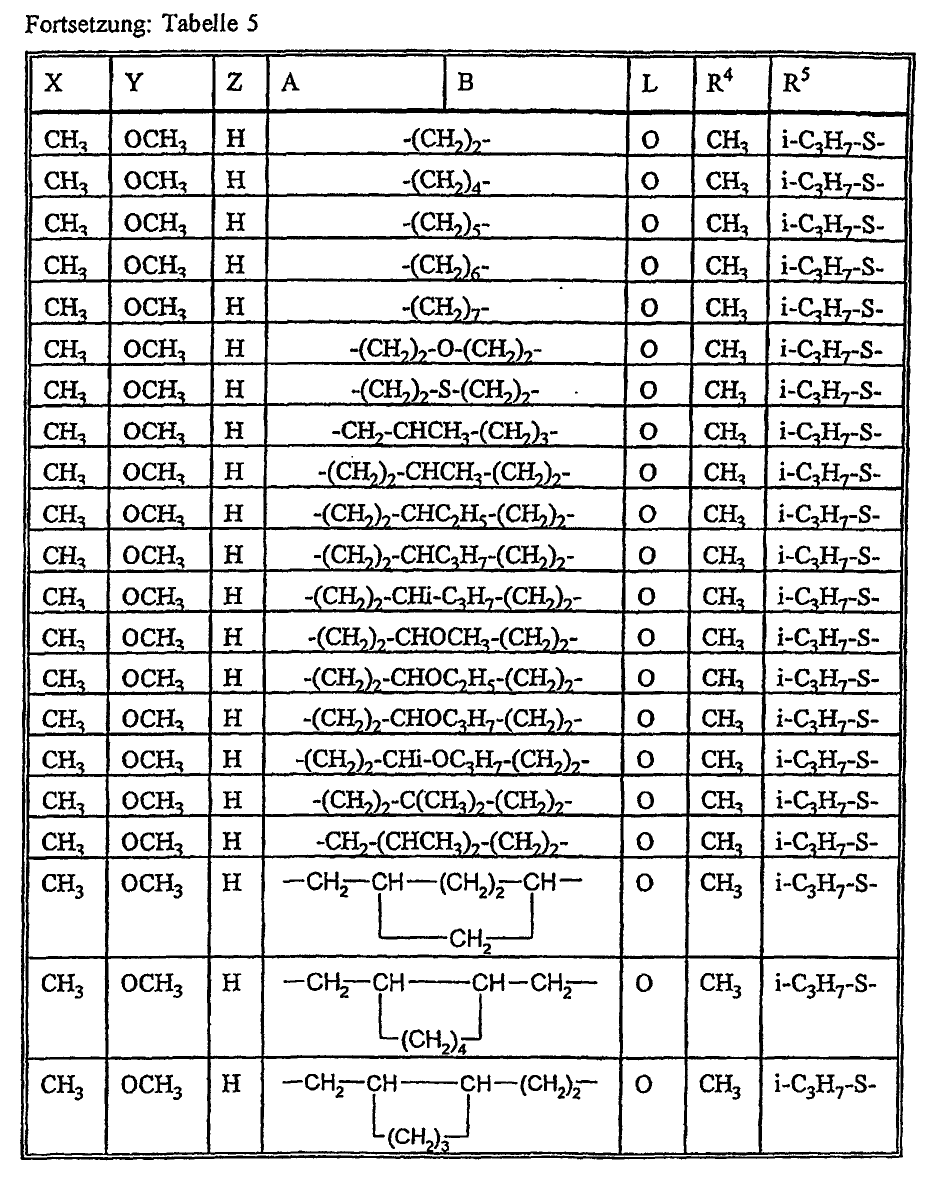

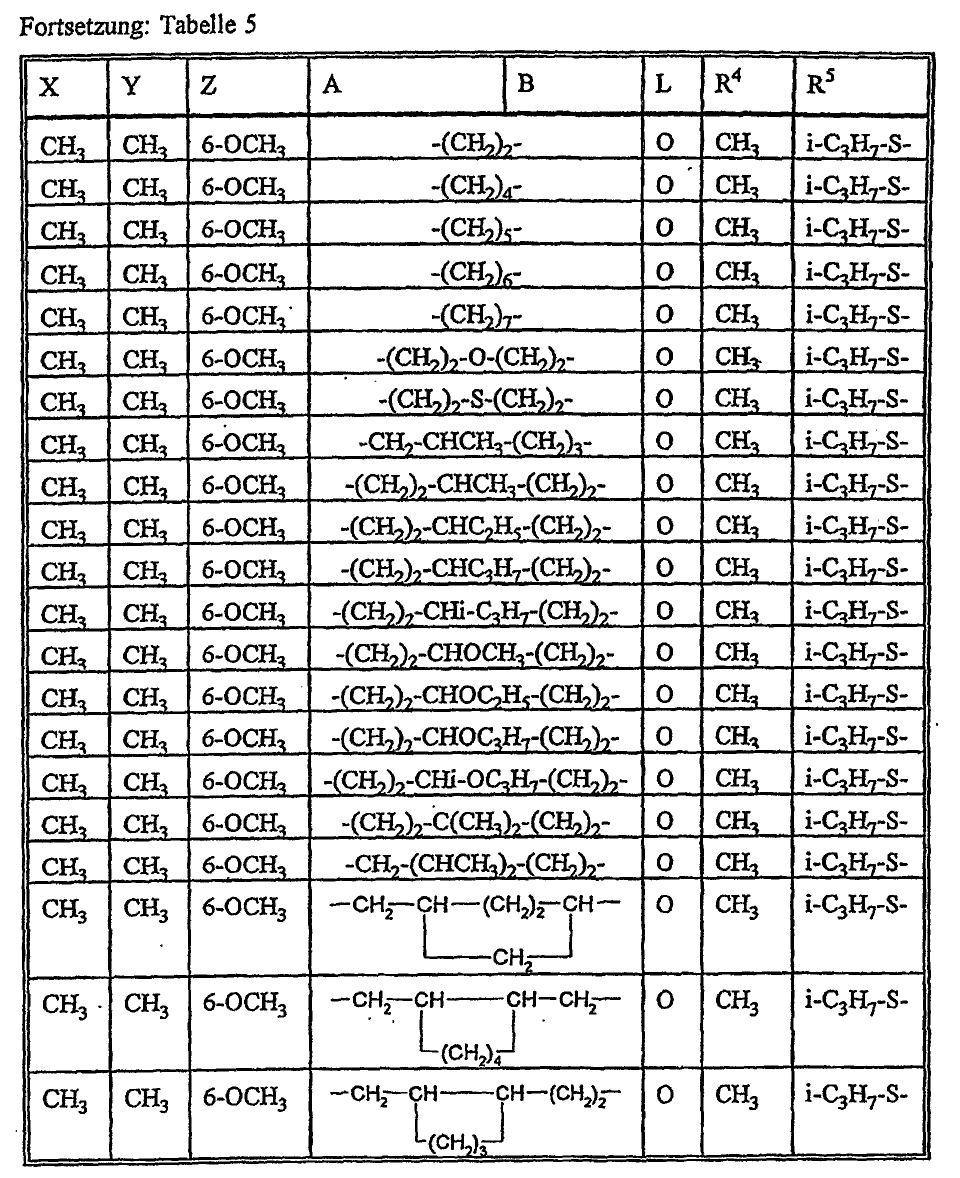

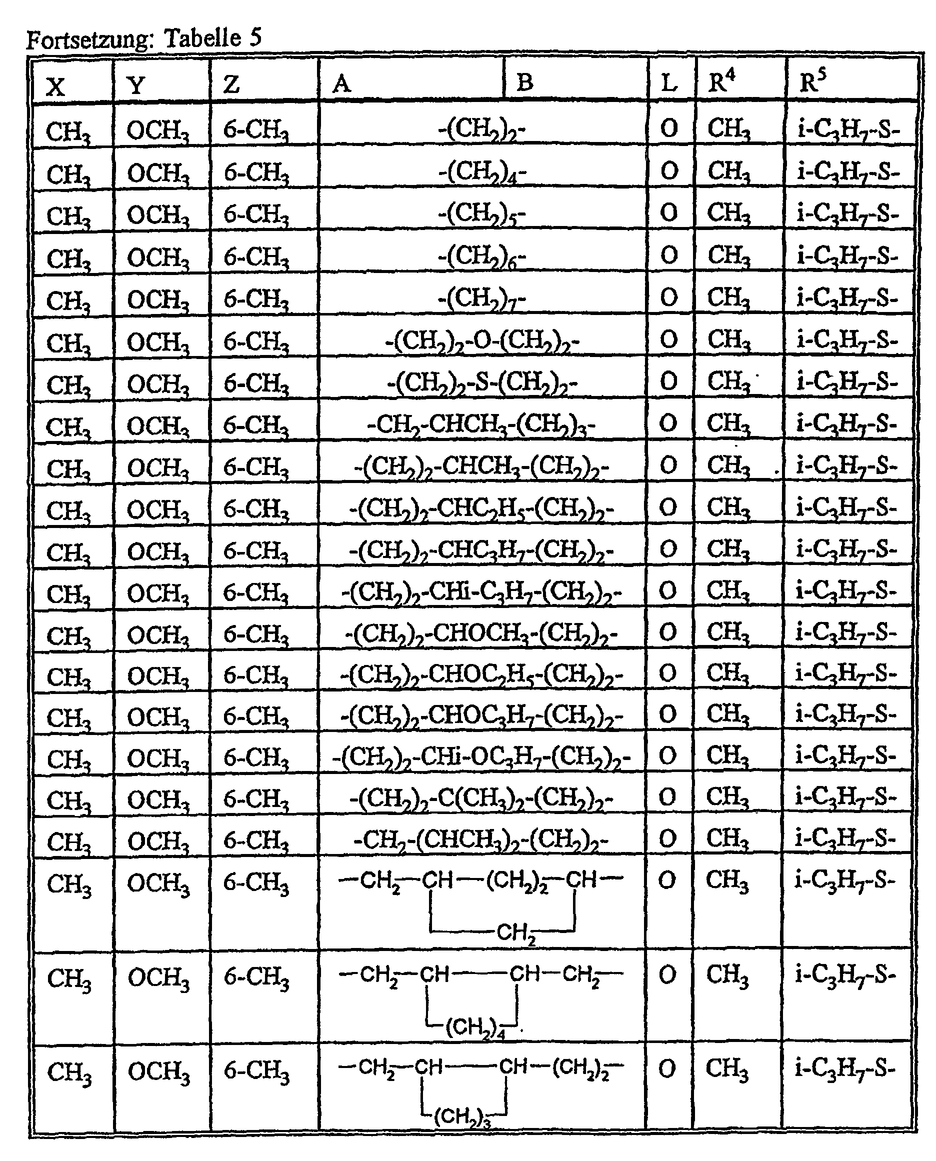

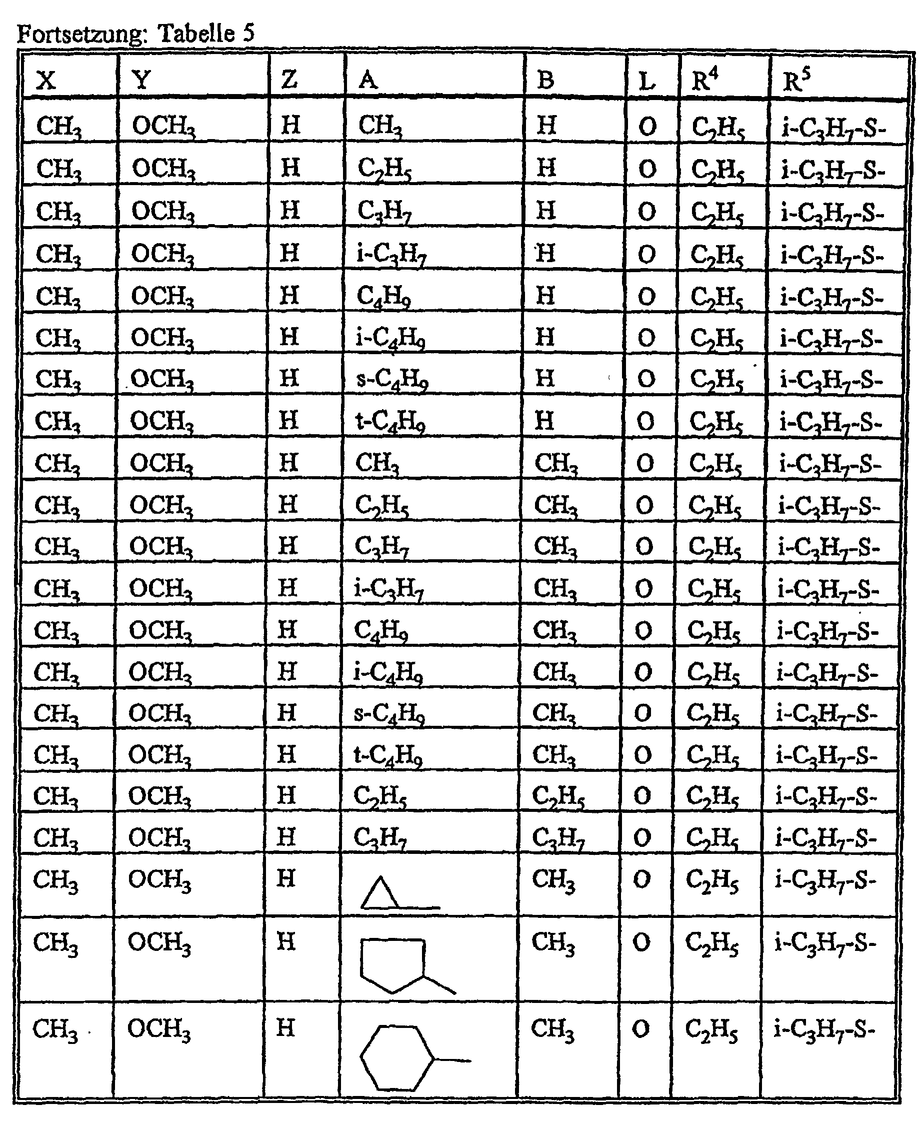

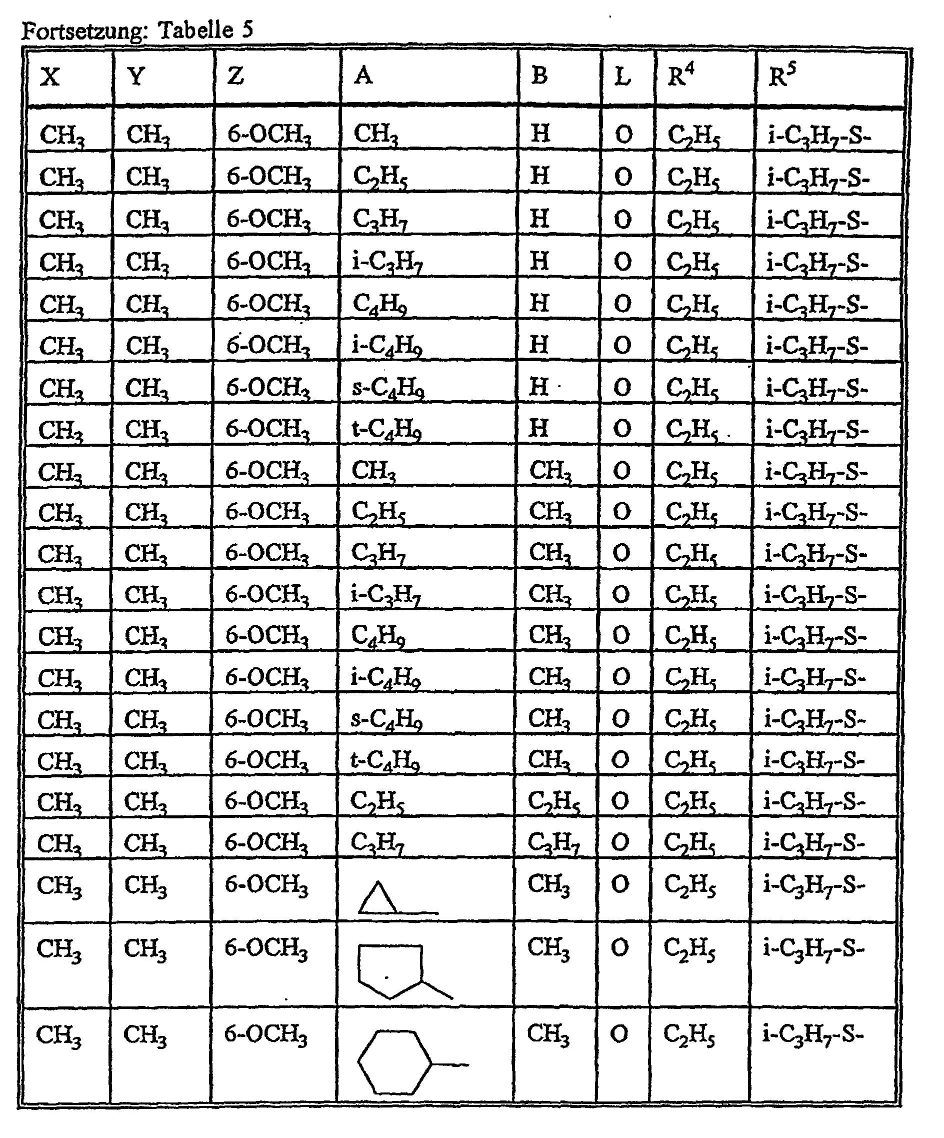

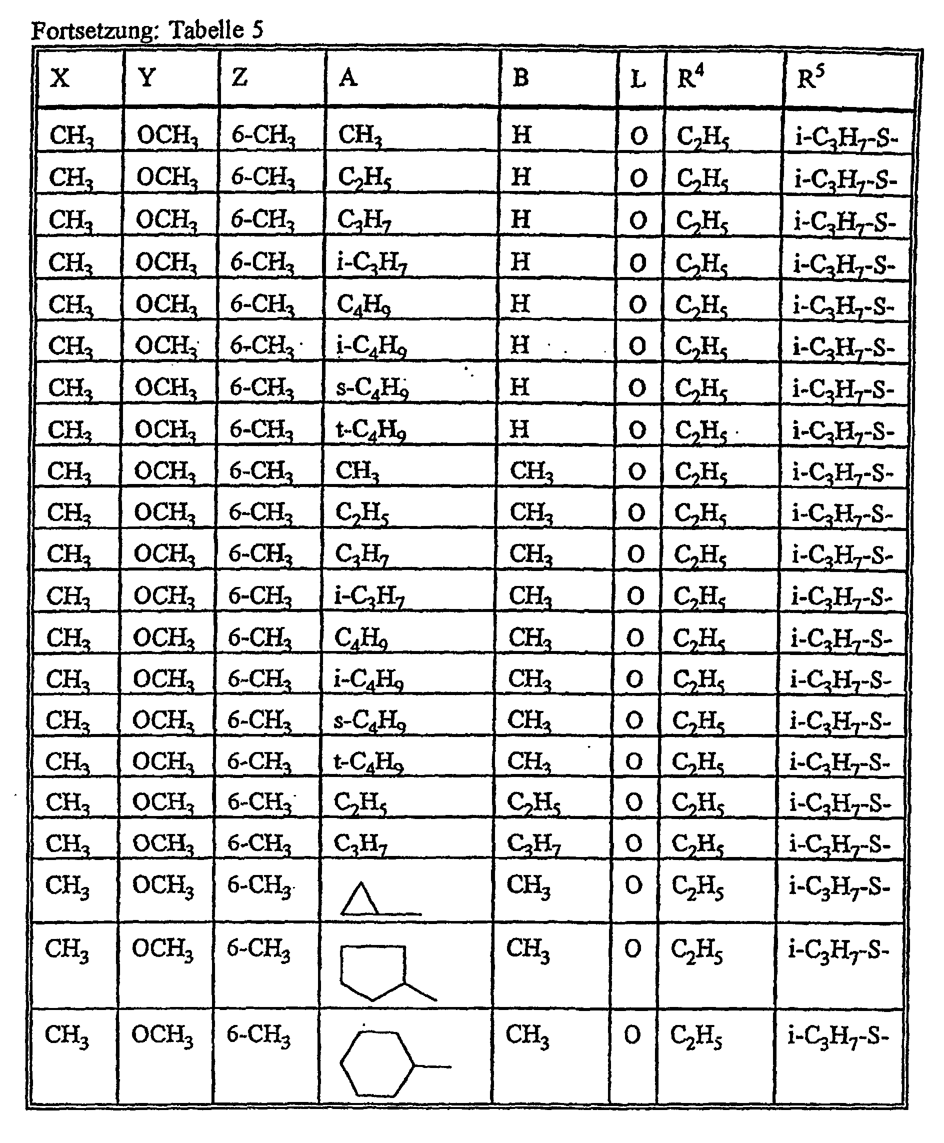

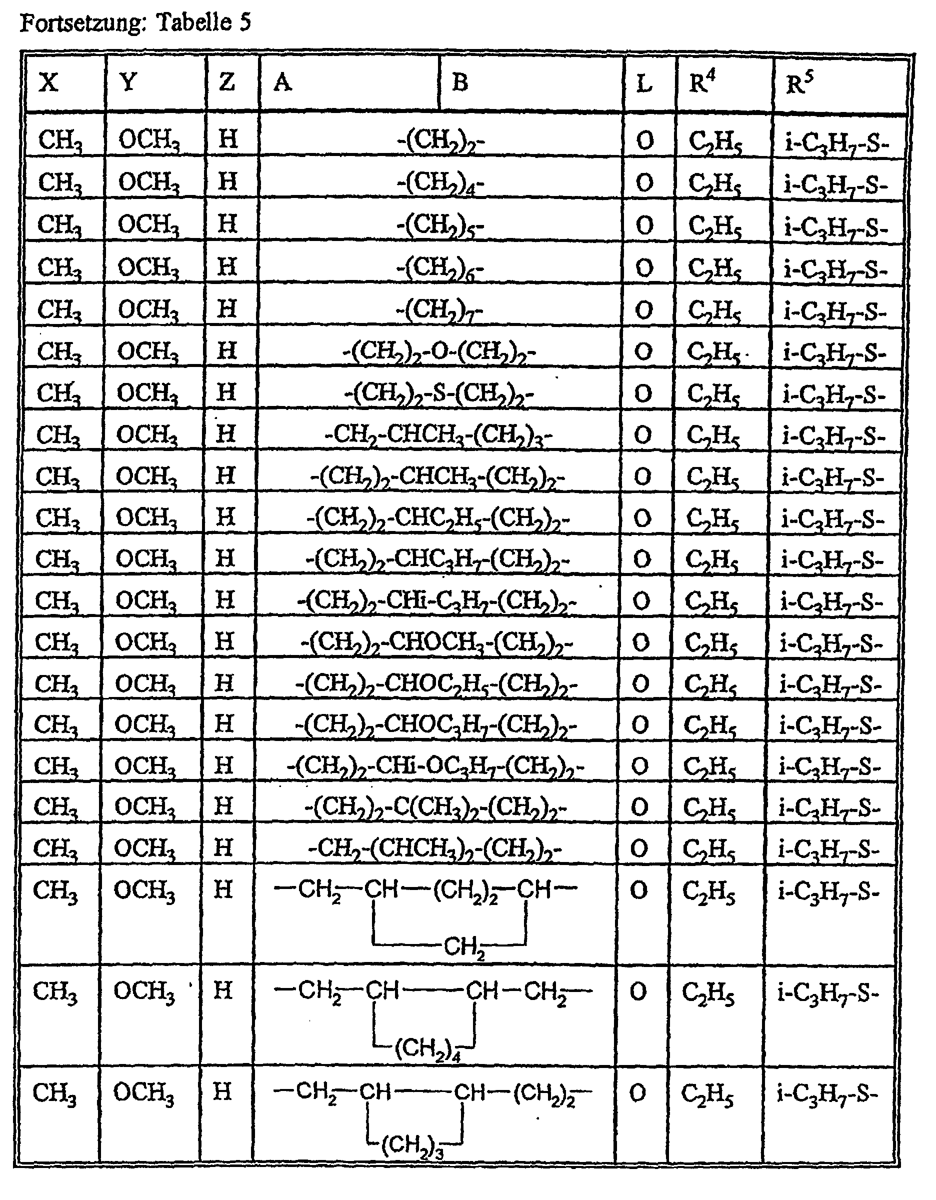

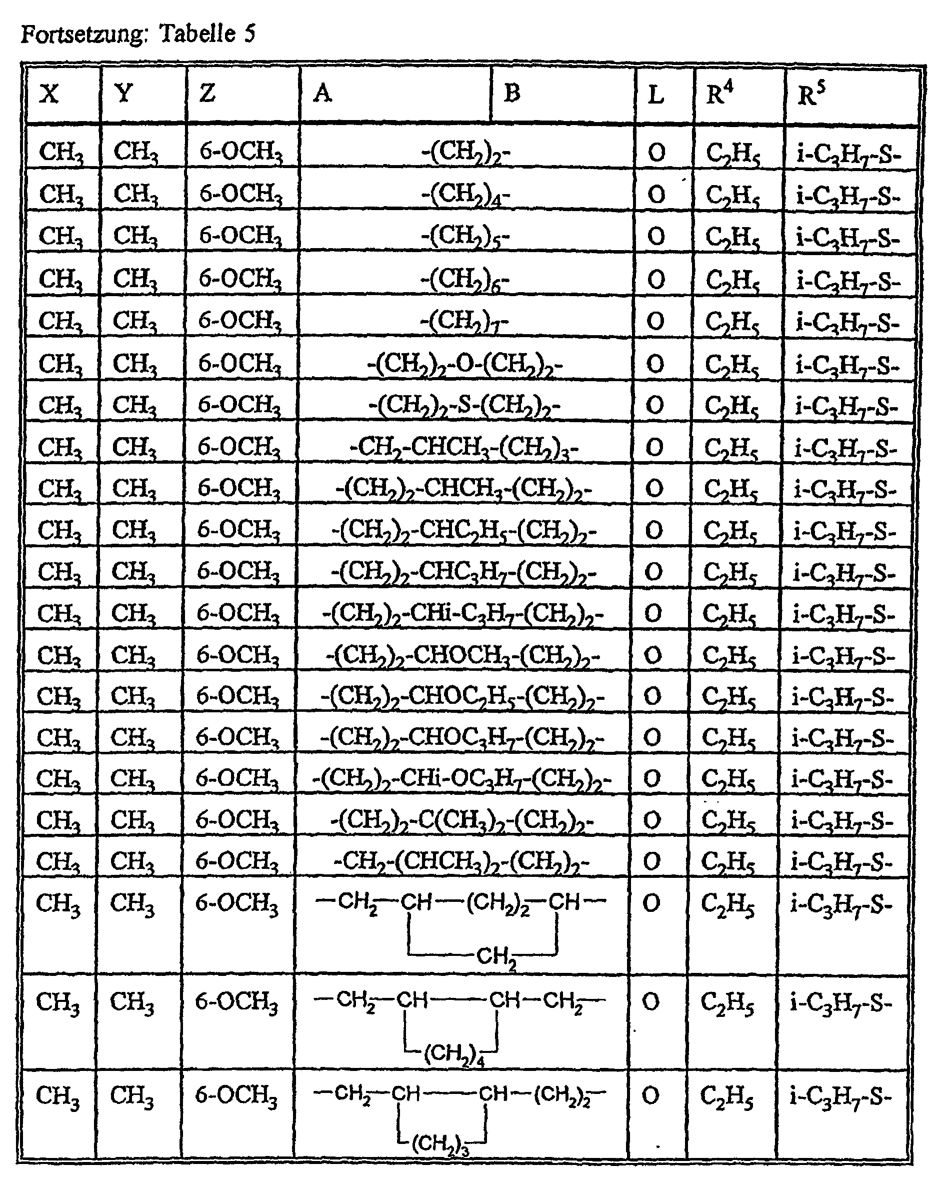

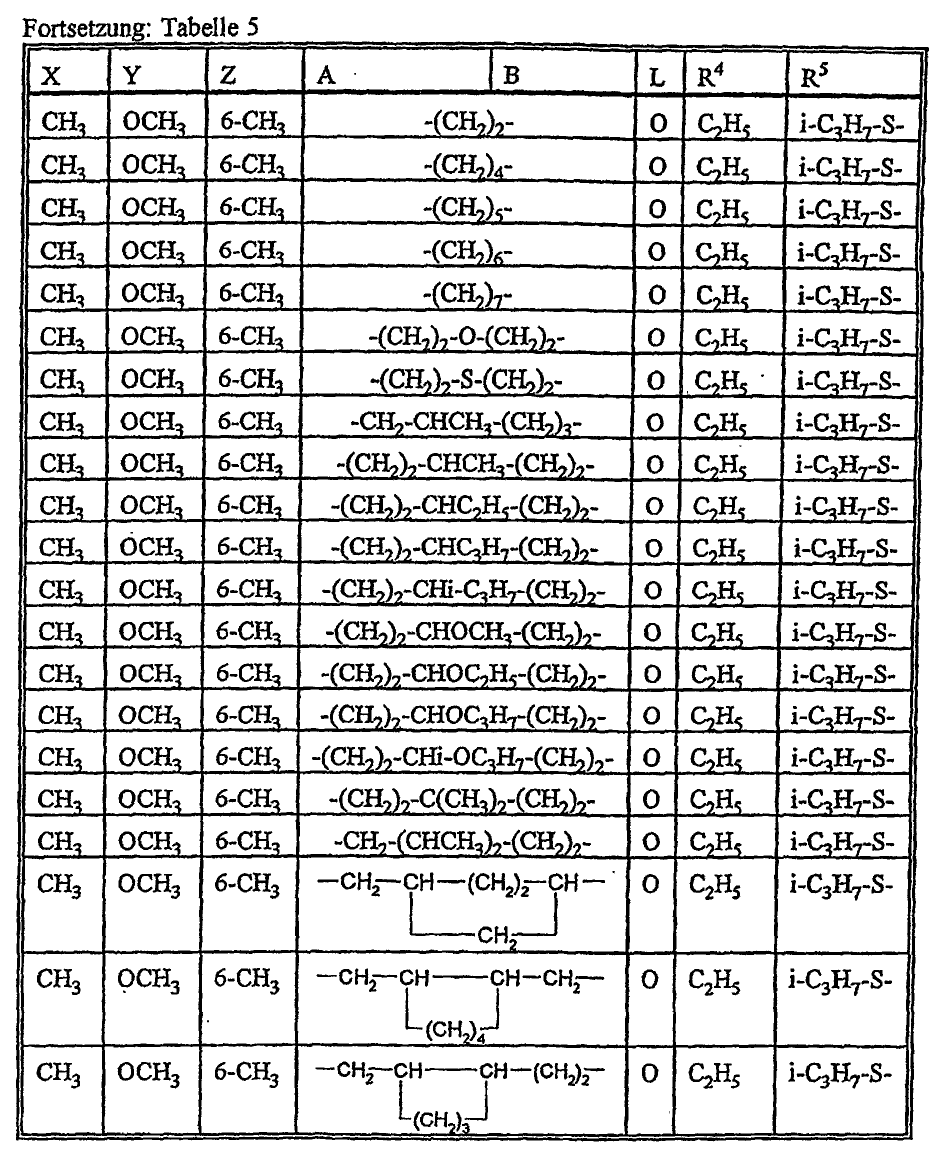

Im einzelnen seien außer den bei den Herstellungsbeispielen genannten Verbindungen die folgenden Verbindungen der Formel (If-a) genannt:

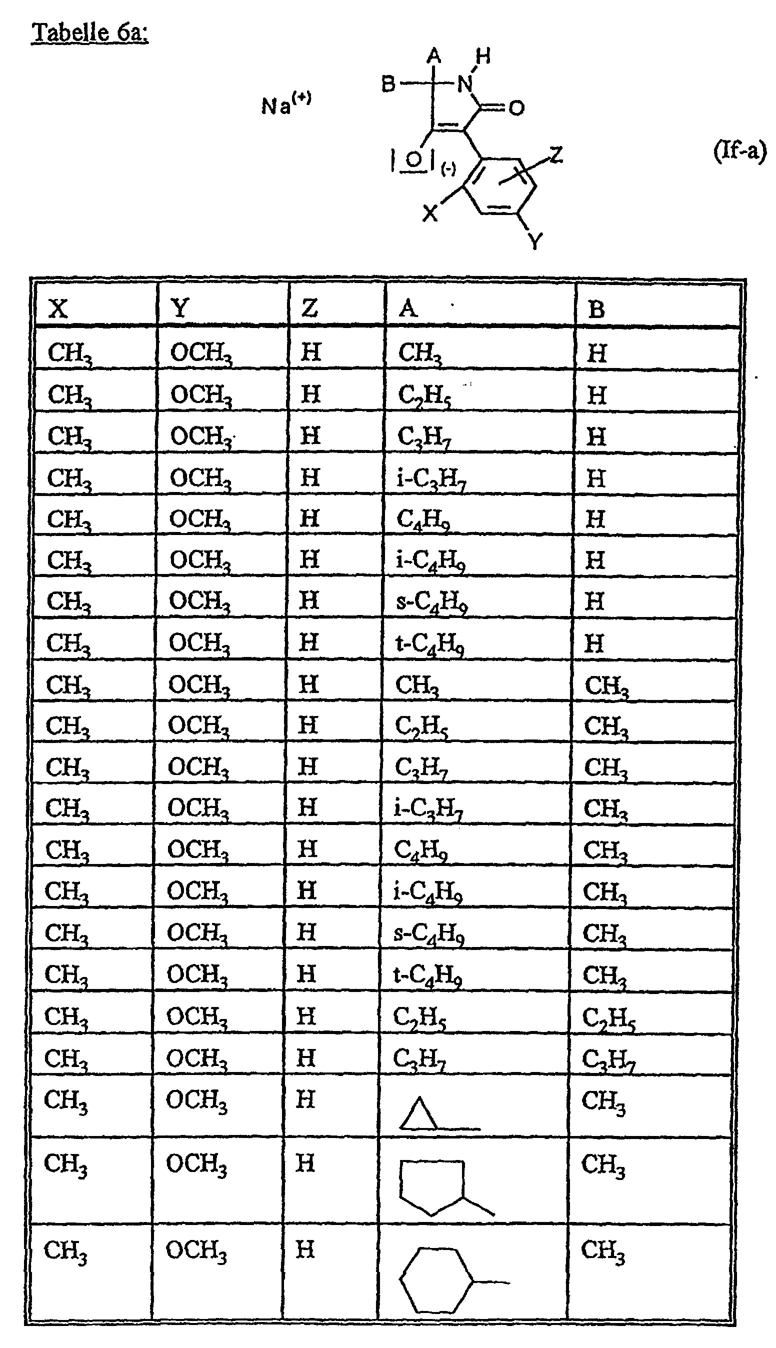

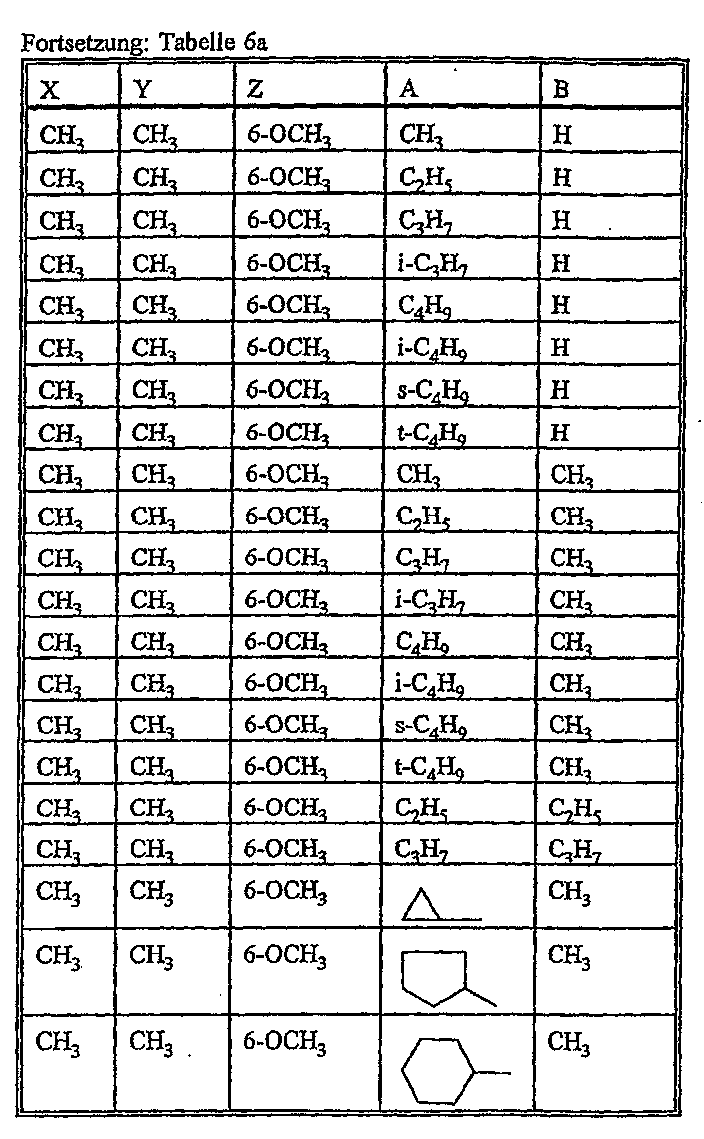

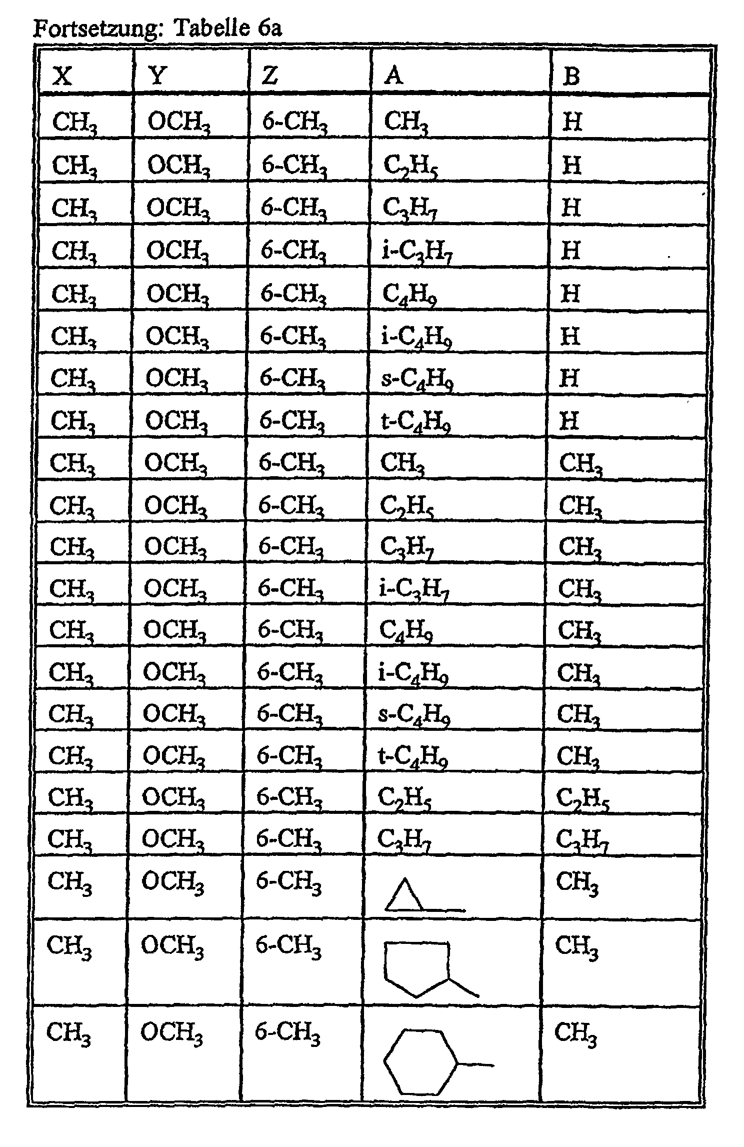

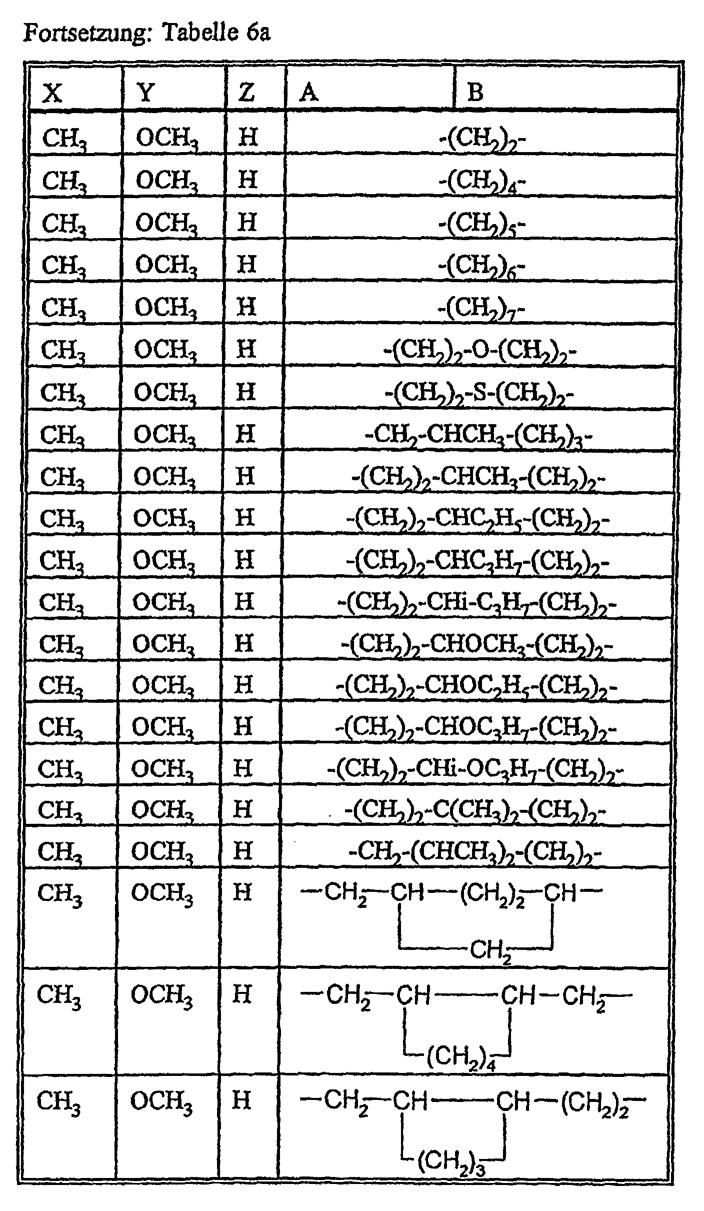

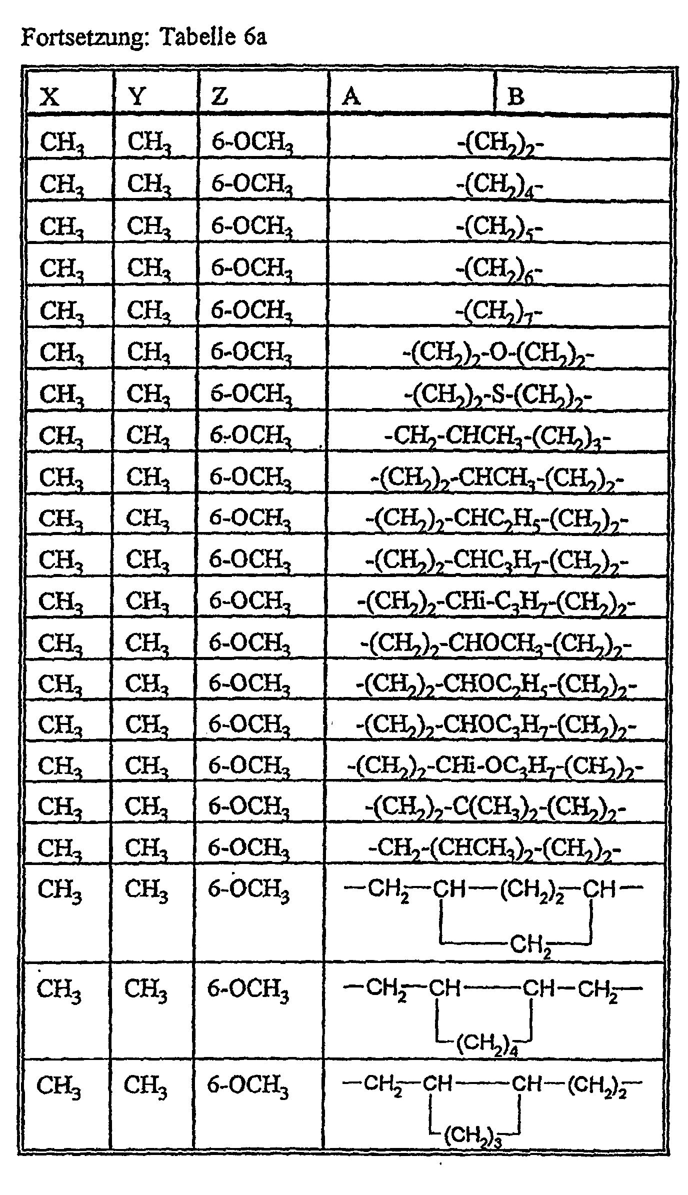

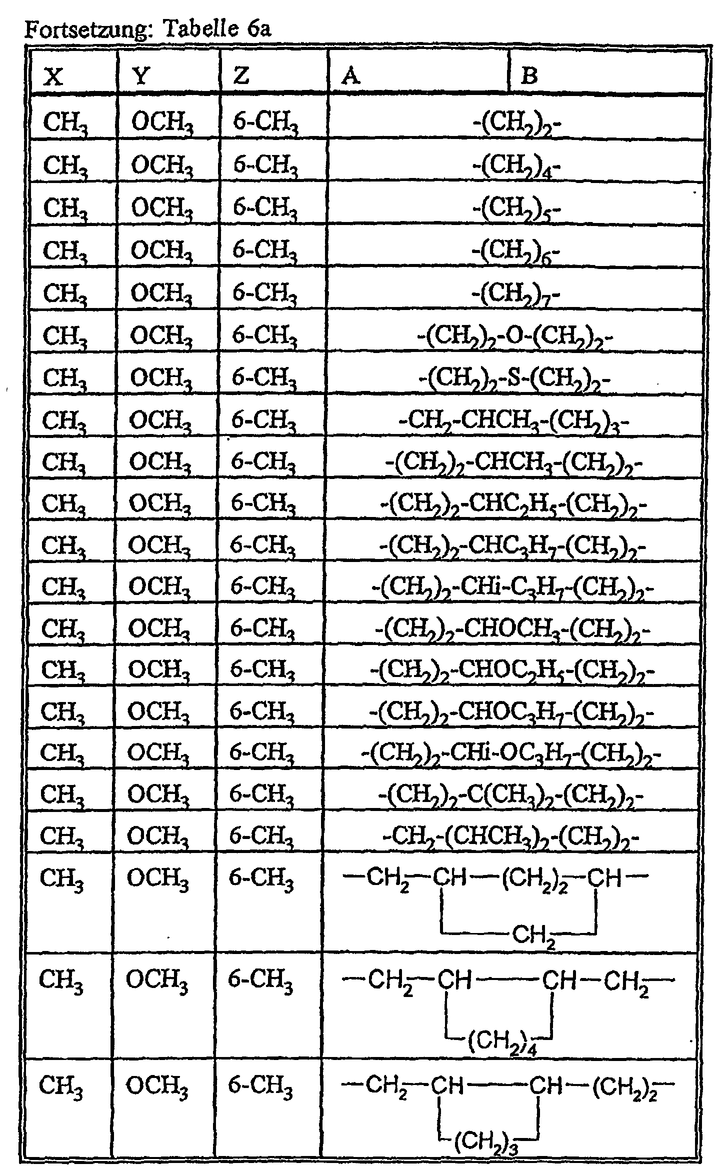

Im einzelnen seien außer den bei den Herstellungsbeispielen genannten Verbindungen die folgenden Verbindungen der Formel (If-b) genannt:

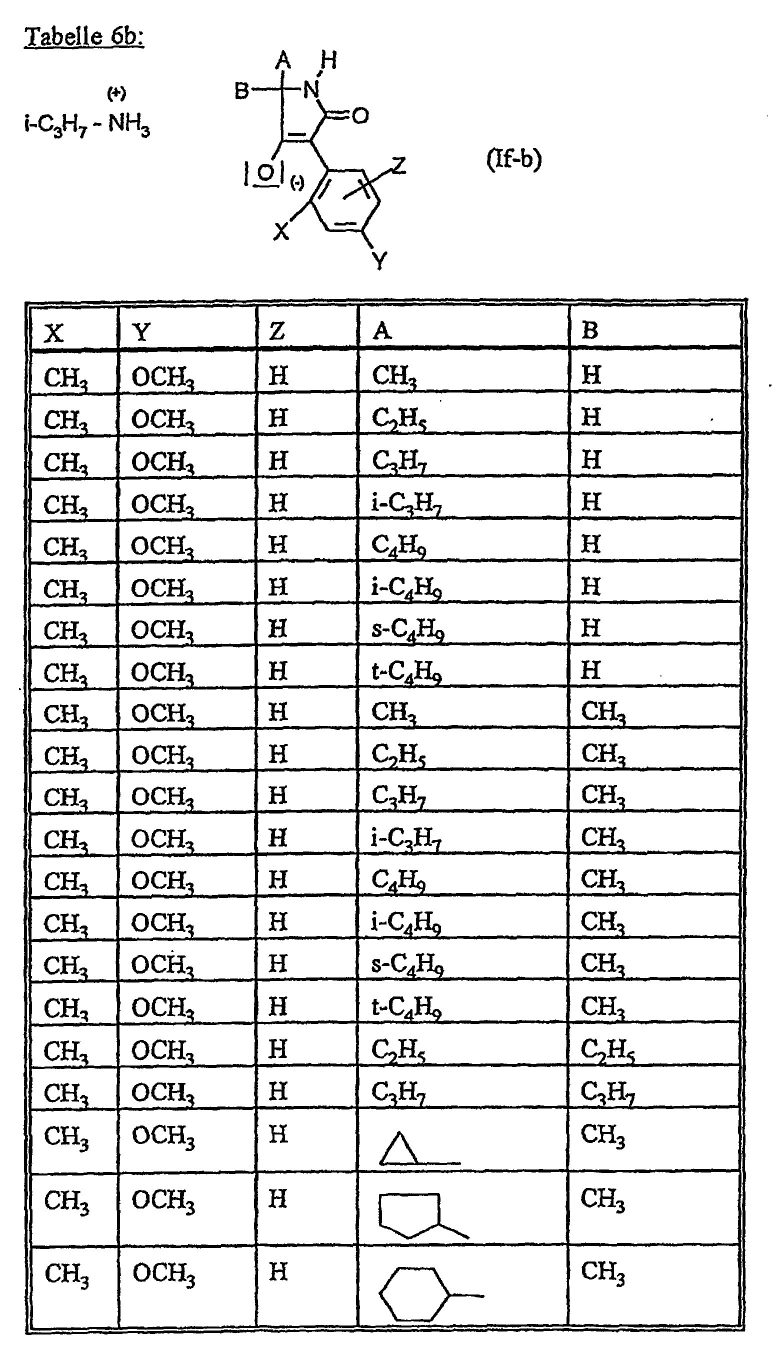

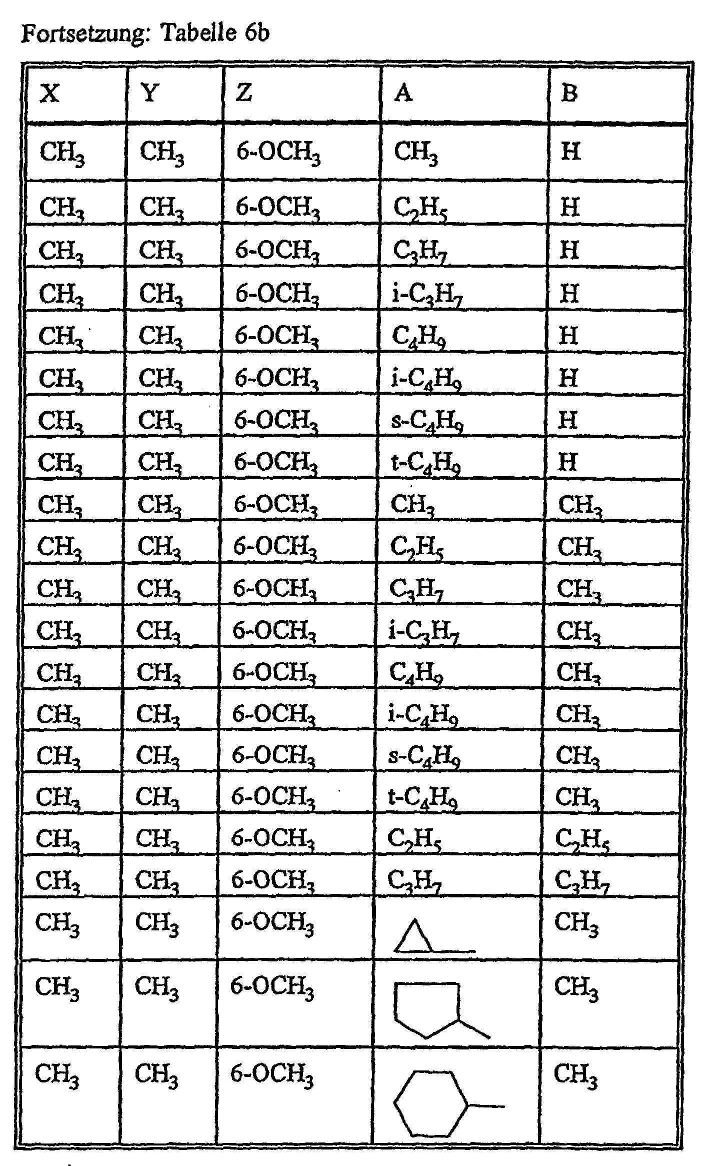

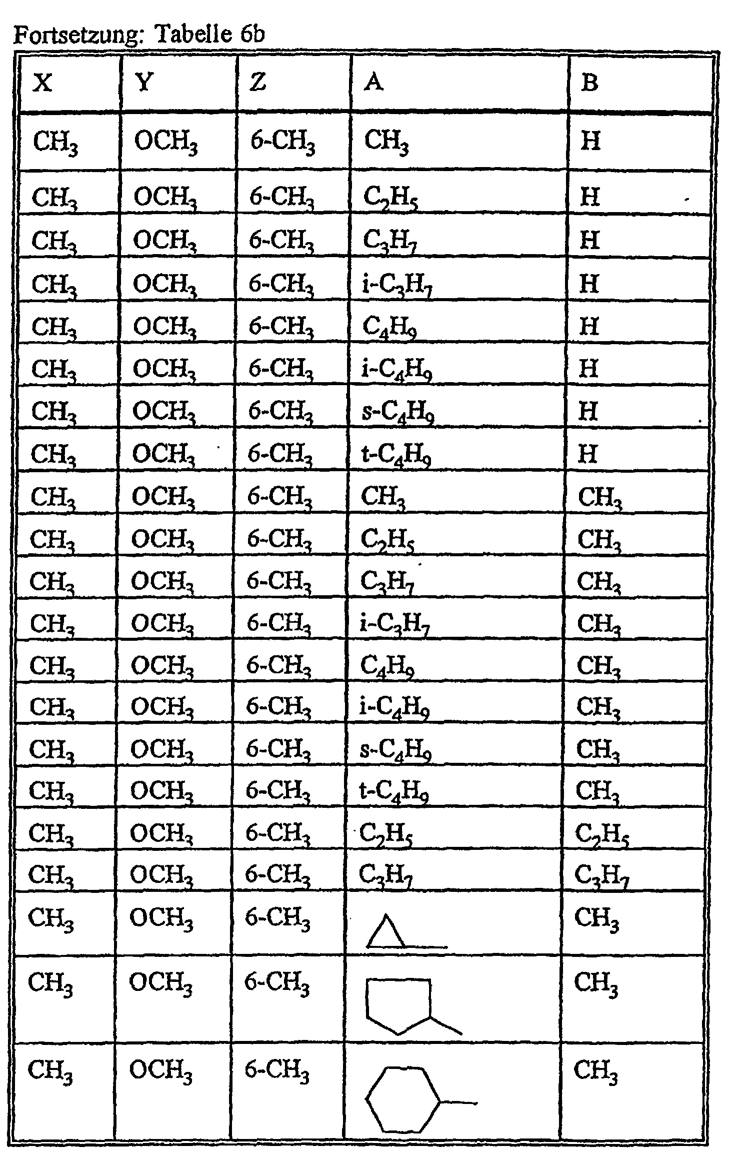

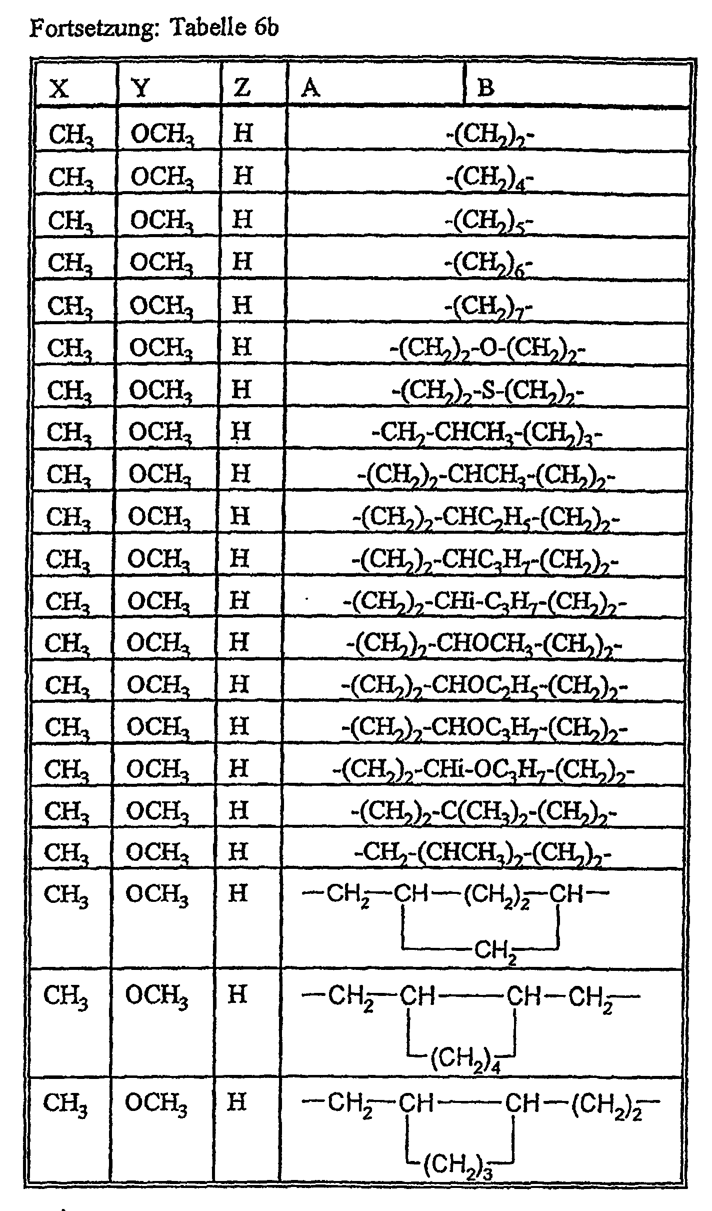

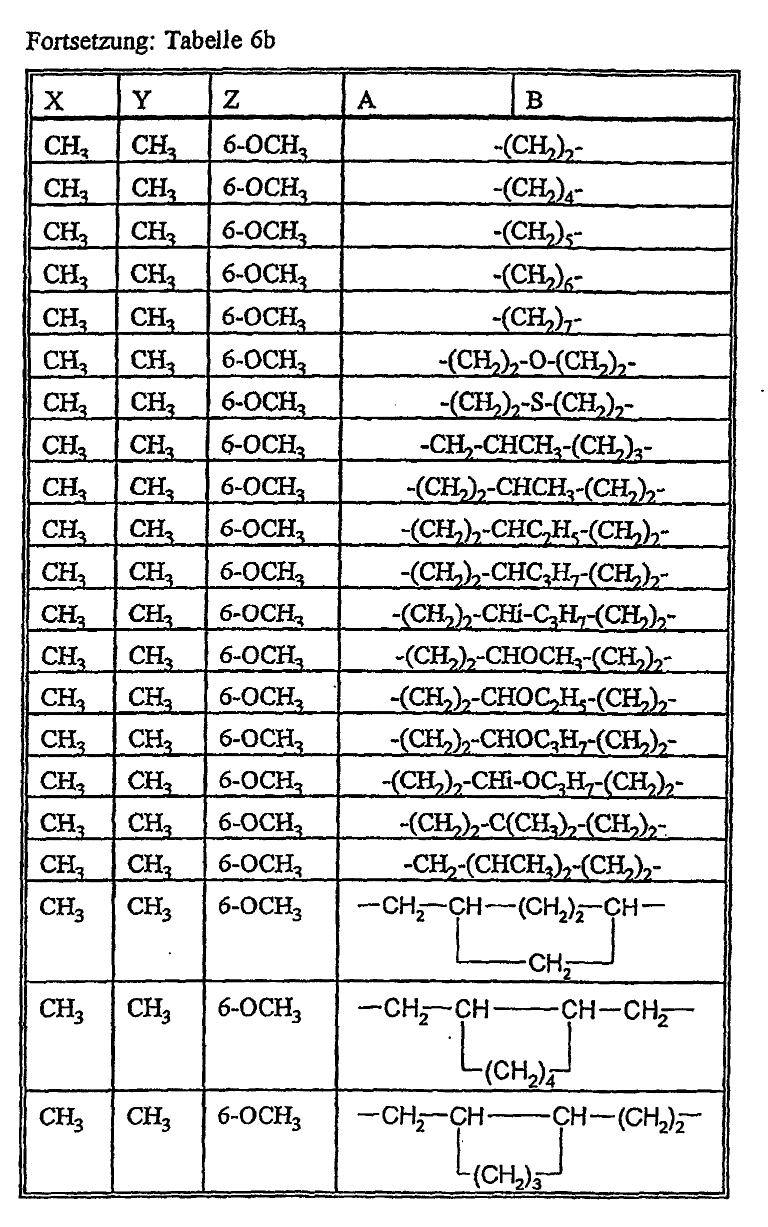

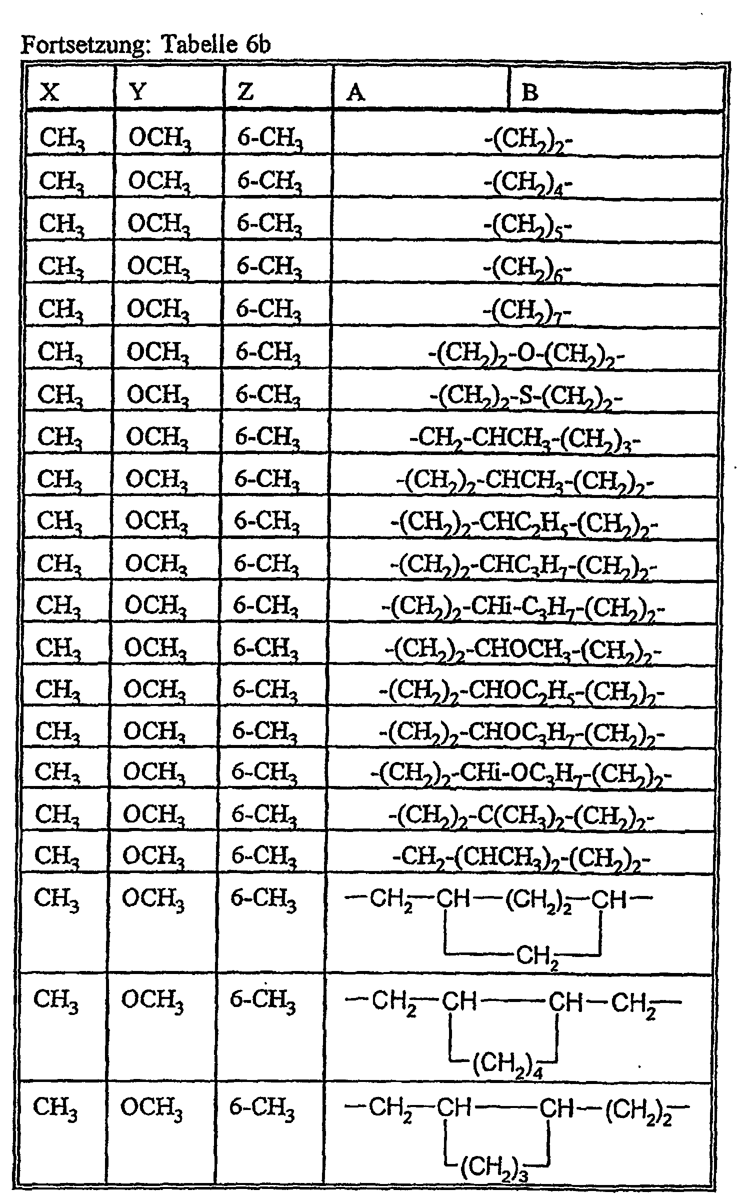

Im einzelnen seien außer den bei den Herstellungsbeispielen genannten Verbindungen die folgenden Verbindungen der Formel (Ig-a) genannt:

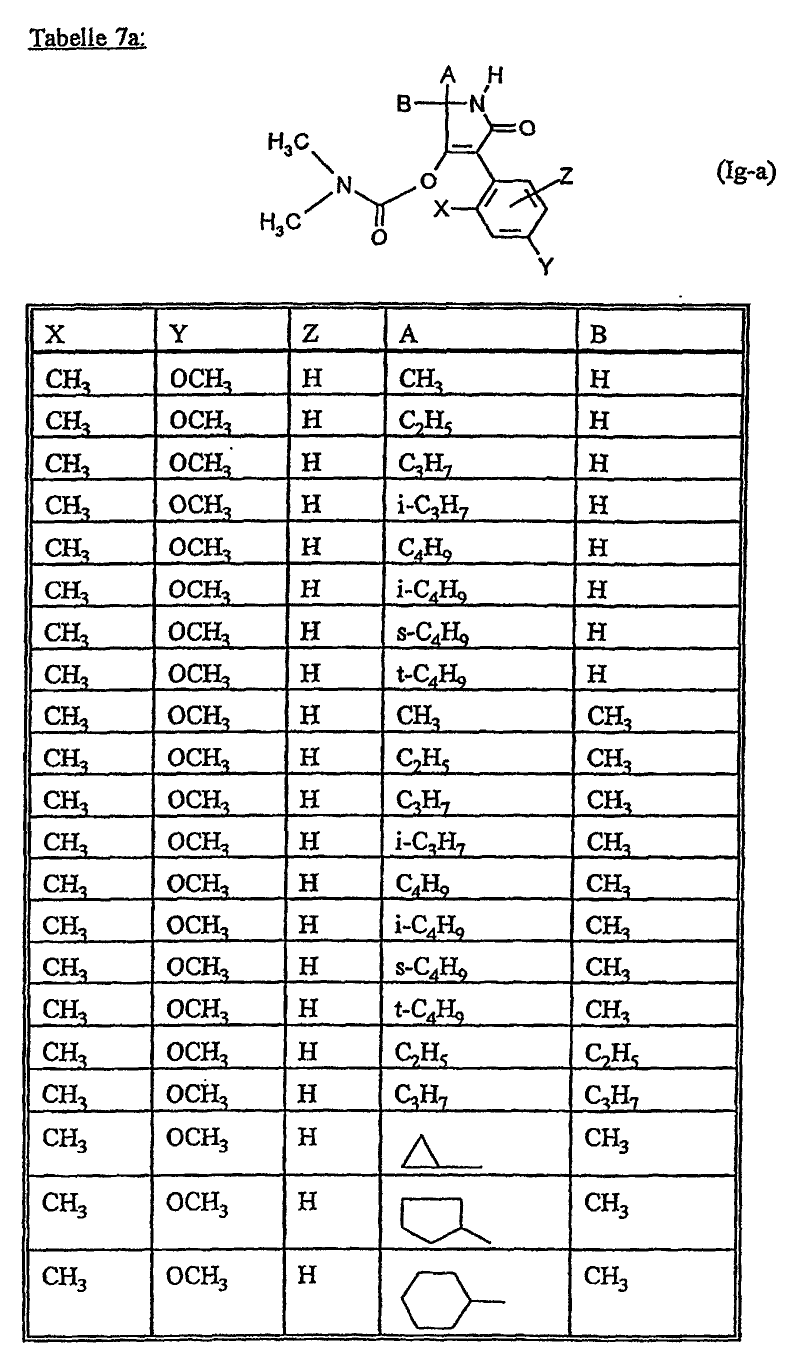

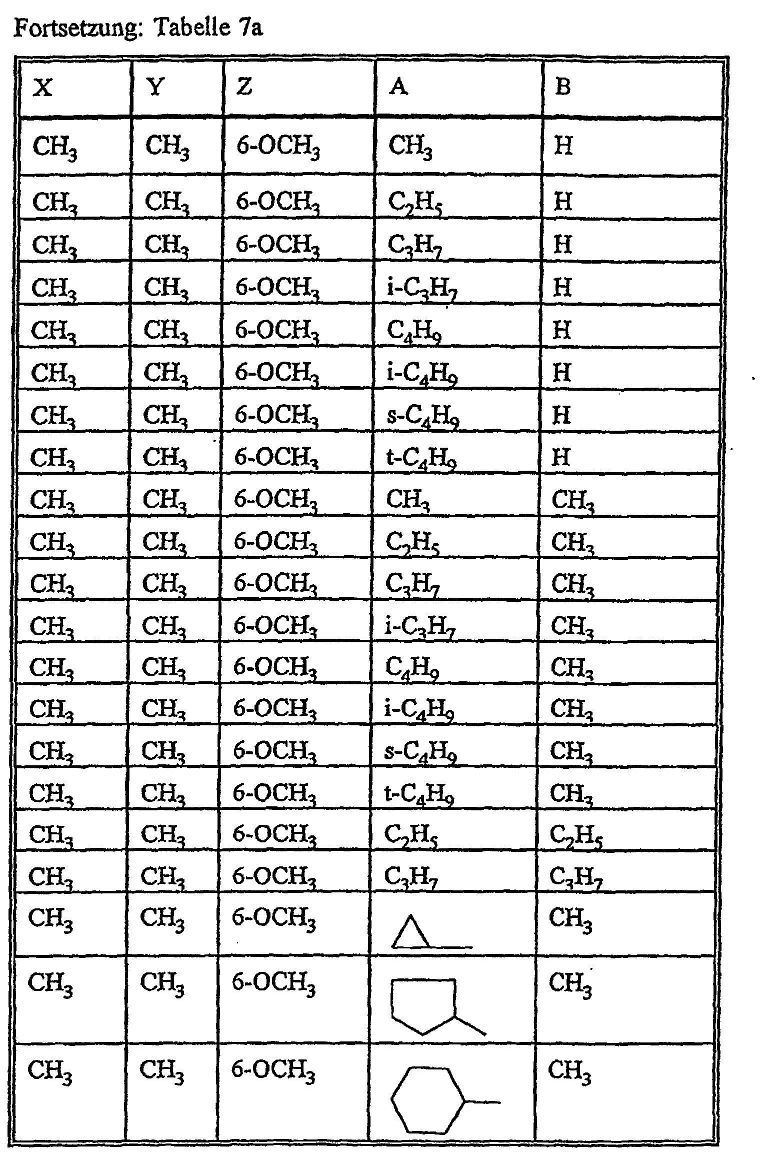

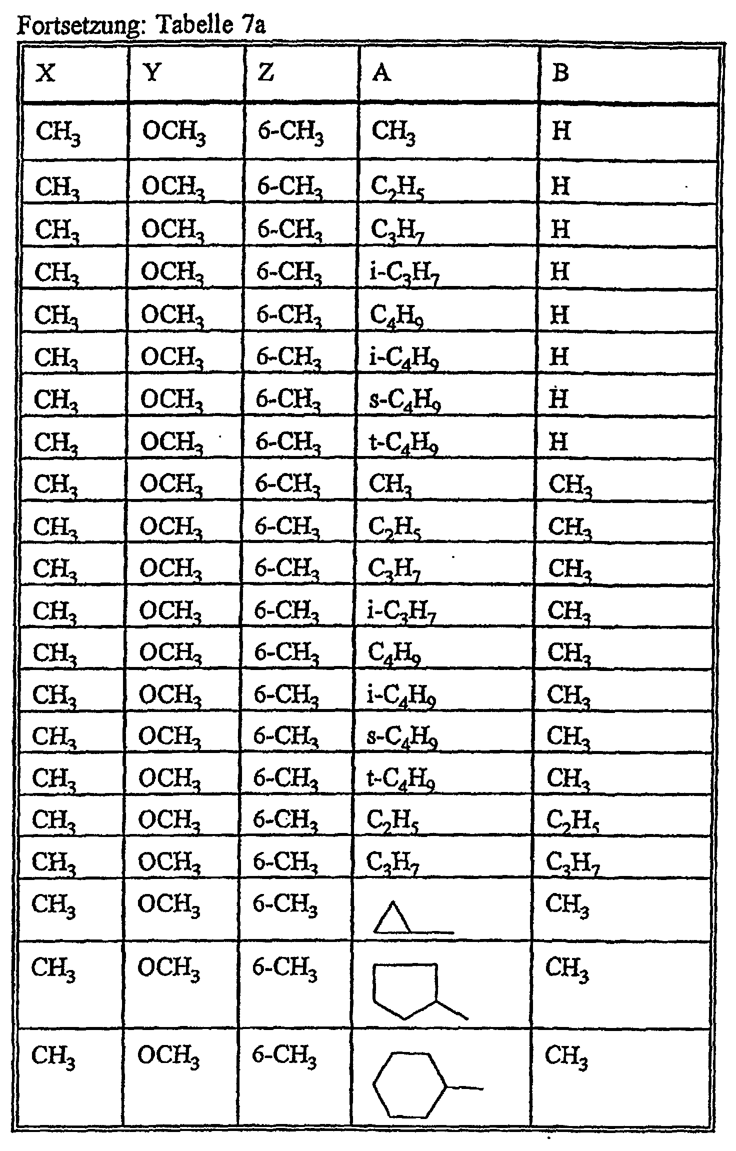

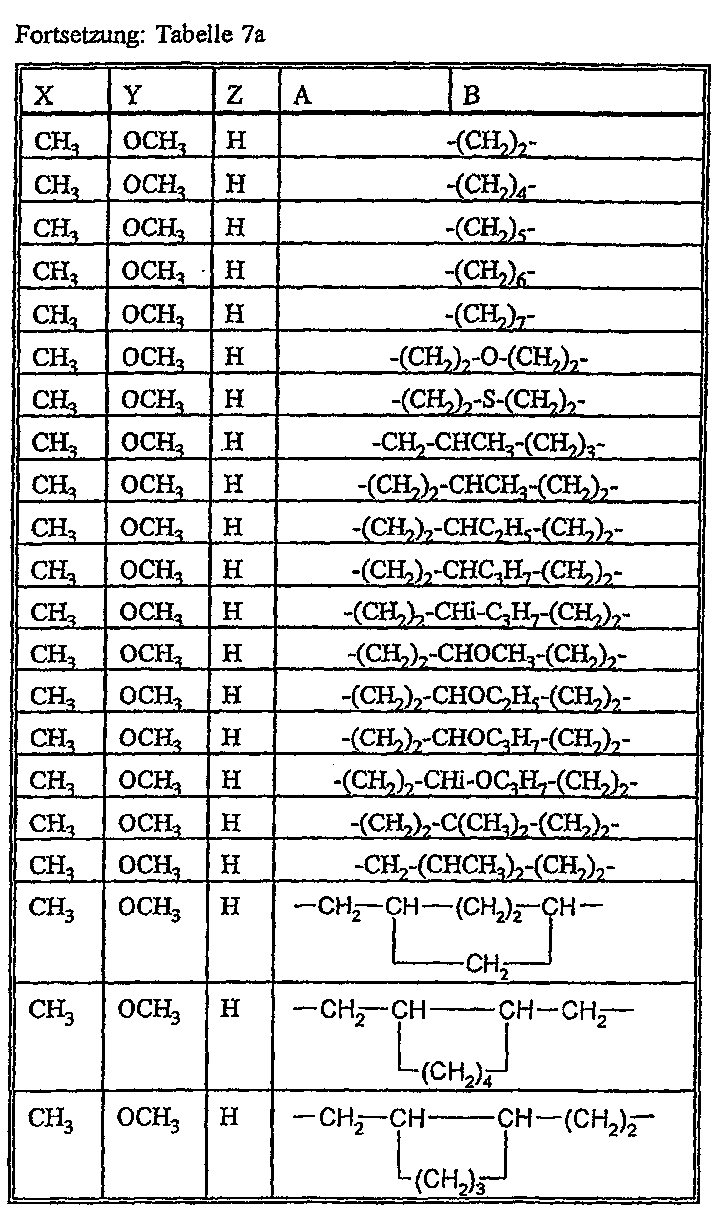

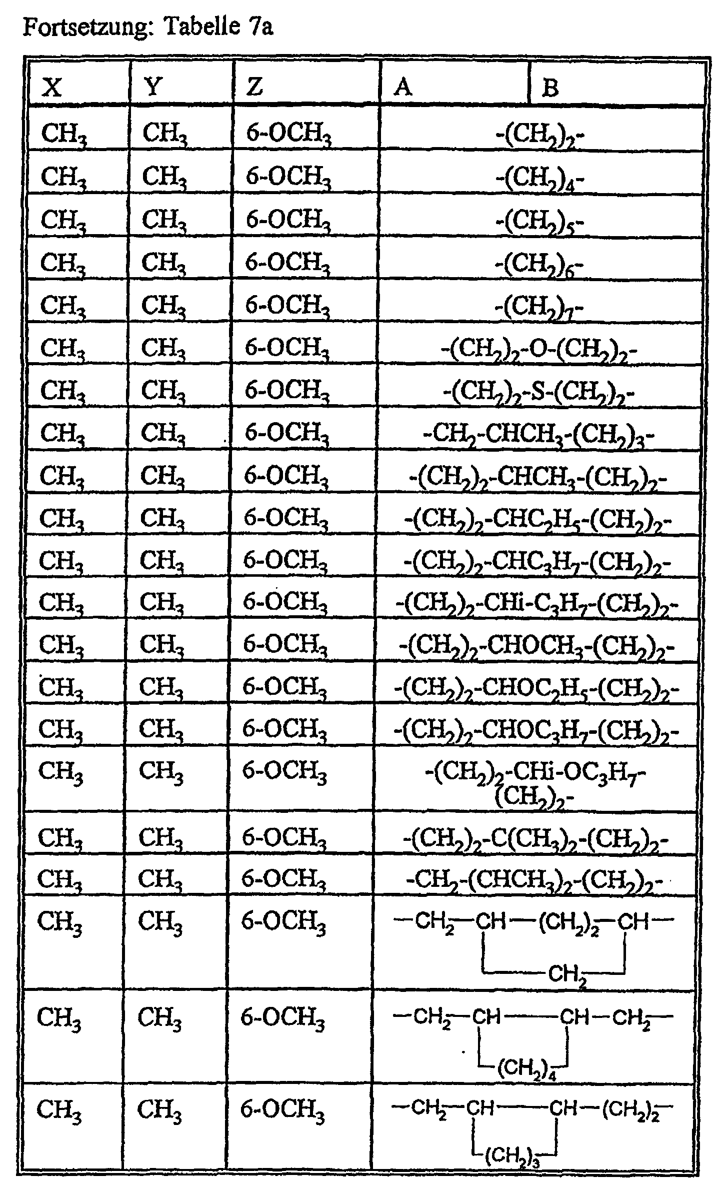

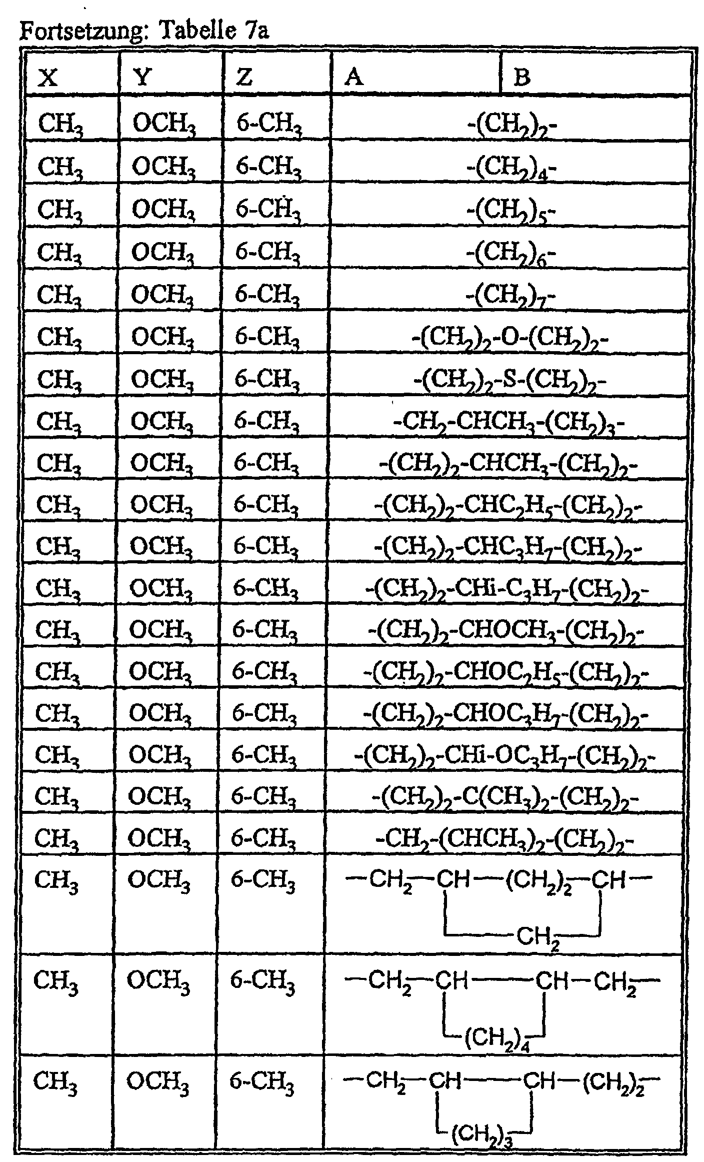

Im einzelnen seien außer den bei den Herstellungsbeispielen genannten Verbindungen die folgenden Verbindungen der Formel (Ig-b) genannt:

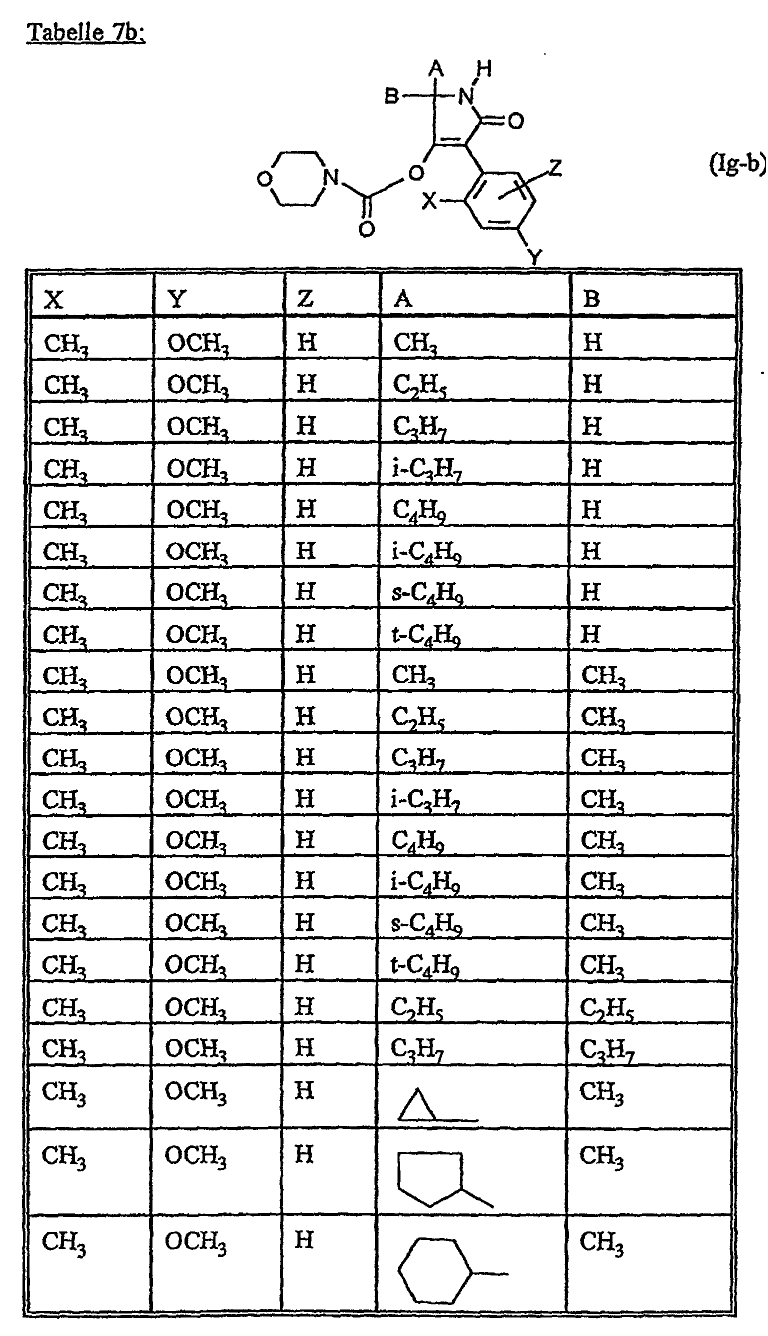

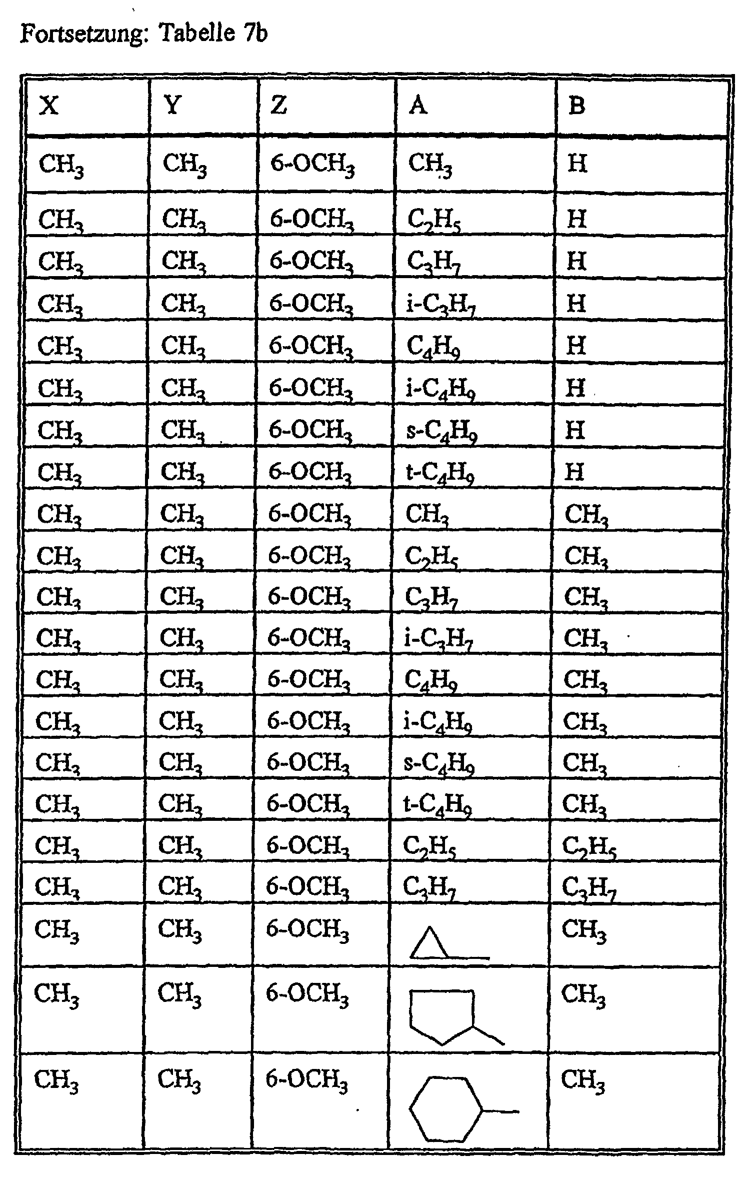

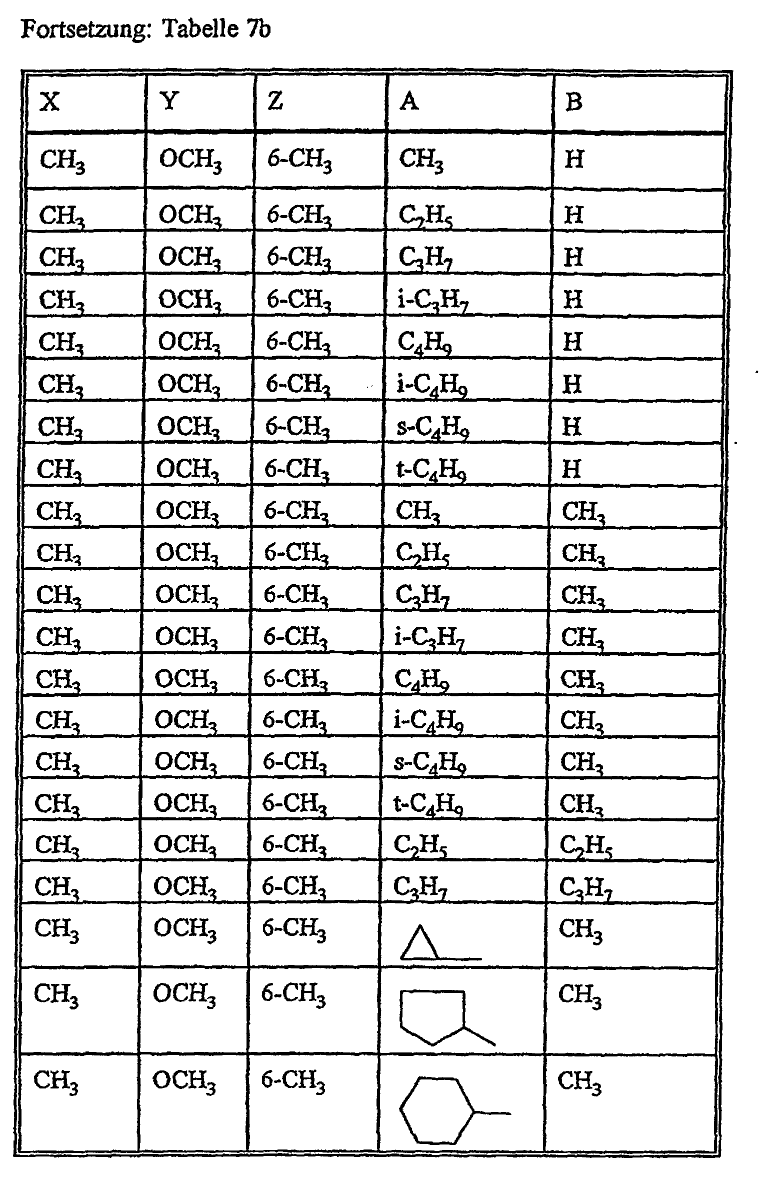

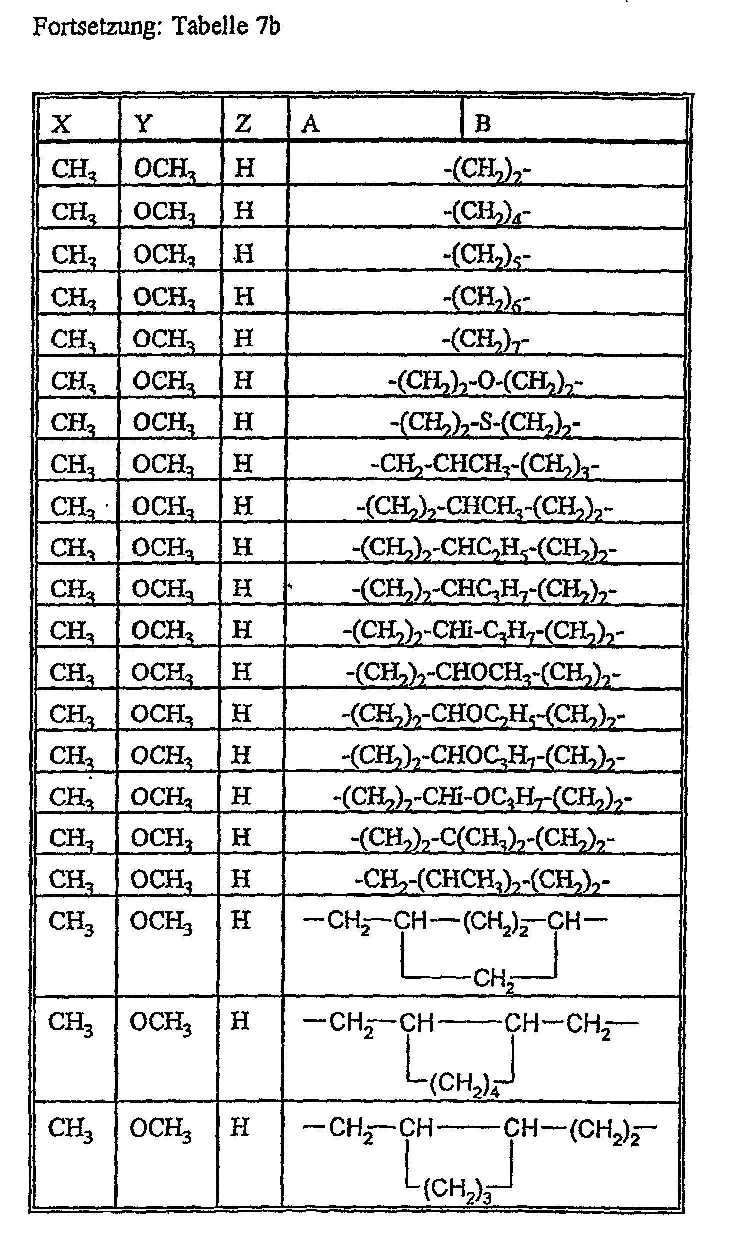

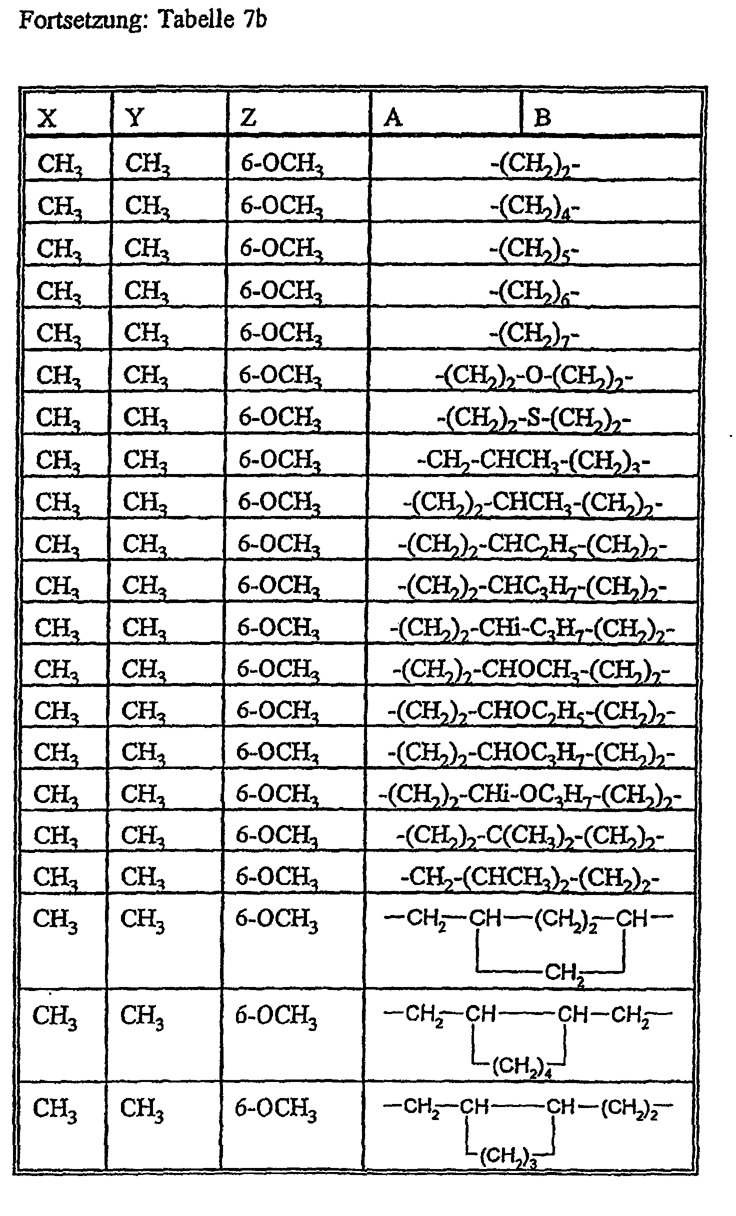

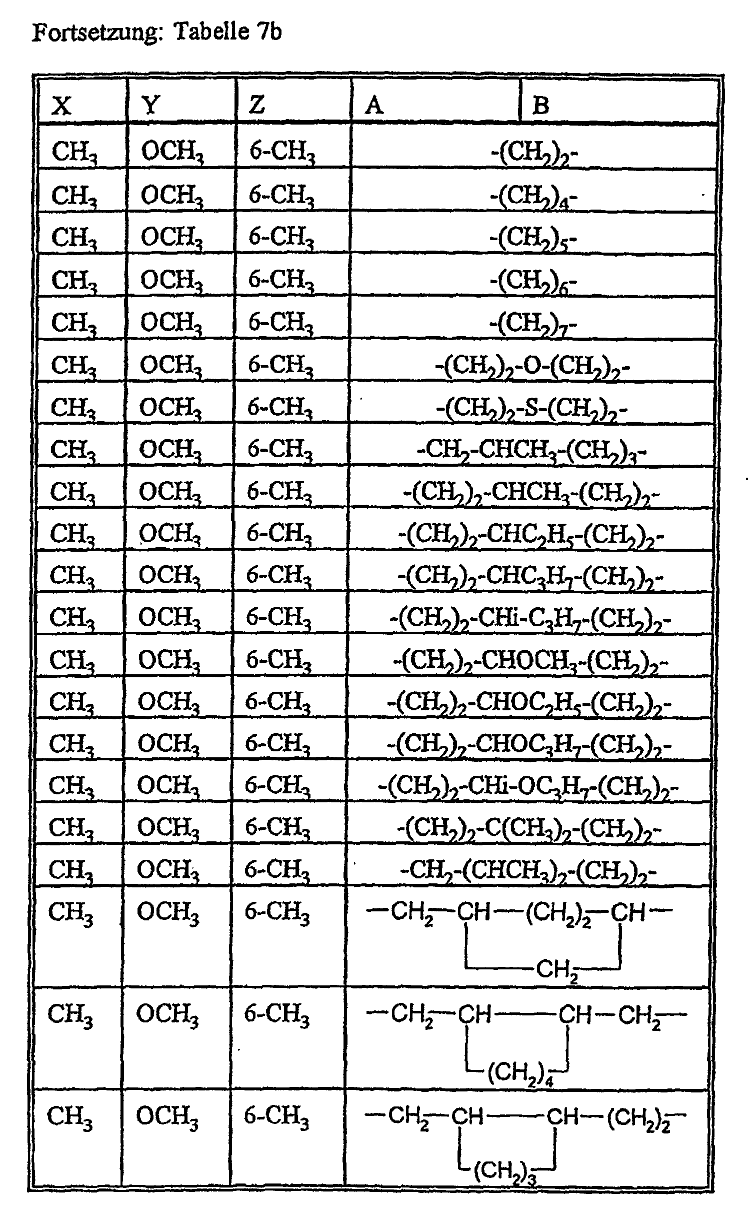

Verwendet man gemäß Verfahren (A) N-(2-Methyl-4-methoxyphenylacetyl)-1-amino-4-ethyl-cyclohexan-carbonsäureethylester als Ausgangsstoff, so kann der Verlauf des erfindungsgemäßen Verfahrens durch folgendes Reaktionsschema wiedergegeben werden:

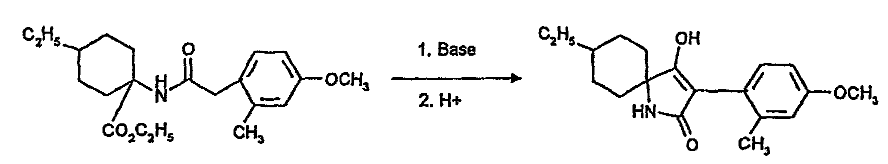

Verwendet man gemäß Verfahren (B_{α}) 3-(2-Methyl-6-methoxyphenyl)-5,5-dimethyl-pyrrolidin-2,4-dion und Pivaloylchlorid als Ausgangsstoffe, so kann der Verlauf des erfindungsgemäßen Verfahrens durch folgendes Reaktionsschema wiedergegeben werden:

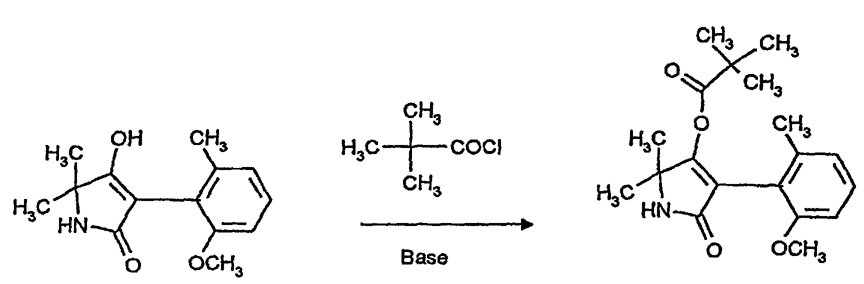

Verwendet man gemäß Verfahren (B_{β}) 3-(2,4-Dimethyl-6-methoxyphenyl)-5-isopropyl-5-methyl-pyrrolidin-2,4-dion und Acetanhydrid als Ausgangsverbindungen, so kann der Verlauf des erfindungsgemäßen Verfahrens durch folgendes Reaktionsschema wiedergegeben werden:

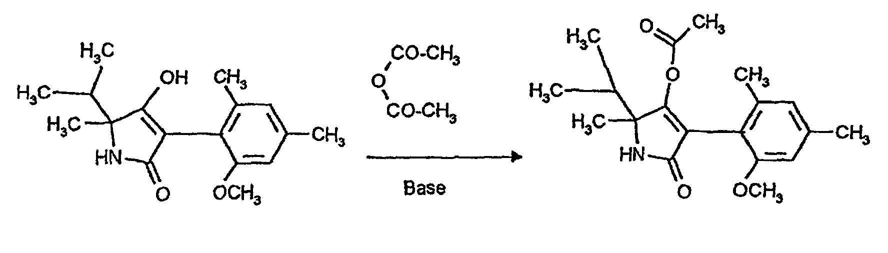

Verwendet man gemäß Verfahren (C) 3-(2-Methoxy-4-methyl-phenyl)-5,5-diethylpyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

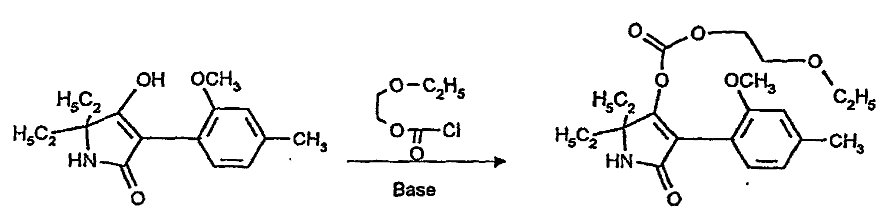

Verwendet man gemäß Verfahren (D_{α}) 3-(2,6-Dimethyl-4-methoxyphenyl)-5.5-pentamethylen-pyrrolidin-2,4-dion und Chlormonothioameisensäuremethylester als Ausgangsprodukte, so kann der Reaktionsverlauf wie folgt wiedergegeben werden:

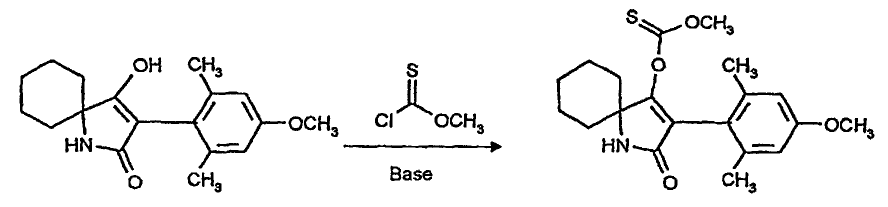

Verwendet man gemäß Verfahren (D_{β}) 3-(2-Methyl-4-ethoxy-phenyl)-5,5-ethylmercaptoethyl-pyrrolidin-2,4-dion, Schwefelkohlenstoff und Methyliodid als Ausgangskomponenten, so kann der Reaktionsverlauf wie folgt wiedergegeben werden:

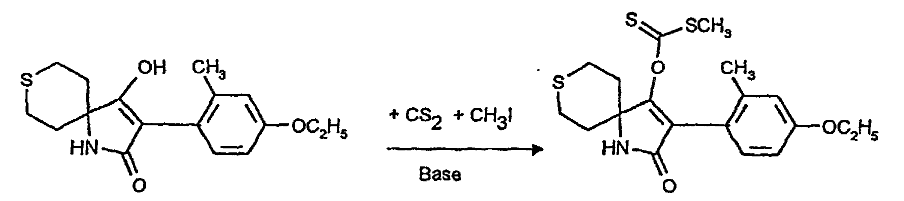

Verwendet man gemäß Verfahren (E) 3-(2-Methyl-4-isopropoxy-phenyl)-5.5-(2-methyl)-pentamethylen-pyrrolidin-2,4-dion und Methansulfonsäurechlorid als Ausgangsprodukt, so kann der Reaktionsverlauf durch folgendes Reaktionsschema wiedergegeben werden:

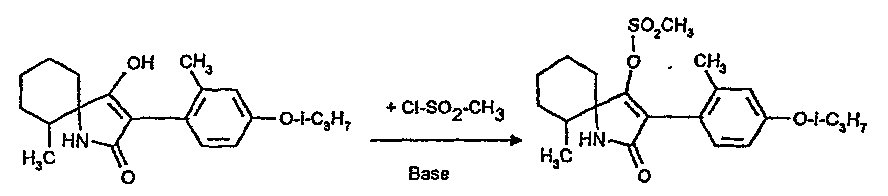

Verwendet man gemäß Verfahren (F) 3-(2-Methoxy-4-methylphenyl)-5-isobutyl-5-methyl-pyrrolidin-2,4-dion und Methanthio-phasphonsäurechlorid-(2,2,2-trifluorethylester) als Ausgangsprodukte, so kann der Reaktionsverlauf durch folgendes Reaktionsschema wiedergegeben werden:

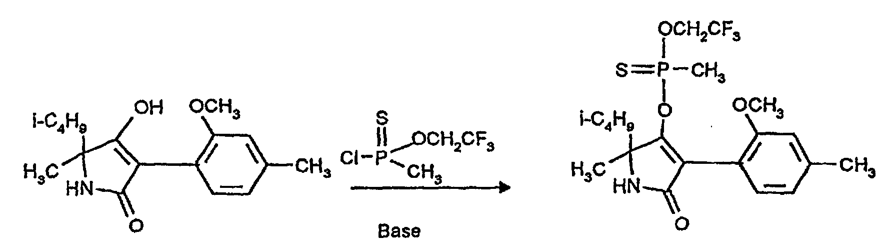

Verwendet man gemäß Verfahren (G) 3-(2-Methyl-4-methoxyphenyl)-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

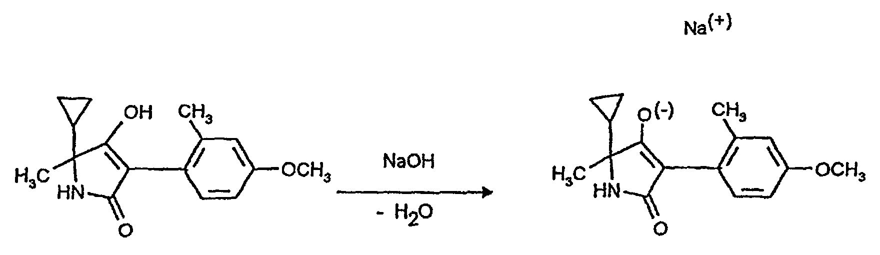

Verwendet man gemäß Verfahren (H_{α}) 3-(2-Methyl-4-ethoxyphenyl)-5,5-hexamethylen-pyrrolidin-2,4-dion und Ethylisocyanat als Ausgangsprodukte, so kann der Reaktionsverlauf durch folgendes Schema wiedergegeben werden:

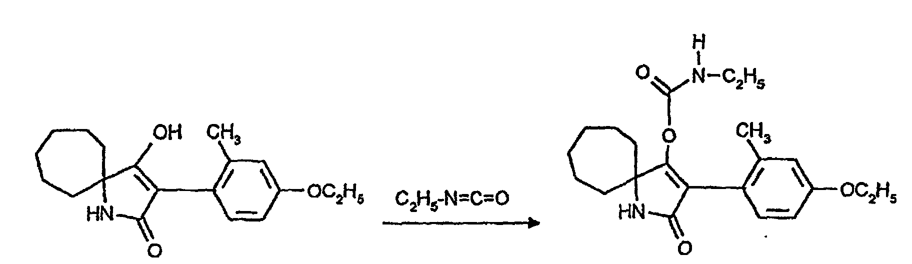

Verwendet man gemäß Verfahren (H_{β}) 3-(2-Methoxy-4-methylphenyl)-5-methylpyrrolidin-2,4-dion und Dimethylcarbamidsäurechlorid als Ausgangsprodukte, so kann der Reaktionsverlauf durch folgendes Schema wiedergegeben werden:

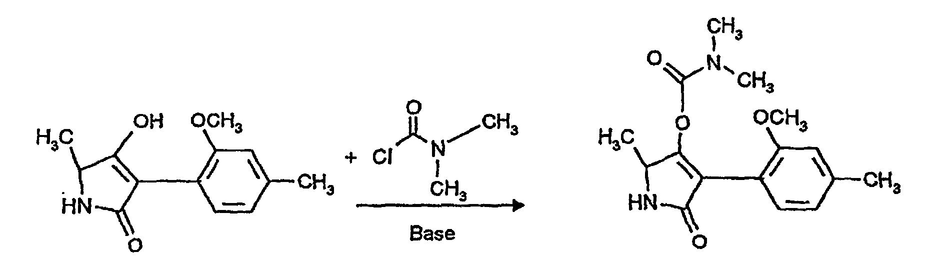

Die bei den erfindungsgemäßen Verfahren (A) als Ausgangsstoffe benötigten Verbindungen der Formel (II) 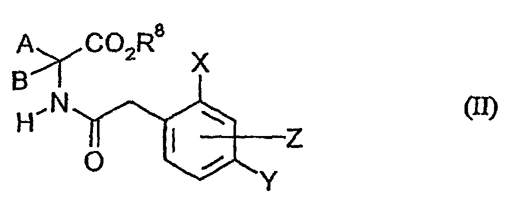 in welcher
- A, B, X, Y, Z und R⁸: die oben angegebene Bedeutung haben,
sind neu.

Man erhält z.B. Acyl-aminosäureester der Formel (II), wenn man Aminosäurederivate der Formel (XIV), 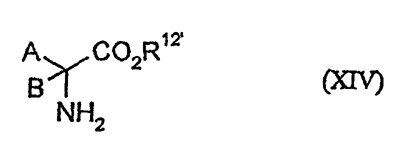 in welcher
- R^{12'}: für Wasserstoff (XIVa) oder Alkyl (XIVb) steht
und
- A und B: die oben angegebene Bedeutung haben,
mit Phenylessigsäurehalogeniden der Formel (XV) 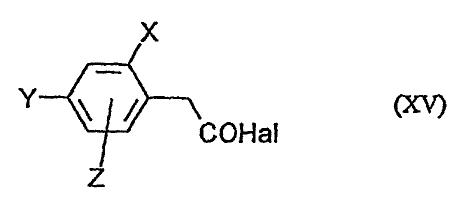 in welcher
- X, Y und Z: die oben angegebene Bedeutung haben und
- Hal: für Chlor oder Brom steht,
acyliert (Chem. Reviews 52, 237-416 (1953); Bhattacharya, Indian J. Chem. 6, 341-5, 1968)
oder wenn man Acylaminosäuren der Formel (IIa), 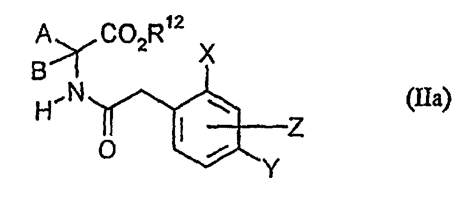 in welcher
- A, B, X, Y und Z: die oben angegebene Bedeutung haben,
und
- R¹²: für Wasserstoff steht,
verestert (Chem. Ind. (London) 1568 (1968)).

Wenn die Substituenten A und B einen Ring bilden, sind die resultierenden substituierten cyclischen Aminocarbonsäuren der Formel (XIVa) im allgemeinen nach der Bucherer-Bergs-Reaktion oder nach der Strecker-Synthese erhältlich und fallen dabei jeweils in unterschiedlichen Isomerenformen an. So erhält man nach den Bedingungen der Bucherer-Bergs-Reaktion vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 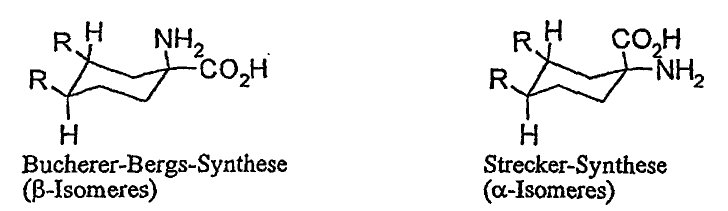 (L. Munday, J. Chem. Soc. 4372 (1961); J.T. Eward, C. Jitrangeri, Can. J. Chem. 53 3339 (1975).

Weiterhin lassen sich die bei dem obigen Verfahren (A) verwendeten Ausgangsstoffe der Formel (II) 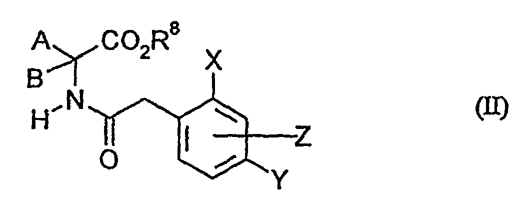 in welcher
- A, B, X_{,} Y, Z und R⁸: die oben angegebene Bedeutung haben,
herstellen, wenn man Aminonitrile der Formel (XVI) 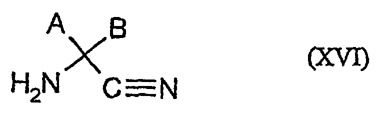 in welcher
- A und B: die oben angegebene Bedeutung haben,
mit Phenylessigsäurehalogeniden der Formel (XV) 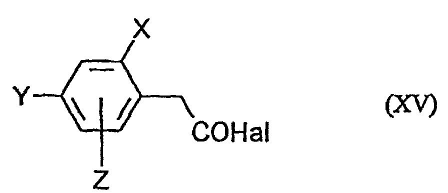 in welcher
- X, Y und Z: die oben angegebene Bedeutung haben und
- Hal: für Chlor oder Brom steht,
zu Verbindungen der Formel (XVII) 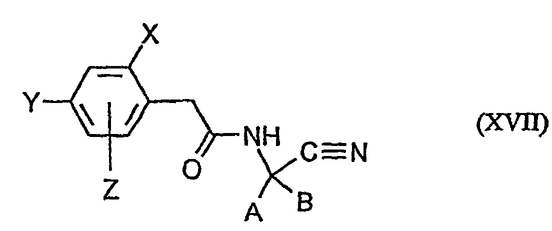 in welcher
- A, B, X, Y und Z: die oben angegebene Bedeutung haben,
umsetzt und diese anschließend einer schwefelsauren Alkoholyse unterwirft.

Die Verbindungen der Formel (XVII) sind ebenfalls neu.

Beispielhaft aber nicht begrenzend seien außer den bei den Herstellungsbeispielen genannten Zwischenprodukten die folgenden Verbindungen der Formel (II) genannt:
N-(2-Methyl-4-methoxyphenylacetyl)-alanin-methylester
N-(2-Methyl-4-methoxyphenylacetyl)-leucin-methylester
N-(2-Methyl-4-methoxyphenylacetyl)-isoleucin-methylester
N-(2-Methyl-4-methoxyphenylacetyl)-valin-methylester
N-(2-Methyl-4-methoxyphenylacetyl)-aminoisobuttersäure-methylester
N-(2-Methyl-4-methoxyphenylacetyl)-2-ethyl-2-aminobuttersäure-methylester
N-(2-Methyl-4-methoxyphenylacetyl)-2-methyl-2-aminovaleriansäure-methylester
N-(2-Methyl-4-methoxyphenylacetyl)-2,3-dimethyl-2-aminovaleriansäuremethylester
N-(2-Methyl-4-methoxyphenylacetyl)-1-amino-cyclopentancarbonsäure-methylester
N-(2-Methyl-4-methoxyphenylacetyl)-1-amino-cyclohexancarbonsäure-methylester
N-(2-Methyl-4-methoxyphenylacetyl)-1-amino-cycloheptancarbonsäure-methylester
N-(2-Methyl-4-methoxyphenylacetyl)-1-amino-cyclooktancarbonsäure-methylester
N-(2,6-Dimethyl-4-methoxyphenylacetyl)-alanin-methylester
N-(2,6-Dimethyl-4-methoxyphenylacetyl)-leucin-methylester
N-(2,6-Dimethyl-4-methoxyphenylacetyl)-isoleucin-methylester
N-(2,6-Dimethyl-4-methoxyphenylacetyl)-valin-methylester
N-(2,6-Dimethyl-4-methoxyphenylacetyl)-aminoisobuttersäure-methylester
N-(2,6-Dimethyl-4-methoxyphenylacetyl)-2-ethyl-2-aminobuttersäure-methylester
N-(2,6-Dimethyl-4-methoxyphenylacetyl)-2-methyl-2-aminovaleriansäure-methylester
N-(2,6-Dimethyl-4-methoxyphenylacetyl)-2,3-dimethyl-2-aminovaleriansäuremethylester
N-(2,6-Dimethyl-4-methoxyphenylacetyl)-1-amino-cyclopentancarbonsäure-methylester
N-(2,6-Dimethyl-4-methoxyphenylacetyl)-1-amino-cyclohexancarbonsäure-methylester
N-(2,6-Dimethyl-4-methoxyphenylacetyl)-1-amino-cycloheptancarbonsäure-methylester
N-(2,6-Dimethyl-4-methoxyphenylacetyl)-1-amino-cyclooktancarbonsäure-methylester
N-(2,4-Dimethyl-6-methoxyphenylacetyl)-alanin-methylester
N-(2,4-Dimethyl-6-methoxyphenylacetyl)-leucin-methylester
N-(2,4-Dimethyl-6-methoxyphenylacetyl)-isoleucin-methylester
N-(2,4-Dimethyl-6-methoxyphenylacetyl)-valin-methylester
N-(2,4-Dimethyl-6-methoxyphenylacetyl)-aminoisobuttersäure-methylester
N-(2,4-Dimethyl-6-methoxyphenylacetyl)-2-ethyl-2-aminobuttersäure-methylester
N-(2,4-Dimethyl-6-methoxyphenylacetyl)-2-methyl-2-aminovaleriansäure-methylester
N-(2,4-Dimethyl-6-methoxyphenylacetyl)-2,3-dimethyl-2-aminovaleriansäuremethylester
N-(2,4-Dimethyl-6-methoxyphenylacetyl)-1-amino-cyclopentancarbonsäure-methylester
N-(2,4-Dimethyl-6-methoxyphenylacetyl)-1-amino-cyclohexancarbonsäure-methylester
N-(2,4-Dimethyl-6-methoxyphenylacetyl)-1-amino-cycloheptancarbonsäure-methylester
N-(2,4-Dimethyl-6-methoxyphenylacetyl)-1-amino-cyclooktancarbonsäure-methylester
N-(2-Methyl-4-methoxy-phenylacetyl)-1-amino-2-methyl-cyclohexancarbonsäure-methylester,
N-(2-Methyl-4-methoxy-phenylacetyl)-1-amino-3-methyl-cyclobexancarbonsäure-methylester,
N-(2-Methyl-4-methoxy-phenylacetyl)-1-amino-4-methyl-cyclohexancarbonsäure-methylester,
N-(2-Methyl-4-methoxy-phenylacetyl)-1-amino-3,4-dimethyl-cyclohexancarbonsäure-methylester,
N-(2-Methyl-4-methoxy-phenylacetyl)-1-amino-4-ethyl-cyclohexancarbonsäure-methylester,
N-(2-Methyl-4-methoxy-phenylacetyl)-1-amino-4-isopropyl-cyclohexancarbonsäure-methylester,
N-(2-Methyl-4-methoxy-phenylacetyl)-1-amino-4-tert.-butyl-cyclohexancarbonsäure-methylester,
N-(2-Methyl-4-methoxy-phenylacetyl)-1-amino-4-methoxy-cyclohexancarbonsäure-methylester,
N-(2,4-Dimethyl-6-methoxy-phenylacetyl)-1-amino-2-methyl-cyclohexancarbonsäure-methylester,
N-(2,4-Dimethyl-6-methoxy-phenylacetyl)-1-amino-3-methyl-cyclohexancarbonsäure-methylester,
N-(2,4-Dimethyl-6-methoxy-phenylacetyl)-1-amino-4-methyl-cyclohexancarbonsäure-methylester,
N-(2,4-Dimethyl-6-methoxy-phenylacetyl)-1-amino-3,4-dimethyl-cyclohexancarbonsäure-methylester,
N-(2,4-Dimethyl-6-methoxy-phenylacetyl)-1-amino-4-ethyl-cyclohexancarbonsäure-methylester,
N-(2,4-Dimethyl-6-methoxy-phenylacetyl)-1-amino-4-isopropyl-cyclohexancarbonsäure-methylester,
N-(2,4-Dimethyl-6-methoxy-phenylacetyl)-1-amino-4-tert.-butyl-cyclohexancarbonsäure-methylester,
N-(2,4-Dimethyl-6-methoxy-phenylacetyl)-1-amino-4-methoxy-cyclohexancarbonsäure-methylester,
N-(2,6-Dimethyl-4-methoxy-phenylacetyl)-1-amino-2-methyl-cyclohexancarbonsäure-methylester,
N-(2,6-Dimethyl-4-methoxy-phenylacetyl)-1-amino-3-methyl-cyclohexancarbonsäure-methylester,
N-(2,6-Dimethyl-4-methoxy-phenylacetyl)-1-amino-4-methyl-cyclohexancarbonsäure-methylester,
N-(2,6-Dimethyl-4-methoxy-phenylacetyl)-1-amino-3,4-dimethyl-cyclohexancarbonsäure-methylester,
N-(2,6-Dimethyl-4-methoxy-phenylacetyl)-1-amino-4-ethyl-cyclohexancarbonsäuremethylester,
N-(2,6-Dimethyl-4-methoxy-phenylacetyl)-1-amino-4-isopropyl-cyclohexancarbonsäure-methylester,
N-(2,6-Dimethyl-4-methoxy-phenylacetyl)-1-amino-4-tert.-butyl-cyclohexancarbonsäure-methylester,
N-(4-Chlor-2-methoxy-phenylacetyl)-1-amino-4-methoxy-cyclohexancarbonsäure-methylester,

Beispielhaft, aber nicht begrenzend, seien außer den bei den Herstellungsbeispielen genannten Zwischenprodukten die folgenden Verbindungen der Formel (IIa) genannt:
N-(2-Methyl-4-methoxyphenylacetyl)-alanin
N-(2-Methyl-4-methoxyphenylacetyl)-leucin
N-(2-Methyl-4-methoxyphenylacetyl)-isoleucin
N-(2-Methyl-4-methoxyphenylacetyl)-valin
N-(2-Methyl-4-methoxyphenylacetyl)-aminoisobuttersäure
N-(2-Methyl-4-methoxyphenylacetyl)-2-ethyl-2-aminobuttersäure
N-(2-Methyl-4-methoxyphenylacetyl)-2-methyl-2-aminovaleriansäure
N-(2-Methyl-4-methoxyphenylacetyl)-2,3-dimethyl-2-aminovaleriansäure
N-(2-Methyl-4-methoxyphenylacetyl)-1-amino-cyclopentancarbonsäure
N-(2-Methyl-4-methoxyphenylacetyl)-1-amino-cyclohexancarbonsäure
N-(2-Methyl-4-methoxyphenylacetyl)-1-amino-cycloheptancarbonsäure
N-(2-Methyl-4-methoxyphenylacetyl)-1-amino-cyclooktancarbonsäure
N-(Methyl-2-methoxyphenylacetyl)-alanin
N-(2,6-Dimethyl-4-methoxyphenylacetyl)-leucin
N-(2,6-Dimethyl-4-2-methoxyphenylacetyl)-isoleucin
N-(2,6-Dimethyl-4-methoxyphenylacetyl)-valin
N-(2,6-Dimethyl-4-methoxyphenylacetyl)-aminoisobuttersäure
N-(2,6-Dimethyl-4-methoxyphenylacetyl)-2-ethyl-2-aminobuttersäure
N-(2,6-Dimethyl-4-methoxyphenylacetyl)-2-methyl-2-aminovaleriansäure
N-(2,6-Dimethyl-4-methoxyphenylacetyl)-2,3-dimethyl-2-aminovaleriansäure
N-(2,6-Dimethyl-4-methoxyphenylacetyl)-1-amino-cyclopentancarbonsäure
N-(2,6-Dimethyl-4-methoxyphenylacetyl)-1-amino-cyclohexancarbonsäure
N-(2,6-Dimethyl-4-methoxyphenylacetyl)-1-amino-cycloheptancarbonsäure
N-(2,6-Dimethyl-4-methoxyphenylacetyl)-1-amino-cyclooktancarbonsäure
N-(2,4-Dimethyl-6-methoxyphenylacetyl)-alanin
N-(2,4-Dimethyl-6-methoxyphenylacetyl)-leucin
N-(2,4-Dimethyl-6-methoxyphenylacetyl)-isoleucin
N-(2,4-Dimethyl-6-methoxyghenylacetyl)-valin
N-(2,4-Dimethyl-6-methoxyphenylacetyl)-aminoisobuttersäure
N-(2,4-Dimethyl-6-methoxyphenylacetyl)-2-ethyl-2-aminobuttersäure
N-(2,4-Dimethyl-6-methoxyphenylacetyl)-2-methyl-2-aminovateriansäure
N-(2,4-Dimethyl-6-methoxyphenylacetyl)-2,3-dimethyl-2-aminovaleriansäure
N-(2,4-Dimethyl-6-methoxyphenylacetyl)-1-amino-cyclopentancarbonsäure
N-(2,4-Dimethyl-6-methoxyphenylacetyl)-1-amino-cyclohexancarbonsäure
N-(2,4-Dimethyl-6-mathoxyphenylacetyl)-1-amino-cycloheptancarbonsäure
N-(2,4-Dimethyl-6-methoxyphenylacetyl)-1-amino-cyclooktancarbonsäure
N-(2-Methyl-4-methoxy-phenylacetyl)-1-amino-2-methyl-cyclohexancarbonsäure
N-(2-Methyl-4-methoxy-phenylacetyl)-1-amino-3-methyl-cyclohexancarbonsäure
N-(2-Methyl-4-methoxy-phenylacetyl)-1-amino-4-methyl-cyclohexancarbonsäure
N-(2-Methyl-4-methoxy-phenylacetyl)-1-amino-3,4-dimethyl-cyclohexancarbonsäure
N-(2-Methyl-4-methoxy-phenylacetyl)-1-amino-4-ethyl-cyclohexancarbonsäure
N-(2-Methyl-4-methoxy-phenylacetyl)-1-amino-4-isopropyl-cyclohexancaräonsäure
N-(2-Methyl-4-methoxy-phenylacetyl)-1-amino-4-tert.-butyl-cyclohexancarbonsäure
N-(2-Methyl-4-methoxy-phenylacetyl)-1-amino-4-methoxy-cyclohexancarbonsäure
N-(2,6-Dimethyl-4-methoxy-phenylacetyl)-1-amino-2-methyl-cyclohexancarbonsäure
N-(2,6-Dimethyl-4-methoxy-phenylacetyl)-1-amino-3-methyl-cyclohexancarbonsäure
N-(2,6-Dimethyl-4-methoxy-phenylacetyl)-1-amino-4-methyl-cyclohexancarbonsäure
N-(2,6-Dimethyl-4-methoxy-phenylacetyl)-1-amino-3,4-dimethyl-cyclohexancarbonsäure
N-(2,6-Dimethyl-4-methoxy-phenylacetyl)-1-amino-4-ethyl-cyclohexancarbonsäure
N-(2,6-Dimethyl-4-methoxy-phenylacetyl)-1-amino-4-isopropyl-cyclohexancarbonsäure
N-(2,6-Dimethyl-4-methoxy-phenylacetyl)-1-amino-4-tert.-butyl-cyclohexancarbonsäure
N-(2,6-Dimethyl-4-methoxy-phenylacetyl)-1-amino-4-methoxy-cyclohexancarbonsäure
N-(2,4-Dimethyl-6-methoxy-phenylacetyl)-1-amino-2-methyl-cyclohexancarbonsäure
N-(2,4-Dimethyl-6-methoxy-phenylacetyl)-1-amino-3-methyl-cyclohexancarbonsäure
N-(2,4-Dimethyl-6-methoxy-phenylacetyl)-1-amino-4-methyl-cyclohexancarbonsäure
N-(2,4-Dimethyl-6-methoxy-phenylacetyl)-1-amino-3,4-dimethyl-cyclohexancarbonsäure
N-(2,4-Dimethyl-6-methoxy-phenylacetyl)-1-amino-4-ethyl-cyclohexancarbonsäure
N-(2,4-Dimethyl-6-methoxy-phenylacetyl)-1-amino-4-isopropyl-cyclohexancarbonsäure
N-(2,4-Dimethyl-6-methoxy-phenylacetyl)-1-amino-4-tert.-butyl-cyclohexancarbonsäure
N-(2,4-Dimethyl-6-methoxy-phenylacetyl)-1-amino-4-methoxy-cyclohexancarbonsäure

Verbindungen der Formel (IIa) sind beispielsweise aus den Phenylessigsäure halogeniden der Formel (XV) und Aminosäuren der Formel (XIVa) nach Schotten-Baumann (Organikum, 9. Auflage, 446 (1970) VEB Deutscher Verlag der Wissenschaften, Berlin) erhältlich.

Die Phenylessigsäurehalogenide der Formel (XV) 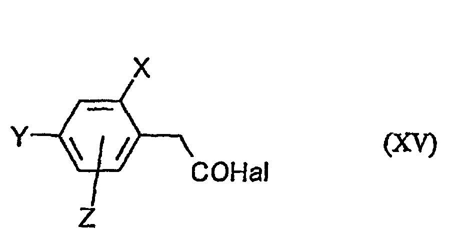 in welcher
- X, Y und Z: die oben angegebene Bedeutung haben und
- Hal: für Brom oder Chlor steht,
sind teilweise neu. Sie lassen sich nach bekannten Verfahren in einfacher Weise aus den entsprechenden bekannten Phenylessigsäuren darstellen.

Die zur Durchführung der erfindungsgemäßen Verfahren (B), (C), (D), (E), (F), (G) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Alkylhalogenide der Formel (VII), Sulfonsäurechloride der Formel (VIII), Phosphorverbindungen der Formel (IX) und Metallverbindungen oder Amine der Formel (X) und (XI) und Isocyanate, Isothiocyanate oder Carbamidsäurechloride der Formel (XIII) sind allgemein bekannte Verbindungen der organischen bzw. anorganischen Chemie.

Das Verfahren (A) ist dadurch gekennzeichnet, daß man Verbindungen der Formel (II) in welcher A, B, X, Y, Z und R⁸ die oben angegebene Bedeutung haben, in Gegenwart von Basen einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Isopropanol, Butanol, iso-Butanol und tert.-Butanol.

Als Basen (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetahamide und -hydride, wie Natriumamid, Natriumhydrid und Calciumhydrid, und außerdem auch Alkalimetallalkoholate, wie Natrium-methylat, Natriumethylat und Kalium-tert.-butylat einsetzbar.

Die Reaktionstemperaturen können bei der Durchführung des erfmdungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung der erfindungsgemäßen Verfahren (A) setzt man pro Mol der Reaktionskomponente der Formel (II) etwa 2 Mol der deprotonierenden Base ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (Bα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (TV) umsetzt.

Verwendet man bei dem erfindungsgemäßen Verfahren (Bβ) als Reaküonskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Die Reaktionstemperaturen können bei dem erfindungsgemäßen Verfahren (Bβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Verwendet man die entsprechenden Chlorameisensäureester bzw. Chlorameisensäurethiolester so kommen als Säurebindemittel bei der Umsetzung nach dem erfindungsgemäßen. Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) bei Verwendung der Chlorameisensäureester bzw. Chlorameisensäurethiolester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester bzw. Chlorameisensäurethiolester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren (D_{α}) setzt man pro Mol Ausgangsverbindung der Formel (Ia) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (D_{β}) setzt man pro Mol Ausgangsverbindung der Formel (Ia) die äquimölare Menge bzw. einen Überschuß Schwefelkohlenstoff zu. Man arbeitet hierbei vorzugsweise bei Temperaturen von 0 bis 50°C und insbesondere bei 20 bis 30°C.

Als Basen können beim Verfahren (Dβ) alle Üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Als Verdünnungsmittel können bei diesem Verfahren alle üblichen Lösungsmittel verwendet werden.

Vorzugsweise verwendbar sind aromatische Kohlenwasserstoffe wie Benzol oder Toluol, Alkohole wie Methanol, Ethanol, Isopropanol oder Ethylenglykol, Nitrile wie Acetonitril, Ether wie Tetrahydrofuran oder Dioxan, Amide wie Dimethylformamid oder andere polare Lösungsmittel wie Dimethylsulfoxid oder Sulfolan.

Oft ist es zweckmäßig zunächst aus der Verbindung der Formel (Ia) durch Zusatz eines Deprotonierungsmittels (wie z.B. Kaliumtertiärbutylat oder Natriumhydrid) das entsprechende Salz herzustellen. Man setzt die Verbindung (Ia) solange mit Schwefelkohlenstoff um, bis die Bildung der Zwischenverbindung abgeschlossen ist, z.B. nach mehrstündigem Rühren bei Raumtemperatur.

Die weitere Umsetzung mit dem Alkylhalogenid der Formel (VII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren (E) setzt man pro Mol Ausgangsverbindung der Formel (Ia) ca. 1 Mol Sulfonsäurechlorid (VIII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Dimethylsulfid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (F) setzt man zum Erhalt von Verbindungen der Struktur (Ie) auf 1 Mol der Verbindung (Ia), 1 bis 2, vorzugsweise 1 bis 1,3 Mol der Phosphorverbindung der Formel (IX) bei Temperaturen zwischen -40°C und 150°C, vorzugsweise zwischen -10 und 110°C um.

Als gegebenenfalls zugesetzte Verdünnungsmittel kommen aller inerten, polaren organischen Lösungsmittel in Prage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (G) ist dadurch gekennzeichnet, daß man Verbindungen der Formel (Ia) mit Metallverbindungen (X) oder Aminen (XI) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (G) wird im allgemeinen unter Normaldruck durchgeführt. Die Reaktionstemperaturen liegen im allgemeinen zwischen -20°C und 100°C, vorzugsweise zwischen 0°C und 50°C.

Bei Herstellungsverfahren (H_{α}) setzt man pro Mol Ausgangsverbindung der Formel (Ia) ca. 1 Mol Isocyanat der Formel (XII) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (H_{β}) setzt man pro Mol Ausgangsverbindung der Formel (Ia) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzusweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.
Die Reaktion kann bei Normaldruck oder unter erhöhtem Druck durchgeführt werdern, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratoriozdes, Melanoplus differentialis, Schistocerca gregaria.

Aus der Ordnung der Dermaptera z.B. Forficula auricularia.
Aus der Ordnung der Isoptera z.B. Reticulitermes spp..

Aus der Ordnung der Anoplura z.B. Phyllaxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammen, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae), gegen die Larven der grünen Reiszikade (Nephotettix cincticeps) oder gegen die Raupen der Kohlschabe (Plutella maculipennis) einsetzen.

Die erfindungsgemäßen Verbindungen weisen auch eine fungizide Wirkung auf, beispielsweise gegen Venturia inaequalis.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräter der Gattungen: Sinapis, Lepidium, Galium, Stellaria, Matricaria, Anthemis, Galinsoga, Chenopodium, Urüca, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindunngen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Bmulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Atkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-[2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Asnpropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Toldophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen, Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen; Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr, Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norilurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosutfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die endungsgemäßen Wirkstoffe wirken nicht nur gegen Pflanzen-, Hygieneund Vorratsschädlinge, sondern auch auf dem veterinärmedizinischen Sektor gegen tierische Ektoparasiten wie Schildzecken, Lederzecken, Räudemilben, Laufmilben, Fliegen (stechend und leckend), parasitierende Fliegenlarven, Läuse, Haarlinge, Federlinge und Flöhe. Beispielsweise zeigen sie eine hervorragende Wirksamkeit gegen Zecken, wie beispielsweise Boophilus microplus.

Die erfindungsgemäßen Wirkstoffe der Formel (I) eignen sich auch zur Bekämpfung von Axthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten Boli, des feed-through-Verfahrens, von Zäpfchen, durch parenterale Verabreichung, wie z.B. durch Injektionen (intramuskulär, subcutan, intravenös, intraperitonal u.a), Implantate, durch nasale Applikation, durch dermale Anwendung in Form beispielsweise des Tauchens oder Badens (Dippen), Sprühens (Spray), Aufgießens (Pour-on und Spoton), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Die Herstellung und die Verwendung der erfindungsgemäßen Stoffe wird durch die folgenden Beispiele veranschaulicht.

### Beispiel (Ia-1):

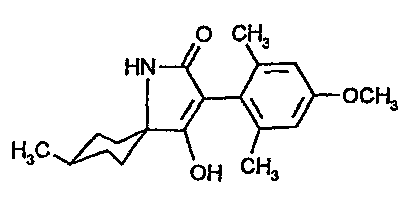

13,96 g (0,124 Mol) Kalium-tert.-butylat werden in 45 ml absolutem THF vorgelegt und unter Rückflußtemperatur eine Lösung von 20,4 g (0,0564 Mol) N-(2,6-Dimethyl-4-methoxyphenyl)acetyl-4-methyl-1-amino-cyclohexancarbonsäuremethylester in 120 ml absolutem Toluol zugetropft. Man erhitzt weitere 1,5 h unter Rückfluß, kühlt ab, fügt 180 ml Wasser hinzu, trennt die wäßrige Phase ab, extrahiert erneut mit 70 ml Wasser, vereinigt die wäßrigen Phasen, säuert bei 10-20°C mit ca. 20 ml konzentrierter Salzsäure an, saugt den Niederschlag ab und trocknet. Nach Verrühren mit Methyl-tert.-Butyl (MTB)-Ether/n-Hexan erhält man 16,8 g (94 % d. Theorie) der oben gezeigten Verbindung vom Schmelzpunkt 169°C.

In Analogie zu Beispiel Ia-1 erhält man die in der folgenden Tabelle aufgeführten Verbindungen:

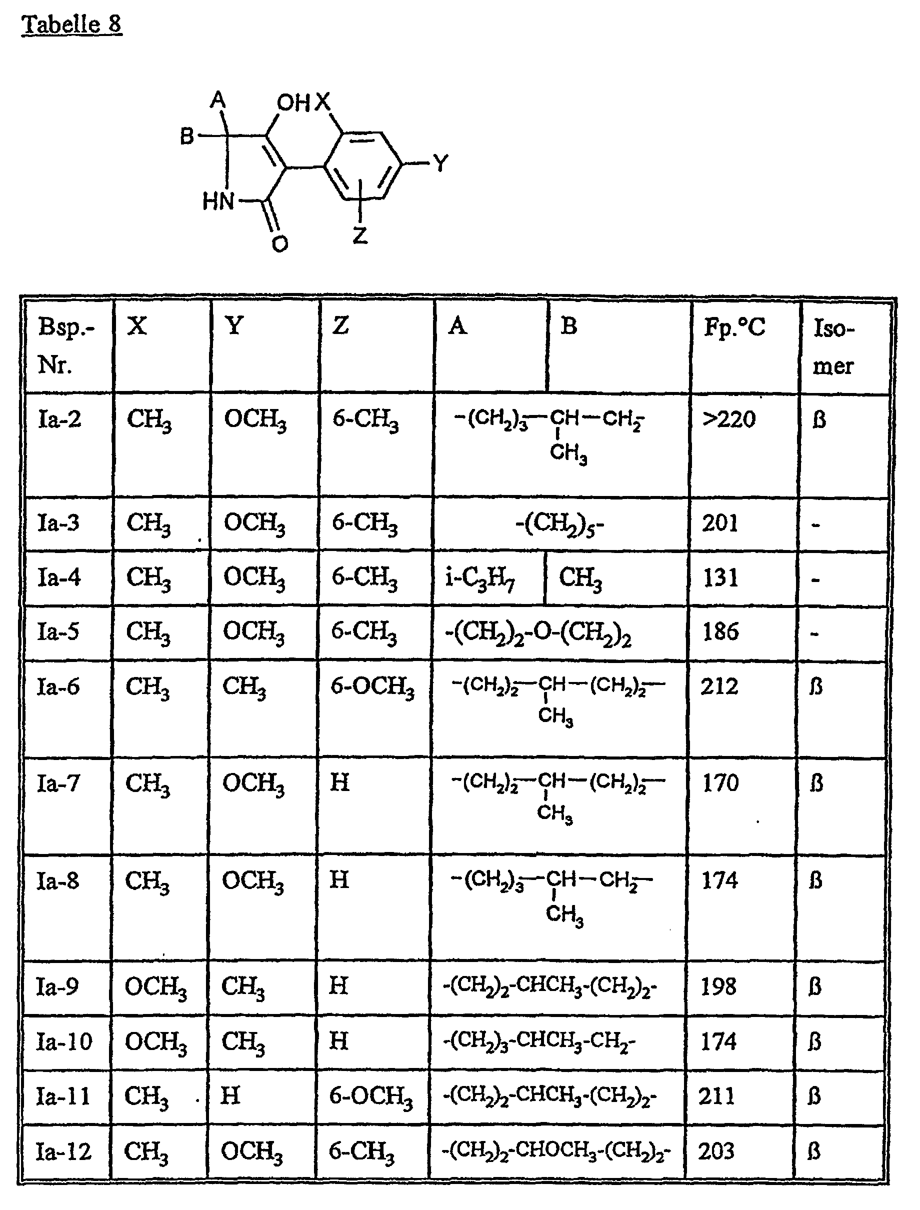

### Beispiel Ib-1

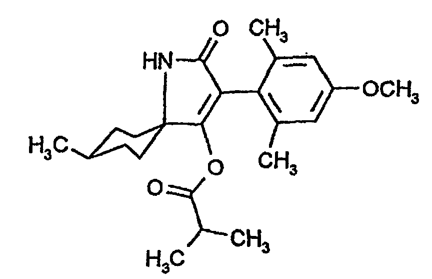

4,73 g (15 mMol) 3-(2,6-Dimethyl-4-methoxyphenyl)-5,5-(3-methyl)-pentamethylen-pyrrolidin-2,4-dion werden in 70 ml absolutem Methylenchlorid suspendiert, mit 2,1 ml Triethylamin versetzt und bei 0-10°C 1,58 ml Isobuttersäurechlorid in 5 ml absolutem Methylenchlorid zugetropft. Man rührt unter dünnschichtchromatographischer Kontrolle bei Raumtemperatur weiter. Nach Beendigung der Reaktion wird die organische Phase 2 mal mit je 100 ml 0,5 N Natronlauge gewaschen, mit Magnesiumsulfat getrocknet und im Vakuum eingedampft. Nach dem Umkristallisieren aus MTB-Ether/n-Hexan erhält man 2,6 g ( 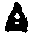 45 % der Theorie) der oben gezeigten Verbindung vom Schmelzpunkt 218°C.

In Analogie zum Beispiel Ib-1 erhält man die in der folgenden Tabelle 9 gezeigten Verbindungen:

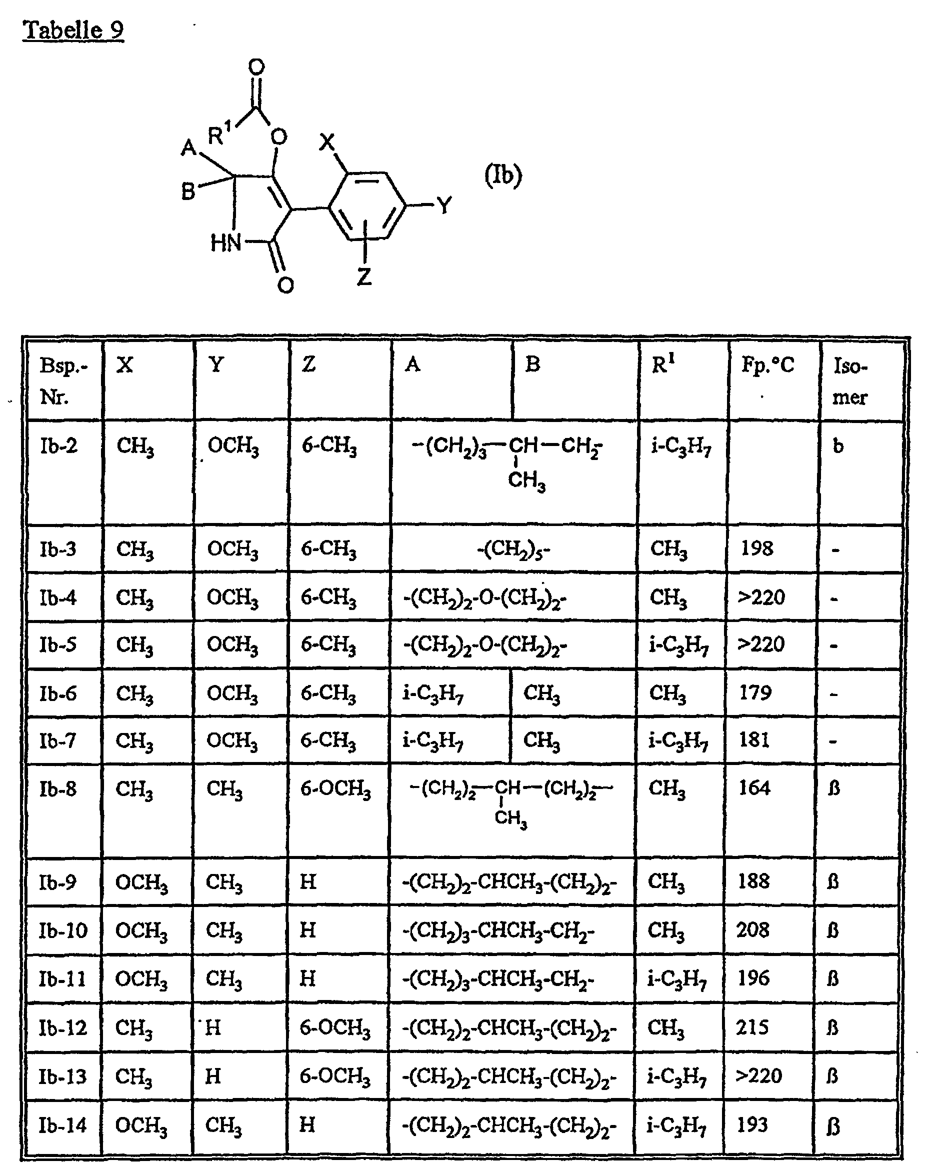

### Beispiel Ic-1

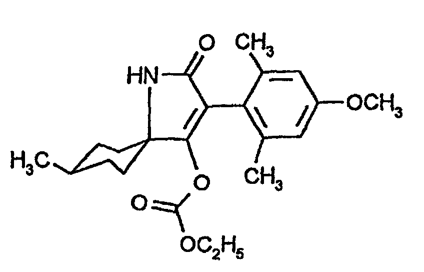

4,73 g (15 mMol) 3-(2,6-Dimethyl-4-methoxyphenyl)-5,5-(3-methyl)-pentamethylen-pyrrolidin-2,4-dion werden in 70 ml absolutem Methylenchlorid. suspendiert, mit 2,1 ml Triethylamin versetzt und bei 0-10°C 1,5 ml Chlorameisensäureethylester in 5 ml absolutem Methylenchlorid zugetropft. Man rührt unter dünnschichtchromatographischer Kontrolle bei Raumtemperatur weiter. Nach Beendigung der Reaktion wird die organische Phase 2 mal mit je 100 ml 0,5 N Natronlauge gewaschen, mit Magnesiumsulfat getrocknet und im Vakuum eingedampft. Nach dem Umkristallisieren aus MTB-Ether/n-Hexan erhält man 3,9 g ( 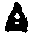 67 % der Theorie) der oben gezeigten Verbindung vom Schmelzpunkt 202°C.

In Analogie zum Beispiel Ic-1 erhält man die in der folgenden Tabelle 10 gezeigten Verbindungen:

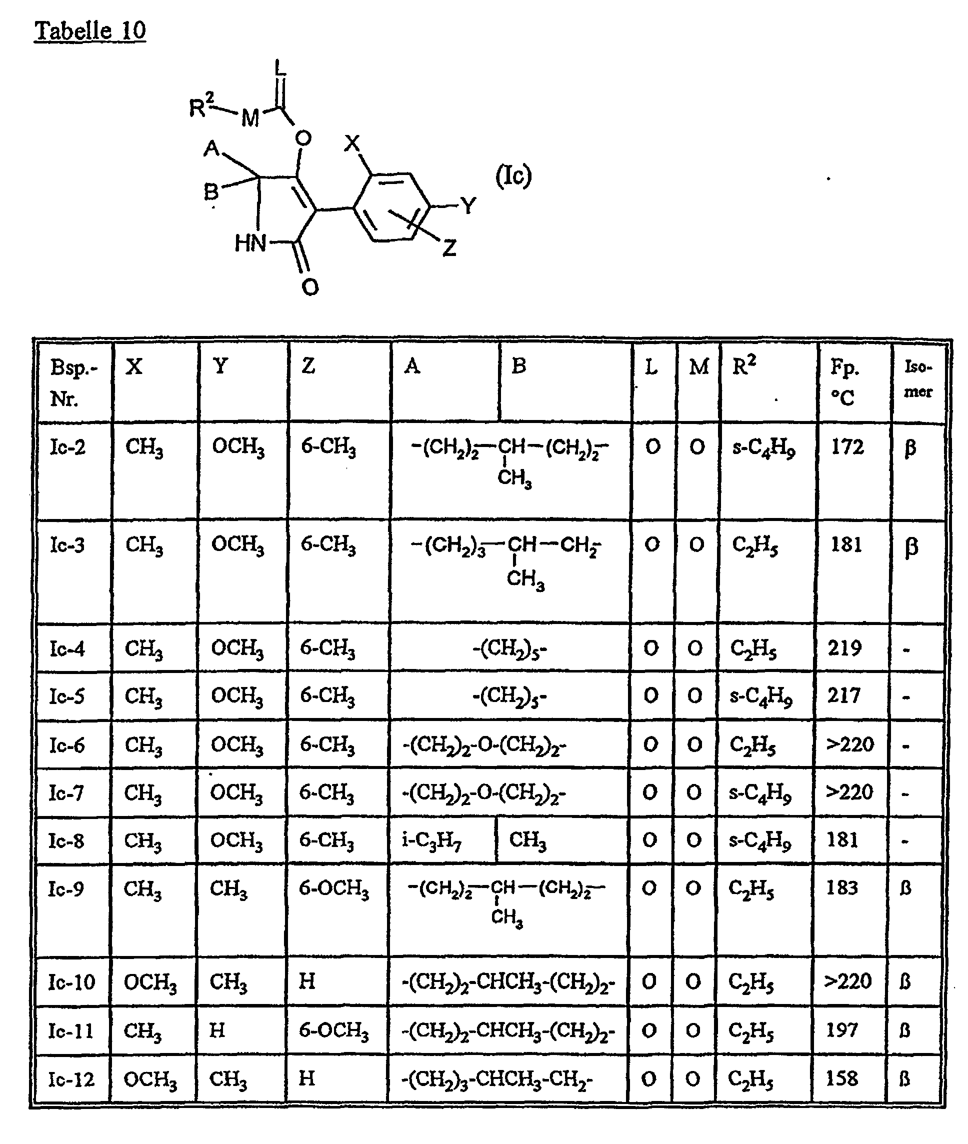

### Beispiel II-1

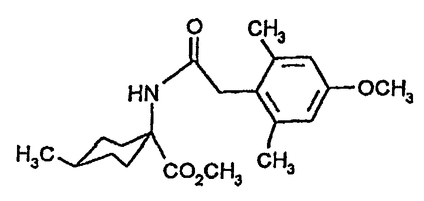

15 g (0,0773 Mol) 2,6-Dimethyl-4-methoxy-phenylessigsäure wurden mit 11,3 ml Thionylchlorid auf 80°C erwärmt bis die Gasentwicklung beendet ist. Überschüssiges Thionylchlorid wird bei 50°C im Vakuum entfernt und der Rückstand in 100 ml absolutem Tetrahydrofuran aufgenommen. Diese Lösung wird bei 0-10°C zu einer Suspension von 16,1 g cis-1-Amino-4-methyl-cyclohexancarbonsäuremethylester und 27,1 ml Triethylamin in 200 ml absulutem Tetrahydrofuran getropft. Man rührt 1 h bei Raumtemperatur nach saugt den Niederschlag ab, wäscht mit Tetrahydrofuran nach und engt im Vakuum ein. Der Rückstand wird in Methylenchlorid aufgenommen, mit 0,5 N HCl gewaschen, mit Magnesiumsulfat getrocknet und eingedampft. Nach dem Umkristallisieren aus MTB-Ether/n-Hexan erhält man 20,4 g ( 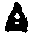 73 % der Theorie) der oben gezeigten Verbindung vom Schmelzpunkt 108°C.

### Beispiel II-2

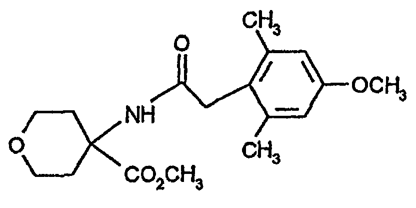

Zu 77,4 g (0,79 Mol) konzentrierter Schwefelsäure tropft man 47,7 g (0,158 Mol) N-(2,6-Dimethyl-4-methoxy-phenylacetyl)-4-amino-pyran-4-carbonsäurenitril suspendiert in 320 ml wasserfreiem Methylenchlorid bei 30 bis 44°C. Man rührt bei 40°C 2 h nach, tropft 109 ml wasserfreies Methanol zu und erwärmt 6 h bei einer Badtemperatur von 40 bis 70°C. Das Reaktionsgemisch wird auf 0,8 kg Eis gegossen, mit Methylenchlorid extrahiert, die organischen Phasen vereint und mit Natriumhydrogencarbonatlösung gewaschen, über Magnesiumsulfat getrocknet und das Lösungsmittel im Vakuum abgedampft. Nach Umkristallisieren aus MTB-Ether/n-Hexan erhält man 37,7 g ( 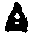 71 % d.Th.) der oben gezeigten Verbindung vom Schmelzpunkt 168°C.

Analog erhält man die in der nachfolgenden Tabelle 11 aufgeführten Substanzen der Formel II:

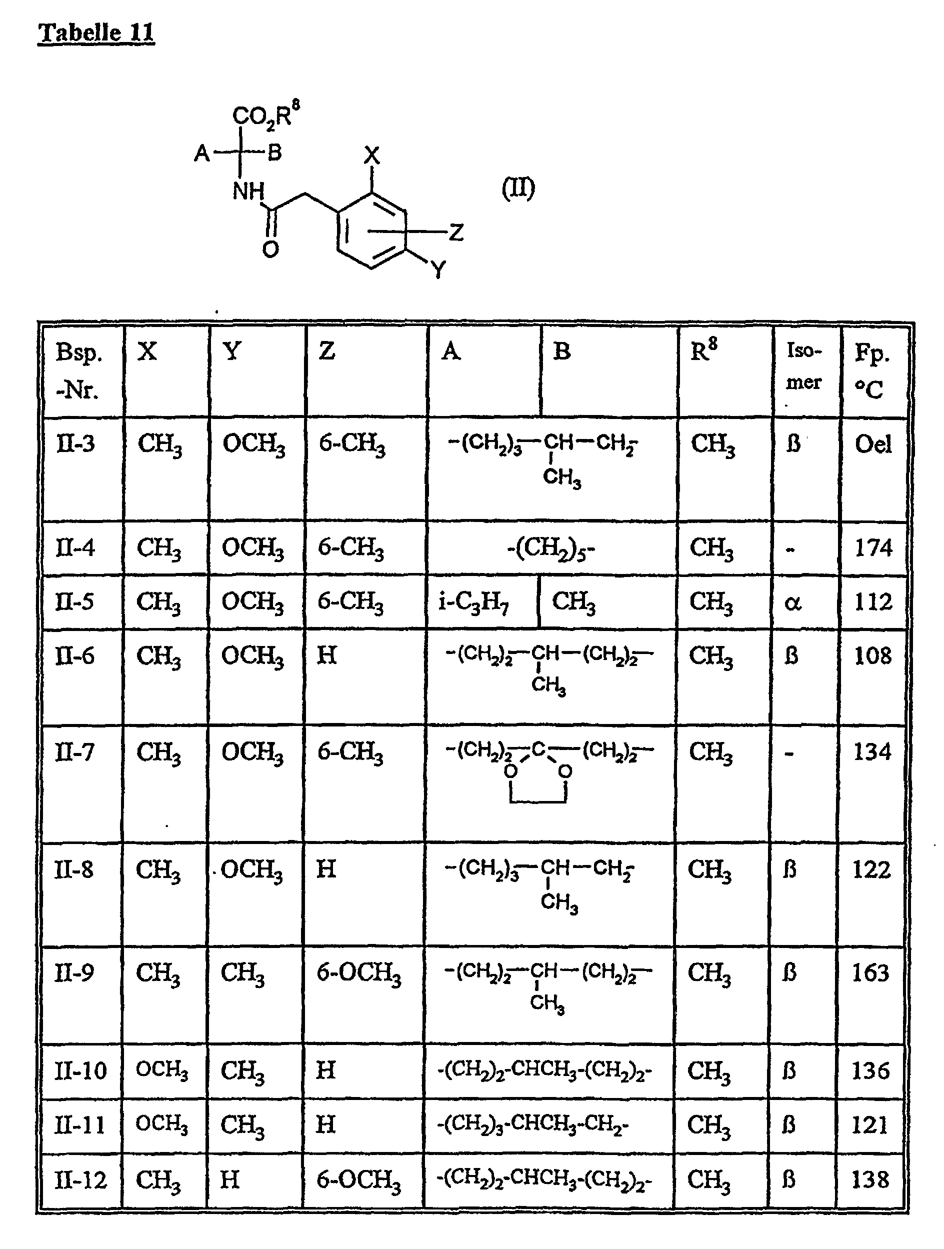

### Beispiel XVII-1

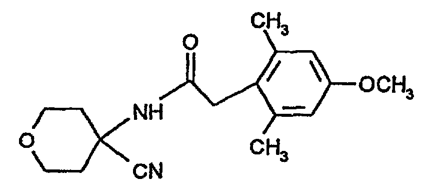

38,6 g (0,2 Mol) 2,6-Dimethyl-4-methoxy-phenylessigsäure werden mit 29,3 ml (0,4 Mol) Thienylchlorid auf 50 bis 60°C erwärmt bis die Gasentwicklung beendet ist. Das überschüssige Thionylchlorid wird im Vakuum abdestilliert der Rückstand in 100 ml wasserfreiem Tetrahydrofuran aufgenommen und bei 0 bis 10°C zu einer Lösung von 25,2 g (0,2 Mol) 4-Amino-pyran-4-carbonsäurenitril und 28 ml (0,2 Mol) Triethylamin in 400 ml wasserfreiem Tetrahydrofuran getropft und 1 h bei Raumtemperatur nachgerührt. Das Reaktionsgemisch wird auf ein Gemisch von 90.0 ml Wasser und 100 ml 2N Salzsäure gegossen, der Niederschlag abgesaugt, getrocknet und aus MTB-Ether/n-Hexan umkristallisiert. Es wurden 47 g ( 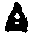 79 % d.Th.) der oben gezeigten Verbindung vom Schmelzpunkt 156°C erhalten.

Analog zum Beispiel XVII-1 wurden die in der Tabelle 12 aufgeführten Verbindungen der Formel (XVII) erhalten.

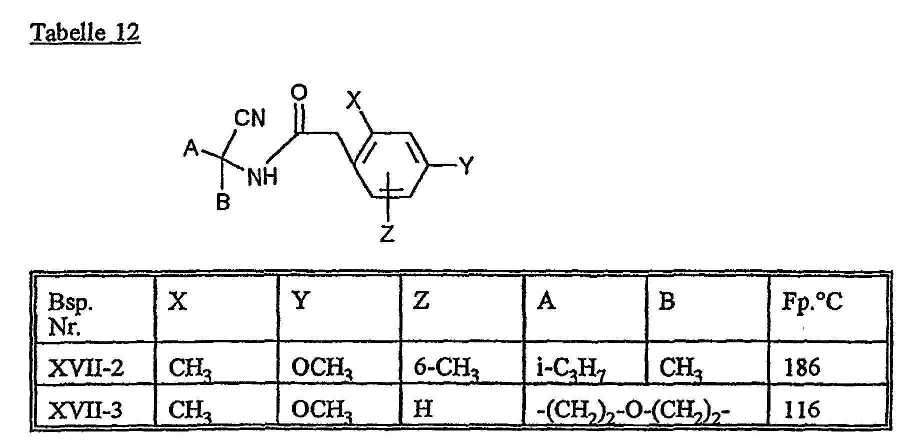

### Anwendungsbeispiele

### Beispiel A

### Myzus-Test

| | | |
|---|---|---|
| Lösungsmittel | 7 Gewichtsteile | Dimethylformamid |
| Emulgator | 1 Gewichtsfell | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß den Herstellungsbeispielen Ia-1, Ia-2, Ia-6 und Ic-1 bei einer beispielhaften Wirkstoffkonzentration von 0,1 % eine Abtötung von m indestens 80 % nach 6 Tagen.

### Beispiel B

### Pre-emergence-Test

| | | |
|---|---|---|
| Lösungsmittel | 3 Gewichtsteile | Aceton |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- O % =: keine Wirkung (wie unbehandelte Kontrolle)
- 100 % =: totale Vernichtung

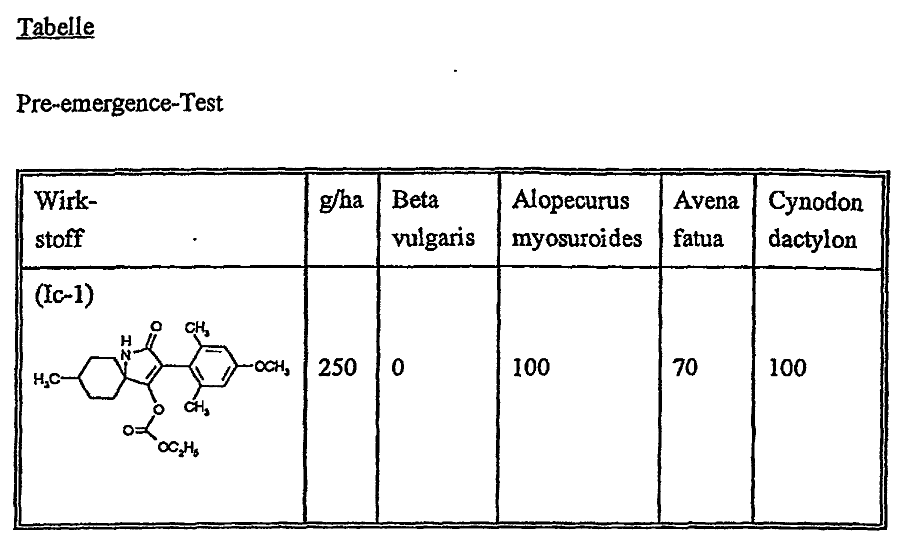

### Beispiel C

### Tetranychus-Test (OP-resistent/Spritzbehandlung)

| | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Stadien der gemeinen Spinnmilbe (Tetranychus urticae) befallen sind, werden mit einer Wirkstoffzubereitung der gewünschten Konzentration gespritzt.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

In diesem Test bewirkte z.B. die Verbindung gemäß Herstellungsbeispiel Ia-6 bei einer beispielhaften Wirkstoffkonzentration von 0,01 % eine Abtötung von 98 % nach 7 Tagen.

## Patentansprüche

1. 1-H-3-Aryl-pyrrolidin-2,4-dion-Derivate der Formel (I) 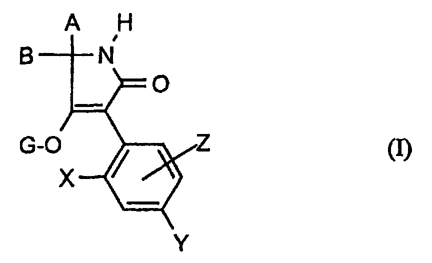 in welcher
A für Wasserstoff oder für jeweils gegebenenfalls einfach oder mehrfach durch Halogen substituiertes C₁-C₁₂-Alkyl, C₂-C₈-Alkenyl, C₁-C₁₀-Alkoxy-C₁-C₈-alkyl, C₁-C₈-Polyalkoxy-C₁-C₈-alkyl, C₁-C₁₀-Alkylthio-C₁-C₆-alkyl, gegebenenfalls durch Halogen, C₁-C₄-Alkyl oder C₁-C₄-Alkoxy substituiertes Cycloalkyl mit 3 bis 8 Ringatomen, das durch Sauerstoff und/oder Schwefel unterbrochen sein kann oder für jeweils gegebenenfalls einfach bis mehrfach durch Halogen, C₁-C₆-Alkyl, C₁-C₆-Halogenalkyl, C₁-C₆-Alkoxy und/oder Nitro substituiertes Phenyl, Naphthyl, Pyridyl, Imidazolyl, Indolyl, Thiazolyl, Furanyl, Thienyl, Phenyl-C₁-C₆-alkyl oder Naphthyl-C₁-C₆-alkyl steht,
B für C₁-C₁₂-Alkyl oder C₁-C₈-Alkoxy-C₁-C₆-alkyl steht oder
A, B und das Kohlenstoffatom an das sie gebunden sind, für einen gesättigten oder ungesättigten C₃-C₁₀-Spirocyclus stehen, der gegebenenfalls einfach oder mehrfach durch C₁-C₆-Alkyyl, C₃-C₈-Cycloalkyl, C₁-C₄-Halogenalkyl, C₁-C₆-Alkoxy, C₁-C₆-Alkylthio, Halogen oder Phenyl substituiert und gegebenenfalls durch Sauerstoff oder Schwefel unterbrochen ist oder
A, B und das Kohlenstoffatom, an das sie gebunden sind, für einen C₃-C₆-Spirocyclus stehen, der durch eine gegebenenfalls durch ein oder zwei Sauerstoff- und/oder Schwefelatome unterbrochene Alkylendiyl-, oder durch eine Alkylendioxyl- oder durch eine Alkylendithioyl-gruppe substituiert ist, die mit dem Kohlenstoffatom, an das sie gebunden ist, einen weiteren fünf- bis achtgliedrigen Spirocyclus bildet oder
A, B und das Kohlenstoffatom, an das sie gebunden sind, für einen C₃-C₈-Spirocyclus stehen, bei dem zwei Substituenten gemeinsam für einen gegebenenfalls durch C₁-C₄-Alkyl, C₁-C₄-Alkoxy oder Halogen substituierten gesättigten oder ungesättigten füng- bis achtgliedrigen Cyclus stehen, der durch Sauerstoff oder Schwefel unterbrochen sein kann,
X für C₁-C₆-Alkyl oder C₁-C₆-Alkoxy steht,
Y für Wasserstoff, C₁-C₆-Alkyl oder C₁-C₆-Alkoxy steht,
Z für Wasserstoff, C₁-C₆-Alkyl oder C₁-C₆-Alkoxy steht,
G für Wasserstoff (a) oder für die Gruppen 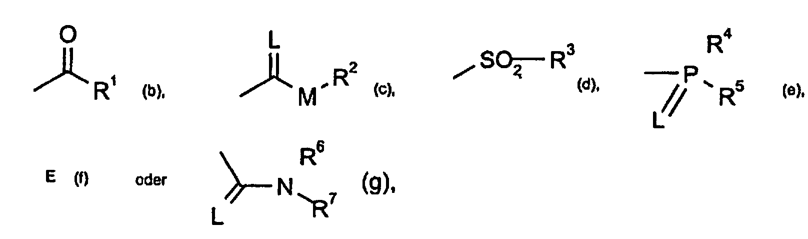
steht, in welchen
E für ein Metallionäquivalent oder ein Ammoniumion steht und
L für Sauerstoffoder Schwefel steht,
M für Sauerstoff oder Schwefel steht,
R¹ für jeweils gegebenenfalls einfach oder mehrfach durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, C₁-C₈-Polyalkoxy-C₂-C₈-alkyl oder gegebenenfalls durch Halogen oder C₁-C₆-Alkyl substituiertes Cycloalkyl mit 3 bis 8 Ringatomen, das durch mindestens ein Sauerstoff und/oder Schwefelatom unterbrochen sein kann,
für gegebenenfalls einfach bis fünffach durch Halogen, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls einfach bis fünffach durch Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls einfach bis dreifach durch Halogen oder C₁-C₆-Alkyl substituiertes Pyridyl, Thienyl, Furanyl, Pyrazolyl, Pyrimidyl oder Thiazolyl,
für gegebenenfalls einfach bis dreifach durch Halogen oder C₁-C₆-Alkyl substituiertes Phenoxy-C₁-C₆-alkyl oder
für gegebenenfalls einfach bis zweifach durch Halogen, Amino oder C₁-C₆-Alkyl substituiertes Pyridinyloxy-C₁-C₆-alkyl, Pyrimidinyloxy-C₁-C₆-alkyl oder Thiazolyloxy-C₁-C₆-alkyl steht,
R² für jeweils gegebenenfalls einfach oder mehrfach durch Halogen substituiertes C₁-C₂₀-Alkyl, C₃-C₂₀-Alkenyl, C₁-C₈-Alkoxy-C₂-C₈-alkyl, C₁-C₈-Polyalkoxy-C₂-C₈-alkyl,
für gegebenenfalls einfach oder mehrfach durch Halogen, C₁-C₄-Alkyl oder C₁-C₄-Alkoxy substituiertes C₃-C₈-Cycloalkyl, oder
für gegebenenfalls einfach bis dreifach durch Halogen, Nitro, C₁-C₆-Alkyl, C₁-C₆-Alkoxy oder C₁-C₆-Halogenalkyl substituiertes Phenyl oder Benzyl steht,
R³, R⁴ und R⁵ unabhängig voneinander für jeweils gegebenenfalls einfach oder mehrfach durch Halogen substituiertes C₁-C₈-Alkyl, C₁-C₈-Alkoxy, C₁-C₈-Alkylamino, Di-(C₁-C₈)-alkylamino, C₁-C₈-Alkylthio, C₃-C₆-Alkenylthio, C₃-C₇-Cycloalkylthio, für jeweils gegebenenfalls einfach oder mehrfach durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen und
R⁶ und R⁷ unabhängig voneinander für Wasserstoff, für jeweils gegebenenfalls einfach oder mehrfach durch Halogen substituiertes C₁-C₈-Alkyl, C₃-C₈-Cycloalkyl, C₁-C₈-Alkoxy, C₃-C₈-Alkenyl, C₁-C₈-Alkoxy-C₂-C₈-alkyl, für gegebenenfalls einfach bis dreifach durch Halogen, C₁-C₈-Halogenalkyl, C₁-C₈-Alkyl oder C₁-C₈-Alkoxy substituiertes Phenyl, gegebenenfalls einfach bis dreifach durch Halogen, C₁-C₈-Alkyl, C₁-C₈-Halogenalkyl und/oder C₁-C₈-Alkoxy substituiertes Benzyl oder gemeinsam mit dem N-Atom, an das sie gebunden sind, für einen gegebenenfalls durch Sauerstoff oder Schwefel unterbrochenen C₃-C₆-Alkylenring stehen,
mit der Maßgabe, dass mindestens einer der Substituenten Y und Z für Alkoxy steht, wenn X für Alkyl steht.

2. 1-H-3-Aryl-pyrrolidin-2,4-dion-Derivate der Formel (I) gemäß Anspruch 1, welche unter Einbeziehung der verschiedenen Bedeutungen (a), (b), (c), (d), (e), (f) und (g) der Gruppe G folgende Strukturen (Ia) bis (Ig) besitzen: 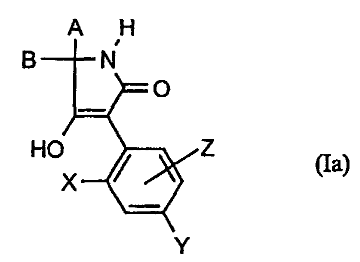 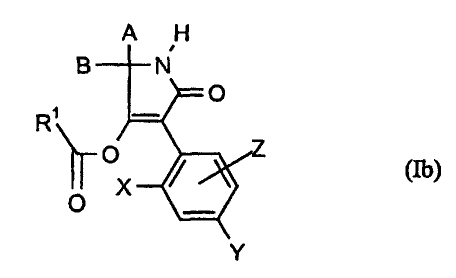 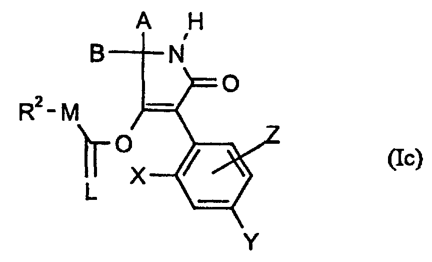 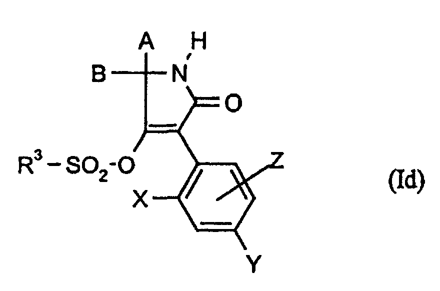 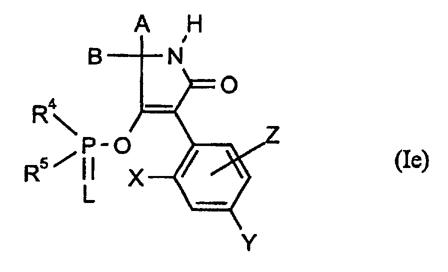 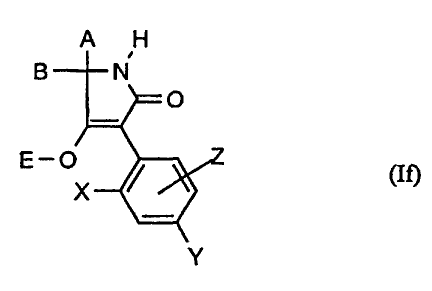 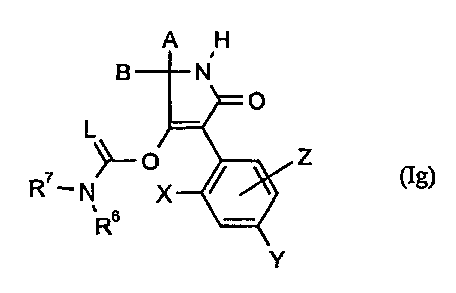 A, B, E, L, M, X, Y, Z, R¹, R², R³, R⁴, R⁵, R⁶ und R⁷ die in Anspruch 1 angegebenen Bedeutungen besitzen.

3. Verfahren zur Herstellung der 1-H-3-Aryl-pyrrolidin-2,4-dion-Derivate der Formel (I) gemäß Anspruch 1, **dadurch gekennzeichnet, dass** man
(A) zum Erhalt von 1-H-3-Aryl-pyrrolidin-2,4-dionen bzw. deren Enolen der Formel (Ia) 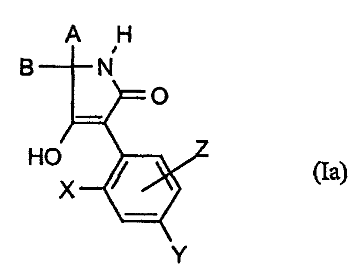 in welcher
A, B, X, Y und Z die in Anspruch 1 angegebene Bedeutung haben,
N-Acylaminosäureester der Formel (II) 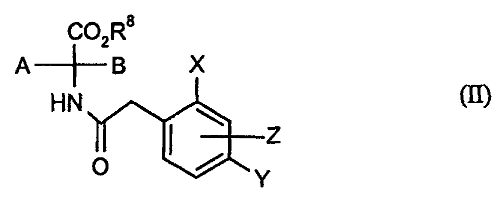 in welcher
A, B, X, Y und Z die in Anspruch 1 angegebene Bedeutung haben,
und
R⁸ für Alkyl steht,
in Gegenwart eines Verdünnungsmittels und in Gegenwart einer Base intramolekular kondensiert;
oder
(B) zum Erhalt von Verbindungen der Formel (Ib) 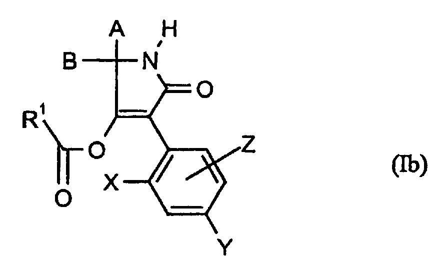 in welcher
A, B, X, Y, Z und R¹ die in Anspruch 1 angegebene Bedeutung haben,
Verbindungen der Formel (Ia), 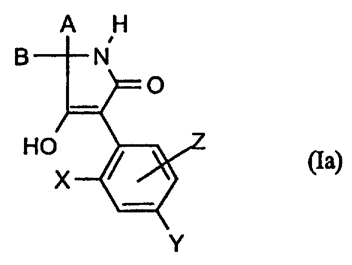 in welcher
A, B, X, Y und Z die in Anspruch 1 angegebene Bedeutung haben,
α) mit Säurehalogeniden der Formel (III) 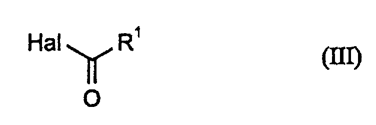 in welcher
R¹ die oben angegebene Bedeutung hat und
Hal für Halogen steht,
gegebenenfalls in Gegenwart eines Verdünnungsmittels und gegebenenfalls in Gegenwart eines Säurebindemittels umsetzt
oder
β) mit Carbonsäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels,
umsetzt;
oder
(C) zum Erhalt von Verbindungen der Formel (Ic-a) 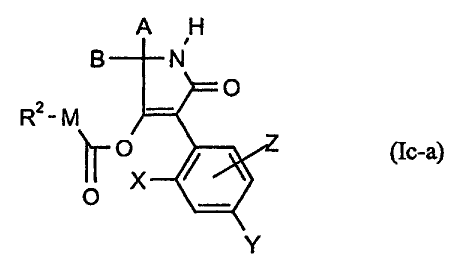 in welcher
A, B, X, Y, Z und R² die oben angegebene Bedeutung haben,
und
M für Sauerstoff oder Schwefel steht,
Verbindungen der Formel (Ia) 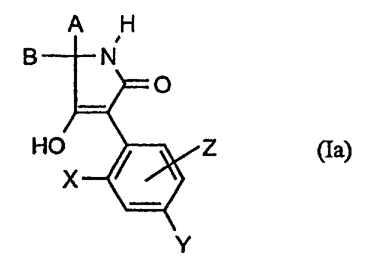 in welcher
A, B, X, Yund Z die oben angegebene Bedeutung haben,
mit Chlorameisensäureester oder Chlorameisensäurethiolester der Formel (V)
R²-M-CO-Cl (V)
in welcher
R² und M die oben angegebene Bedeutung haben,
gegebenenfalls in Gegenwart eines Verdünnungsmittels und gegebenenfalls in Gegenwart eines Säurebindemittels umsetzt;
oder
(D) zum Erhalt von Verbindungen der Formel (Ic-b) 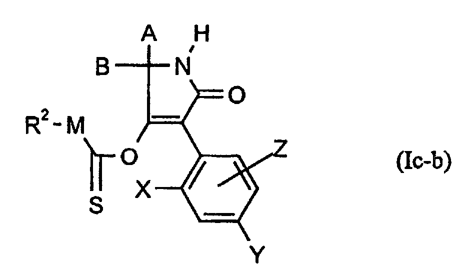 in welcher
A, B, R², X, Y und Z die oben angegebene Bedeutung haben
und
M für Sauerstoff oder Schwefel steht,
Verbindungen der Formel (Ia) 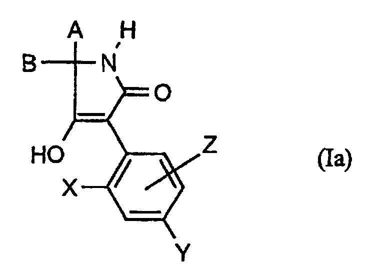 in welcher
A, B, X, Y und Z die oben angegebene Bedeutung haben,
α) mit Chlormonothioameisensäureestern oder Chlordithioameisensäureestern der Formel (VI) 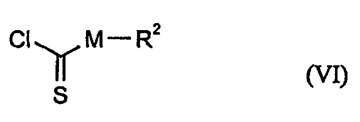 in welcher
M und R² die oben angegebene Bedeutung haben,
gegebenenfalls in Gegenwart eines Verdünnungsmittels und gegebenenfalls in Gegenwart eines Säurebindemittels umsetzt,
oder
β) mit Schwefelkohlenstoff und anschließend mit Alkylhalogeniden der Formel (VII)
R²-Hal (VII)
in welcher
R² die oben angegebene Bedeutung hat
und
Hal für Chlor, Brom oder Iod steht,
gegebenenfalls in Gegenwart eines Verdünnungsmittels und gegebenenfalls in Gegenwart einer Base umsetzt;
oder
(E) zum Erhalt von Verbindungen der Formel (Id) 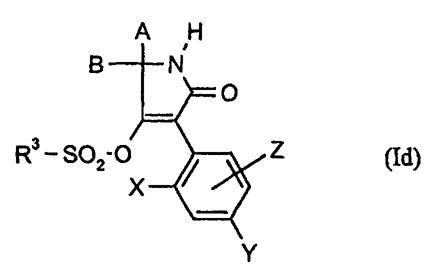 in welcher
A, B, X, Y, Z und R³ die in Anspruch 1 angegebene Bedeutung haben,
Verbindungen der Formel (Ia) 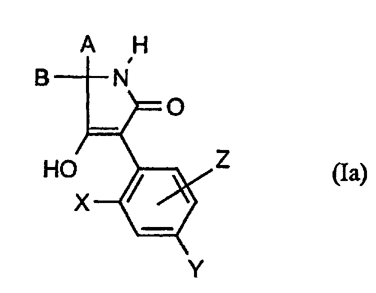 in welcher
A, B, X, Y und Z die oben angegebene Bedeutung haben,
mit Sulfonsäurechloriden der Formel (VIII)
R³-SO₂-Cl (VIII)
in welcher
R³ die oben angegebene Bedeutung hat,
gegebenenfalls in Gegenwart eines Verdünnungsmittels und gegebenenfalls in Gegenwart eines Säurebindemittels,
umsetzt;
oder
(F) zum Erhalt von 3-Aryl-pyrrolidin-2,4-dionen der Formel (Ie) 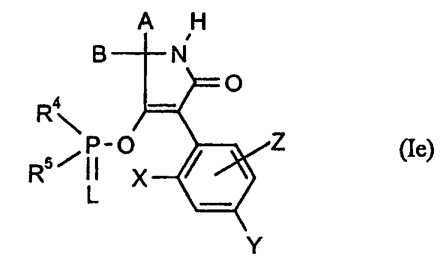 in welcher
A, B, L, X, Y, Z, R⁴ und R⁵ die in Anspruch 1 angegebene Bedeutung haben,
1-H-3-Aryl-pyrrolidin-2,4-dione der Formel (Ia) bzw. deren Enole 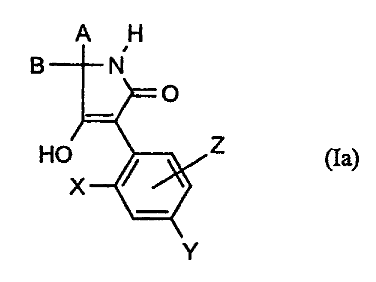 in welcher
A, B, X, Y und Z die oben angegebene Bedeutung haben,
mit Phosphorverbindungen der Formel (IX) 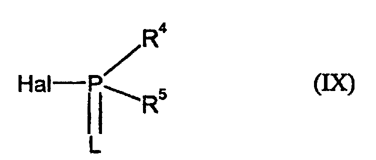 in welcher
L, R⁴ und R⁵ die oben angegebene Bedeutung haben
und
Hal für Halogen, insbesondere Chlor oder Brom steht,
gegebenenfalls in Gegenwart eines Verdünnungsmittels und gegebenenfalls in Gegenwart eines Säurebindemittels umsetzt;
oder
(G) zum Erhalt von Verbindungen der Formel (If) 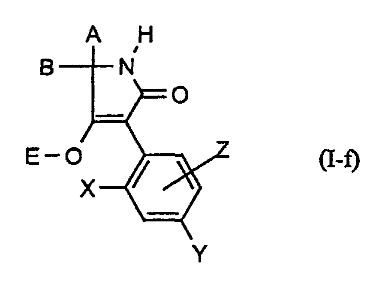 in welcher
A, B, X, Y und Z die oben angegebene Bedeutung haben,
und
E für ein Metallionäquivalent oder für ein Ammoniumion steht,
Verbindungen der Formel (Ia) 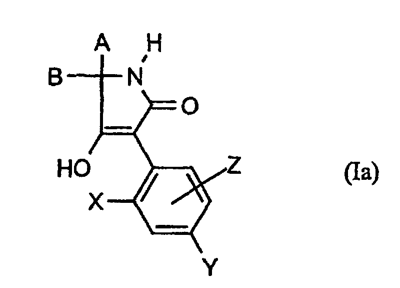 in welcher
A, B, X, Y und Z die oben angegebene Bedeutung haben,
mit Metallhydroxiden, Metallalkoxiden oder Aminen der Formeln (X) und (XI) 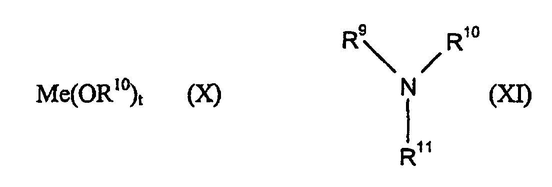 in welchen
Me für ein- oder zweiwertige Metallionen,
t für die Zahl 1 oder 2 und
R⁹, R¹⁰ und R¹¹ unabhängig voneinander für Wasserstoff und/oder Alkyl
stehen,
gegebenenfalls in Gegenwart eines Verdünnungsmittels, umsetzt,
oder
(H) zum Erhalt von Verbindungen der Formel (Ig) 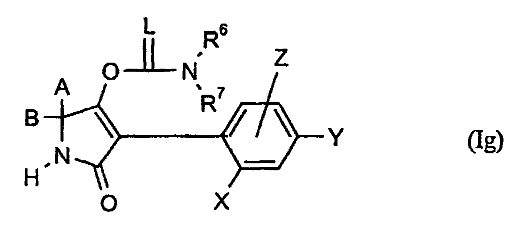 in welcher
A, B, L, X, Y, Z, R⁶ und R⁷ die in Anspruch 1 angegebene Bedeutung haben,
Verbindungen der Formel (Ia) 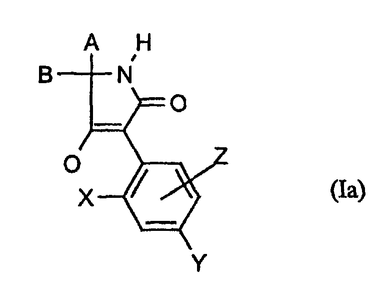 in welcher
A, B, X, Y und Z die oben angegebene Bedeutung haben,
α) mit Isocyanaten oder Isothiocyanaten der Formel (XII)
R⁶-N=C=L (XII)
in welcher
R⁶ die oben angegebene Bedeutung hat
gegebenenfalls in Gegenwart eines Verdünnungsmittels und gegebenenfalls in Gegenwart eines Katalysators
oder
b) mit Carbamidsäurechloriden oder Thiocarbamidsäurechloriden der Formel (XIII) 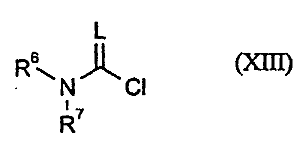 in welcher
L, R⁶ und R⁷ die oben angegebene Bedeutung haben
gegebenenfalls in Gegenwart eines Verdünnungsmittels und gegebenenfalls in Gegenwart eines Säurebindemittels,
umsetzt.

4. 1-H-3-Aryl-pyrrolidin-2,4-dione der Formel (I) gemäß Anspruch 1, in welcher
A für Wasserstoff, für jeweils gegebenenfalls einfach bis sechsfach durch Fluor oder Chlor substituiertes C₁-C₁₀-Alkyl, C₂-C₆-Alkenyl, C₁-C₈-Alkoxy-C₁-C₆-alkyl, C₁-C₆-Polyalkoxy-C₁-C₆-alkyl, C₁-C₈-Alkylthio-C₁-C₆-alkyl, gegebenenfalls durch Fluor, Chlor, C₁-C₃-Alkyl oder C₁-C₃-Alkoxy substituiertes Cycloalkyl mit 3 bis 7 Ringatomen, das durch 1 bis 2 Sauerstoff- und/oder Schwefelatome unterbrochen sein kann oder für jeweils gegebenenfalls einfach bis zweifach durch Fluor, Chlor, Brom, C₁-C₄-Alkyl, C₁-C₄-Halogenalkyl, C₁-C₄-Allcoxy oder Nitro substituiertes Phenyl, Pyridyl, Furanyl, Thienyl, Imidazolyl, Indolyl oder Phenyl-C₁-C₄-alkyl steht,
B für C₁-C₁₀-Alkyl oder C₁-C₆-Alkoxy-C₁-C₄-alkyl steht oder
A, B und das Kohlenstoffatom an das sie gebunden sind, für einen gesättigten oder ungesättigten C₃-C₉-Spirocyclus stehen, der gegebenenfalls einfach oder mehrfach durch C₁-C₆-Alkyl, C₃-C₈-Cycloalkyl, C₁-C₃-Halogenalkyl, C₁-C₆-Alkoxy, C₁-C₆-Alkylthio, Fluor, Chlor oder Phenyl substituiert und gegebenenfalls durch Sauerstoff oder Schwefel unterbrochen ist oder
A, B und das Kohlenstoffatom, an das sie gebunden sind, für einen C₃-C₆-Spirocyclus stehen, der durch eine gegebenenfalls durch ein oder zwei Sauerstoff- oder Schwefelatome unterbrochenen Alkylendiyl- oder durch eine Alkylendioxyl- oder durch eine Alkylendithiol-Gruppe substituiert ist, die mit dem Kohlenstoffatom, an das sie gebunden ist, einen weiteren fünf- bis siebengliedrigen Spirocyclus bildet oder
A,B und das Kohlenstoffatom, an das sie gebunden sind, für einen C₃-C₆-Spirocyclus stehen, bei dem zwei Substituenten gemeinsam für einen gegebenenfalls durch C₁-C₃-Alkyl, C₁-C₃-Alkoxy, Fluor, Chlor oder Brom substituierten gesättigten oder ungesättigten, fünf- bis siebengliedrigen Cyclus stehen, der durch Sauerstoff oder Schwefel unterbrochen sein kann,
X für C₁-C₅-Alkyl oder C₁-C₄-Alkoxy steht,
Y für Wasserstoff, C₁-C₅-Alkyl oder C₁-C₄-Alkoxy steht,
Z für Wasserstoff, C₁-C₄-Alkyl oder C₁-C₄-Alkoxy steht,
in welchen
G für Wasserstoff (a) oder für die Gruppen 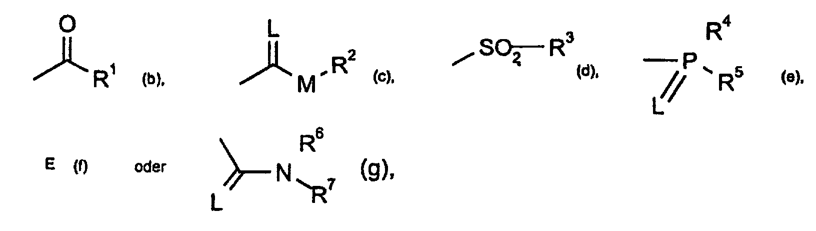 steht,
in welchen
E für ein Metallionäquivalent oder ein Ammoniumion steht,
L für Sauerstoff oder Schwefel steht,
M für Sauerstoff oder Schwefel steht,
R¹ für jeweils gegebenenfalls einfach bis sechsfach durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl, C₁-C₆-Polyalkoxy-C₂-C₆-alkyl oder gegebenenfalls durch Fluor, Chlor oder C₁-C₅-Alkyl substituiertes Cycloalkyl mit 3 bis 7 Ringatomen, das durch 1 oder 2 Sauerstoff- und/oder Schwefelatome unterbrochen sein kann,
für gegebenenfalls einfach bis dreifach durch Fluor, Chlor, Brom Nitro, C₁-C₄-Alkyl, C₁-C₄-Alkoxy, C₁-C₃-Halogenalkyl, C₁-C₃-Halogenalkoxy, C₁-C₄-Alkylthio und/oder C₁-C₄-Alkylsulfonyl substituiertes Phenyl,
für gegebenenfalls einfach bis dreifach durch Fluor, Chlor, Brom, C₁-C₄-Alkyl, C₁-C₄-Alkoxy, C₁-C₃-Halogenalkyl, C₁-C₃-Halogenalkoxy substituiertes Phenyl-C₁-C₄-alkyl,
für gegebenenfalls einfach bis zweifach durch Fluor, Chlor, Brom oder C₁-C₄-Alkyl substituiertes Pyridyl, Thienyl, Furanyl, Pyrazolyl, Pyrimidyl oder Thiazolyl,
für gegebenenfalls einfach bis zweifach durch Fluor, Chlor, Brom oder C₁-C₄-Alkyl substituiertes Phenoxy C₁-C₅-alkyl oder
für gegebenenfalls einfach bis zweifach durch Fluor, Chlor, Brom, Amino oder C₁-C₄-Alkyl substituiertes Pyrimidinyloxy-C₁-C₅-alkyl, Pyridinyloxy-C₁-C₅-alkyl, Thiazolyloxy-C₁-C₅-alkyl steht,
R² für jeweils gegebenenfalls einfach bis sechsfach durch Fluor und/oder Chlor substituiertes C₁-C₁₆ Alkyl, C₃-C₁₆-Alkenyl, C₁-C₆-Alkoxy-C₁-C₆-alkyl, C₁-C₆-Polyalkoxy-C₂-C₆-alkyl,
für gegebenenfalls einfach bis sechsfach durch Fluor, Chlor, C₁-C₃-Alkyl oder C₁-C₃-Alkoxy-substituiertes C₃-C₇-Cycloalkyl,
für gegebenenfalls einfach bis zweifach durch Fluor, Chlor, Brom, Nitro, C₁-C₄-Alkyl, C₁-C₃-Alkoxy oder C₁-C₃-Halogenalkyl substituiertes Phenyl oder Benzyl steht,
R³, R⁴ und R⁵ unabhängig voneinander für jeweils gegebenenfalls einfach bis sechsfach durch Fluor oder Chlor substituiertes C₁-C₆-Alkyl, C₁-C₆-Alkoxy, C₁-C₆-Alkylamino, Di-(C₁-C₆)-alkylamino, C₁-C₆-Alkylthio, C₃-C₄-Alkenylthio, C₃-C₆-Cycloalkylthio, für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio stehen und
R⁶ und R⁷ unabhängig voneinander für Wasserstoff, für jeweils gegebenenfalls einfach bis sechsfach durch Fluor oder Chlor substituiertes C₁-C₆-Alkyl, C₃-C₆-Cycloalkyl, C₁-C₆-Alkoxy, C₃-C₆-Alkenyl, C₁-C₆-Alkoxy-C₂-C₆-alkyl, für gegebenenfalls einfach bis zweifach durch Fluor, Chlor, Brom, C₁-C₅-Halogenalkyl, C₁-C₅-Alkyl oder C₁-C₅-Alkoxy substituiertes Phenyl, für gegebenenfalls einfach bis zweifach durch Fluor, Chlor, Brom, C₁-C₅-Alkyl, C₁-C₅-Halogenalkyl oder C₁-C₅-Alkoxy substituiertes Benzyl steht, oder gemeinsam mit dem N-Atom, an das sie gebunden sind, für einen gegebenenfalls durch Sauerstoff oder Schwefel unterbrochenen C₃-C₆-Alkylenring stehen,
mit der Maßgabe, dass mindestens einer der Substituenten Y und Z für Alkoxy steht, wenn X für Alkyl steht.

5. 1-H-3-Aryl-pyrrolidin-2,4-dione der Formel (I) gemäß Anspruch 1, in welcher
A für Wasserstoff, für jeweils gegebenenfalls einfach bis dreifach durch Fluor oder Chlor substituiertes C₁-C₈-Alkyl, C₂-C₄-Alkenyl, C₁-C₆-Alkoxy-C₁-C₄-alkyl, C₁-C₄-Polyalkoxy-C₁-C₄-alkyl, C₁-C₆-Alkylthio-C₁-C₄-alkyl, gegebenenfalls durch Fluor, Chlor, Methyl, Ethyl, Methoxy oder Ethoxy substituiertes Cycloalkyl mit 3 bis 6 Ringatomen, das durch 1 bis 2 Sauerstoff- und/oder Schwefelatome unterbrochen sein kann oder für jeweils gegebenenfalls einfach bis zweifach durch Fluor, Chlor, Brom, Methyl, Ethyl, Propyl, iso-Propyl, Methoxy, Ethoxy, Trifluormethyl und/oder Nitro substituiertes Phenyl, Furanyl, Thienyl, Imidazolyl, Indolyl, Pyridyl oder Benzyl steht,
B für C₁-C₈-Alkyl oder C₁-C₄-Alkoxy-C₁-C₂-alkyl steht oder
A, B und das Kohlenstoffatom an das sie gebunden sind, für einen gesättigten oder ungesättigten C₃-C₈-Spirocyclus stehen, der gegebenenfalls einfach oder mehrfach durch Methyl, Ethyl, Propyl, Isopropyl, Butyl, iso-Butyl, sec.-Butyl, tert,-Butyl, Cyclohexyl, Trifluormethyl, Methoxy, Ethoxy, Propoxy, iso-Propoxy, Butoxy, iso-Butoxy, sek.-Butoxy, tert.-Butoxy, Methylthio, Ethylthio, Fluor, Chlor oder Phenyl substituiert und gegebenenfalls durch Sauerstoff oder Schwefel unterbrochen ist oder
A, B und das Kohlenstoffatom, an das sie gebunden sind, für einen C₅-C₆-Spirocyclus stehen, der durch eine gegebenenfalls durch ein Sauerstoff- oder Schwefelatom unterbrochene Alkylendiyl- oder durch eine Alkylendioxyl-Gruppe substituiert ist, die mit dem Kohlenstoffatom, an das sie gebunden ist, einen weiteren fünf- bis siebengliedrigen Spirocyclus bildet oder
A,B und das Kohlenstoffatom, an das sie gebunden sind, für einen C₃-C₆-Spirocyclus stehen, bei dem zwei Substituenten gemeinsam für einen gesättigten oder ungesättigten fünf- oder sechsgliedrigen Cyclus stehen, der durch Sauerstoff oder Schwefel unterbrochen sein kann,
X für Methyl, Ethyl, Propyl, iso-Propyl, Methoxy, Ethoxy, Propoxy oder iso-Propoxy steht,
Y für Wasserstoff, Methyl, Ethyl, Propyl, iso-Propyl, Butyl, iso-Butyl, sek.-Butyl, tert.-Butyl, Methoxy, Ethoxy, Propoxy oder iso-Propoxy steht,
Z für Wasserstoff, Methyl, Ethyl, Propyl, iso-Propyl, Methoxy, Ethoxy, Propoxy oder iso-Propoxy steht,
G für Wasserstoff (a) oder für die Gruppen 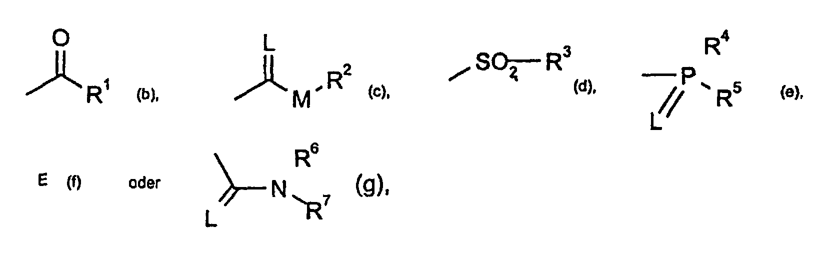 steht,
in welchen
E für ein Metallionäquivalent oder ein Ammoniumion steht und
L für Sauerstoff oder Schwefel steht,
M für Sauerstoff oder Schwefel steht,
R¹ für jeweils gegebenenfalls einfach bis dreifach durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, C₁-C₄-Polyalkoxy-C₂-C₄-alkyl oder gegebenenfalls durch Fluor, Chlor, Methyl, Ethyl, Propyl, i-Propyl, Butyl, i-Butyl oder tert.-Butyl substituiertes Cycloalkyl mit 3 bis 6 Ringatomen, das durch 1 bis 2 Sauerstoff- und/oder Schwefelatome unterbrochen sein kann,
für gegebenenfalls einfach bis zweifach durch Fluor, Chlor, Brom, Methyl, Ethyl, Propyl, i-Propyl, Methoxy, Ethoxy, Trifluormethyl, Trifluormethoxy, Methylthio, Ethylthio, Methylsulfonyl, Ethylsulfonyl oder Nitro substituiertes Phenyl,
für gegebenenfalls einfach bis zweifach durch Fluor, Chlor, Brom, Methyl, Ethyl, Propyl, i-Propyl, Methoxy, Ethoxy, Trifluormethyl oder Trifluormethoxy substituiertes Phenyl-C₁-C₃-alkyl steht,
für gegebenenfalls einfach bis zweifach durch Fluor, Chlor, Brom, Methyl oder Ethyl substituiertes Thienyl, Furanyl oder Pyridyl,
für gegebenenfalls einfach bis zweifach durch Fluor, Chlor, Methyl oder Ethyl substituiertes Phenoxy-C₁-C₄-alkyl, oder
für jeweils gegebenenfalls einfach bis zweifach durch Fluor, Chlor, Amino, Methyl oder Ethyl substituiertes Pyridyloxy-C₁-C₄-alkyl, Pyrimidyloxy-C₁-C₄-alkyl und Thiazolyloxy-C₁-C₄-alkyl steht,
R² für jeweils gegebenenfalls einfach bis dreifach durch Fluor oder Chlor substituiertes C₁-C₁₄-Alkyl, C₃-C₁₄-Alkenyl, C₁-C₄-Alkoxy-C₁-C₆-alkyl, C₁-C₄-Polyalkoxy-C₂-C₆-alkyl,
für gegebenenfalls einfach bis dreifach durch Fluor, Chlor, Methyl, Ethyl, Propyl, iso-Propyl oder Methoxy substituiertes C₃-C₆-Cycloalkyl,
oder für jeweils gegebenenfalls einfach bis zweifach durch Fluor, Chlor, Nitro, Methyl, Ethyl, Propyl, i-Propyl, Methoxy, Ethoxy oder Trifluorrriethyl substituiertes Phenyl oder Benzyl steht,
R³, R⁴ und R⁵ unabhängig voneinander für jeweils gegebenenfalls einfach bis dreifach durch Fluor oder Chlor substituiertes C₁-C₄-Alkyl, C₁-C₄-Alkoxy, C₁-C₄-Alkylamino, Di-(C₁-C₄)-alkylamino, C₁-C₄-Alkylthio, für jeweils gegebenenfalls einfach bis zweifach durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio, C₁-C₂-Alkyl oder C₁-C₂-Fluoralkyl substituiertes Phenyl, Phenoxy oder Phenylthio stehen und
R⁶ und R⁷ unabhängig voneinander für Wasserstoff, für jeweils gegebenenfalls einfach bis dreifach durch Fluor oder Chlor substituiertes C₁-C₄-Alkyl, C₃-C₆-Cycloalkyyl, C₁-C₄-Alkoxy, C₃-C₄-Alkenyl, C₁-C₄-Alkoxy-C₂-C₄-alkyl, für gegebenenfalls einfach bis zweifach durch Fluor, Chlor, Brom, C₁-C₄-Halogenalkyl, C₁-C₄-Alkyl und/oder C₁-C₄-Alkoxy substituiertes Phenyl, für gegebenenfalls einfach bis zweifach durch Fluor, Chlor, Brom, C₁-C₄-Alkyl, C₁-C₄-Halogenalkyl und/oder C₁-C₄-Alkoxy substituiertes Benzyl, oder gemeinsam mit dem N-Atom, an das sie gebunden sind, für einen gegebenenfalls durch Sauerstoff oder Schwefel unterbrochenen C₃-C₆-Alkylenring stehen,
mit der Maßgabe, dass mindestens einer der Substituenten Y und Z für Alkoxy steht, wenn X für Alkyl steht.

6. Verbindungen der Formel (II) 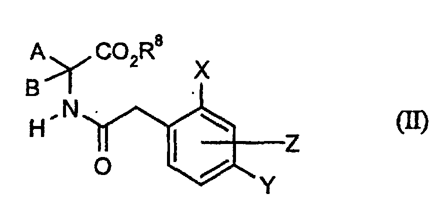 in welcher
A, B, X, Y und Z die in Anspruch 1 angegebene Bedeutung haben und
R⁸ für Alkyl steht.

7. Verfahren zur Herstellung der Acyl-aminosäureester der Formel (II) gemäß Anspruch 7, **dadurch gekennzeichnet, dass** man Aminosäurederivate der Formel (XIV), 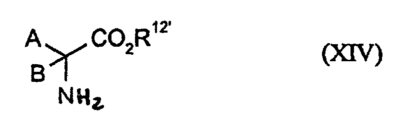 in welcher
R^{12'} für Wasserstoff (XIVa) oder Alkyl (XIVb) steht
und
A und B die oben angegebene Bedeutung haben,
mit Phenylessigsäurehalogeniden der Formel (XV) 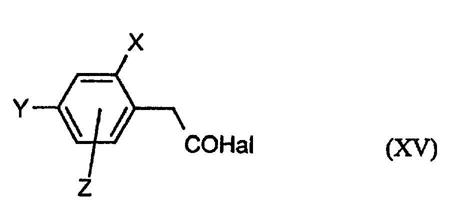 in welcher
X, Y und Z die oben angegebene Bedeutung haben und
Hal für Chlor oder Brom steht,
acyliert,
oder dass man Acylaminosäuren der Formel (IIa), 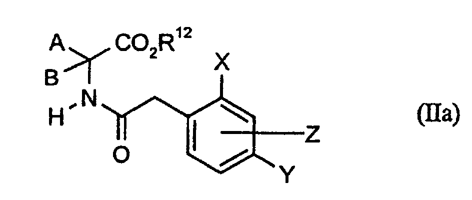 in welcher
A, B, X, Y und Z die oben angegebene Bedeutung haben,
und
R¹² für Wasserstoff steht,
verestert oder
dass man Aminonitrile der Formel (XVI) 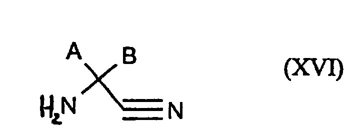 in welcher
A und B die oben angegebene Bedeutung haben,
mit Phenylessigsäurehalogeniden der Formel (XV) 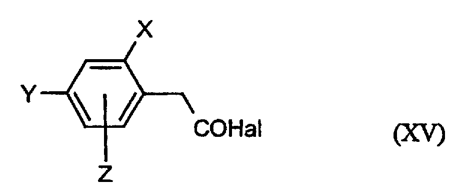 in welcher
X, Y und Z die oben angegebene Bedeutung haben und
Hal für Chlor oder Brom steht,
zu Verbindungen der Formel (XVII) 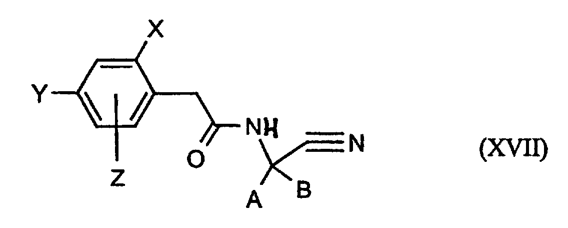 in welcher
A, B, X, Y und Z die oben angegebene Bedeutung haben,
umsetzt, und diese anschließend einer schwefelsauren Alkoholyse unterwirft.

8. Verbindungen der Formel (XVII) 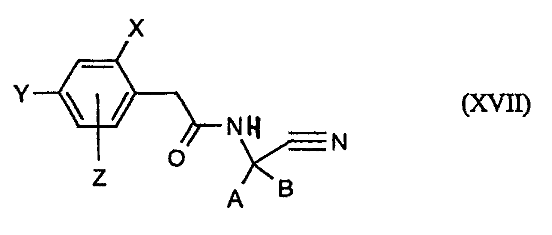 in welcher
A, B, X, Y und Z die in Anspruch 1 angegebene Bedeutung haben.

9. Verfahren zur Herstellung von Verbindungen der Formel (XVII) gemäß Anspruch 9, **dadurch gekennzeichnet, dass** man Aminonitrile der Formel (XVI) 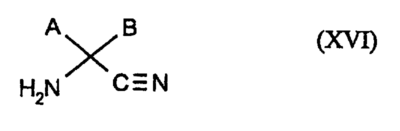 in welcher
A und B die in Anspruch 1 angegebene Bedeutung haben,
mit Phenylessigsäurehalogeniden der Formel (XV) 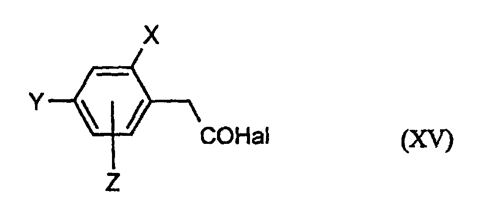 in welcher
X, Y und Z die in Anspruch 1 angegebene Bedeutung haben und
Hal für Chlor oder Brom steht,
umsetzt.

10. Schädlingsbekämpfungsmittel und Unkrautbekämpfungsmittel, **gekennzeichnet durch** einen Gehalt an mindestens einem 1-H-3-Aryl-pyrrolidin-2,4-dion-derivat der Formel (I) gemäß Anspruch 1.

11. Verwendung von 1-H-3-Aryl-pyrrolidin-2,4-dion-derivat der Formel (I) gemäß Anspruch 1 zur Bekämpfung von Schädlingen und Unkräutern.

12. Verfahren zur Bekämpfung von Schädlingen und Unkräutern, **dadurch gekennzeichnet, dass** man 1-H-3-Aryl-pyrrolidin-2,4-dion-derivate der Formel (I) gemäß Anspruch 1 auf Schädlinge und/oder ihren Lebensraum einwirken lässt oder auf Unkräuter und/oder ihren Lebensraum einwirken lässt.

13. Verfahren zur Herstellung von Schädlingsbekämpfungsmitteln und Unkrautbekämpfungsmitteln, **dadurch gekennzeichnet, dass** man 1-H-3-Aryl-pyrrolidin-2,4-dion-derivate der Formel (I) gemäß Anspruch 1 mit Streckmitteln und/oder oberflächenaktiven Mitteln vermischt.

## Claims

1. 1H-3-Aryl-pyrrolidine-2,4-dione derivatives of the formula (I) 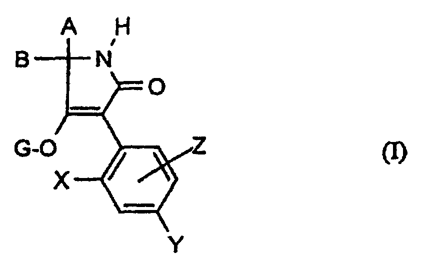 in which
A represents hydrogen or represents in each case optionally mono- or poly-halogeno-substituted C₁-C₁₂-alkyl, C₂-C₈-alkenyl, C₁-C₁₀-alkoxy-C₁-C₈-alkyl, C₁-C₈-polyalkoxy-C₁-C₈-alkyl, C₁-C₁₀-alkylthio-C₁-C₆-alkyl, cycloalkyl which has from 3 to 8 ring atoms, is optionally substituted by halogen, C₁-C₄-alkyl or C₁-C₄-alkoxy and can be interrupted by oxygen and/or sulphur, or represents phenyl, naphthyl, pyridyl, imidazolyl, indolyl, thiazolyl, furanyl, thienyl, phenyl-C₁-C₆-alkyl or naphthyl-C₁-C₆-alkyl, each of which is optionally substituted one or more times by halogen, C₁-C₆-alkyl, C₁-C₆-halogenoalkyl, C₁-C₆-alkoxy and/or nitro,
B represents C₁-C₁₂-alkyl or C₁-C₈-alkoxy-C₁-C₆-alkyl or
A, B and the carbon atom to which they are attached represent a saturated or unsaturated C₃-C₁₀ spirocyclic radical which is optionally monosubstituted or polysubstituted by C₁-C₆-alkyl, C₃-C₈-cycloalkyl, C₁-C₄-halogenoalkyl, C₁-C₆-alkoxy, C₁-C₆-alkylthio, halogen or phenyl and is optionally interrupted by oxygen or sulphur, or
A, B and the carbon atom to which they are attached represent a C₃-C₆ spirocyclic radical which is substituted by an alkylenediyl group optionally interrupted by one or two oxygen and/or sulphur atoms, or is substituted by an alkylenedioxyl or an alkylenedithioyl group which forms, with the carbon atom to which it is attached, a further five- to eight-membered spirocyclic radical, or
A, B and the carbon atom to which they are attached represent a C₃-C₈ spirocyclic radical in which two substituents together represent a saturated or unsaturated five- to eight-membered ring which is optionally substituted by C₁-C₄-alkyl, C₁-C₄-alkoxy or halogen and may be interrupted by oxygen or sulphur,
X represents C₁-C₆-alkyl or C₁-C₆-alkoxy,
Y represents hydrogen, C₁-C₆-alkyl or C₁-C₆-alkoxy,
Z represents hydrogen, C₁-C₆-alkyl or C₁-C₆-alkoxy,
G represents hydrogen (a) or represents the groups 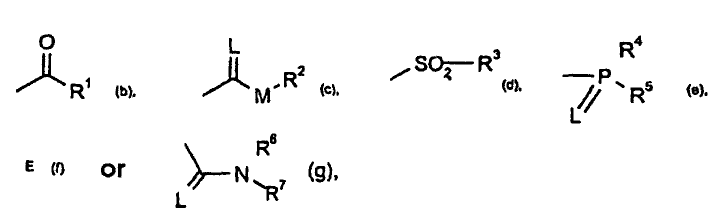
in which
E represents a metal ion equivalent or an ammonium ion, and
L represents oxygen or sulphur,
M represents oxygen or sulphur,
R¹ represents in each case optionally mono- or poly-halogeno-substituted C₁-C₂₀-alkyl, C₂-C₂₀-alkenyl, C₁-C₈-alkoxy-C₁-C₈-alkyl, C₁-C₈-alkylthio-C₁-C₈-alkyl, C₁-C₈-polyalkoxy-C₂-C₈-alkyl or optionally halo- or C₁-C₆-alkyl-substituted cycloalkyl having 3 to 8 ring atoms, which may be interrupted by at least one oxygen and/or sulphur atom,
represents optionally mono- to penta-halogeno-, -nitro-, -C₁-C₆-alkyl-, -C₁-C₆-alkoxy-, -C₁-C₆-halogenoalkyl-, -C₁-C₆-halogenoalkoxy-, -C₁-C₆-alkylthio- or -C₁-C₆-alkylsulphonyl-substituted phenyl,
represents optionally mono- to penta-halogeno-, -C₁-C₆-alkyl-, -C₁-C₆-alkoxy-, -C₁-C₆-halogenoalkyl- or -C₁-C₆-halogenoalkoxy-substituted phenyl-C₁-C₆-alkyl,
represents optionally mono- to tri-halogeno- or -C₁-C₆-alkyl-substituted pyridyl, thienyl, furanyl, pyrazolyl, pyrimidyl or thiazolyl,
represents optionally mono- to tri-halogeno- or -C₁-C₆-alkyl-substituted phenoxy-C₁-C₆-alkyl, or
represents optionally mono- or di-halogeno-, -amino- or -C₁-C₆-alkyl-substituted pyridinyloxy-C₁-C₆-alkyl, pyrimidinyloxy-C₁-C₆-alkyl or thiazolyloxy-C₁-C₆-alkyl,
R² represents in each case optionally mono- or poly-halogeno-substituted C₁-C₂₀-alkyl, C₃-C₂₀-alkenyl, C₁-C₈-alkoxy-C₂-C₈-alkyl, C₁-C₈-polyalkoxy-C₂-C₈-alkyl,
represents optionally mono- or poly-halogeno-, -C₁-C₄-alkyl- and/or -C₁-C₄-alkoxy-substituted C₃-C₈-cycloalkyl, or
represents optionally mono- to tri-halogeno-, -nitro-, -C₁-C₆-alkyl-, -C₁-C₆-alkoxy- or -C₁-C₆-halogenoalkyl-substituted phenyl or benzyl,
R³, R⁴ and R⁵ independently of one another represent in each case optionally mono- or poly-halogeno-substituted C₁-C₈-alkyl, C₁-C₈-alkoxy, C₁-C₈-alkylamino, di-(C₁-C₈)-alkylamino, C₁-C₈-alkylthio, C₃-C₆-alkenylthio or C₃-C₇-cycloalkylthio, or represent phenyl, phenoxy or phenylthio, each of which is optionally mono- or poly-substituted by halogen, nitro, cyano, C₁-C₄-alkoxy, C₁-C₄-halogenoalkoxy, C₁-C₄-alkylthio, C₁-C₄-halogenoalkylthio, C₁-C₄-alkyl or C₁-C₄-halogenoalkyl, and
R⁶ and R⁷ independently of one another represent hydrogen, represent in each case optionally mono- or poly-halogeno-substituted C₁-C₈-alkyl, C₃-C₈-cycloalkyl, C₁-C₈-alkoxy, C₃-C₈-alkenyl or C₁-C₈-alkoxy-C₂-C₈-alkyl, represent optionally mono- to tri-halogeno-, -C₁-C₈-halogenoalkyl-, -C₁-C₈-alkyl- or -C₁-C₈-alkoxy-substituted phenyl, optionally mono- to tri-halogeno-, -C₁-C₈-alkyl-, -C₂-C₈-halogenoalkyl- and/or -C₁-C₈-alkoxy-substituted benzyl, or represent, together with the N atom to which they are attached, a C₃-C₆-alkylene ring which is optionally interrupted by oxygen or sulphur,
with the proviso that at least one of the substituents Y and Z represents alkoxy if X represents alkyl.

2. 1H-3-Aryl-pyrrolidine-2,4-dione derivatives of the formula (I) according to Claim 1, which, taking into account the various meanings (a), (b), (c), (d), (e), (f) and (g) of the group G, possess the following structures (Ia) to (Ig): 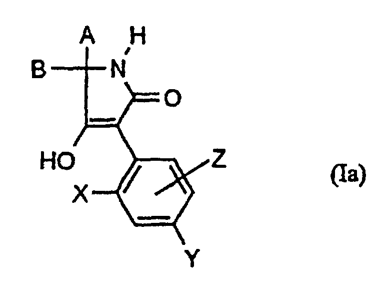 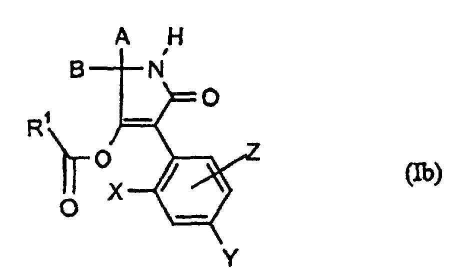 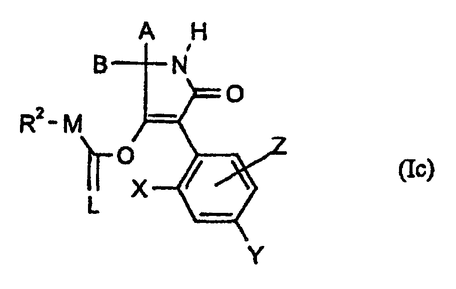 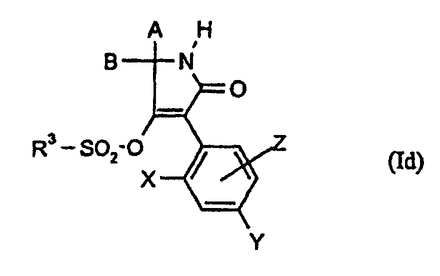 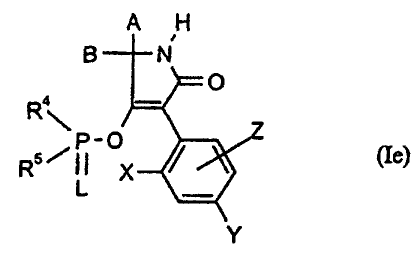 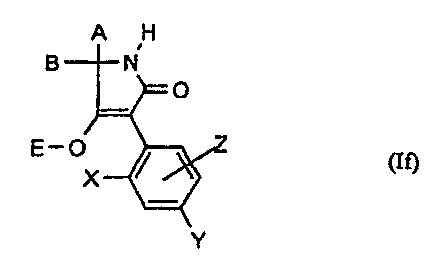 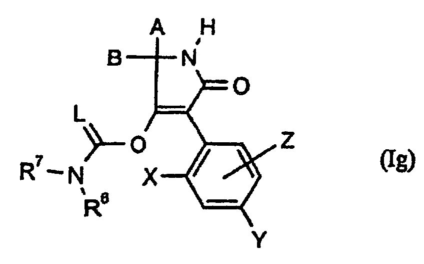 in which
A, B, E, L, M, X, Y, Z, R¹, R², R³, R⁴, R⁵, R⁶ and R⁷ possess the meanings given in Claim 1.

3. Process for the preparation of the 1H-3-aryl-pyrrolidine-2,4-dione derivatives of the formula (I) according to Claim 1, **characterized in that**
(A) to obtain 1H-3-aryl-pyrrolidine-2,4-diones and/or enols thereof, of the formula (Ia) 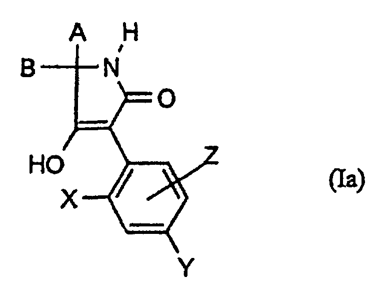 in which
A, B, X, Y and Z have the meaning given in Claim 1,
N-acylamino acid esters of the formula (II) 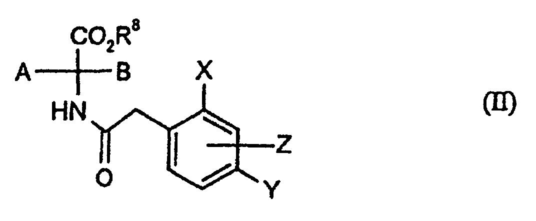 in which
A, B, X, Y and Z have the meaning given in Claim 1,
and
R⁸ represents alkyl,
are subjected to intramolecular condensation in the presence of a diluent and in the presence of a base;
or
(B) to obtain compounds of the formula (Ib) 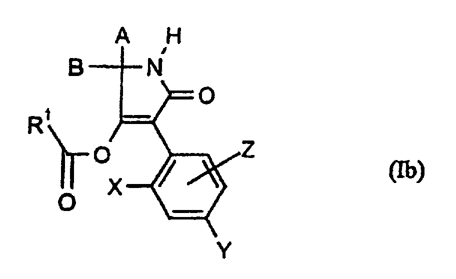 in which
A, B, X, Y, Z and R¹ have the meaning given in Claim 1,
compounds of the formula (Ia) 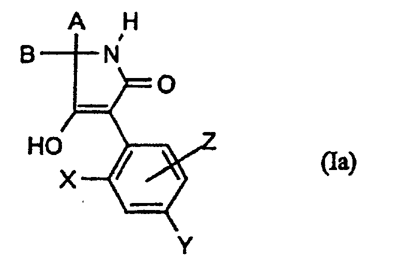 in which
A, B, X, Y and Z have the meaning given in Claim 1,
α) are reacted with acid halides of the formula (III) 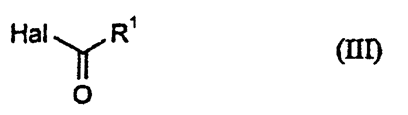 in which
R¹ has the meaning given above and
Hal represents halogen,
optionally in the presence of a diluent and optionally in the presence of an acid-binding agent,
or
β) are reacted with carboxylic anhydrides of the formula (IV)
R¹-CO-O-CO-R¹ (IV)
in which
R¹ has the meaning given above,
optionally in the presence of a diluent and optionally in the presence of an acid-binding agent;
or
(C) to obtain compounds of the formula (Ic-a) 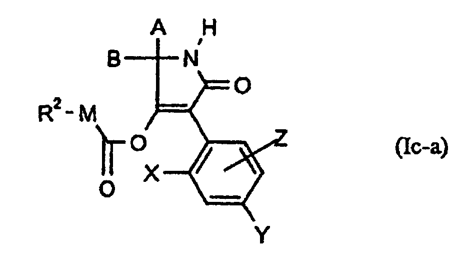 in which
A, B, X, Y, Z and R² have the meaning given above,
and
M represents oxygen or sulphur,
compounds of the formula (Ia) 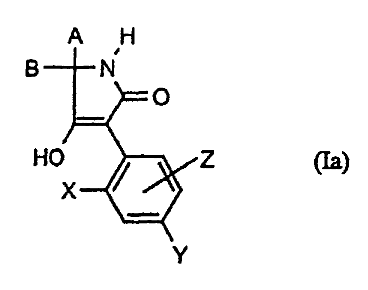 in which
A, B, X, Y and Z have the meaning given above
are reacted with chloroformic ester or chloroformic thioester of the formula (V)
R²-M-CO-Cl (V)
in which
R² and M have the meaning given above,
optionally in the presence of a diluent and optionally in the presence of an acid-binding agent;
or
(D) to obtain compounds of the formula (Ic-b) 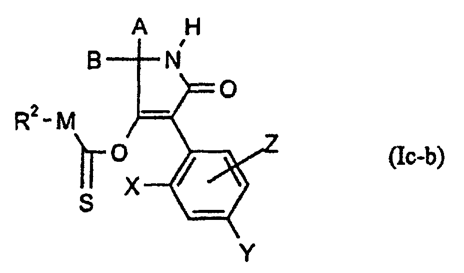 in which
A, B, R², X, Y and Z have the meaning given above
and
M represents oxygen or sulphur,
compounds of the formula (Ia) 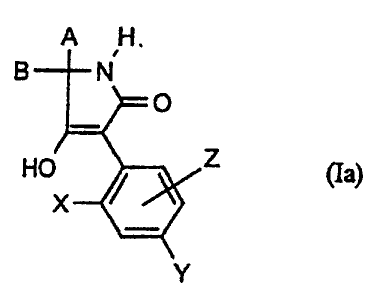 in which
A, B, X, Y and Z have the meaning given above,
α) are reacted with chloromonothioformic esters or chlorodithioformic esters of the formula (VI) 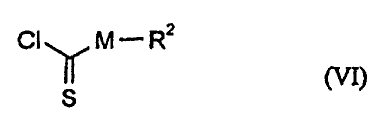 in which
M and R² have the meaning given above
optionally in the presence of a diluent and optionally in the presence of an acid-binding agent,
or
β) are reacted with carbon disulphide and then with alkyl halides of the formula (VII)
R²-Hal (VII)
in which
R² has the meaning given above
and
Hal represents chlorine, bromine or iodine
optionally in the presence of a diluent and optionally in the presence of a base;
or
(E) to obtain compounds of the formula (Id) 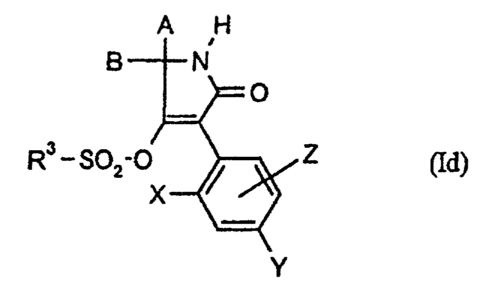 in which
A, B, X, Y, Z and R³ have the meaning given in Claim 1,
compounds of the formula (Ia) 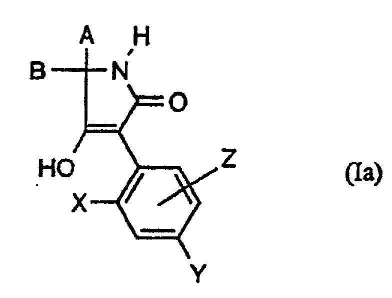 in which
A, B, X, Y and Z have the meaning given above
are reacted with sulphonyl chlorides of the formula (VIII)
R³-SO₂-Cl (VIII)
in which
R³ has the meaning given above
optionally in the presence of a diluent and optionally in the presence of an acid-binding agent;
or
(F) to obtain 3-aryl-pyrrolidine-2,4-diones of the formula (Ie) 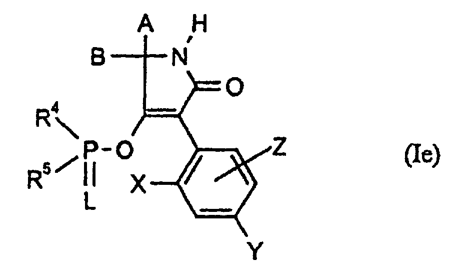 in which
A, B, L, X, Y, Z, R⁴ and R⁵ have the meaning given in Claim 1,
1H-3-aryl-pyrrolidine-2,4-diones of the formula (Ia) and/or enols thereof 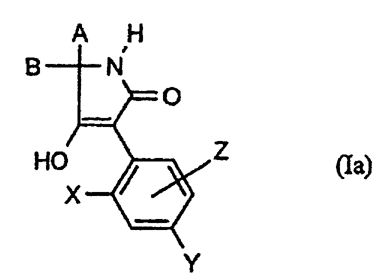 in which
A, B, X, Y and Z have the meaning given above,
are reacted with phosphorus compounds of the formula (IX) 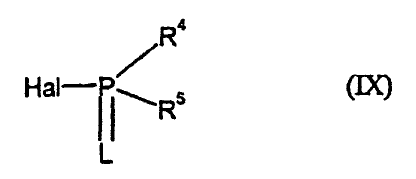 in which
L, R⁴ and R⁵ have the meaning given above
and
Hal represents halogen, especially chlorine or bromine,
optionally in the presence of a diluent and optionally in the presence of an acid-binding agent;
or
(G) to obtain compounds of the formula (If) 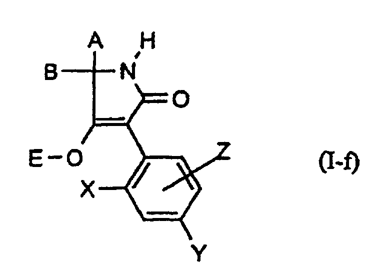 in which
A, B, X, Y and Z have the meaning given above
and
E represents a metal ion equivalent or an ammonium ion,
compounds of the formula (Ia) 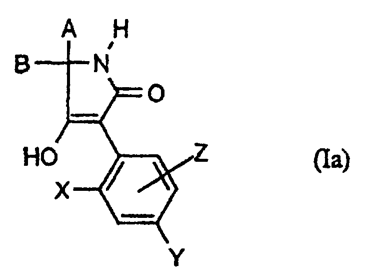 in which
A, B, X, Y and Z have the meaning given above
are reacted with metal hydroxides, metal alkoxides or amines of the formulae (X) and (XI) 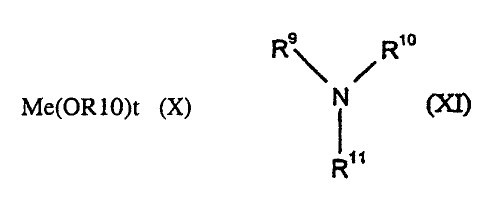 in which
Me represents mono- or divalent metal ions,
t represents the number 1 or 2 and
R⁹, R¹⁰ and R¹¹ independently of one another represent hydrogen and/or alkyl
optionally in the presence of a diluent,
or
(H) to obtain compounds of the formula (Ig) 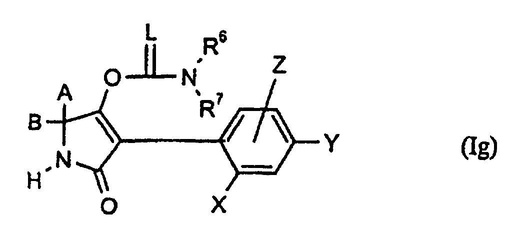 in which
A, B, L, X, Y, Z, R⁶ and R⁷ have the meaning given in Claim 1,
compounds of the formula (Ia) 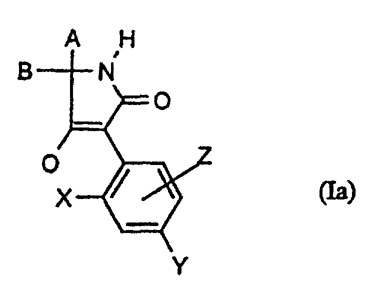 in which
A, B, X, Y and Z have the meaning given above
• ) are reacted with isocyanates or isothiocyanates of the formula (XII)
R⁶-N=C=L (XII)
in which
R⁶ has the meaning given above
optionally in the presence of a diluent and optionally in the presence of a catalyst
or
β) are reacted with arbamoyl chlorides or thiocarbamoyl chlorides of the formula (XIII) 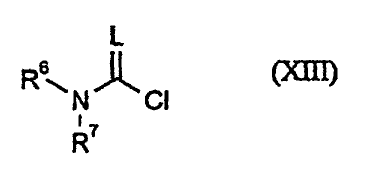 in which
L, R⁶ and R⁷ have the meaning given above
optionally in the presence of a diluent and optionally in the presence of an acid-binding agent.

4. 1H-3-Aryl-pyrrolidine-2,4-diones of the formula (I) according to Claim 1,
in which
A represents hydrogen, represents in each case optionally mono- to hexa-fluoro- or -chloro-substituted C₁-C₁₀-alkyl, C₂-C₆-alkenyl, C₁-C₈-alkoxy-C₁-C₆-alkyl, C₁-C₆-polyalkoxy-C₁-C₆-alkyl or C₁-C₈-alkylthio-C₁-C₆-alkyl, represents optionally fluoro-, chloro-, C₁-C₃-alkyl- or C₁-C₃-alkoxy-substituted cycloalkyl having 3 to 7 ring atoms which may be interrupted by 1 or 2 oxygen and/or sulphur atoms, or represents in each case optionally mono- or di-fluoro-, -chloro-, -bromo-, -C₁-C₄-alkyl-, -C₁-C₄-halogenoalkyl-, -C₁-C₄-alkoxy- or -nitro-substituted phenyl, pyridyl, furanyl, thienyl, imidazolyl, indolyl or phenyl-C₁-C₄-alkyl,
B represents C₁-C₁₀-alkyl or C₁-C₆-alkoxy-C₁-C₄-alkyl, or
A, B and the carbon atom to which they are attached represent a saturated or unsaturated C₃-C₉ spirocyclic radical which is optionally substituted one or more times by C₁-C₆-alkyl, C₃-C₈-cycloalkyl, C₁-C₃-halogenoalkyl, C₁-C₆-alkoxy, C₁-C₆-alkylthio, fluorine, chlorine or phenyl and is optionally interrupted by oxygen or sulphur, or
A, B and the carbon atom to which they are attached represent a C₃-C₆ spirocyclic radical which is substituted by an alkylenediyl group optionally interrupted by one or two oxygen or sulphur atoms, or is substituted by an alkylenedioxy or by an alkylenedithio group which forms, with the carbon atom to which it is attached, a further five- to seven-membered spirocyclic radical, or
A, B and the carbon atom to which they are attached represent a C₃-C₆ spirocyclic radical in which two substituents together represent a saturated or unsaturated five- to seven-membered ring which is optionally substituted by C₁-C₃-alkyl, C₁-C₃-alkoxy, fluorine, chlorine or bromine and may be interrupted by oxygen or sulphur,
X represents C₁-C₅-alkyl or C₁-C₄-alkoxy,
Y represents hydrogen, C₁-C₅-alkyl or C₁-C₄-alkoxy,
Z represents hydrogen, C₁-C₄-alkyl or C₁-C₄-alkoxy,
in which
G represents hydrogen (a) or represents the groups 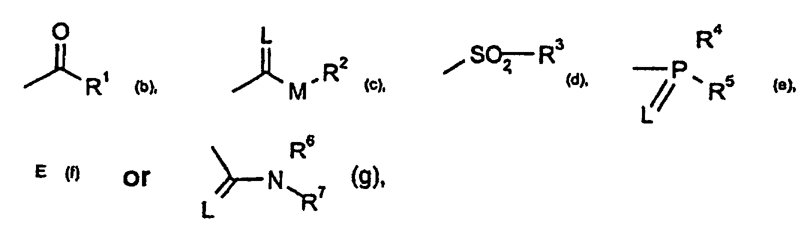
in which
E represents a metal ion equivalent or an ammonium ion,
L represents oxygen or sulphur,
M represents oxygen or sulphur,
R¹ represents in each case optionally mono- to hexa-fluoro- or -chloro-substituted C₁-C₁₆-alkyl, C₂-C₁₆-alkenyl, C₁-C₆-alkoxy-C₁-C₆-alkyl, C₁-C₆-alkylthio-C₁-C₆-alkyl, C₁-C₆-polyalkoxy-C₂-C₆-alkyl or optionally fluoro-, chloro- or C₁-C₅-alkyl-substituted cycloalkyl having 3 to 7 ring atoms, which may be interrupted by 1 or 2 oxygen and/or sulphur atoms,
represents optionally mono- to tri-fluoro-, -chloro-, -bromo-, -nitro-, -C₁-C₄-alkyl-, -C₁-C₄-alkoxy-, -C₁-C₃-halogenoalkyl-, -C₁-C₃-halogenoalkoxy-, -C₁-C₄-alkylthio- and/or C₁-C₄-alkylsulphonyl-substituted phenyl,
represents optionally mono- to tri-fluoro-, -chloro-, -bromo-, -C₁-C₄-alkyl-, -C₁-C₄-alkoxy-, -C₁-C₃-halogenoalkyl-, or -C₁-C₃-halogenoalkoxy-substituted phenyl-C₁-C₄-alkyl,
represents optionally mono- or di-fluoro-, -chloro-, -bromo- or -C₁-C₄-alkyl-substituted pyridyl, thienyl, furanyl, pyrazolyl, pyrimidyl or thiazolyl,
represents optionally mono- or di-fluoro-, -chloro-, -bromo- or -C₁-C₄-alkyl-substituted phenoxy-C₁-C₅-alkyl, or
represents optionally mono- or di-fluoro-, -chloro-, -bromo-, -amino- or -C₁-C₄-alkyl-substituted pyrimidinyloxy-C₁-C₅-alkyl, pyridinyloxy-C₁-C₅-alkyl or thiazolyloxy-C₁-C₅-alkyl,
R² represents in each case optionally mono- to hexa-fluoro- and/or -chloro-substituted C₁-C₁₆-alkyl, C₃-C₁₆-alkenyl, C₁-C₆-alkoxy-C₁-C₆-alkyl or C₁-C₆-polyalkoxy-C₂-C₆-alkyl,
represents optionally mono- to hexa-fluoro-, -chloro-, -C₁-C₃-alkyl- or -C₁-C₃-alkoxy-substituted C₃-C₇-cycloalkyl,
represents optionally mono- or di-fluoro-, -chloro-, -bromo-, -nitro-, -C₁-C₄-alkyl-, -C₁-C₃-alkoxy- or -C₁-C₃-halogenoalkyl-substituted phenyl or benzyl,
R³, R⁴ and R⁵ independently of one another represent in each case optionally mono- to hexa-fluoro- or -chloro-substituted C₁-C₆-alkyl, C₁-C₆-alkoxy-, C₁-C₆-alkylamino, di-(C₁-C₆)-alkylamino, C₁-C₆-alkylthio, C₃-C₄-alkenylthio or C₃-C₆-cycloalkylthio, or represent phenyl, phenoxy or phenylthio each of which is optionally substituted once or twice by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl,
R⁶ and R⁷ independently of one another represent hydrogen, in each case optionally mono- to hexa-fluoro- or -chloro-substituted C₁-C₆-alkyl, C₃-C₆-cycloalkyl, C₁-C₆-alkoxy, C₃-C₆-alkenyl or C₁-C₆-alkoxy-C₂-C₆-alkyl, represent phenyl which is optionally substituted once or twice by fluorine, chlorine, bromine, C₁-C₅-halogenoalkyl, C₁-C₅-alkyl or C₁-C₅-alkoxy, represent benzyl which is optionally substituted once or twice by fluorine, chlorine, bromine, C₁-C₅-alkyl, C₁-C₅-halogenoalkyl or C₁-C₅-alkoxy, or represent, together with the N atom to which they are attached, a C₃-C₆-alkylene ring which is optionally interrupted by oxygen or sulphur,
with the proviso that at least one of the substituents Y and Z represents alkoxy if X represents alkyl.

5. 1H-3-Aryl-pyrrolidine-2,4-diones of the formula (I) according to Claim 1, in which
A represents hydrogen, in each case optionally mono- to tri-fluoro- or -chloro-substituted C₁-C₈-alkyl, C₂-C₄-alkenyl, C₁-C₆-alkoxy-C₁-C₄-alkyl, C₁-C₄-polyalkoxy-C₁-C₄-alkyl or C₁-C₆-alkylthio-C₁-C₄-alkyl, optionally fluoro-, chloro-, methyl-, ethyl-, methoxy- or ethoxy-substituted cycloalkyl having 3 to 6 ring atoms which may be interrupted by 1 or 2 oxygen and/or sulphur atoms, or represents in each case optionally mono- or di-fluoro-, -chloro-, -bromo-, methyl-, -ethyl-, -propyl-, -isopropyl, -methoxy-, -ethoxy-, -trifluoromethyl- and/or -nitro-substituted phenyl, furanyl, thienyl, imidazolyl, indolyl, pyridyl or benzyl,
B represents C₁-C₈-alkyl or C₁-C₄-alkoxy-C₁-C₂-alkyl, or
A, B and the carbon atom to which they are attached represent a saturated or unsaturated C₃-C₈ spirocyclic radical which is optionally substituted one or more times by methyl, ethyl, propyl, isopropyl, butyl, isobutyl, sec-butyl, tert-butyl, cyclohexyl, trifluoromethyl, methoxy, ethoxy, propoxy, isopropoxy, butoxy, isobutoxy, sec-butoxy, tert-butoxy, methylthio, ethylthio, fluorine, chlorine or phenyl and is optionally interrupted by oxygen or sulphur, or
A, B and the carbon atom to which they are attached represent a C₅-C₆ spirocyclic radical which is substituted by an alkylenediyl group optionally interrupted by an oxygen or sulphur atom, or is substituted by an alkylenedioxy group which forms, with the carbon atom to which it is attached, a further five- to seven-membered spirocyclic radical, or
A, B and the carbon atom to which they are attached represent a C₃-C₆ spirocyclic radical in which two substituents together represent a saturated or unsaturated five- or six-membered ring which may be interrupted by oxygen or sulphur,
X represents methyl, ethyl, propyl, isopropyl, methoxy, ethoxy, propoxy or isopropoxy,
Y represents hydrogen, methyl, ethyl, propyl, isopropyl, butyl, isobutyl, sec-butyl, tert-butyl, methoxy, ethoxy, propoxy or isopropoxy,
Z represents hydrogen, methyl, ethyl, propyl, isopropyl, methoxy, ethoxy, propoxy or isopropoxy,
G represents hydrogen (a) or represents the groups 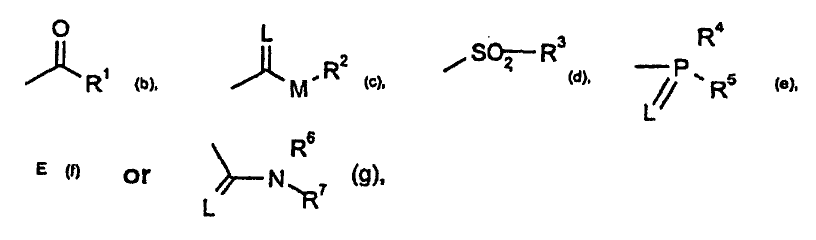
in which
E represents a metal ion equivalent or an ammonium ion and
L represents oxygen or sulphur,
M represents oxygen or sulphur,
R¹ represents in each case optionally mono- to tri-fluoro- or -chloro-substituted C₁-C₁₄-alkyl, C₂-C₁₄-alkenyl, C₁-C₄-alkoxy-C₁-C₆-alkyl, C₁-C₄-alkylthio-C₁-C₆-alkyl, or C₁-C₄-polyalkoxy-C₂-C₄-alkyl, or represents optionally fluoro-, chloro-, methyl-, ethyl-, propyl-, isopropyl-, butyl-, isobutyl- or t-butyl-substituted cycloalkyl having 3 to 6 ring atoms, which may be interrupted by 1 or 2 oxygen and/or sulphur atoms,
represents optionally mono- or di-fluoro-, -chloro-, -bromo-, -methyl-, -ethyl-, -propyl-, -isopropyl-, -methoxy-, -ethoxy-, -trifluoromethyl-, -trifluoromethoxy-, -methylthio-, -ethylthio-, -methylsulphonyl-, -ethylsulphonyl- or -nitro-substituted phenyl,
represents optionally mono- or di-fluoro-, -chloro-, -bromo-, -methyl-, -ethyl-, -propyl-, -isopropyl-, -methoxy-, -ethoxy-, -trifluoromethyl- or -trifluoromethoxy-substituted phenyl-C₁-C₃-alkyl,
represents optionally mono- or di-fluoro-, -chloro-, -bromo-, -methyl- or -ethyl-substituted thienyl, furanyl or pyridyl,
represents optionally mono- or di-fluoro-, -chloro-, -methyl- or -ethyl-substituted phenoxy-C₁-C₄-alkyl, or
represents in each case optionally mono- to di-fluoro-, -chloro-, -amino-, -methyl- or -ethyl-substituted pyridyloxy-C₁-C₄-alkyl, pyrimidinyloxy-C₁-C₄-alkyl or thiazolyloxy-C₁-C₄-alkyl,
R² represents in each case optionally mono- to tri-fluoro- or -chloro-substituted C₁-C₁₄-alkyl, C₃-C₁₄-alkenyl, C₁-C₄-alkoxy-C₁-C₆-alkyl or C₁-C₄-polyalkoxy-C₂-C₆-alkyl,
represents optionally mono- to tri-fluoro-, -chloro-, -methyl-, -ethyl-, -propyl-, -isopropyl- or -methoxy-substituted C₃-C₆-cycloalkyl,
or represents in each case optionally mono- or di-fluoro-, -chloro-, -nitro-, -methyl-, -ethyl-, -propyl-, -isopropyl-, -methoxy-, -ethoxy- or -trifluoromethyl-substituted phenyl or benzyl,
R³, R⁴, and R⁵ independently of one another represent in each case optionally mono- to tri-fluoro- or -chloro-substituted C₁-C₄-alkyl, C₁-C₄-alkoxy, C₁-C₄-alkylamino, di-(C₁-C₄)-alkylamino or C₁-C₄-alkylthio, or represent phenyl, phenoxy or phenylthio each of which is optionally mono- or di-substituted by fluorine, chlorine, bromine, nitro, cyano, C₁-C₂-alkoxy, C₁-C₂-fluoroalkoxy, C₁-C₂-alkylthio, C₁-C₂-fluoroalkylthio, C₁-C₂-alkyl or C₁-C₂-fluoroalkyl, and
R⁶ and R⁷ independently of one another represent hydrogen, represent in each case optionally mono- to tri-fluoro- or -chloro-substituted C₁-C₄-alkyl, C₃-C₆-cycloalkyl, C₁-C₄-alkoxy, C₃-C₄-alkenyl, C₁-C₄-alkoxy-C₂-C₄-alkyl, represent phenyl which is optionally substituted once or twice by fluorine, chlorine, bromine, C₁-C₄-halogenoalkyl, C₁-C₄-alkyl and/or C₁-C₄-alkoxy, represent benzyl which is optionally substituted once or twice by fluorine, chlorine, bromine, C₁-C₄-alkyl, C₁-C₄-halogenoalkyl and/or C₁-C₄-alkoxy, or represent, together with the N atom to which they are attached, a C₃-C₆-alkylene ring which is optionally interrupted by oxygen or sulphur,
with the proviso that at least one of the substituents Y and Z represents alkoxy if X represents alkyl.

6. Compounds of the formula (II) 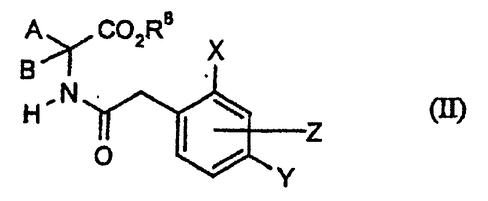 in which
A, B, X, Y and Z have the meaning given in Claim 1 and
R⁸ represents alkyl.

7. Process for the preparation of the acyl-amino acid esters of the formula (II) according to Claim 7, **characterized in that** amino acid derivatives of the formula (XIV) 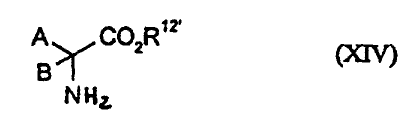 in which
R^{12'} represents hydrogen (XIVa) or alkyl (XIVb)
and
A and B have the meaning given above
are acylated with phenylacetyl halides of the formula (XV) 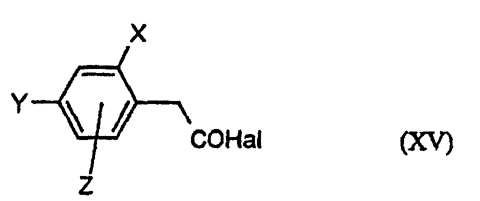 in which
X, Y and Z have the meaning given above
Hal represents chlorine or bromine,
or **in that** acylamino acids of the formula (IIa) 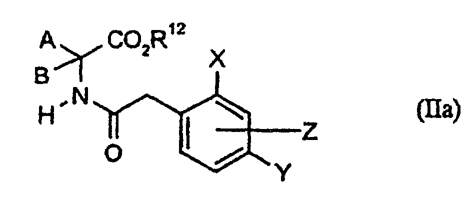 in which
A, B, X, Y and Z have the meaning given above
and
R¹² represents hydrogen
are esterified
or
**in that** aminonitriles of the formula (XVI) 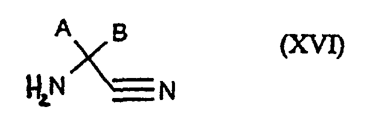 in which
A and B have the meaning given above
are reacted with phenylacetyl halides of the formula (XV) 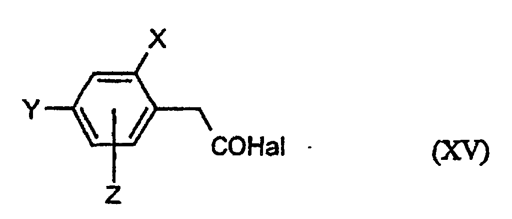 in which
X, Y and Z have the meaning given above and
Hal represents chlorine or bromine,
to give compounds of the formula (XVII) 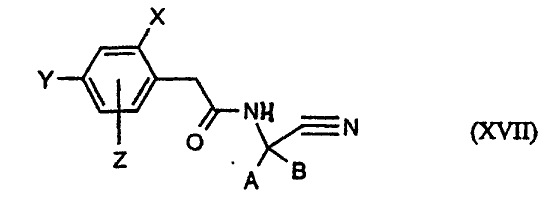 in which
A, B, X, Y and Z have the meaning given above
and these compounds are subsequently subjected to alcoholysis in the presence of sulphuric acid.

8. Compounds of the formula (XVII) 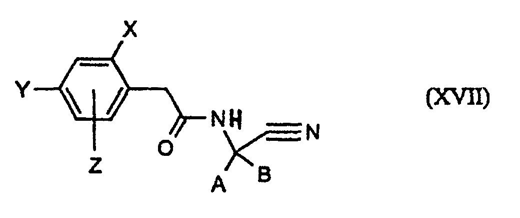 in which
A, B, X, Y and Z have the meaning given in Claim 1.

9. Process for the preparation of compounds of the formula (XVII) according to Claim 9, **characterized in that** aminonitriles of the formula (XVI) 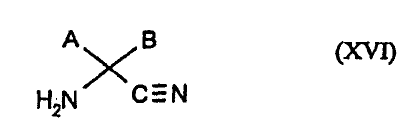 in which
A and B have the meaning given in Claim 1
are reacted with phenylacetyl halides of the formula (XV) 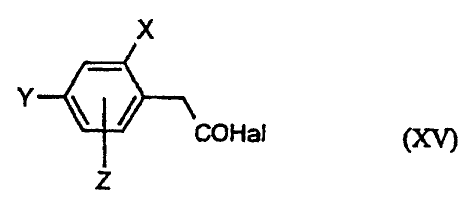 in which
X, Y and Z have the meaning given in Claim 1 and
Hal represents chlorine or bromine.

10. Pesticides and weed control compositions, **characterized by** a content of at least one 1H-3-aryl-pyrrolidine-2,4-dione derivative of the formula (I) according to Claim 1.

11. Use of 1H-3-aryl-pyrrolidine-2,4-dione derivatives of the formula (I) according to Claim 1 for controlling pests and weeds.

12. Method of controlling pests and weeds, **characterized in that** 1H-3-arylpyrrolidine-2,4-drone derivatives of the formula (I) according to Claim 1 are allowed to act on pests and/or their habitat or on weeds and/or their habitat.

13. Process for the preparation of pesticides and weed control compositions, **characterized in that** 1H-3-aryl-pyrrolidine-2,4-dione derivatives of the formula (I) according to Claim 1 are mixed with extenders and/or surface-active agents.

## Revendications

1. Dérivés de 1-H-3-arylpyrrolidin-2,4-dione de la formule (I) : 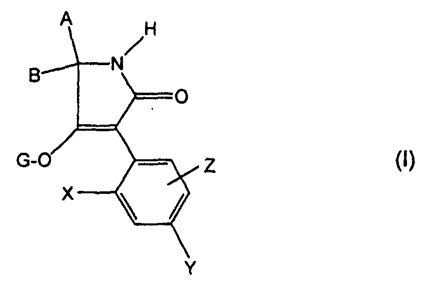 dans laquelle :
A représente l'atome d'hydrogène ou un radical alcoyle en C₁-C₁₂, alcényle en C₂-C₈, (alcoxy en C₁-C₁₀)alcoyle en C₁-C₈, poly(alcoxy en C₁-C₈)alcoyle en C₁-C₈, (alcoylthio en C₁-C₁₀)alcoyle en C₁-C₆, le cas échéant substitué une ou plusieurs fois par un atome d'halogène, un radical cycloalcoyle en 3 à 8 atomes cycliques, le cas échéant substitué par un atome d'halogène, un radical alcoyle en C₁-C₄ ou alcoxy en C₁-C₄, qui peut être interrompu par des atomes d'oxygène et/ou de soufre ou le radical phényle, naphtyle, pyridyle, imidazolyle, indolyle, thiazolyle, furannyle, thiényle, phényl(alcoyle en C₁-C₆) ou naphtyl(alcoyle en C₁-C₆). le cas échéant substitué une ou plusieurs fois par un atome d'halogène, un radical alcoyle en C₁-C₆, halogénoalcoyle en C₁-C₆, alcoxy en C₁-C₆ et/ou nitro,
B représente un radical alcoyle en C₁-C₁₂ ou (alcoxy en C₁-C₈)alcoyle en C₁-C₆, ou
A, B et l'atome de carbone sur lequel ils sont liés, représentent un cycle spiro en C₃-C₁₀ saturé ou insaturé, qui est le cas échéant, substitué une ou plusieurs fois par un radical alcoyle en C₁-C₆, cycloalcoyle en C₃-C₈, halogénoalcoyle en C₁-C₄, alcoxy en C₁-C₆, alcoylthio en C₁-C₆, un atome d'halogène ou le radical phényle et est le cas échéant interrompu par un atome d'oxygène ou de soufre, ou
A, B et l'atome de carbone sur lequel ils sont liés, représentent un cycle spiro en C₃-C₆, qui est le cas échéant, substitué par un radical alcoylènediyle le cas échéant interrompu par un ou deux atomes d'oxygène et/ou de soufre, ou par un radical alcoylènedioxyle ou alcoylènedithioyle, qui avec l'atome de carbone sur lequel il est lié, forme un autre cycle spiro de cinq à huit membres, ou
A, B et l'atome de carbone sur lequel ils sont liés, représentent un cycle spiro en C₃-C₈, dans lequel deux substituants représentent ensemble un cycle de 5 à 8 membres saturé ou insaturé, le cas échéant substitué par un radical alcoyle en C₁-C₄, alcoxy en C₁-C₄ ou un atome d'halogène, lequel peut être interrompu par un atome d'oxygène ou de soufre ;
X représente un radical alcoyle en C₁-C₆ ou alcoxy en C₁-C₆ ;
Y représente l'atome d'hydrogène, un radical alcoyle en C₁-C₆ ou alcoxy en C₁-C₆ ;
Z représente l'atome d'hydrogène, un radical alcoyle en C₁-C₆ ou alcoxy en C₁-C₆ ;
G représente l'atome d'hydrogène (a) ou les radicaux : 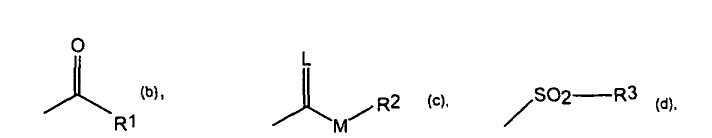 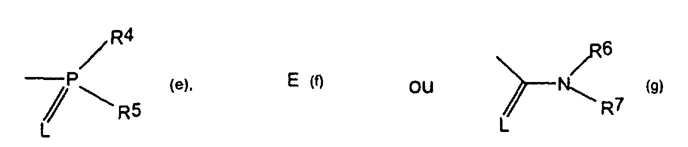 dans lesquels :
E représente un équivalent d'ion métallique ou un ion ammonium, et
L représente l'atome d'oxygène ou de soufre,
M représente l'atome d'oxygène ou de soufre,
R¹ représente un radical alcoyle en C₁-C₂₀, alcényle en C₂-C₂₀, (alcoxy en C₁-C₈)alcoyle en C₁-C₈, (alcoylthio en C₁-C₈)alcoyle en C₁-C₈, poly(alcoxy en C₁-C₈)alcoyle en C₂-C₈, le cas échéant substitué une ou plusieurs fois par un atome d'halogène, ou un radical cycloalcoyle en 3 à 8 atomes cycliques, le cas échéant substitué par un atome d'halogène ou un radical alcoyle en C₁-C₆, lequel peut être interrompu par au moins un atome d'oxygène et/ou de soufre,
représente le radical phényle le cas échant substitué une à cinq fois par un atome d'halogène, le radical nitro, un radical alcoyle en C₁-C₆, alcoxy en C₁-C₆, halogénoalcoyle en C₁-C₆, halogénoalcoxy en C₁-C₆, alcoylthio en C₁-C₆ ou alcoylsulfonyle en C₁-C₆,
représente un radical phényl(alcoyle en C₁-C₆) le cas échant substitué une à cinq fois par un atome d'halogène, un radical alcoyle en C₁-C₆, alcoxy en C₁-C₆, halogénoalcoyle en C₁-C₆ ou halogénoalcoxy en C₁-C₆,
représente un radical pyridyle, thiényle, furannyle, pyrazolyle, pyrimidyle ou thiazolyle le cas échant substitué une à trois fois par un atome d'halogène ou un radical alcoyle en C₁-C₆,
représente un radical phénoxy(alcoyle en C₁-C₆) le cas échant substitué une à trois fois par un atome d'halogène ou un radical alcoyle en C₁-C₆, ou
représente un radical pyridinyloxy(alcoyle en C₁-C₆), pyrimidinyloxy(alcoyle en C₁-C₆), ou thiazolyloxy(alcoyle en C₁-C₆), le cas échant substitué une à deux fois par un atome d'halogène ou un radical amino ou alcoyle en C₁-C₆,
R² représente un radical alcoyle en C₁-C₂₀, alcényle en C₃-C₂₀, (alcoxy en C₁-C₈)alcoyle en C₂-C₈, poly(alcoxy en C₁-C₈)alcoyle en C₂-C₈, le cas échéant substitué une ou plusieurs fois par un atome d'halogène,
représente un radical cycloalcoyle en C₃-C₈, le cas échéant substitué une ou plusieurs fois par un atome d'halogène, un radical alcoyle en C₁-C₄ ou alcoxy en C₁-C₄,
représente le radical phényle ou benzyle, le cas échant substitué une à trois fois par un atome d'halogène, le radical nitro, un radical alcoyle en C₁-C₆, alcoxy en C₁-C₆ ou halogénoalcoyle en C₁-C₆ ;
R³ , R⁴ et R⁵ représentent indépendamment l'un de l'autre, un radical alcoyle en C₁-C₈, alcoxy en C₁-C₈, alcoylamino en C₁-C₈, di(alcoyl en C₁-C₈)amino, alcoylthio en C₁-C₈, alcénylthio en C₃-C₆, cycloalcoylthio en C₃-C₇, le cas échéant substitué une ou plusieurs fois par un atome d'halogène ; le radical phényle, phénoxy ou phénylthio, le cas échéant substitué une ou plusieurs fois par un atome d'halogène, le radical nitro, cyano, un radical alcoxy en C₁-C₄, halogénoalcoxy en C₁-C₄, alcoylthio en C₁-C₄, halogénoalcoylthio en C₁-C₄, alcoyle en C₁-C₄ ou halogénoalcoyle en C₁-C₄ ;
R⁶ et R⁷ représentent indépendamment l'un de l'autre, l'atome d'hydrogène, un radical alcoyle en C₁-C₈, cycloalcoyle en C₃-C₈, alcoxy en C₁-C₈, alcényle en C₃-C₈, (alcoxy en C₁-C₈)alcoyle en C₂-C₈, le cas échéant substitué une ou plusieurs fois par un atome d'halogène ; le radical phényle le cas échéant substitué une à trois fois par un atome d'halogène, un radical halogénoalcoyle en C₁-C₈, alcoyle en C₁-C₈ ou alcoxy en C₁-C₈ ; un radical benzyle le cas échéant substitué une à trois fois par un atome d'halogène, un radical alcoyle en C₁-C₈, halogénoalcoyle en C₁-C₈ et/ou alcoxy en C₁-C₈ ; ou avec l'atome d'azote sur lequel ils sont liés, un cycle alcoylène en C₃-C₆ le cas échéant interrompu par un atome d'oxygène ou de soufre,
avec la condition qu'au moins un des substituants Y et Z représente alcoxy lorsque X représente alcoyle.

2. Dérivés de 1-H-3-arylpyrrolidin-2,4-dione de la formule (I) suivant la revendication 1, qui possèdent en incorporant les différentes significations (a), (b), (c), (d), (e), (f) et (g) du radical G, les structures (Ia) à (Ig) : 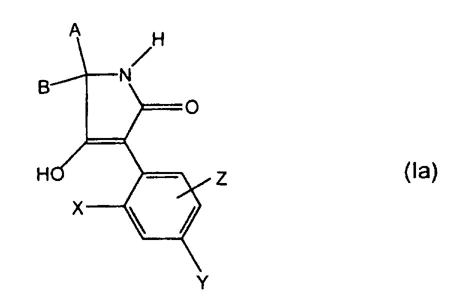 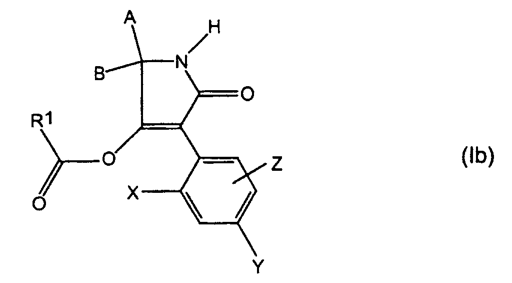 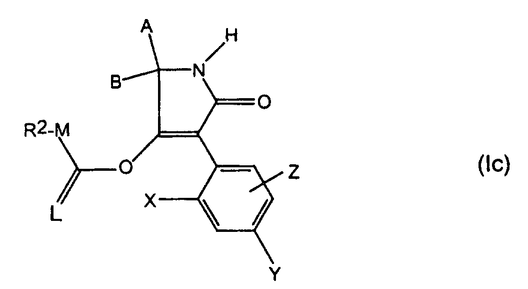 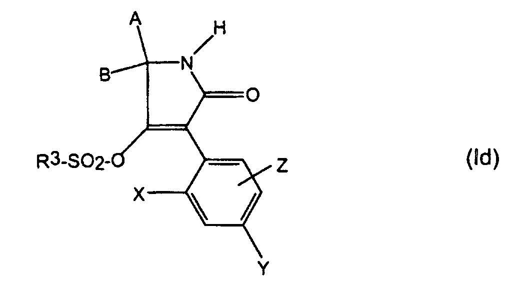 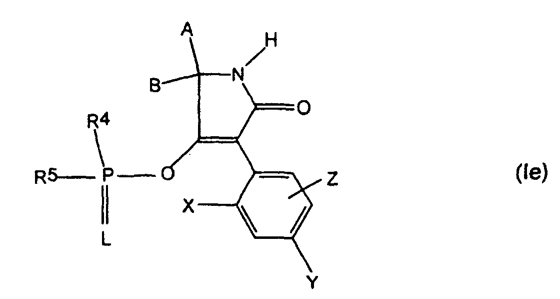 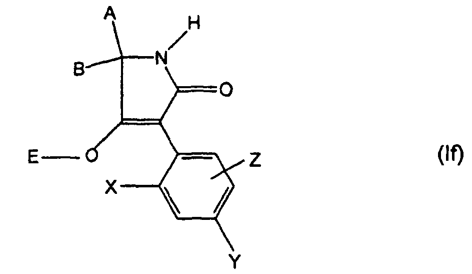 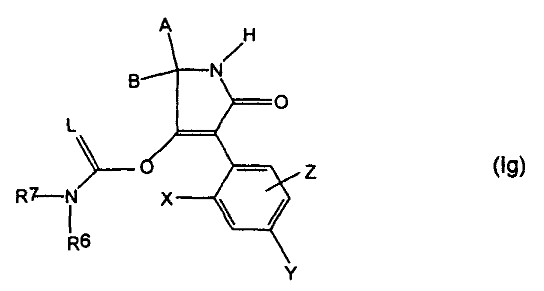 où
A, B, E, L, M, X, Y, Z, R¹, R², R³, R⁴, R⁵, R⁶ et R⁷ possèdent les significations indiquées à la revendication 1.

3. Procédé de préparation de dérivés de 1-H-3-arylpyrrolidin-2,4-dione de la formule (I) suivant la revendication 1, **caractérisé en ce que** :
(A) pour l'obtention des 1-H-3-arylpyrrolidin-2,4-diones ou de leurs énols de formule (Ia) : 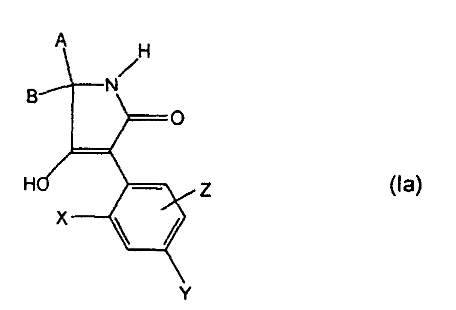 dans laquelle :
A, B, X, Y et Z ont les significations indiquées à la revendication 1,
on condense de manière intramoléculaire, des esters d'acide N-acylaminé de la formule (II) : 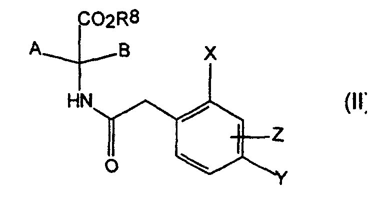 dans laquelle :
A, B, X, Y et Z ont les significations indiquées à la revendication 1,
et R⁸ représente un radical alcoyle,
en présence d'un agent de dilution et en présence d'une base, ou
(B) pour l'obtention des composés de la formule (Ib) : 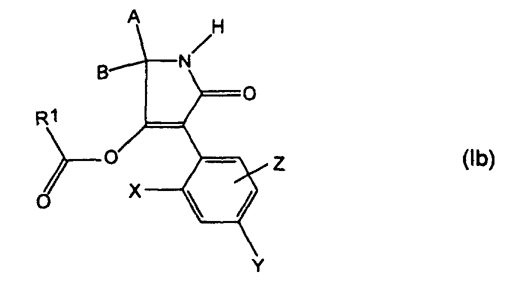 dans laquelle :
A, B, X, Y, Z et R¹ ont les significations indiquées à la revendication 1,
on fait réagir des composés de formule (Ia) : 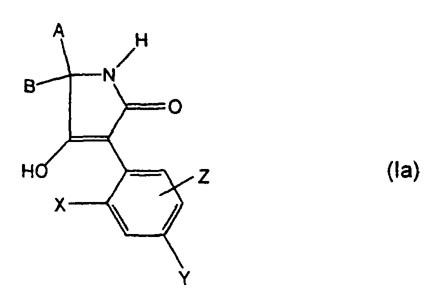 dans laquelle :
A, B, X, Y et Z ont les significations indiquées à la revendication 1,
α) avec des halogénures d'acide de formule (III) : 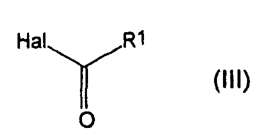 dans laquelle :
R¹ a la signification indiquée ci-dessus, et
Hal représente halogène,
le cas échéant en présence d'un agent de dilution et le cas échéant, en présence d'un agent liant les acides, ou
β) avec des anhydrides d'acide carboxylique de formule (IV) :
R¹-CO-O-CO-R¹ (IV)
dans laquelle :
R¹ a la signification indiquée ci-dessus,
le cas échéant en présence d'un agent de dilution et le cas échéant, en présence d'un agent liant les acides, ou
(C) pour l'obtention de composés de formule (Ic-a) : 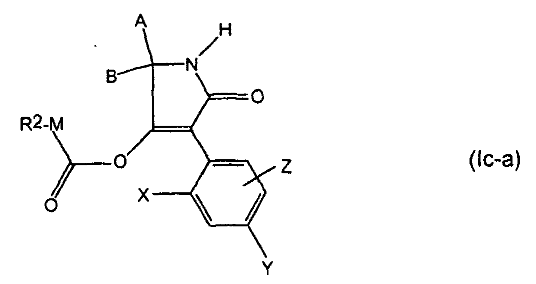 dans laquelle :
A, B, X, Y, Z et R² ont les significations indiquées ci-dessus et
M représente l'atome d'oxygène ou de soufre,
on fait réagir des composés de formule (Ia) : 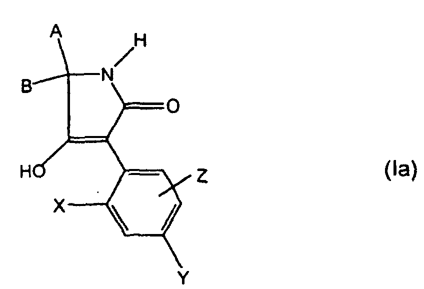 dans laquelle :
A, B, X, Y et Z ont les significations indiquées ci-dessus,
avec un ester d'acide chloroformique ou un thiolester d'acide chloroformique de formule (V) :
R²-M-CO-Cl (V)
dans laquelle
R² et M ont la signification indiquée ci-dessus,
le cas échéant en présence d'un agent de dilution et le cas échéant, en présence d'un agent liant les acides ;
ou
(D) pour l'obtention de composés de formule (Ic-b) : 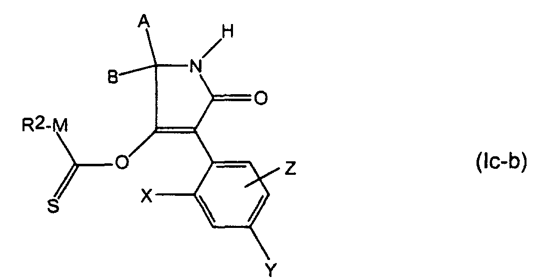 dans laquelle :
A, B, X, Y, Z et R² ont les significations indiquées et
M représente l'atome d'oxygène ou de soufre,
on fait réagir des composés de formule (Ia) : 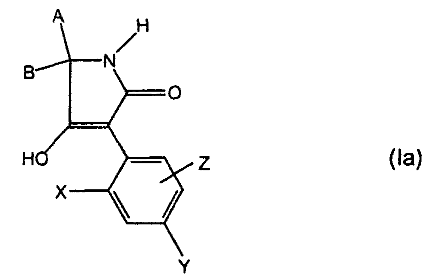 dans laquelle :
A, B, X, Y et Z ont les significations indiquées ci-dessus,
α) avec des esters d'acide chloromonothioformique ou des esters d'acide chlorodithioformique de formule (VI) : 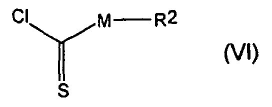 dans laquelle
R² et M ont la signification indiquée ci-dessus,
le cas échéant en présence d'un agent de dilution et le cas échéant, en présence d'un agent liant les acides, ou
β) avec le sulfure d'hydrogène et ensuite, avec des halogénures d'alkyle de la formule (VII) :
R²-Hal (VII)
dans laquelle
R² a la signification indiquée ci-dessus, et
Hal représente l'atome de chlore, de brome ou d'iode,
le cas échéant en présence d'un agent de dilution et le cas échéant, en présence d'une base ;
ou
(E) pour l'obtention des composés de formule (Id) : 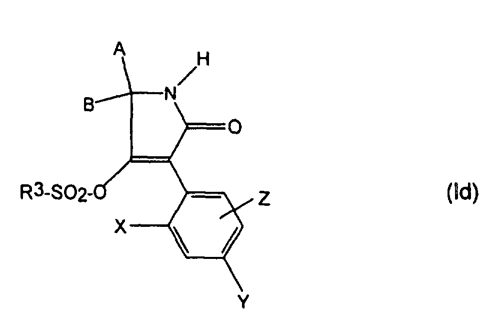 dans laquelle :
A, B, X, Y, Z et R³ ont les significations indiquées à la revendication 1,
on fait réagir des composés de formule (Ia) : 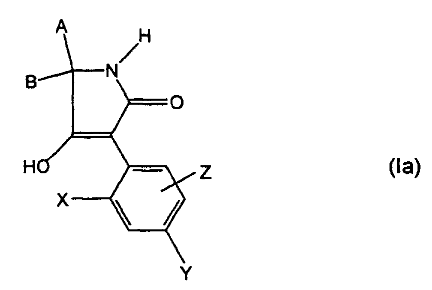 dans laquelle :
A, B, X, Y et Z ont les significations indiquées ci-dessus,
avec des chlorures d'acide sulfonique de formule (VIII) :
R³-SO₂-Cl (VIII)
dans laquelle
R³ a la signification indiquée ci-dessus, et
le cas échéant en présence d'un agent de dilution et le cas échéant, en présence d'un agent liant les acides ;
ou
(F) pour l'obtention de 3-arylpyrrolidin-2,4-diones de formule (Ie) : 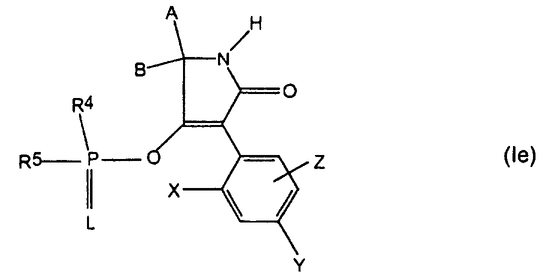 dans laquelle :
A, B, L, X, Y, Z, R⁴ et R⁵ ont les significations indiquées à la revendication 1,
on fait réagir des 1-H-3-arylpyrrolidin-2,4-diones de formule (Ia) ou leurs énols : 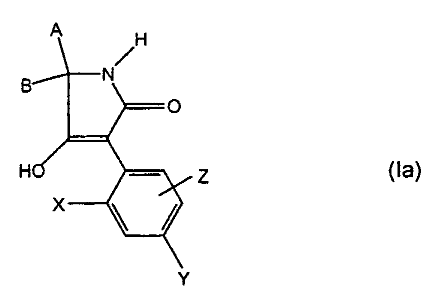 dans laquelle :
A, B, X, Y et Z ont les significations indiquées ci-dessus,
avec des composés du phosphore de formule (IX) : 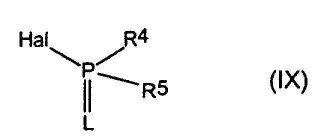 dans laquelle :
L, R⁴ et R⁵ ont les significations indiquées ci-dessus, et
Hal représente un atome d'halogène, en particulier l'atome de chlore ou de brome,
le cas échéant en présence d'un agent de dilution et le cas échéant, en présence d'un agent liant les acides ;
ou
(G) pour l'obtention de composés de formule (If) : 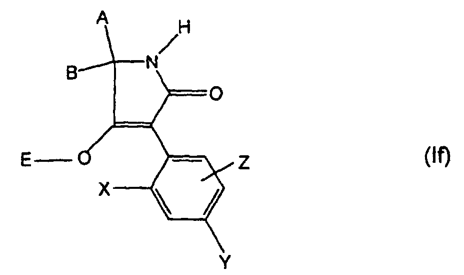 dans laquelle :
A, B, X, Y et Z ont les significations indiquées ci-dessus, et
E représente un équivalent d'ion métallique ou un ion ammonium,
on fait réagir des composés de formule (Ia) : 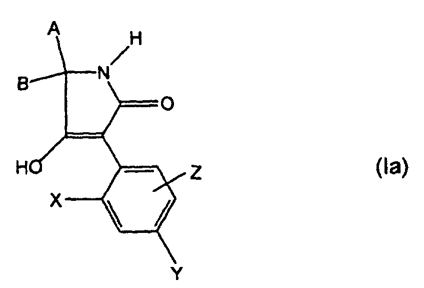 dans laquelle :
A, B, X, Y et Z ont les significations indiquées ci-dessus,
avec des hydroxydes métalliques, des alkoxydes métalliques ou des amines des formules (X) et (XI) : 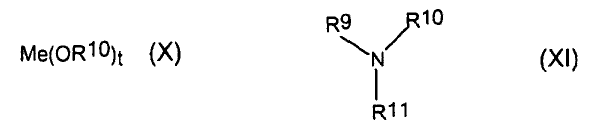 dans laquelle :
Me représente un ion métallique mono- ou bivalent,
t représente le nombre 1 ou 2, et
R⁹, R¹⁰ et R¹¹ représentent indépendamment l'un de l'autre, l'atome d'hydrogène et/ou un radical alcoyle,
le cas échéant en présence d'un agent de dilution,
ou
(H) pour l'obtention de composés de formule (Ig) : 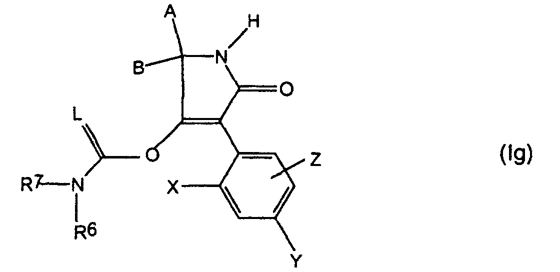 dans laquelle :
A, B, L, X, Y, Z, R⁶ et R⁷ ont les significations indiquées à la revendication 1, et
on fait réagir des composés de formule (Ia) : 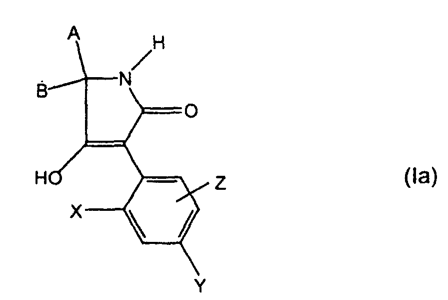 dans laquelle :
A, B, X, Y et Z ont les significations indiquées ci-dessus,
α) avec des isocyanates ou isothiocyanates de formule (XII) :
R⁶-N=C=L (XII)
dans laquelle
R⁶ a la signification indiquée ci-dessus,
le cas échéant en présence d'un agent de dilution, le cas échéant en présence d'un catalyseur,
ou
β) avec des chlorures d'acide carbamique ou des chlorures d'acide thiocarbamique de formule (XIII) : 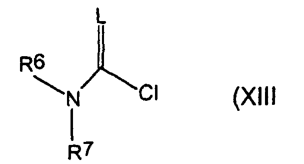 dans laquelle :
L, R⁶ et R⁷ ont la signification indiquée ci-dessus,
le cas échéant en présence d'un agent de dilution et le cas échéant en présence d'un agent liant les acides.

4. 1-H-3-Arylpyrrolidin-2,4-diones de la formule (I) selon la revendication 1, dans laquelle :
A représente l'atome d'hydrogène ou un radical alcoyle en C₁-C₁₀, alcényle en C₂-C₆, (alcoxy en C₁-C₈)alcoyle en C₁-C₆, poly(alcoxy en C₁-C₆) alcoyle en C₁-C₆, (alcoylthio en C₁-C₈) alcoyle en C₁-C₆, le cas échéant substitués une à six fois par l'atome de fluor ou de chlore, un radical cycloalcoyle en 3 à 7 atomes cycliques, le cas échéant substitué par l'atome de fluor, de chlore, un radical alcoyle en C₁-C₃ ou alcoxy en C₁-C₃, qui peut être interrompu par 1 ou 2 atomes d'oxygène et/ou de soufre ou le radical phényle, pyridyle, furannyle, thiényle, imidazolyle, indolyle ou phényl(alcoyle en C₁-C₄) , le cas échéant substitué une à six fois par l'atome de fluor, de chlore, de brome, un radical alcoyle en C₁-C₄, halogénoalcoyle en C₁-C₉, alcoxy en C₁-C₄ ou nitro,
B représente un radical alcoyle en C₁-C₁₀ ou (alcoxy en C₁-C₆) alcoyle en C₁-C₉, ou
A, B et l'atome de carbone sur lequel ils sont liés, représentent un cycle spiro en C₃-C₉ saturé ou insaturé, qui est le cas échéant, substitué une ou plusieurs fois par un radical alcoyle en C₁-C₆, cycloalcoyle en C₃-C₈, halogénoalcoyle en C₁-C₃, alcoxy en C₁-C₆, alcoylthio en C₁-C₆, l'atome de fluor, de chlore ou le radical phényle et le cas échéant interrompu par un atome d'oxygène ou de soufre, ou
A, B et l'atome de carbone sur lequel ils sont liés, représentent un cycle spiro en C₃-C₆, qui est le cas échéant, substitué par un radical alcoylènediyle le cas échéant interrompu par un ou deux atomes d'oxygène ou de soufre, ou par un radical alcoylènedioxyle ou alcoylènedithiol, qui avec l'atome de carbone sur lequel il est lié, forme un autre cycle spiro de cinq à sept membres, ou
A, B et l'atome de carbone sur lequel ils sont liés, représentent un cycle spiro en C₃-C₆, dans lequel deux substituants représentent ensemble un cycle de cinq à sept membres saturé ou insaturé, le cas échéant substitué par un radical alcoyle en C₁-C₃, alcoxy en C₁-C₃ ou l'atome de fluor, de chlore ou de brome, lequel peut être interrompu par un atome d'oxygène ou de soufre ;
X représente un radical alcoyle en C₁-C₅ ou alcoxy en C₁-C₅ ;
Y représente l'atome d'hydrogène, un radical alcoyle en C₁-C₅ ou alcoxy en C₁-C₄ ;
Z représente l'atome d'hydrogène, un radical alcoyle en C₁-C₄ ou alcoxy en C₁-C₄ ;
G représente l'atome d'hydrogène (a) ou les radicaux : 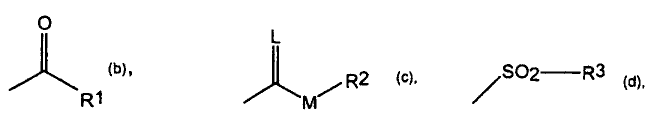 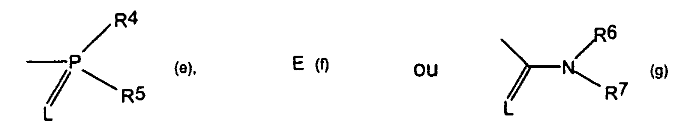 dans lesquels :
E représente un équivalent d'ion métallique ou un ion ammonium, et
L représente l'atome d'oxygène ou de soufre,
M représente l'atome d'oxygène ou de soufre,
R¹ représente un radical alcoyle en C₁-C₁₆, alcényle en C₂-C₁₆, (alcoxy en C₁-C₆) alcoyle en C₁-C₆, (alcoylthio en C₁-C₆)alcoyle en C₁-C₆, poly(alcoxy en C₁-C₆)alcoyle en C₂-C₆, le cas échéant substitué une à six fois par l'atome de fluor ou de chlore, ou un radical cycloalcoyle en 3 à 7 atomes cycliques, le cas échéant substitué par l'atome de fluor, de chlore ou un radical alcoyle en C₁-C₅, lequel peut être interrompu par 1 ou 2 atomes d'oxygène et/ou de soufre,
représente le radical phényle le cas échant substitué une à trois fois par l'atome de fluor, de chlore, de brome, le radical nitro, un radical alcoyle en C₁-C₄ , alcoxy en C₁-C₄, halogénoalcoyle en C₁-C₃, halogénoalcoxy en C₁-C₃, alcoylthio en C₁-C₄ ou alcoylsulfonyle en C₁-C₄,
représente un radical phényl(alcoyle en C₁-C₄) le cas échant substitué une à trois fois par l'atome de fluor, de chlore, de brome, un radical alcoyle en C₁-C₄, alcoxy en C₁-C₄, halogénoalcoyle en C₁-C₃ ou halogénoalcoxy en C₁-C₃,
représente un radical pyridyle, thiényle, furannyle, pyrazolyle, pyrimidyle ou thiazolyle le cas échant substitué une à deux fois par l'atome de fluor, de chlore, de brome, ou un radical alcoyle en C₁-C₄,
représente un radical phénoxy(alcoyle en C₁-C₅) le cas échant substitué une à deux fois par l'atome de fluor, de chlore, de brome, ou un radical alcoyle en C₁-C₄, ou
représente un radical pyrimidinyloxy(alcoyle en C₁-C₅), pyridinyloxy(alcoyle en C₁-C₅), ou thiazolyloxy(alcoyle en C₁-C₅), le cas échant substitué une à deux fois par l'atome de fluor, de chlore, de brome, ou un radical amino ou alcoyle en C₁-C₄,
R² représente un radical alcoyle en C₁-C₁₆, alcényle en C₃-C₁₆, (alcoxy en C₁-C₆) alcoyle en C₁-C₆, poly(alcoxy en C₁-C₆ ) alcoyle en C₂-C₆, le cas échéant substitué une à six fois par l'atome de fluor et/ou de chlore,
représente un radical cycloalcoyle en C₃-C₇, le cas échéant substitué une à six fois par l'atome de fluor, de chlore, un radical alcoyle en C₁-C₃ ou alcoxy en C₁-C₃,
représente le radical phényle ou benzyle, le cas échant substitué une à deux fois par l'atome de fluor, de chlore, de brome, le radical nitro, un radical alcoyle en C₁-C₄, alcoxy en C₁-C₃ ou halogénoalcoyle en C₁-C₃ ;
R³ , R⁴ et R⁵ représentent indépendamment l'un de l'autre, un radical alcoyle en C₁-C₆, alcoxy en C₁-C₆, alcoylamino en C₁-C₆, di(alcoyl en C₁-C₆) amino, alcoylthio en C₁-C₆, alcénylthio en C₃-C₄, cycloalcoylthio en C₃-C₆, le cas échéant substitué une à six fois par l'atome de fluor ou de chlore ; le radical phényle, phénoxy ou phénylthio, le cas échéant substitué une à deux fois par l'atome de fluor, de chlore, de brôme, le radical nitro,. cyano, un radical alcoxy en C₁-C₃, halogénoalcoxy en C₁-C₃, alcoylthio en C₁-C₃, halogénoalcoylthio en C₁-C₃, alcoyle en C₁-C₃ ou halogénoalcoyle en C₁-C₃;
R⁶ et R⁷ représentent indépendamment l'un de l'autre, l'atome d' hydrogène, un radical alcoyle en C₁-C₆, cycloalcoyle en C₃-C₆, alcoxy en C₁-C₆, alcényle en C₃-C₆, (alcoxy en C₁-C₆)alcoyle en C₂-C₆, le cas échéant substitué une à six fois par l'atome de fluor ou de chlore ; le radical phényle le cas échéant substitué une à deux fois par l'atome de fluor, de chlore, de brome, un radical halogénoalcoyle en C₁-C₅, alcoyle en C₁-C₅ ou alcoxy en C₁-C₅; le radical benzyle le cas échéant substitué une à deux fois par l'atome de fluor, de chlore, de brome, alcoyle en C₁-C₅, un radical halogénoalcoyle en C₁-C₅ ou alcoxy en C₁-C₅ : ou avec l'atome d'azote sur lequel ils sont liés, un cycle alcoylène en C₃-C₆ le cas échéant interrompu par un atome d'oxygène ou de soufre,
avec la condition qu'au moins un des substituants Y et Z représente alcoxy lorsque X représente alcoyle.

5. 1-H-3-Arylpyrrolidin-2,4-diones de la formule (I) selon la revendication 1, dans laquelle :
A représente l'atome d'hydrogène ou un radical alcoyle en C₁-C₈, alcényle en C₂-C₄, (alcoxy en C₁-C₆) alcoyle en C₁-C₄, poly(alcoxy en C₁-C₄)alcoyle en C₁-C₄, (alcoylthio en C₁-C₆) alcoyle en C₁-C₄, le cas échéant substitués une à trois fois par l'atome de fluor ou de chlore, un radical cycloalcoyle en 3 à 6 atomes cycliques, le cas échéant substitué par l'atome de fluor, de chlore, le radical méthyle, éthyle, méthoxy ou éthoxy, qui peut être interrompu par 1 ou 2 atomes d'oxygène et/ou de soufre, ou le radical phényle, furannyle, thiényle, imidazolyle, indolyle, pyridyle ou benzyle, le cas échéant substitué une à deux fois par l'atome de fluor, de chlore, de brome, le radical méthyle, éthyle, propyle, isopropyle, méthoxy, éthoxy, trifluorométhyle et/ou nitro,
B représente un radical alcoyle en C₁-C₈ ou (alcoxy en C₁-C₄)alcoyle en C₁-C₂, ou
A, B et l'atome de carbone sur lequel ils sont liés, représentent un cycle spiro en C₃-C₈ saturé ou insaturé, qui est le cas échéant, substitué une ou plusieurs fois par le radical méthyle, éthyle, propyle, isopropyle, butyle, isobutyle, s-butyle, t-butyle, cyclohexyle, trifluorométhyle, méthoxy, éthoxy, propoxy, isopropoxy, butoxy, isobutoxy, s-butoxy, t-butoxy, méthylthio, éthylthio, l'atome de fluor, de chlore ou le radical phényle et le cas échéant interrompu par un atome d'oxygène ou de soufre, ou
A, B et l'atome de carbone sur lequel ils sont liés, représentent un cycle spiro en C₅-C₆, qui est le cas échéant, substitué par un radical alcoylènediyle le cas échéant interrompu par un ou deux atomes d'oxygène et/ou de soufre, ou par un radical alcoylènedioxyle, qui avec l'atome de carbone sur lequel il est lié, forme un autre cycle spiro de cinq à sept membres, ou
A, B et l'atome de carbone sur lequel ils sont liés, représentent un cycle spiro en C₃-C₆, dans lequel deux substituants représentent ensemble un cycle de cinq ou six membres saturé ou insaturé, lequel peut être interrompu par un atome d'oxygène ou de soufre ;
X représente le radical méthyle, éthyle, propyle, isopropyle, méthoxy, éthoxy, propoxy ou isopropoxy ;
Y représente l'atome d'hydrogène, le radical méthyle, éthyle, propyle, isopropyle, butyle, isobutyle, s-butyle, t-butyle, méthoxy, éthoxy, propoxy ou isopropoxy ;
Z représente l'atome d'hydrogène, le radical méthyle, éthyle, propyle, isopropyle, méthoxy, éthoxy, propoxy ou isopropoxy ;
G représente l'atome d'hydrogène (a) ou les radicaux : 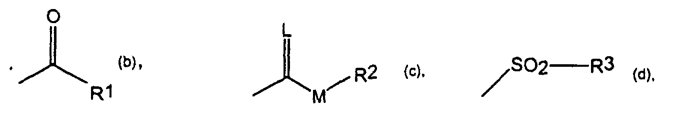 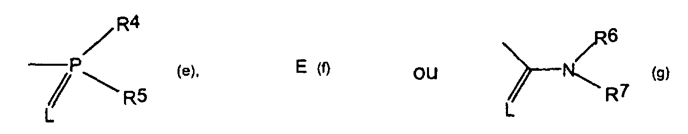 dans lesquels :
E représente un équivalent d'ion métallique ou un ion ammonium, et
L représente l'atome d'oxygène ou de soufre,
M représente l'atome d'oxygène ou de soufre,
R¹ représente un radical alcoyle en C₁-C₁₄, alcényle en C₂-C₁₄, (alcoxy en C₁-C₄)alcoyle en C₁-C₆, (alcoylthio en C₁-C₄ ) alcoyle en C₁-C₆, poly(alcoxy en C₁-C₄) alcoyle en C₂-C₄, le cas échéant substitué une à trois fois par l'atome de fluor ou de chlore, ou un radical cycloalcoyle en 3 à 6 atomes cycliques, le cas échéant substitué par l'atome de fluor, de chlore, le radical méthyle, éthyle, propyle, isopropyle, butyle, isobutyle, ou t-butyle, lequel peut être interrompu par 1 ou 2 atomes d'oxygène et/ou de soufre,
représente le radical phényle le cas échant substitué une à deux fois par l'atome de fluor, de chlore, de brome, le radical méthyle, éthyle, propyle, isopropyle, méthoxy, éthoxy, trifluorométhyle, trifluorométhoxy, méthylthio, éthylthio, méthylsulfonyle, éthylsulfonyle, ou nitro,
représente un radical phényl(alcoyle en C₁-C₃) le cas échéant substitué une à deux fois par l'atome de fluor, de chlore, de brome, le radical méthyle, éthyle, propyle, isopropyle, méthoxy, éthoxy, trifluorométhyle ou trifluorométhoxy,
représente un radical thiényle, furannyle ou pyridyle, le cas échant substitué une à deux fois par l'atome de fluor, de chlore, de brome, le radical méthyle ou éthyle,
représente un radical phénoxy(alcoyle en C₁-C₄) le cas échant substitué une à deux fois par l'atome de fluor, de chlore, de brome, le radical méthyle ou éthyle, ou
représente un radical pyridyloxy(alcoyle en C₁-C₄), pyrimidyloxy(alcoyle en C₁-C₄) , ou thiazolyloxy(alcoyle en C₁-C₄), le cas échant substitué une à deux fois par l'atome de fluor, de chlore, le radical amino, méthyle ou éthyle ;
R² représente un radical alcoyle en C₁-C₁₄, alcényle en C₃-C₁₄, (alcoxy en C₁-C₉) alcoyle en C₁-C₆, poly(alcoxy en C₁-C₄) alcoyle en C₂-C₆, le cas échéant substitué une à trois fois par l'atome de fluor ou de chlore,
représente un radical cycloalcoyle en C₃-C₆, le cas échéant substitué une à trois fois par l'atome de fluor, de chlore, le radical méthyle, éthyle, propyle, isopropyle ou méthoxy,
représente le radical phényle ou benzyle, le cas échant substitué une à deux fois par l'atome de fluor, de chlore, le radical nitro, méthyle, éthyle, propyle, isopropyle, méthoxy, éthoxy ou trifluorométhyle ;
R³, R⁴ et R⁵ représentent indépendamment l'un de l'autre, un radical alcoyle en C₁-C₄, alcoxy en C₁-C₄, alcoylamino en C₁-C₄, di(alcoyl en C₁-C₄)amino, alcoylthio en C₁-C₄, le cas échéant substitué une à trois fois par l'atome de fluor ou de chlore ; le radical phényle, phénoxy ou phénylthio, le cas échéant substitué une à deux fois par l'atome de fluor, de chlore, de brome, le radical nitro, cyano, un radical alcoxy en C₁-C₂, fluoroalcoxy en C₁-C₂, alcoylthio en C₁-C₂, fluoroalcoylthio en C₁-C₂, alcoyle en C₁-C₂ ou fluoroalcoyle en C₁-C₂ ;
R⁶ et R⁷ représentent indépendamment l'un de l'autre, l'atome d'hydrogène, un radical alcoyle en C₁-C₄, cycloalcoyle en C₃-C₆, alcoxy en C₁-C₄, alcenyle en C₃-C₄, (alcoxy en C₁-C₄)alcoyle en C₂-C₄, le cas échéant substitué une à trois fois par l'atome de fluor ou de chlore ; le radical phényle le cas échéant substitué une à deux fois par l'atome de fluor, de chlore, de brome, un radical halogénoalcoyle en C₁-C₄, alcoyle en C₁-C₄ et/ou alcoxy en C₁-C₄ ; le radical benzyle le cas échéant substitué une à deux fois par l'atome de fluor, de chlore, de brome, un radical alcoyle en C₁-C₄, halogénoalcoyle en C₁-C₄ ou alcoxy en C₁-C₄ ; ou avec l'atome d'azote sur lequel ils sont liés, un cycle alcoylène en C₃-C₆ le cas échéant interrompu par un atome d'oxygène ou de soufre,
avec la condition qu'au moins un des substituants Y et Z représente alcoxy lorsque X représente alcoyle.

6. Composés de formule (II) : 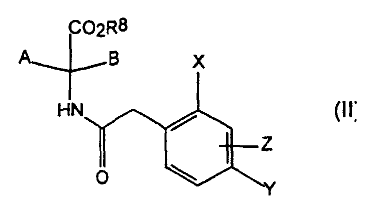 dans laquelle :
A, B, X, Y et Z ont les significations indiquées à la revendication 1,
et R⁸ représente un radical alcoyle

7. Procédé de préparation des esters d'acide acylaminé de formule (II) suivant la revendication 6, **caractérisé en ce que** l'on acyle des dérivés d'acide aminé de formule (XIV) : 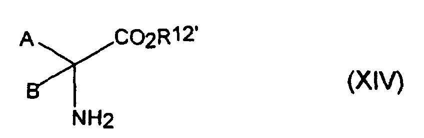 dans laquelle
R^{12'} représente l'atome d' hydrogène (XIVa) ou un radical alcoyle (XIVb), et .
A et B ont la signification indiquée ci-dessus, avec des halogénures phénylacétiques de formule (XV) : 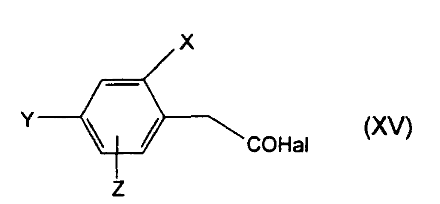 dans laquelle :
X, Y et Z ont la signification indiquée ci-dessus, et
Hal représente l'atome de chlore ou de brome,
ou que l'on estérifie des acides acylaminés de formule (IIa) : 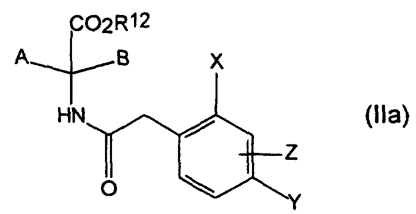 dans laquelle :
A, B, X, Y et Z ont la signification indiquée ci-dessus, et
R¹² représente l'atome d'hydrogène, ou
que l'on fait réagir des aminonitriles de formule (XVI) : 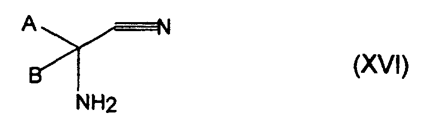 dans laquelle :
A et B ont la signification indiquée ci-dessus,
avec des halogénures phénylacétiques de formule (XV) : 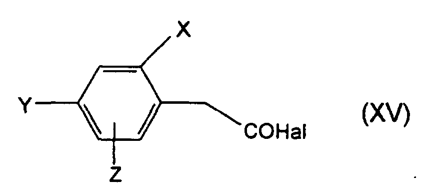 dans laquelle :
X, Y et Z ont la signification indiquée ci-dessus, et
Hal représente l'atome de chlore ou de brome,
pour obtenir les composés de formule (XVII) : 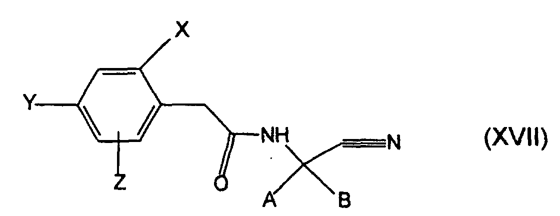 dans laquelle :
A, B, X, Y et Z ont la signification indiquée ci-dessus, et
ceux-ci subissent ensuite une alcoolyse dans l'acide sulfurique.

8. Composés de formule (XVII) : 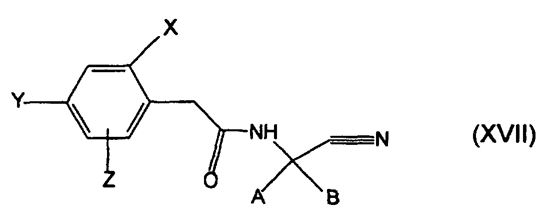 dans laquelle :
A, B, X, Y et Z ont la signification indiquée à la revendication 1.

9. Procédé de préparation de composés de formule (XVII) suivant la revendication 8, **caractérisé en ce que** l'on fait réagir des aminonitriles de formule (XVI) : 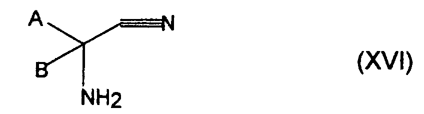 dans laquelle :
A et B ont la signification indiquée à la revendication 1,
avec des halogénures phénylacétiques de formule (XV) : 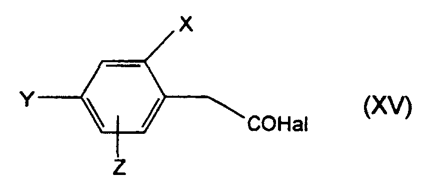 dans laquelle :
X, Y et Z ont la signification indiquée ci-dessus, et
Hal représente l'atome de chlore ou de brome

10. Agents de lutte contre les parasites et agents herbicides, **caractérisés par** une teneur en au moins un dérivé 1-H-3-arylpyrrolidin-2,4-dione de formule (I) suivant la revendication 1.

11. Utilisation d'un dérivé 1-H-3-arylpyrrolidin-2,4-dione de formule (I) suivant la revendication 1, pour lutter contre les parasites et mauvaises herbes.

12. Procédé pour lutter contre les parasites et mauvaises herbes, **caractérisé en ce que** l'on fait agir des dérivés 1-H-3-arylpyrrolidin-2,4-diones de formule (I) suivant la revendication 1, sur les parasites et/ou leur biotope ou sur les mauvaises herbes et/ou leur biotope.

13. Procédé de préparation d'agents de lutte contre les parasites et d'agents herbicides, **caractérisé en ce que** on mélange des dérivés 1-H-3-arylpyrrolidin-2,4-diones de formule (I) suivant la revendication 1, avec des diluants et/ou agents tensioactifs.
